# EUROPEAN PATENT APPLICATION

(11) **EP 4 675 353 A1**
(43) Date of publication of application: **07.01.2026**
(21) Application number: 25186358.5
(22) Date of filing: 30.06.2025
(51) Int. Cl.: G03F 7/004, G03F 7/038, C07C 25/18, C07C 309/73, C07C 309/75, C07C 381/12, C07D 327/08, C07D 333/76, C08F 12/20, C08F 12/22, C08F 12/30, C08F 12/32, C09D 125/18

(54) **ONIUM SALT MONOMER, POLYMER, CHEMICALLY AMPLIFIED NEGATIVE RESIST COMPOSITION, AND RESIST PATTERN FORMING PROCESS**

(30) Priority: 05.07.2024 JP 2024108822
(71) Applicant: Shin-Etsu Chemical Co., Ltd., Tokyo 100-0005 (JP)
(72) Inventor: FUKUSHIMA, Masahiro, Niigata (JP)
(74) Representative: Ter Meer Steinmeister & Partner

(57) **Abstract**

Provided are an onium salt monomer which has excellent etching resistance, organic solvent solubility, and appropriate acid strength, and can generate an acid with low diffusion, a base polymer comprising a polymer-bonded acid generator using the same, a chemically amplified negative resist composition using the same, and a resist pattern forming process using the chemically amplified negative resist composition.

An onium salt monomer has the formula (A1):

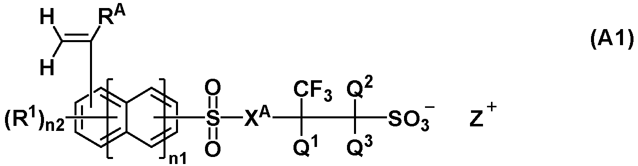

## Description

### TECHNICAL FIELD

The present invention relates to an onium salt monomer, a polymer, a chemically amplified negative resist composition, and a resist pattern forming process.

### BACKGROUND ART

To meet the demand for higher integration density and operating speed of LSIs, the effort to reduce the pattern rule is in rapid progress. Acid-catalyzed chemically amplified resist compositions are most often used in forming resist patterns with a feature size of 0.2 µm or less. High-energy radiation such as UV, deep-UV or EB is used as the light source for exposure of these resist compositions. In particular, while EB lithography is utilized as the ultra-fine microfabrication technique, it is also indispensable in processing photomask blanks to form photomasks for use in semiconductor device fabrication.

Polymers comprising a major proportion of aromatic structure having an acidic side chain, for example, polyhydroxystyrene are useful in resist materials for the KrF excimer laser lithography. These polymers are not used in resist materials for the ArF excimer laser lithography because they exhibit strong absorption at a wavelength of around 200 nm. However, these polymers are expected to form useful resist materials for the EB and EUV lithography for forming patterns of smaller size than the processing limit of ArF excimer laser because they offer high etching resistance.

Resist compositions for photolithography include positive ones in which exposed areas are dissolved away and negative ones in which exposed areas are left as a pattern. A viable composition is selected among them depending on the desired resist pattern. In general, the chemically amplified negative resist composition comprises a polymer which is normally soluble in an aqueous alkaline developer, an acid generator which is decomposed to generate an acid upon exposure to light, and a crosslinker which causes the polymer to crosslink in the presence of the acid serving as a catalyst, thus rendering the polymer insoluble in the developer (sometimes, the crosslinker is incorporated in the polymer). Most often a quencher is added for controlling the diffusion of the acid generated upon light exposure.

A number of negative resist compositions of a type using a unit derived from phenols as an alkali-soluble unit constituting polymers which dissolve in the aqueous alkaline developer were developed, especially as adapted for exposure to KrF excimer laser light. These compositions have not been used in the ArF excimer laser lithography because the phenolic units are not transmissive to exposure light having a wavelength of 150 to 220 nm. However, recently, as a negative resist composition for EB or EUV exposure, which is an exposure method for obtaining a finer pattern, these resist compositions have attracted attention again, and as a resist composition that imparts significantly high resolution even when used in a thin film, for example, those described in Patent Documents 1, 2, and 3 have been proposed.

In addition to those described above, many materials for chemically amplified negative resist compositions have been developed. For example, as a material that insolubilizes an alkali-soluble polymer that provides a negative mechanism used in a resist composition by the action of an acid generated when irradiated with high-energy radiation, a crosslinker as used in Patent Documents 1 to 3 is used, and many crosslinkers have been developed. Many attempts have been made to impart the function of the crosslinker to a polymer, a method of incorporating a styrene unit in which an alkoxymethoxy group is substituted (Patent Document 4), a method of incorporating a repeat unit having an alkoxymethylamino group (Patent Document 5), a method of incorporating a repeat unit having an epoxy group (Patent Document 6), a method of incorporating a styrene-based repeat unit having an acid-releasable group (Patent Document 7), a method of incorporating an adamantyl-based repeat unit having an acid-releasable hydroxy group (Patent Document 8), a method of incorporating an aliphatic hydrocarbon group having an acid-releasable hydroxy group and an alicyclic hydrocarbon-based repeat unit (Patent Documents 9, 10, and 11), and the like have been proposed, and a material having an acid-releasable hydroxy group has been proposed in Patent Document 12 and the like.

In order to suppress acid diffusion, a polymer comprising repeat units derived from an onium salt of sulfonic acid having a polymerizable unsaturated bond has been proposed (Patent Document 13). Such a so-called polymer-bonded acid generator has a feature that acid diffusion is significantly short because a polymeric sulfonic acid is generated by exposure. The sensitivity can also be improved by increasing the ratio of the acid generator. In the case of an additive-type acid generator, the amount of addition is increased, the sensitivity is increased, but in this case, the acid diffusion distance is also increased. Since the acid diffuses non-uniformly, line edge roughness (LER) and dimensional uniformity (CDU) are degraded as the acid diffusion increases. In the balance of sensitivity, LER, and CDU, the polymer-bonded acid generator has high ability, and development of an acid generator that generates an acid with more suitable strength is desired.

Patent Documents 14 and 15 describe examples in which acid diffusion is controlled by bonding sulfonic acid generated by exposure as an acid generator that generates an acid having a more suitable strength to a polymer used for a resist composition to suppress diffusion. Such a method in which a repeat unit that generates an acid by exposure is contained in a base polymer to suppress acid diffusion is effective for obtaining a pattern with a small LER. However, depending on the structure and incorporation rate of such a repeat unit, there may be a problem in solubility of the base polymer to which a repeat unit that generates an acid by exposure is bonded in an organic solvent.

In accordance with the progress of miniaturization of a resist pattern in recent years, collapse of the resist pattern during development and poor resistance in an etching process are problematic. Although the acid diffusion of the generated acid is suppressed to some extent by the incorporation of the repeat unit derived from the onium salt of sulfonic acid having the polymerizable unsaturated bond, there is still room for improvement of various lithographic performances, collapse of the resist pattern, and poor etching resistance. In order to meet the demand for miniaturization in the future, it is extremely important to develop a polymeric acid generator that achieves both lithography performance and collapse of a resist pattern, and etching resistance.

### Citation List

| | |
|---|---|
| Patent Document 1: | JP-A 2010-276910 |
| Patent Document 2: | JP-A 2010-164933 |
| Patent Document 3: | JP-A 2008-249762 |
| Patent Document 4: | JP-A H05-232702 |
| Patent Document 5: | JP-A H08-202037 |
| Patent Document 6: | JP-A 2001-226430 |
| Patent Document 7: | JP-A 2003-337414 |
| Patent Document 8: | JP-A 2001-154357 |
| Patent Document 9: | USP 7300739 |
| Patent Document 10: | USP 7393624 |
| Patent Document 11: | USP 7563558 |
| Patent Document 12: | JP-A 2013-164588 |
| Patent Document 13: | JP-A 2012-177834 |
| Patent Document 14: | JP-A 2011-22564 |
| Patent Document 15: | WO 2015/194330 |

### SUMMARY OF THE INVENTION

An object of the present invention is to provide an onium salt monomer which has excellent etching resistance, organic solvent solubility, and appropriate acid strength, and can generate an acid with low diffusion, a base polymer comprising a polymer-bonded acid generator using the same, a chemically amplified negative resist composition using the same, and a resist pattern forming process using the chemically amplified negative resist composition.

As a result of intensive studies to achieve the above object, the present inventors have found that by using, as a polymer-bonded acid generator, a polymer comprising repeat units derived from an onium salt containing an anion having a structure in which an aromatic ring substituted with a vinyl group and a partially fluorinated sulfonate anion are connected by a sulfonate ester bond or a sulfonate amide bond as a material of a chemically amplified negative resist composition, solvent solubility is excellent particularly in EB lithography and EUV lithography, lithographic performance such as resolution, LER, and pattern profile is excellent, it is resistant to pattern collapse even in fine pattern formation, and a pattern profile is also excellent, thereby completing the present invention.

That is, the present invention provides an onium salt monomer, a polymer, a chemically amplified negative resist composition, and a resist pattern forming process.
1. An onium salt monomer having the formula (A1): 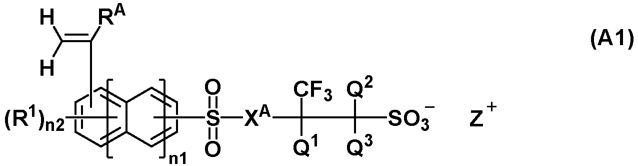 wherein n1 is 0 or 1, n2 is 0, 1, 2, 3, or 4,
   R^{A} is a hydrogen atom, a fluorine atom, a methyl group, or a trifluoromethyl group,
   R¹ is a C₁-C₂₀ hydrocarbyl group which may contain a halogen atom, a nitro group, a cyano group, a hydroxy group, or a heteroatom, when n2 is 2 or more, a plurality of R¹ may bond together to form a ring with the carbon atoms to which they are attached,
   X^{A} is a single bond, -O-, or -N(H)-,
   Q¹ to Q³ are each independently a hydrogen atom, a fluorine atom, or a trifluoromethyl group, and
   Z⁺ is an onium cation.
2. The onium salt monomer of 1 having the formula (A1-1): 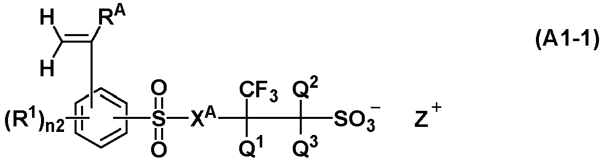 wherein R^{A}, R¹, X^{A}, Q¹ to Q³, n2, and Z⁺ are as defined above.
3. The onium salt monomer of 2 having the formula (A1-2): 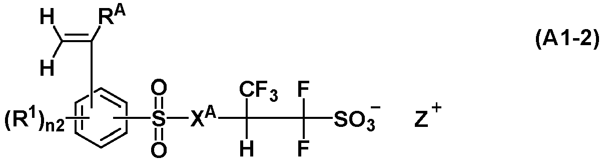 wherein R^{A}, R¹, X^{A}, n2, and Z⁺ are as defined above.
4. The onium salt monomer of any one of 1 to 3 wherein Z⁺ is a sulfonium cation having the formula (cation-1) or an iodonium cation having the formula (cation-2): 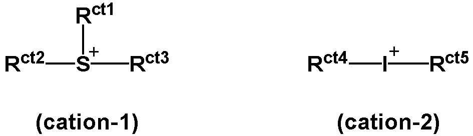 wherein R^{ct1} to R^{ct5} are each independently a halogen atom or a C₁-C₃₀ hydrocarbyl group which may contain a heteroatom, and R^{ct1} and R^{ct2} may bond together to form a ring with the sulfur atoms to which they are attached.
5. A polymer comprising repeat units derived from the onium salt monomer of any one of 1 to 4.
6. The polymer of 5, further comprising repeat units having the formula (A2): 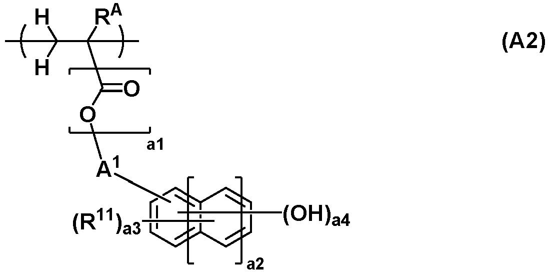 wherein a1 is 0 or 1, a2 is 0, 1, or 2, a3 is an integer satisfying 0 ≤ a3 ≤ 5+2(a2)-a4, a4 is 1, 2, or 3,
   R^{A} is a hydrogen atom, a fluorine atom, a methyl group, or a trifluoromethyl group,
   R¹¹ is a halogen atom, an optionally halogenated C₁-C₆ saturated hydrocarbyl group, an optionally halogenated C₁-C₆ saturated hydrocarbyloxy group, or an optionally halogenated C₂-C₈ saturated hydrocarbylcarbonyloxy group, and
   A¹ is a single bond or a C₁-C₁₀ saturated hydrocarbylene group in which some -CH₂-may be replaced by -O-.
7. The polymer of 5 or 6, further comprising repeat units of at least one type selected from repeat units having the formula (A3) and repeat units having the formula (A4): 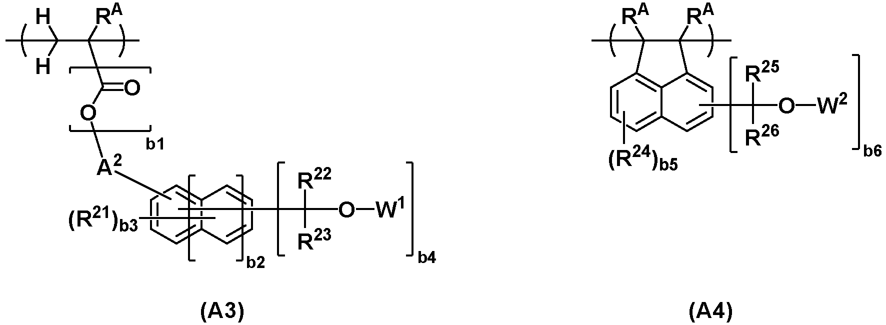 wherein b1 is 0 or 1, b2 is 0, 1, or 2, b3 is an integer satisfying 0 ≤ b3 ≤ 5+2(b2)-b4, b4 is 1, 2, or 3, b5 is 0, 1, or 2, b6 is 1 or 2,
   R^{A} is each independently a hydrogen atom, a fluorine atom, a methyl group, or a trifluoromethyl group,
   R²¹ is a C₁-C₂₀ hydrocarbyl group which may contain a halogen atom or a heteroatom,
   R²² and R²³ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²² and R²³ are not hydrogen atoms at the same time, R²² and R²³ may bond together to form a ring with the carbon atoms to which they are attached, some -CH₂- in the ring may be replaced by -O- or -S-,
   R²⁴ is a C₁-C₂₀ hydrocarbyl group which may contain a halogen atom or a heteroatom,
   R²⁵ and R²⁶ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²⁵ and R²⁶ are not hydrogen atoms at the same time, R²⁵ and R²⁶ may bond together to form a ring with the carbon atoms to which they are attached, some -CH₂- in the ring may be replaced by -O- or -S-,
   A² is a single bond or a C₁-C₁₀ saturated hydrocarbylene group in which some -CH₂-may be replaced by -O-, and
   W¹ and W² are each independently a hydrogen atom, a C₁-C₁₀ aliphatic hydrocarbyl group, a C₂-C₁₀ aliphatic hydrocarbylcarbonyl group, or an optionally substituted C₆-C₁₅ aryl group.
8. The polymer of any one of 5 to 7, further comprising repeat units of at least one type selected from repeat units having the formula (A5), repeat units having the formula (A6), and repeat units having the formula (A7): 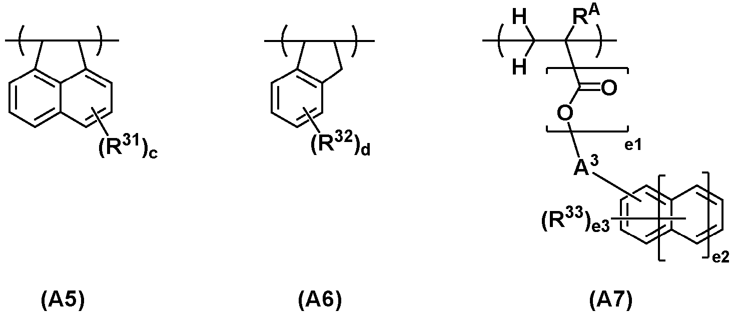 wherein c and d are each independently 0, 1, 2, 3, or 4, e1 is 0 or 1, e2 is 0, 1, or 2, e3 is 0, 1, 2, 3, 4, or 5,
   R^{A} is a hydrogen atom, a fluorine atom, a methyl group, or a trifluoromethyl group,
   R³¹ and R³² are each independently a hydroxy group, a halogen atom, an optionally halogenated C₁-C₈ saturated hydrocarbyl group, an optionally halogenated C₁-C₈ saturated hydrocarbyloxy group, or an optionally halogenated C₂-C₈ saturated hydrocarbylcarbonyloxy group,
   R³³ is a C₁-C₂₀ saturated hydrocarbyl group, a C₁-C₂₀ saturated hydrocarbyloxy group, a C₂-C₂₀ saturated hydrocarbylcarbonyloxy group, a C₂-C₂₀ saturated hydrocarbyloxyhydrocarbyl group, a C₂-C₂₀ saturated hydrocarbylthiohydrocarbyl group, a halogen atom, a nitro group, a cyano group, a C₁-C₂₀ saturated hydrocarbylsulfinyl group, or a C₁-C₂₀ saturated hydrocarbylsulfonyl group, and
   A³ is a single bond or a C₁-C₁₀ saturated hydrocarbylene group in which some -CH₂-may be replaced by -O-.
9. A chemically amplified negative resist composition comprising (A) a base polymer containing the polymer of any one of 5 to 8.
10. The chemically amplified negative resist composition of 9 wherein the polymer functions as a polymer-bonded acid generator.
11. The chemically amplified negative resist composition of 9 or 10 wherein the polymer comprises repeat units having the formula (A2-1), repeat units having the formula (A3-1), (A3-2), or (A4-1), and repeat units having formula (A1): 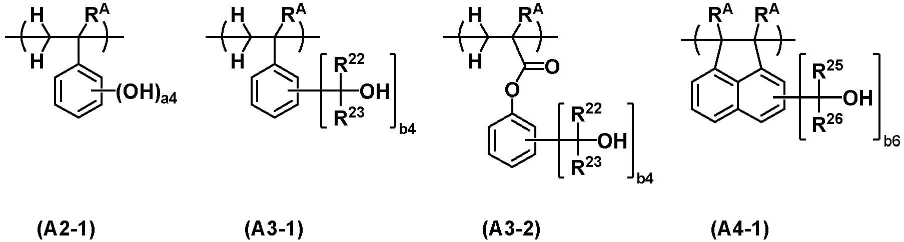 wherein a4 is 1, 2, or 3, b4 is 1, 2, or 3, b6 is 1 or 2,
   R^{A} is each independently a hydrogen atom, a fluorine atom, a methyl group, or a trifluoromethyl group,
   R²² and R²³ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²² and R²³ are not hydrogen atoms at the same time, R²² and R²³ may bond together to form a ring with the carbon atoms to which they are attached, some -CH₂- in the ring may be replaced by -O- or -S-,
   R²⁵ and R²⁶ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²⁵ and R²⁶ are not hydrogen atoms at the same time, and R²⁵ and R²⁶ may bond together to form a ring with the carbon atoms to which they are attached, and some -CH₂- in the ring may be replaced by -O- or -S-.
12. The chemically amplified negative resist composition of any one of 9 to 11 wherein the base polymer further contains a polymer comprising repeat units having the formula (A2) and repeat units having the formula (A3) or (A4), but not repeat units having formula (A1): 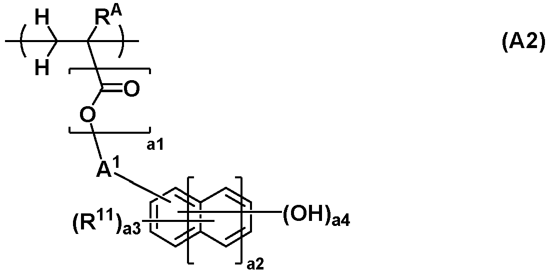
   wherein a1 is 0 or 1, a2 is 0, 1, or 2, a3 is an integer satisfying 0 ≤ a3 ≤ 5+2(a2)-a4, a4 is 1, 2, or 3,
      R^{A} is a hydrogen atom, a fluorine atom, a methyl group, or a trifluoromethyl group,
      R¹¹ is a halogen atom, an optionally halogenated C₁-C₆ saturated hydrocarbyl group, an optionally halogenated C₁-C₆ saturated hydrocarbyloxy group, or an optionally halogenated C₂-C₈ saturated hydrocarbylcarbonyloxy group,
      A¹ is a single bond or a C₁-C₁₀ saturated hydrocarbylene group in which some -CH₂-may be replaced by -O-, 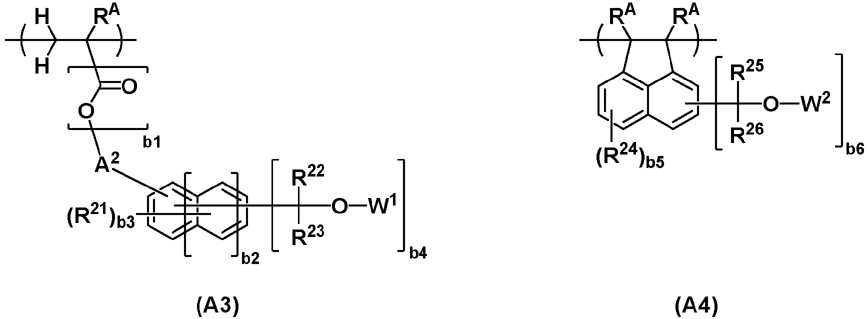
   wherein b1 is 0 or 1, b2 is 0, 1, or 2, b3 is an integer satisfying 0 ≤ b3 ≤ 5+2(b2)-b4, b4 is 1, 2, or 3, b5 is 0, 1, or 2, b6 is 1 or 2,
      R^{A} is each independently a hydrogen atom, a fluorine atom, a methyl group, or a trifluoromethyl group,
      R²¹ is a C₁-C₂₀ hydrocarbyl group which may contain a halogen atom or a heteroatom,
      R²² and R²³ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²² and R²³ are not hydrogen atoms at the same time, R²² and R²³ may bond together to form a ring with the carbon atoms to which they are attached, some -CH₂- in the ring may be replaced by -O- or -S-,
      R²⁴ is a C₁-C₂₀ hydrocarbyl group which may contain a halogen atom or a heteroatom,
      R²⁵ and R²⁶ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²⁵ and R²⁶ are not hydrogen atoms at the same time, and R²⁵ and R²⁶ may bond together to form a ring with the carbon atoms to which they are attached, some -CH₂- in the ring may be replaced by -O- or -S-,
      A² is a single bond or a C₁-C₁₀ saturated hydrocarbylene group in which some -CH₂-may be replaced by -O-, and
      W¹ and W² are each independently a hydrogen atom, a C₁-C₁₀ aliphatic hydrocarbyl group, a C₂-C₁₀ aliphatic hydrocarbylcarbonyl group, or an optionally substituted C₆-C₁₅ aryl group.
13. The chemically amplified negative resist composition of any one of 9 to 12 wherein repeat units having an aromatic ring structure account for 60 mol% or more of the overall repeat units of the polymer in the base polymer.
14. The chemically amplified negative resist composition of any one of 9 to 13, further comprising (B) a crosslinker.
15. The chemically amplified negative resist composition of any one of 9 to 14 which is free of a crosslinker.
16. The chemically amplified negative resist composition of any one of 9 to 15, further comprising (C) a fluorinated polymer comprising repeat units of at least one type selected from repeat units having the formula (C1), repeat units having the formula (C2), repeat units having the formula (C3), and repeat units having the formula (C4), and optionally repeat units of at least one type selected from repeat units having the formula (C5) and repeat units having the formula (C6): 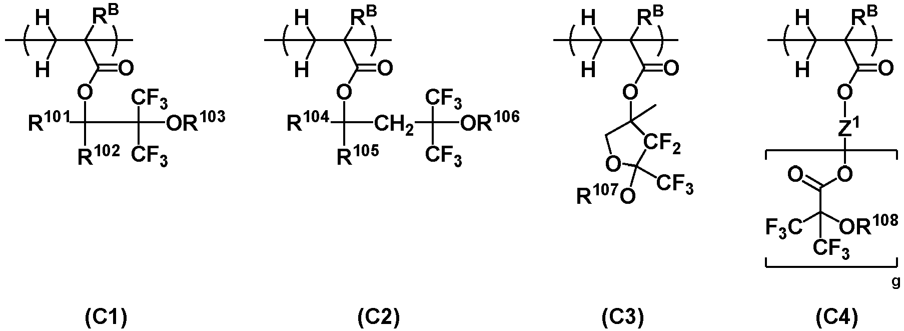 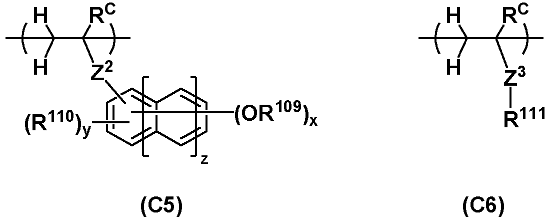 wherein x is 1, 2, or 3, y is an integer satisfying 0 ≤ y ≤ 5+2z-x, z is 0 or 1, g is 1, 2, or 3,
   R^{B} is each independently a hydrogen atom, a fluorine atom, a methyl group, or a trifluoromethyl group,
   R^{C} is each independently a hydrogen atom or a methyl group,
   R¹⁰¹, R¹⁰², R¹⁰⁴, and R¹⁰⁵ are each independently a hydrogen atom or a C₁-C₁₀ saturated hydrocarbyl group,
   R¹⁰³, R¹⁰⁶, R¹⁰⁷, and R¹⁰⁸ are each independently a hydrogen atom, a C₁-C₁₅ hydrocarbyl group, a C₁-C₁₅ fluorinated hydrocarbyl group, or an acid labile group, and when R¹⁰³, R¹⁰⁶, R¹⁰⁷, and R¹⁰⁸ each are a hydrocarbyl group or a fluorinated hydrocarbyl group, an ether bond or a carbonyl group may intervene in a carbon-carbon bond,
   R¹⁰⁹ is a hydrogen atom or a C₁-C₅ straight or branched hydrocarbyl group in which a heteroatom-containing group may intervene in a carbon-carbon bond,
   R¹¹⁰ is a C₁-C₅ straight or branched hydrocarbyl group in which a heteroatom containing group may intervene in a carbon-carbon bond,
   R¹¹¹ is a C₁-C₂₀ saturated hydrocarbyl group in which at least one hydrogen atom is substituted by a fluorine atom, and in which some -CH₂- may be replaced by an ester bond or an ether bond,
   Z¹ is a C₁-C₂₀ (g+1)-valent hydrocarbon group or C₁-C₂₀ (g+1)-valent fluorinated hydrocarbon group,
   Z² is a single bond, *-C(=O)-O-, or *-C(=O)-NH-, * designates a point of attachment to the carbon atom in the backbone,
   Z³ is a single bond, -O-, *-C(=O)-O-Z³¹-Z³²-, or *-C(=O)-NH-Z³¹-Z³²-, Z³¹ is a single bond or a C₁-C₁₀ saturated hydrocarbylene group, Z³² is a single bond, an ester bond, an ether bond, or a sulfonamide bond, and
   * designates a point of attachment to the carbon atom in the backbone.
17. The chemically amplified negative resist composition of any one of 9 to 16, further comprising (D) a quencher.
18. The chemically amplified negative resist composition of any one of 9 to 17, further comprising (E) an organic solvent.
19. The chemically amplified negative resist composition of any one of 9 to 18, further comprising (F) another photoacid generator.
20. A resist pattern forming process comprising the steps of: applying the chemically amplified negative resist composition of any one of 9 to 19 onto a substrate to form a resist film thereon, exposing the resist film patternwise to high-energy radiation, and developing the exposed resist film in an alkaline developer.
21. The resist pattern forming process of 20 wherein the high-energy radiation is EUV of wavelength 3 to 15 nm or EB.
22. The resist pattern forming process of 20 or 21 wherein the substrate has the outermost surface of a material containing at least one element selected from chromium, silicon, tantalum, molybdenum, cobalt, nickel, tungsten, and tin.
23. The resist pattern forming process of any one of 20 to 22 wherein the substrate is a mask blank of transmission or reflection type.
24. A mask blank of transmission or reflection type which is coated with the chemically amplified negative resist composition of any one of 9 to 19.

### ADVANTAGEOUS EFFECTS OF THE INVENTION

The chemically amplified negative resist composition of the present invention can effectively control acid diffusion due to exposure during pattern formation by the action of the copolymerization unit derived from the onium salt monomer having formula (A1), and when a resist film is formed using the chemically amplified negative resist composition to form a pattern, a pattern having extremely high resolution, high pattern fidelity, and reduced LER can be obtained.

### DESCRIPTION OF THE PREFERRED EMBODIMENT

Hereinafter, the present invention is described in detail. It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Onium salt monomer

The present invention provides an onium salt monomer having the formula (A1).

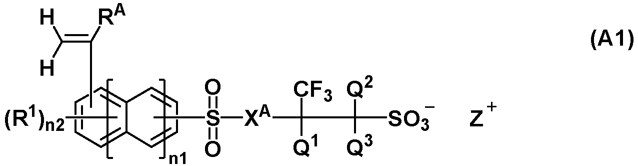

In formula (A1), n1 is 0 or 1. The relevant structure is a benzene ring when n1=0, and a naphthalene ring when n1=1. The benzene ring corresponding to n1=0 is preferred from the viewpoint of solvent solubility. n2 is 0, 1, 2, 3, or 4, and is preferably 0 or 1 from the viewpoint of reactant availability.

In formula (A1), R^{A} is a hydrogen atom, a fluorine atom, a methyl group, or a trifluoromethyl group. Among them, a hydrogen atom and a methyl group are preferred, and a hydrogen atom is more preferred.

In formula (A1), R¹ is a C₁-C₂₀ hydrocarbyl group which may contain a halogen atom, a nitro group, a cyano group, a hydroxy group, or a heteroatom. The halogen atom is preferably a fluorine atom, a chlorine atom, a bromine atom, or an iodine atom, more preferably a fluorine atom or an iodine atom. The hydrocarbyl group may be saturated or unsaturated, and may be straight, branched, or cyclic. Specific examples thereof include C₁-C₂₀ alkyl groups such as a methyl group, an ethyl group, an n-propyl group, an isopropyl group, an n-butyl group, an isobutyl group, a sec-butyl group, a tert-butyl group, an n-pentyl group, an n-hexyl group, an n-octyl group, an n-nonyl group, an n-decyl group, an undecyl group, a dodecyl group, a tridecyl group, a tetradecyl group, a pentadecyl group, a heptadecyl group, an octadecyl group, a nonadecyl group, and an icosyl group; C₃-C₂₀ cyclic saturated hydrocarbyl groups such as a cyclopropyl group, a cyclopentyl group, a cyclohexyl group, a cyclopropylmethyl group, a 4-methylcyclohexyl group, a cyclohexylmethyl group, a norbornyl group, and an adamantyl group; C₂-C₂₀ alkenyl groups such as a vinyl group, an allyl group, a propenyl group, a butenyl group, and a hexenyl group; C₃-C₂₀ cyclic unsaturated hydrocarbyl groups such as a cyclohexenyl group; C₆-C₂₀ aryl groups such as a phenyl group and a naphthyl group; C₇-C₂₀ aralkyl groups such as a benzyl group, a 1-phenylethyl group, and a 2-phenylethyl group; and combinations thereof. Inter alia, aryl groups are preferred. In the hydrocarbyl group, some or all of the hydrogen atoms may be substituted by a group containing a heteroatom such as an oxygen atom, a sulfur atom, a nitrogen atom, or a halogen atom, and some -CH₂- may be replaced by a group containing a heteroatom such as an oxygen atom, a sulfur atom, or a nitrogen atom, so that the group may contain a hydroxy group, a cyano group, a fluorine atom, a chlorine atom, a bromine atom, an iodine atom, a carbonyl group, an ether bond, an ester bond, a sulfonate ester bond, a carbonate bond, a lactone ring, a sultone ring, a carboxylic anhydride (-C(=O)-O-C(=O)-), or a haloalkyl group. When n3 is 2, 3, or 4, each R¹ may be identical or different.

When n2 is 2, 3, or 4, a plurality of R¹ may bond together to form a ring with the carbon atoms to which they are attached. Specific examples of the ring thus formed include a cyclopropane ring, a cyclobutane ring, a cyclopentane ring, a cyclohexane ring, a norbornane ring, and an adamantane ring. In the ring, some or all of the hydrogen atoms may be substituted by a group containing a heteroatom such as an oxygen atom, a sulfur atom, a nitrogen atom, or a halogen atom, and some -CH₂- may be replaced by a group containing a heteroatom such as an oxygen atom, a sulfur atom, or a nitrogen atom, so that the group may contain a hydroxy group, a fluorine atom, a chlorine atom, a bromine atom, an iodine atom, a cyano group, a carbonyl group, an ether bond, an ester bond, a sulfonate ester bond, a carbonate bond, a lactone ring, a sultone ring, a carboxylic anhydride (-C(=O)-O-C(=O)-), or a haloalkyl group.

In formula (A1), X^{A} is a single bond, -O-, or -N(H)-. From the viewpoint of reactant availability, X^{A} is preferably -O-.

In formula (A1), Q¹ to Q³ are each independently a hydrogen atom, a fluorine atom, or a trifluoromethyl group. From the viewpoint of reactant availability, Q¹ is preferably a hydrogen atom or a trifluoromethyl group. Q² and Q³ are preferably a hydrogen atom or a fluorine atom. When Q¹ is a hydrogen atom, Q² and Q³ are preferably a fluorine atom. When Q¹ is a trifluoromethyl group, Q² and Q³ are preferably a hydrogen atom.

Of the onium salt monomers having formula (A1), those having the formula (A1-1) are preferred.

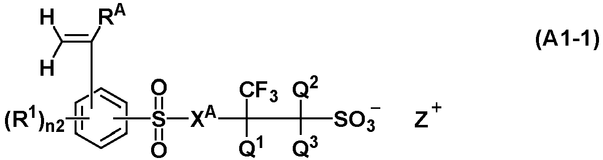

Herein R^{A}, R¹, X^{A}, Q¹ to Q³, n2, and Z⁺ are as defined above.

Of the onium salt monomers having formula (A1-1), those having the formula (A1-2) are preferred.

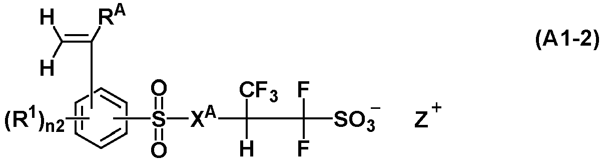

Herein R^{A}, R¹, X^{A}, n2, and Z⁺ are as defined above.

Specific examples of the anion of the onium salt monomer having formula (A1) are shown below, but are not limited thereto. In the formula below, R^{A} is as defined above, and Me is a methyl group. The binding sites of various substituents on the aromatic ring may be interchanged with each other.

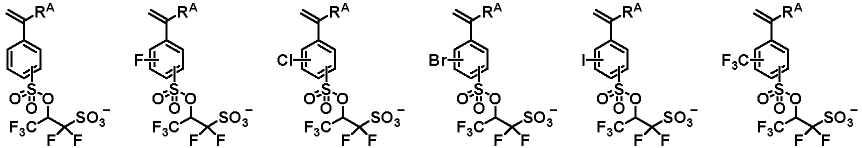

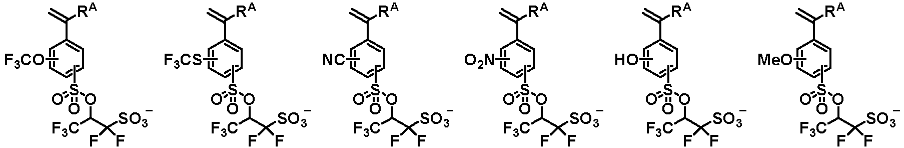

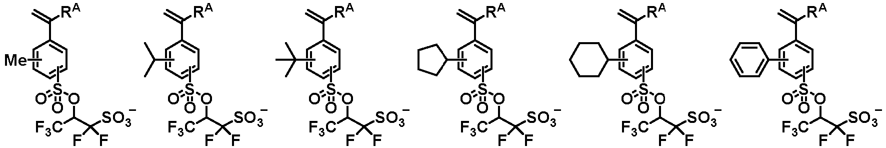

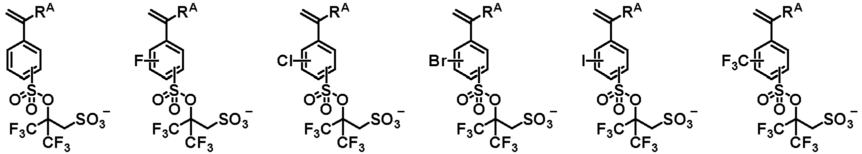

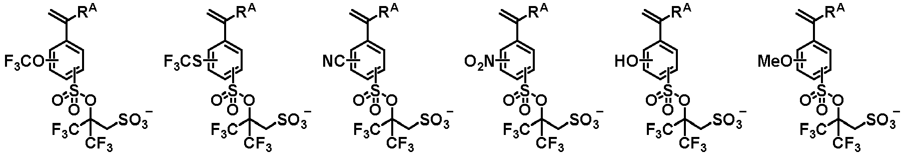

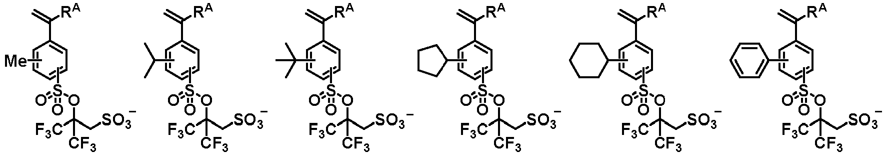

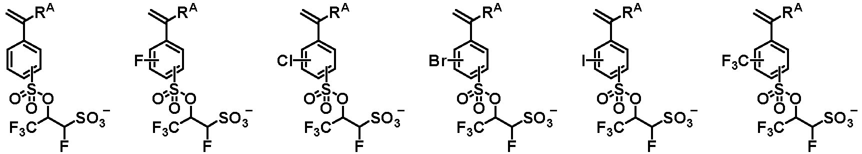

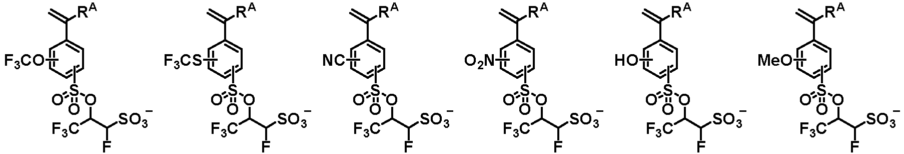

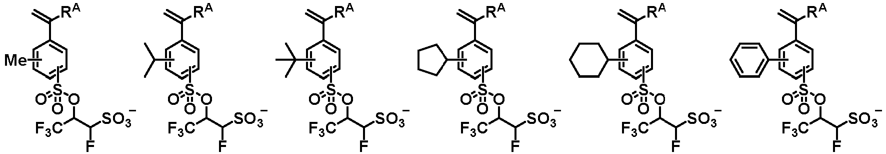

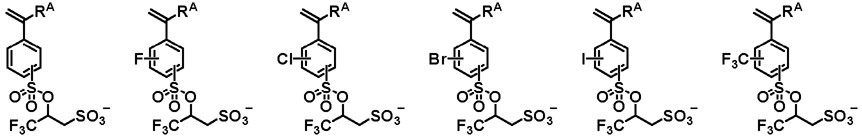

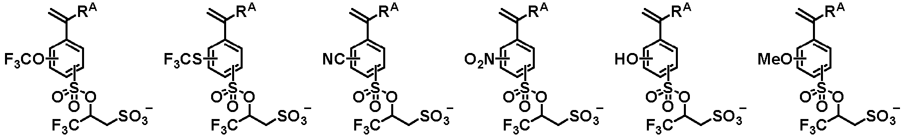

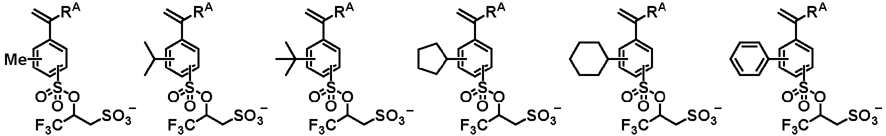

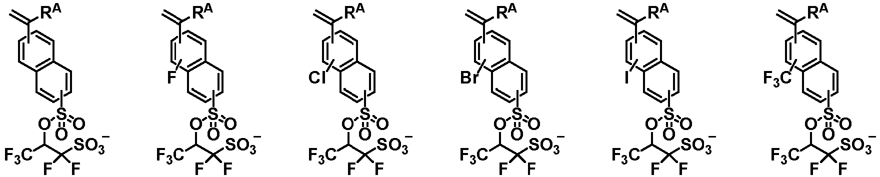

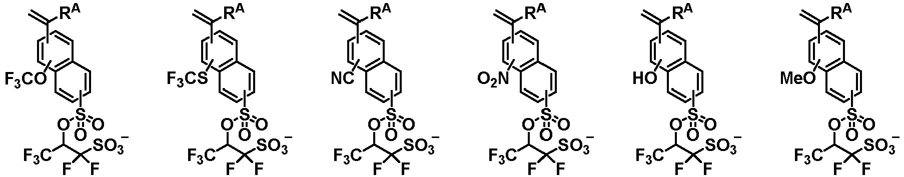

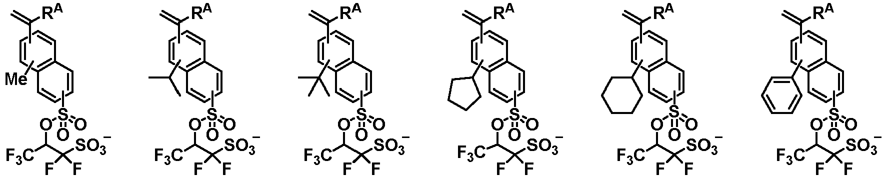

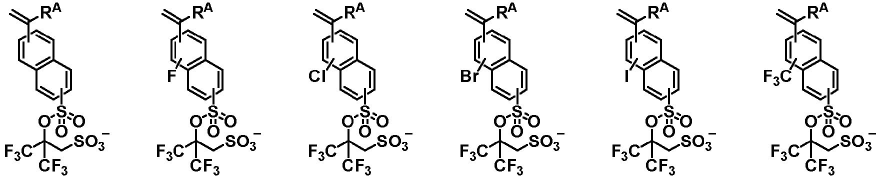

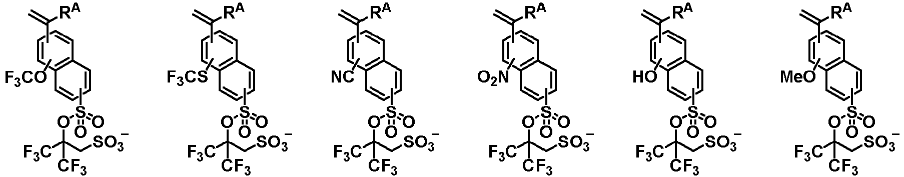

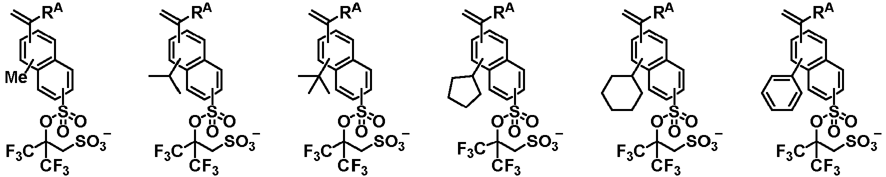

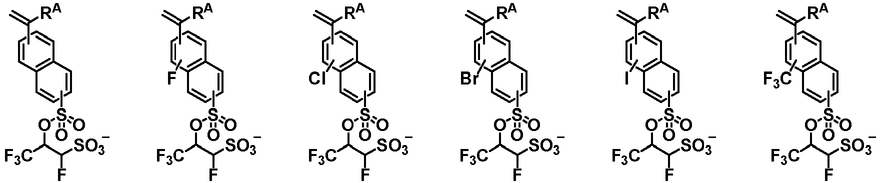

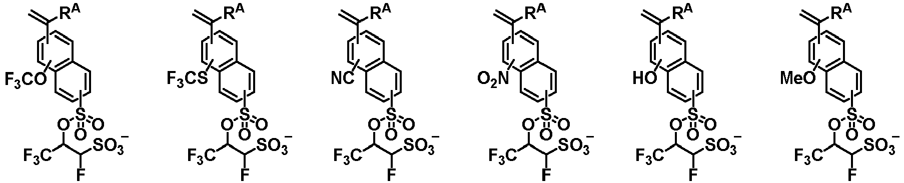

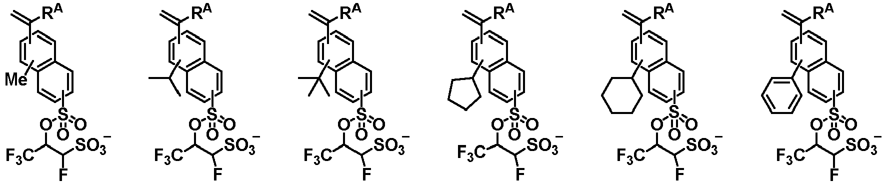

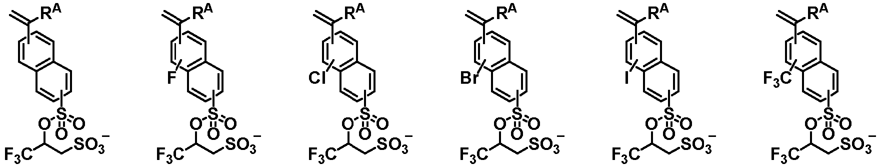

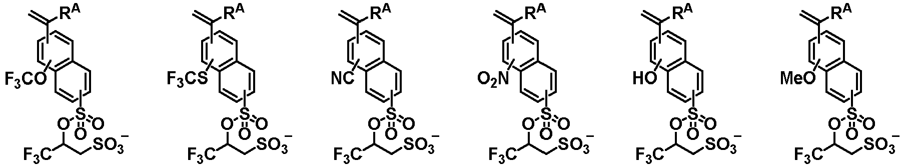

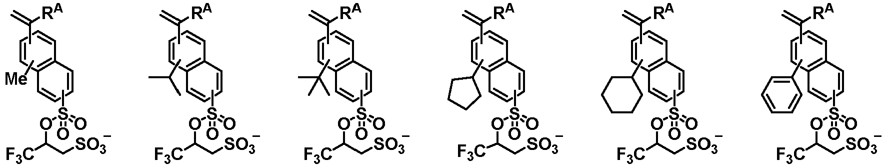

In formula (A1), Z⁺ is an onium cation. The onium cation is a sulfonium cation having the formula (cation-1) or an iodonium cation having the formula (cation-2).

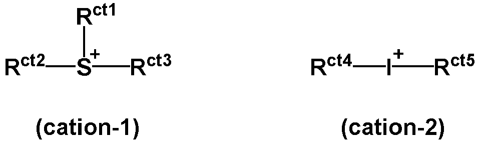

In formula (cation-1) and formula (cation-2), R^{ct1} to R^{ct5} are each independently a halogen atom or a C₁-C₃₀ hydrocarbyl group which may contain a heteroatom.

Specific examples of the halogen atom represented by R^{ct1} to R^{ct5} include a fluorine atom, a chlorine atom, a bromine atom, and an iodine atom.

The hydrocarbyl groups R^{ct1} to R^{ct5} may be saturated or unsaturated, and may be straight, branched, or cyclic. Specific examples thereof include C₁-C₃₀ alkyl groups such as a methyl group, an ethyl group, an n-propyl group, an isopropyl group, an n-butyl group, an isobutyl group, a sec-butyl group, and a tert-butyl group; C₃-C₃₀ cyclic saturated hydrocarbyl groups such as a cyclopropyl group, a cyclopentyl group, a cyclohexyl group, a cyclopropylmethyl group, a 4-methylcyclohexyl group, a cyclohexylmethyl group, a norbornyl group, and an adamantyl group; C₂-C₃₀ alkenyl groups such as a vinyl group, an allyl group, a propenyl group, a butenyl group, and a hexenyl group; C₃-C₃₀ cyclic unsaturated hydrocarbyl groups such as a cyclohexenyl group; C₆-C₃₀ aryl groups such as a phenyl group, a naphthyl group, and a thienyl group; C₇-C₃₀ aralkyl groups such as a benzyl group, a 1-phenylethyl group, and a 2-phenylethyl group; and combinations thereof, and aryl groups are preferred. In the hydrocarbyl group, some or all of the hydrogen atoms may be substituted by a group containing a heteroatom such as an oxygen atom, a sulfur atom, a nitrogen atom, or a halogen atom, and some -CH₂- may be replaced by a group containing a heteroatom such as an oxygen atom, a sulfur atom, or a nitrogen atom, so that the group may contain a hydroxy group, a fluorine atom, a chlorine atom, a bromine atom, an iodine atom, a cyano group, a nitro group, a carbonyl group, an ether bond, an ester bond, a sulfonate ester bond, a carbonate bond, a lactone ring, a sultone ring, a carboxylic anhydride (-C(=O)-O-C(=O)-), or a haloalkyl group.

R^{ct1} and R^{ct2} may bond together to form a ring with the sulfur atoms to which they are attached. Examples of the structure of the ring are shown below.

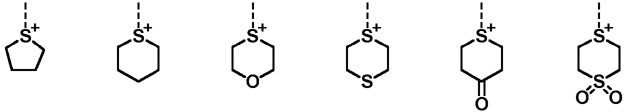

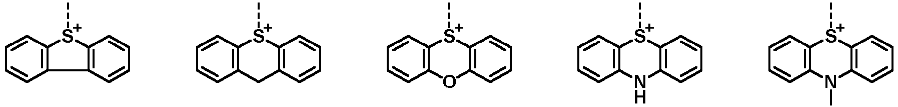

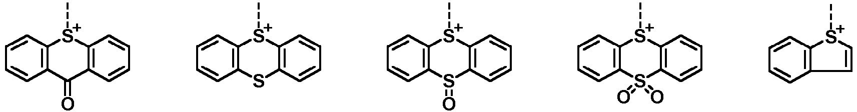

Herein the broken line designates a point of attachment to R^{ct3}.

Specific examples of the sulfonium cation having formula (cation-1) are shown below, but are not limited thereto.

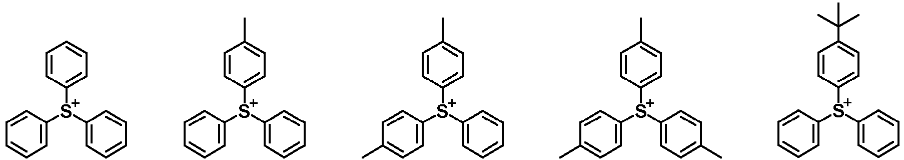

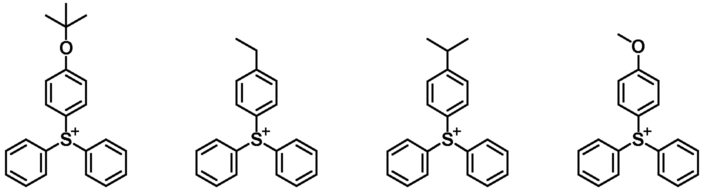

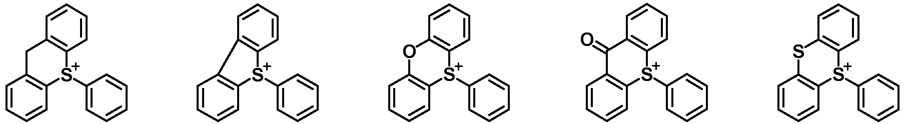

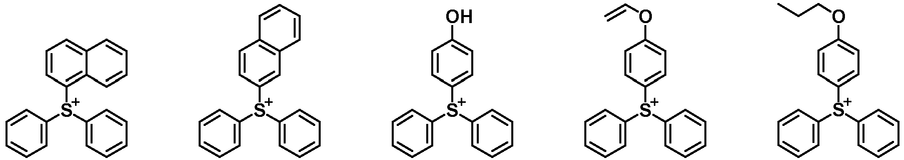

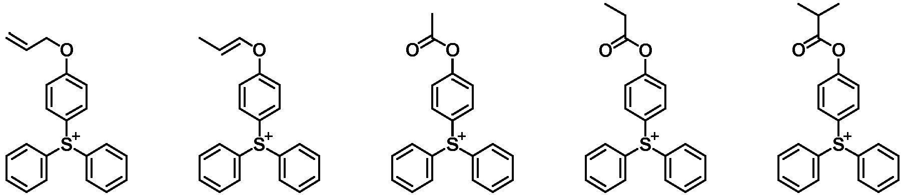

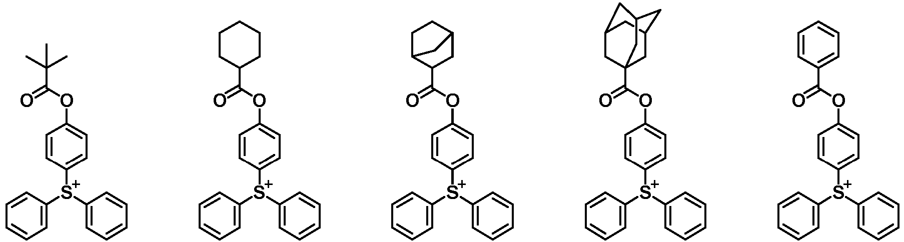

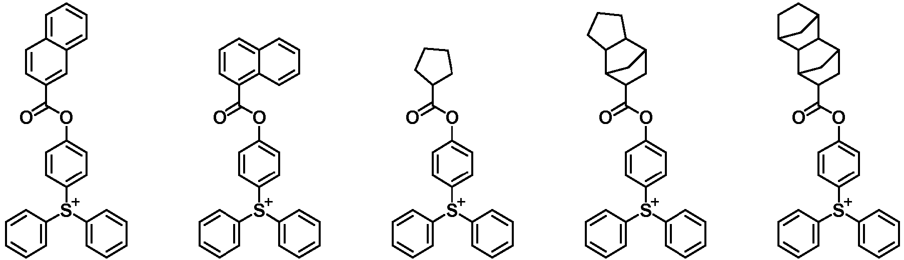

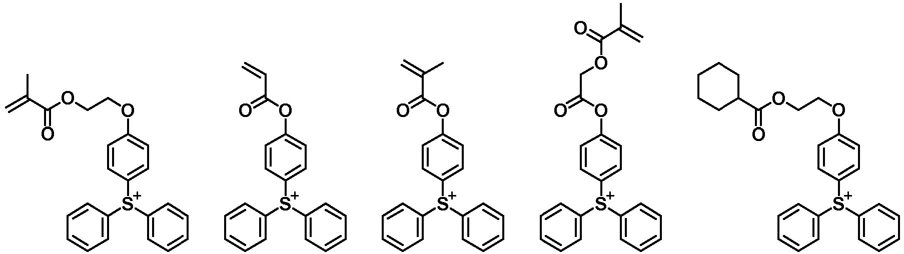

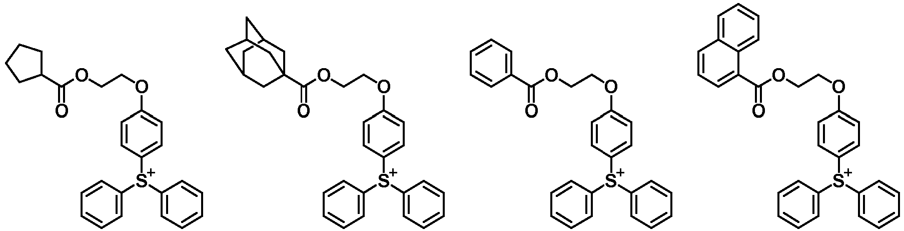

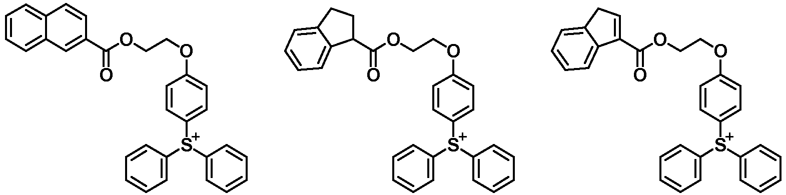

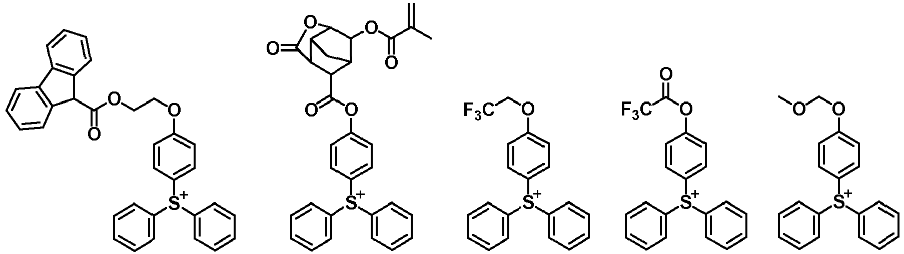

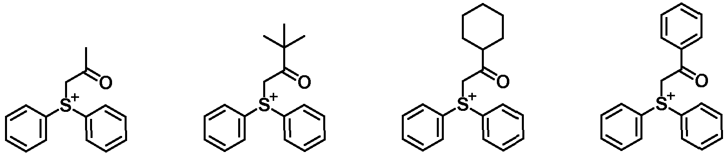

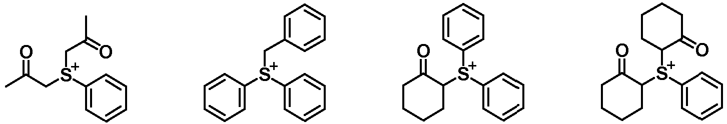

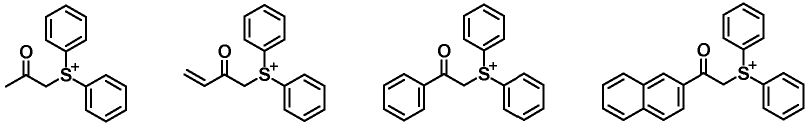

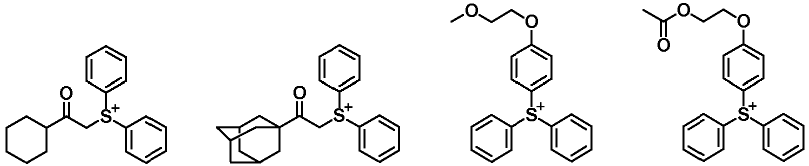

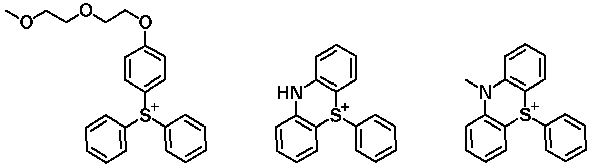

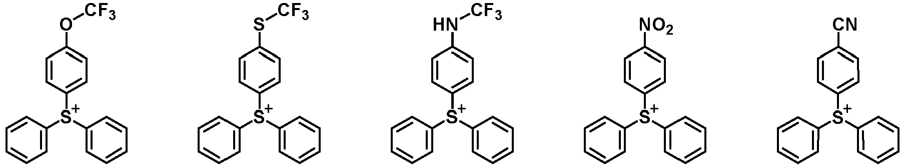

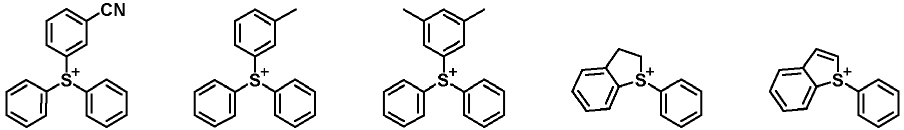

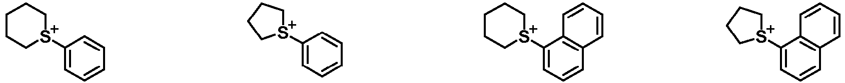

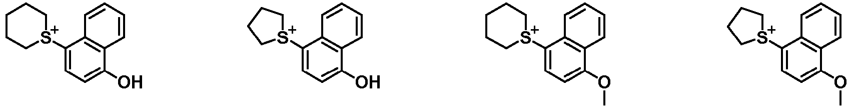

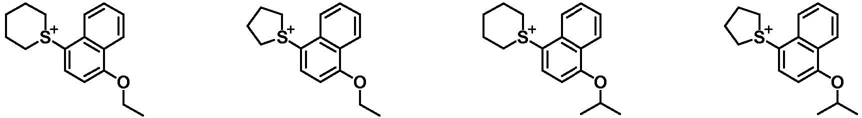

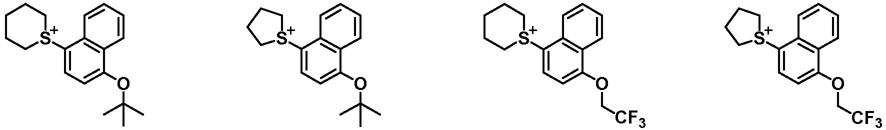

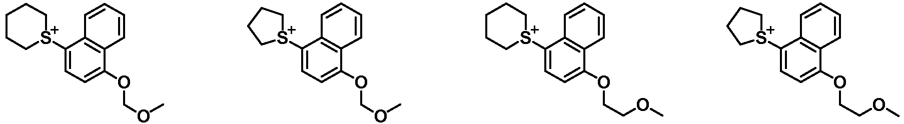

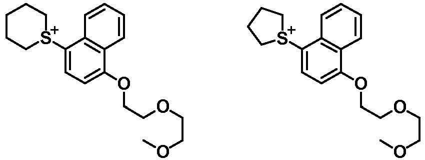

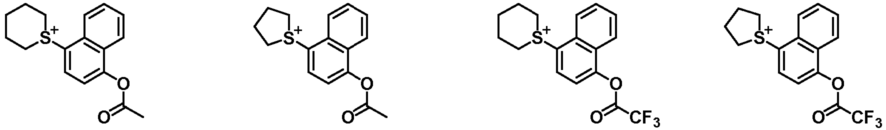

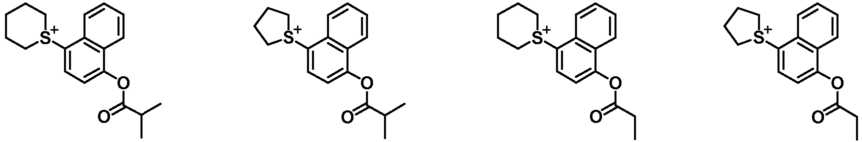

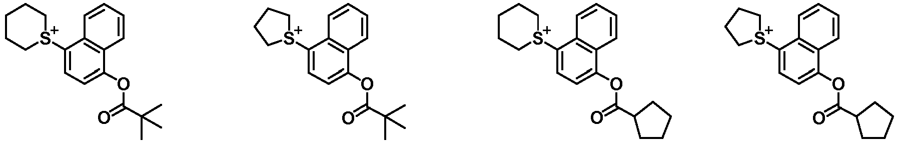

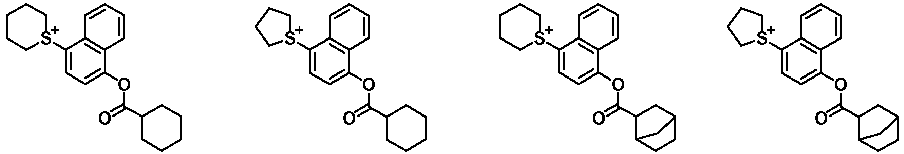

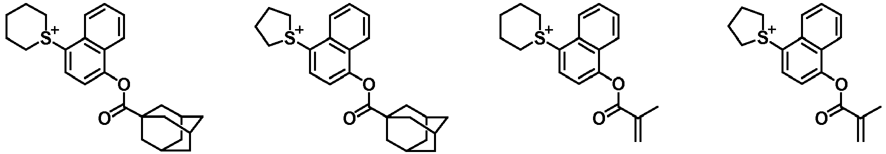

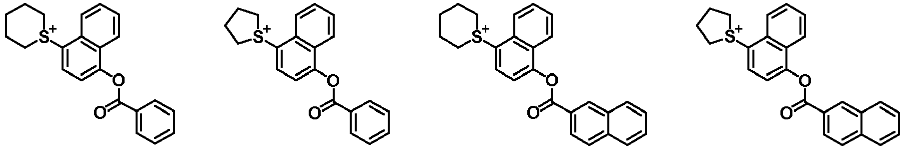

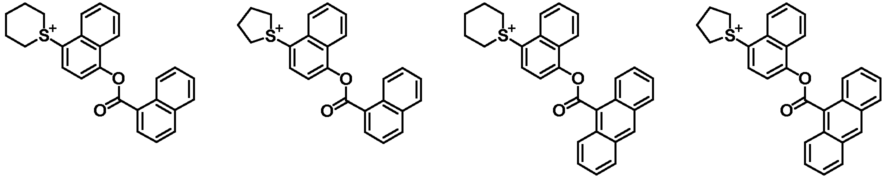

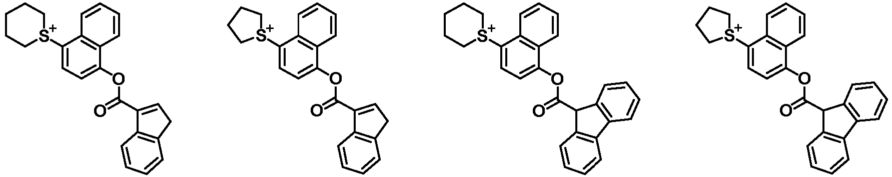

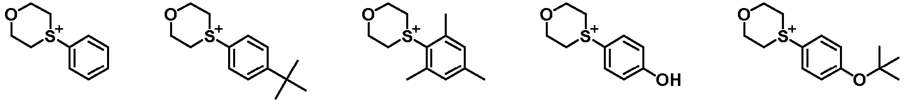

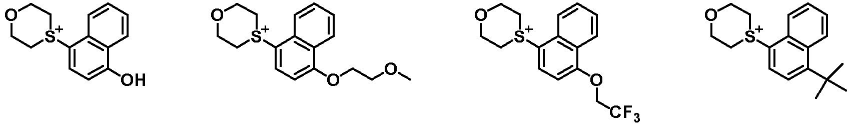

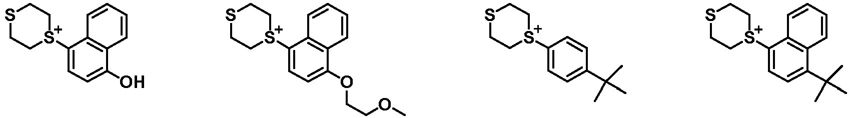

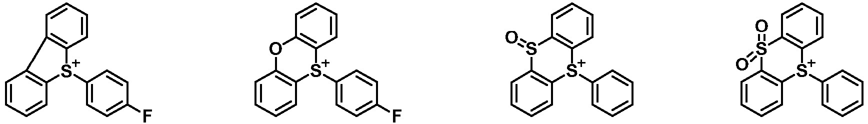

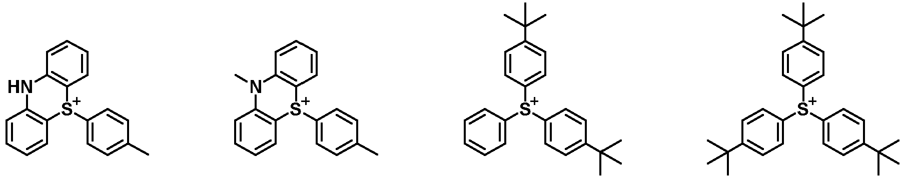

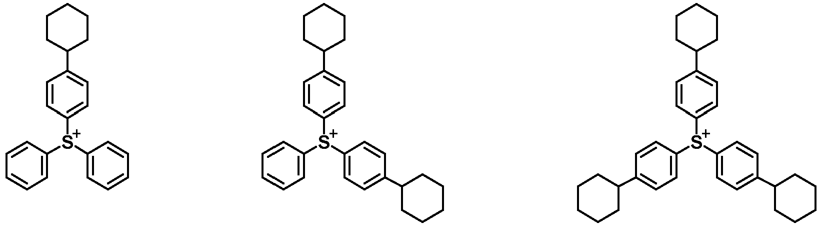

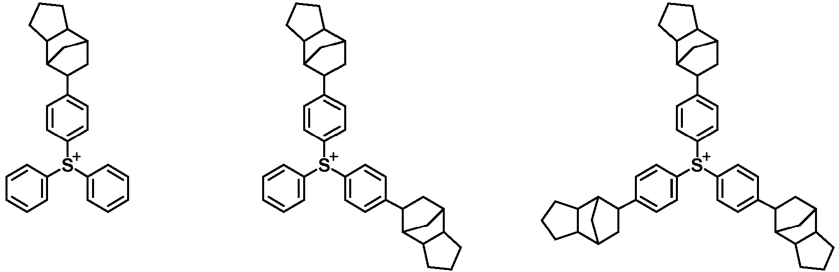

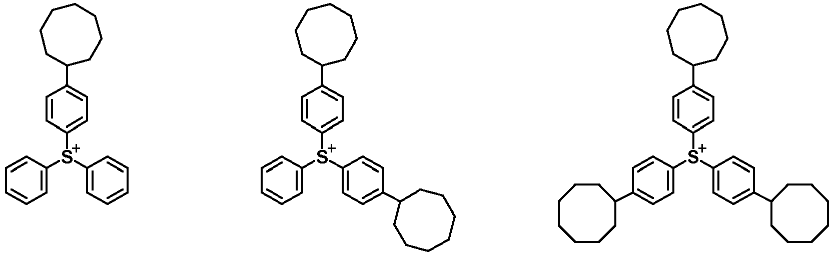

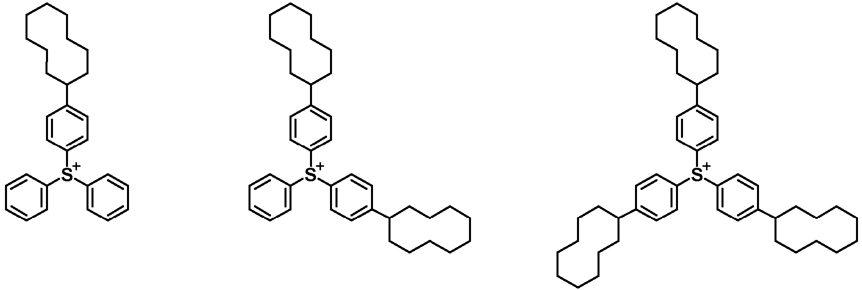

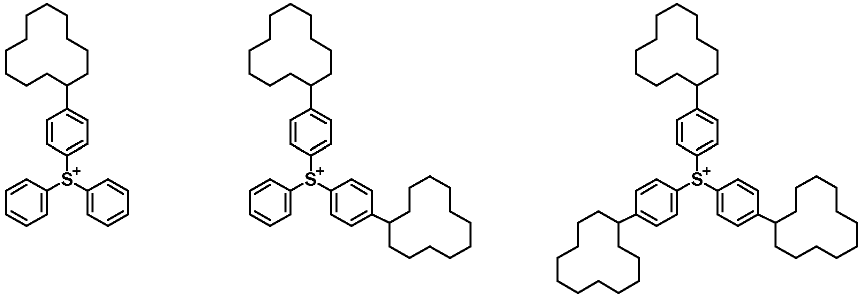

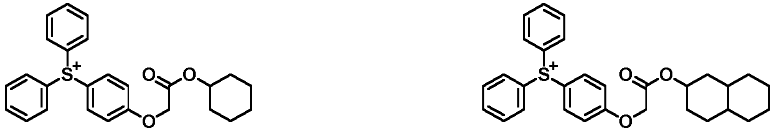

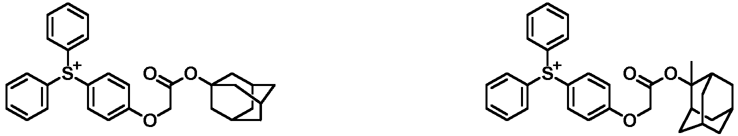

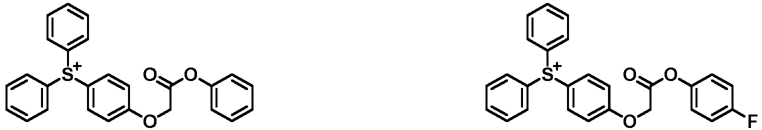

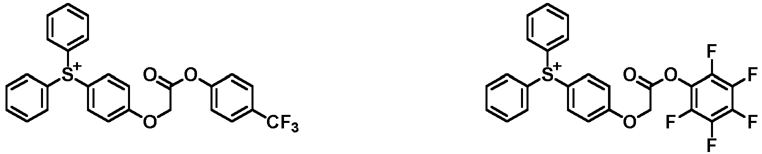

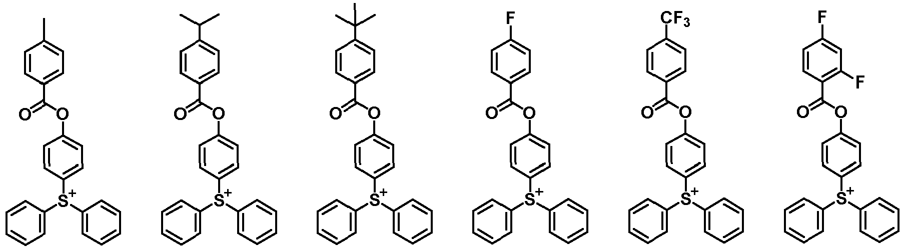

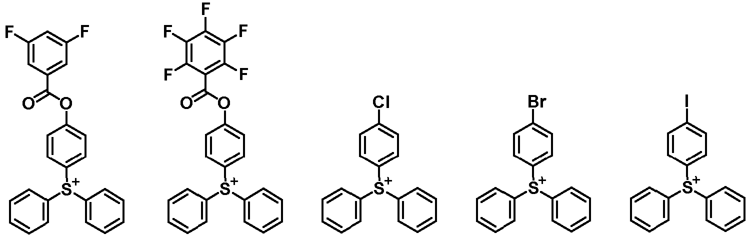

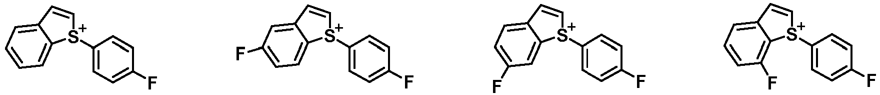

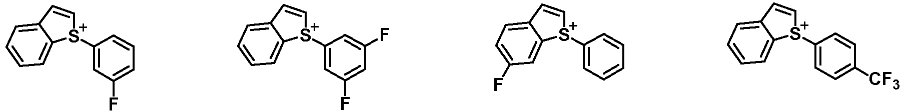

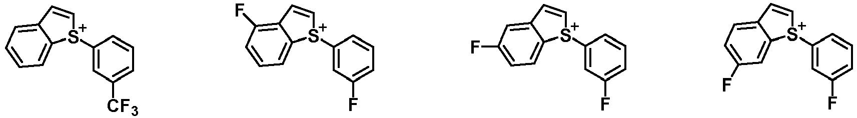

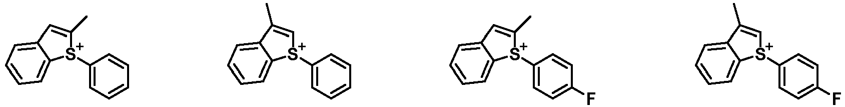

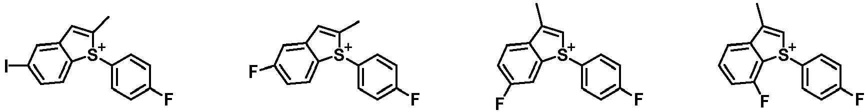

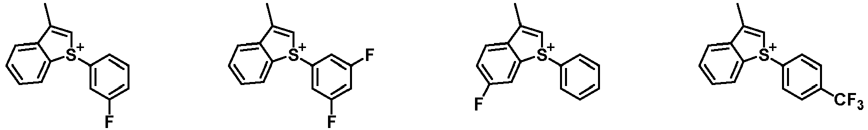

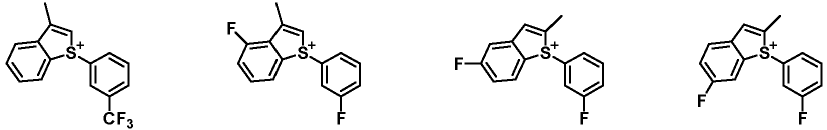

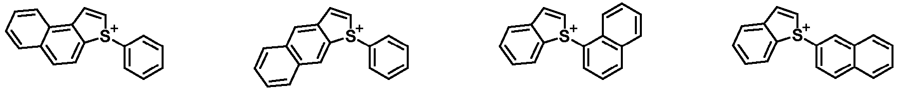

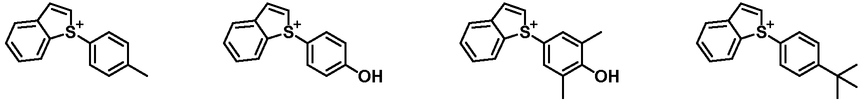

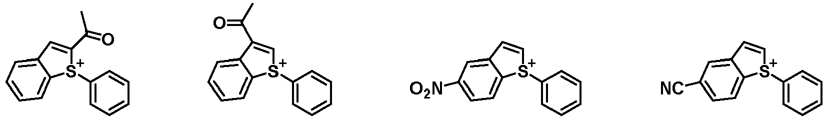

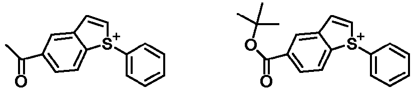

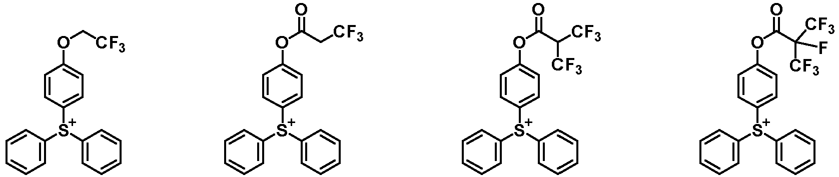

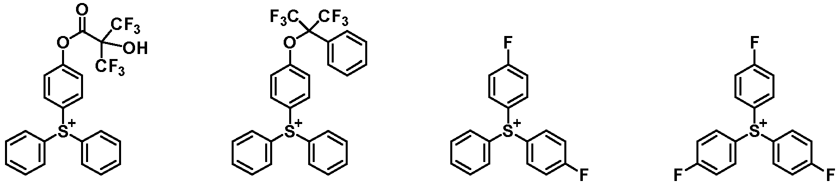

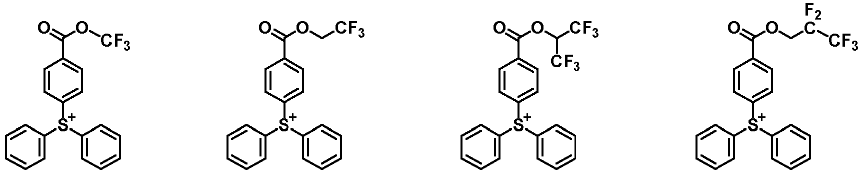

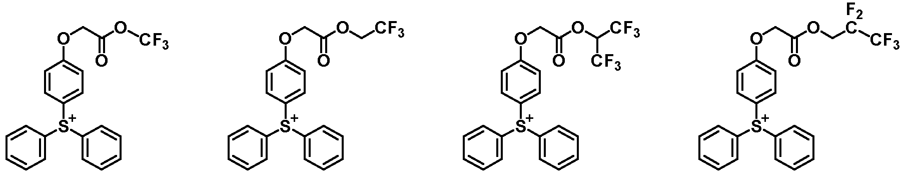

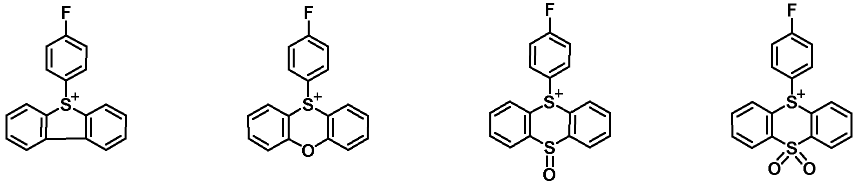

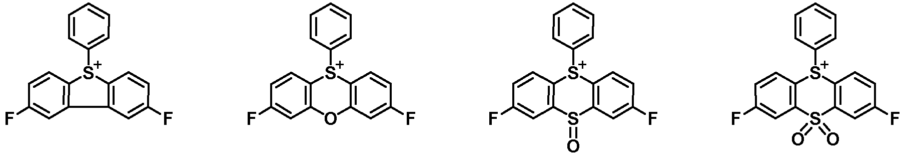

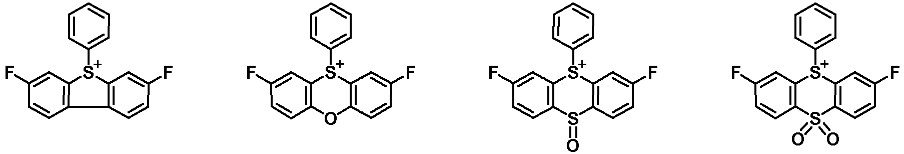

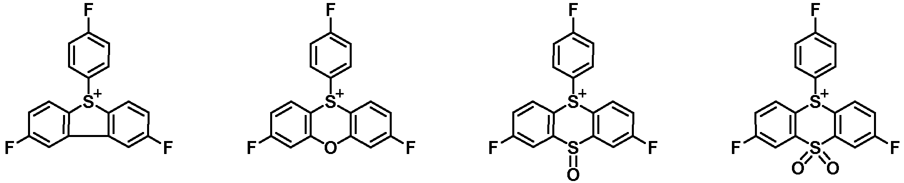

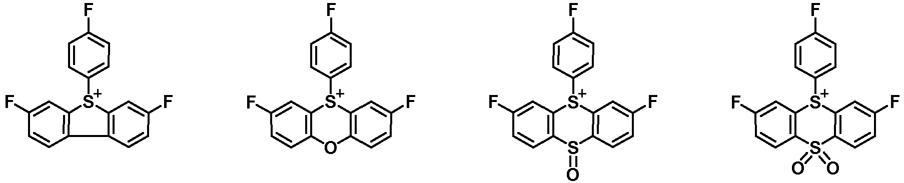

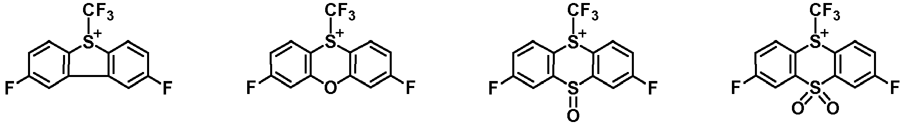

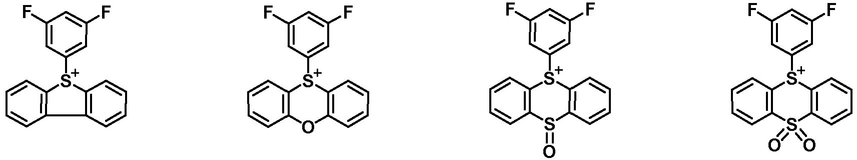

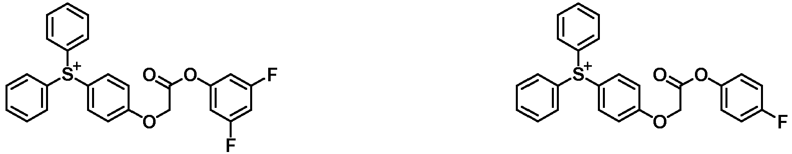

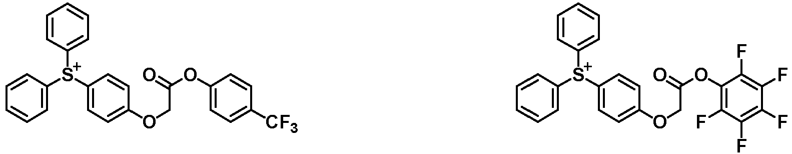

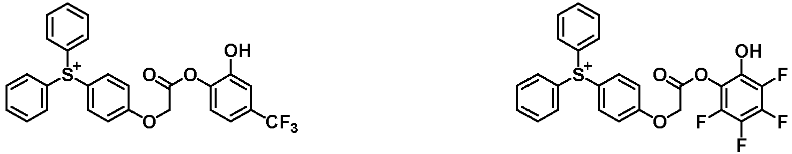

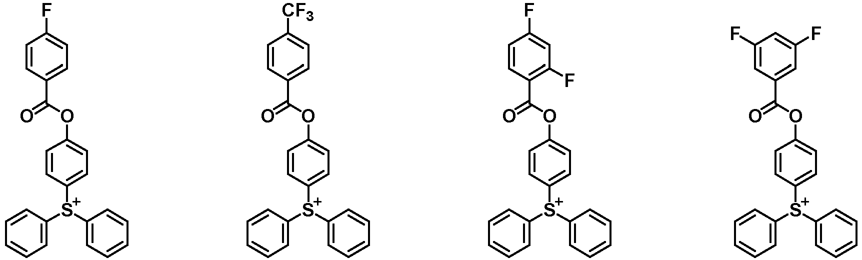

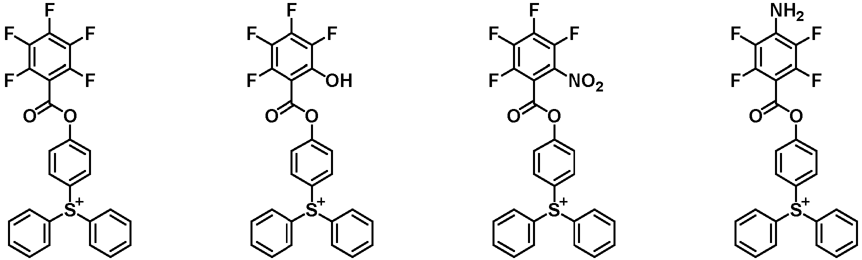

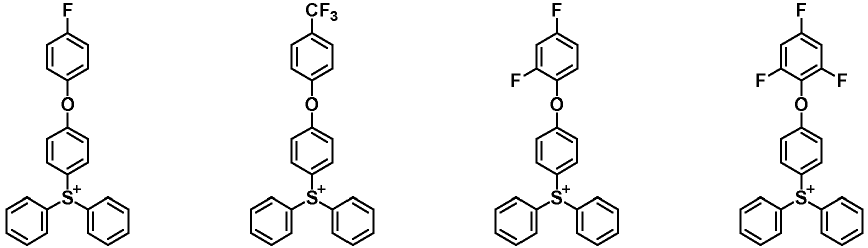

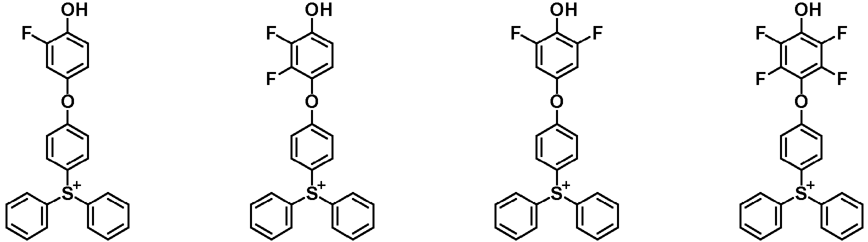

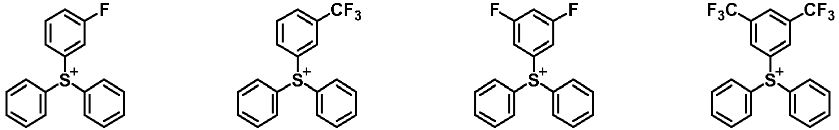

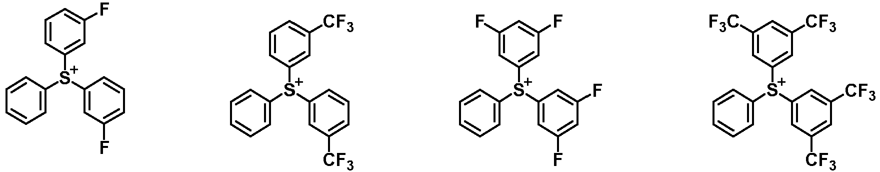

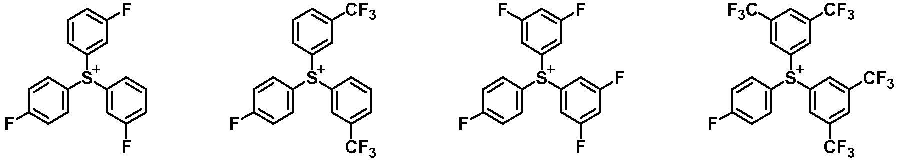

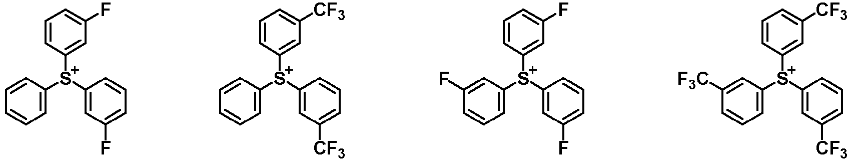

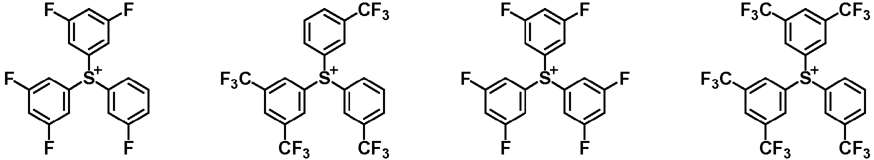

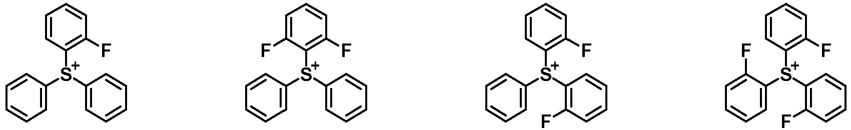

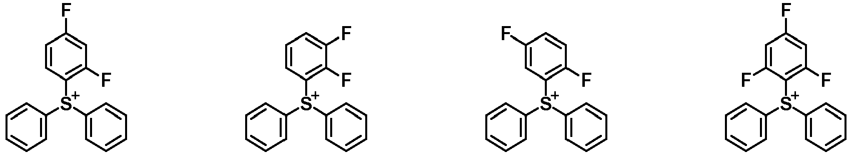

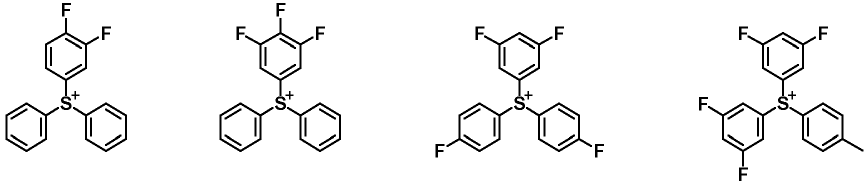

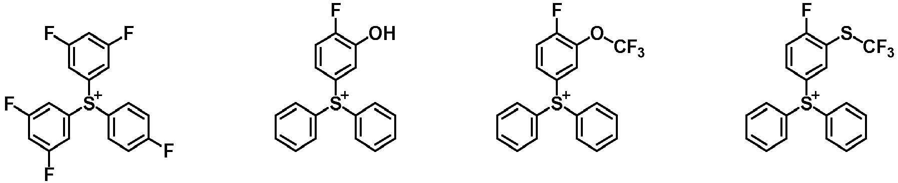

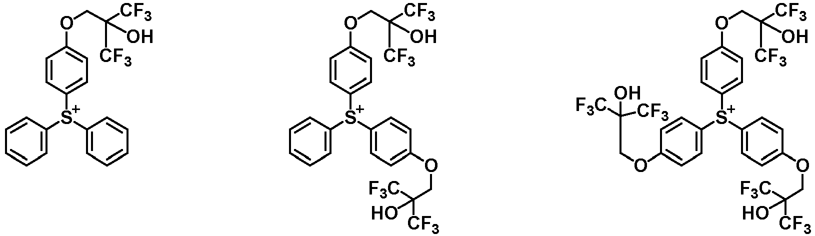

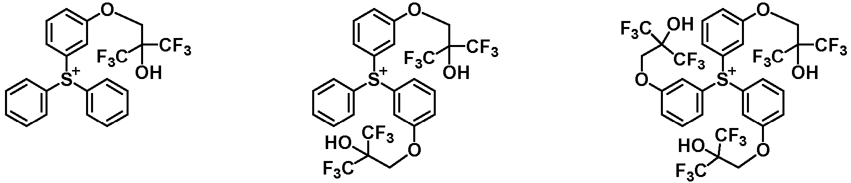

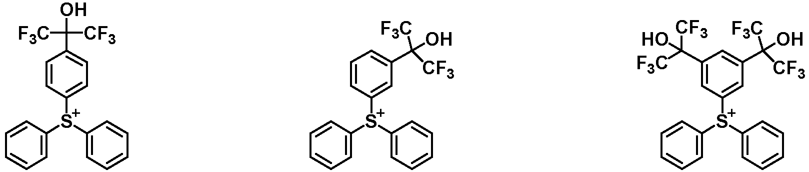

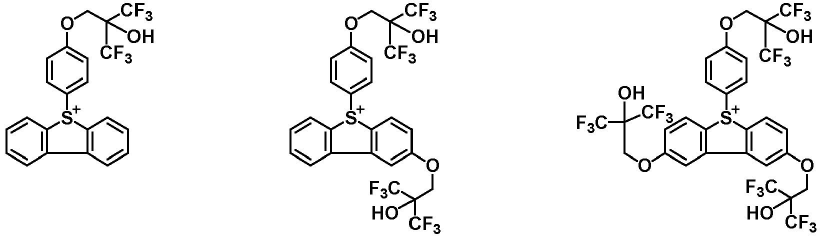

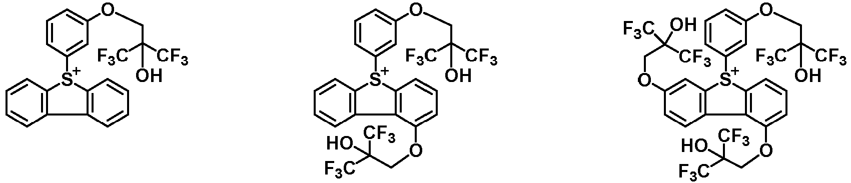

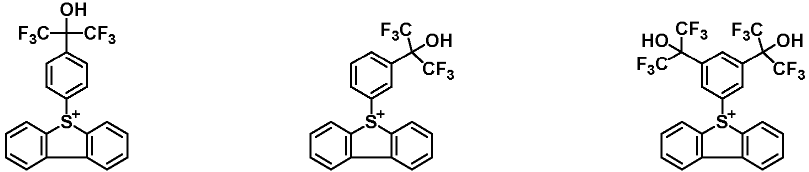

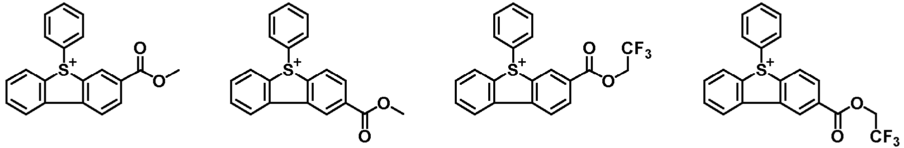

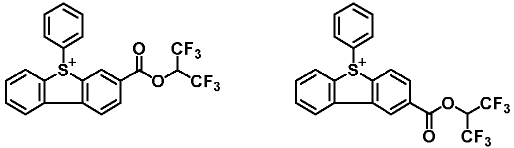

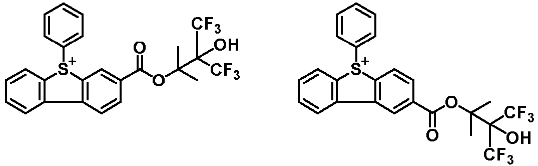

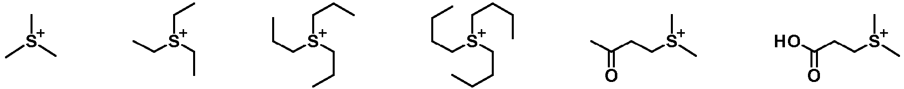

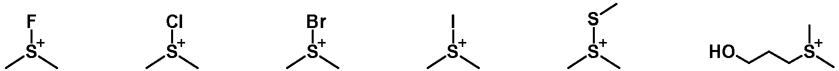

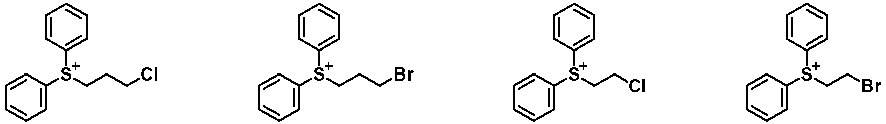

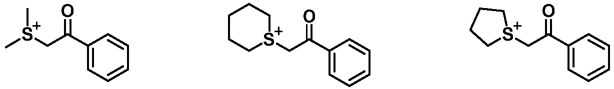

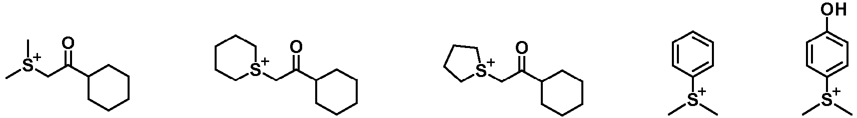

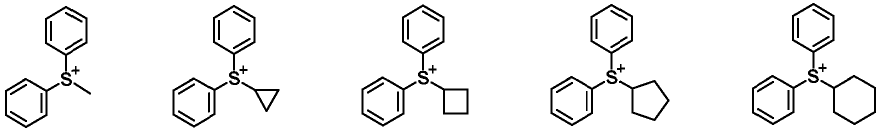

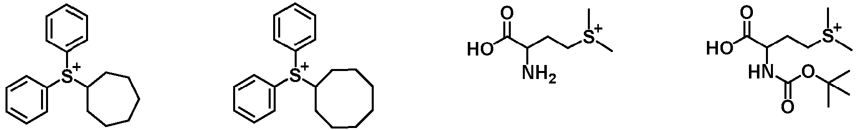

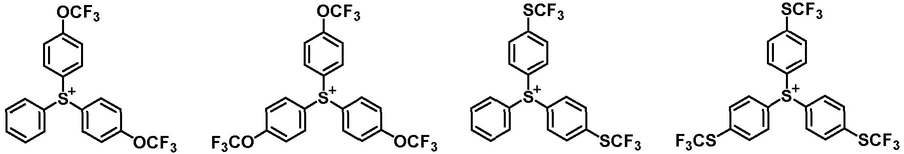

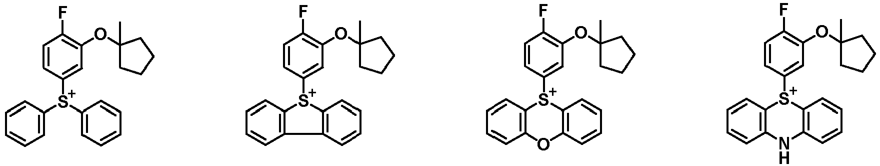

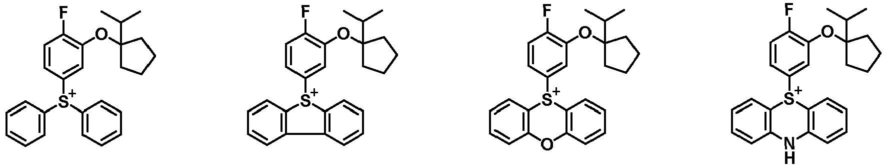

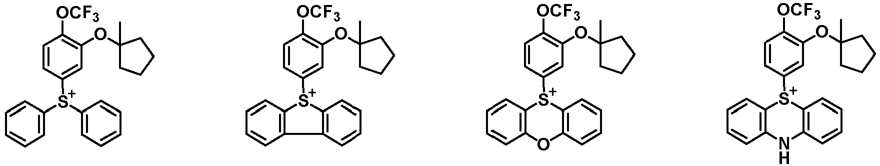

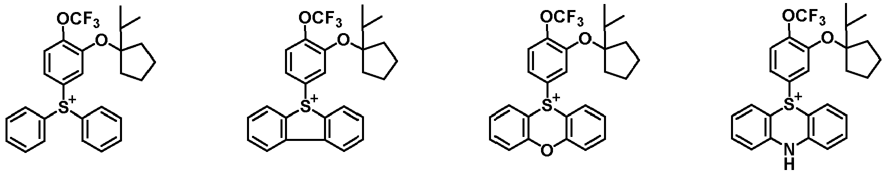

Specific examples of the iodonium cation having formula (cation-2) are shown below, but are not limited thereto.

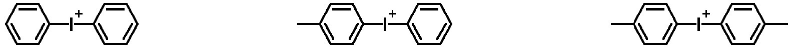

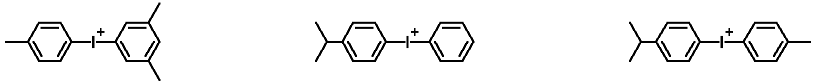

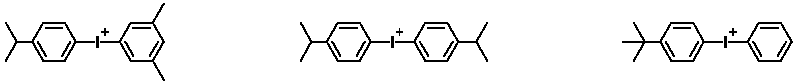

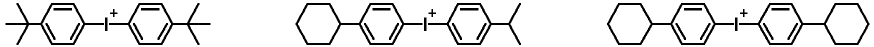

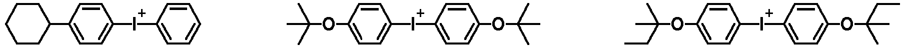

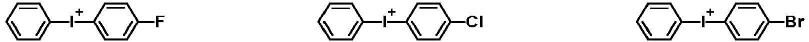

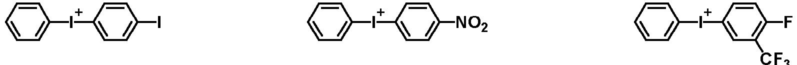

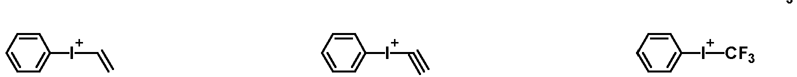

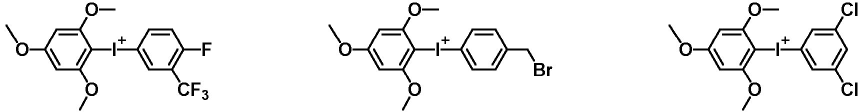

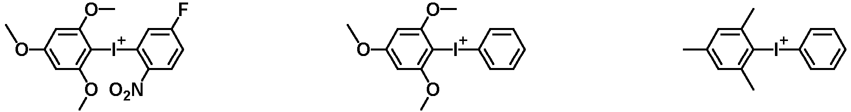

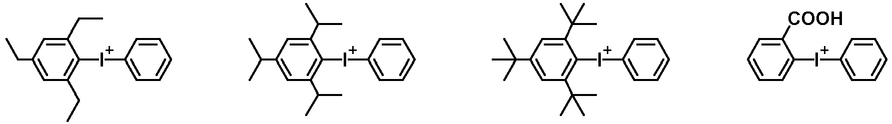

Specific examples of the monomer having formula (A1) include any combination of the anion and the cation described above.

The monomer having formula (A1) can be synthesized, for example, in the same manner as the sulfonium salt having a polymerizable anion described in JP 5201363, but is not limited thereto.

### Polymer

The polymer of the present invention comprises repeat units derived from an onium salt monomer having formula (A1) (hereinafter, also referred to as repeat units A1) (hereinafter, the polymer is also referred to as polymer A).

The repeat unit A1 is a unit that generates an acid by exposure, and the polymer A functions as a polymer-bonded photoacid generator in the resist composition.

The polymer A preferably further comprises repeat units A2 having a phenolic hydroxy group having the formula (A2). The repeat unit A2 is a repeat unit that imparts etching resistance and imparts adhesion to a substrate and solubility in alkaline developer.

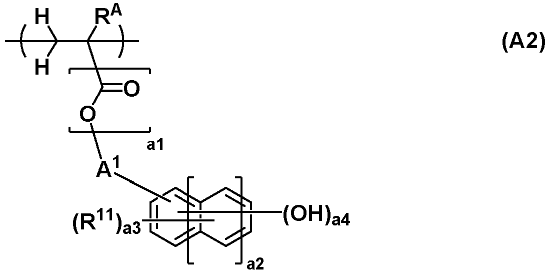

**In** formula (A2), a1 is 0 or 1, a2 is 0, 1, or 2, when a2 is 0, a2 represents a benzene structure, when a2 is 1, a2 represents a naphthalene structure, and when a2 is 2, a2 represents an anthracene structure, a3 is an integer satisfying 0 ≤ a3 ≤ 5+2(a2)-a4, a4 is 1, 2, or 3, and when a2 is 0, preferably, a3 is 0, 1, 2, or 3, and a4 is 1, 2, or 3, and when a2 is 1 or 2, preferably, a3 is 0, 1, 2, 3, or 4, and a4 is 1, 2, or 3.

In formula (A2), R^{A} is a hydrogen atom, a fluorine atom, a methyl group, or a trifluoromethyl group.

In formula (A2), R¹¹ is a halogen atom, an optionally halogenated C₁-C₆ saturated hydrocarbyl group, an optionally halogenated C₁-C₆ saturated hydrocarbyloxy group, or an optionally halogenated C₂-C₈ saturated hydrocarbylcarbonyloxy group. The saturated hydrocarbyl group and saturated hydrocarbyl moiety in the saturated hydrocarbyloxy group and saturated hydrocarbylcarbonyloxy group may be straight, branched, or cyclic. Specific examples thereof include alkyl groups such as a methyl group, an ethyl group, an n-propyl group, an isopropyl group, a butyl group, a pentyl group, a hexyl group, and structural isomers thereof; cycloalkyl groups such as a cyclopropyl group, a cyclobutyl group, a cyclopentyl group, and a cyclohexyl group; and combinations thereof. A carbon count within the upper limit ensures a sufficient solubility in alkaline developer. When a3 is 2 or more, each R¹¹ may be identical or different.

In formula (A2), A¹ is a single bond or a C₁-C₁₀ saturated hydrocarbylene group in which some -CH₂- may be replaced by -O-. The saturated hydrocarbylene group may be straight, branched, or cyclic, and examples thereof include C₁-C₁₀ alkanediyl groups such as a methylene group, an ethane-1,2-diyl group, a propane-1,3-diyl group, a butane-1,4-diyl group, a pentane-1,5-diyl group, a hexane-1,6-diyl group, and structural isomers thereof; C₃-C₁₀ cyclic saturated hydrocarbylene groups such as a cyclopropanediyl group, a cyclobutanediyl group, a cyclopentanediyl group, and a cyclohexanediyl group; and combinations thereof. For the saturated hydrocarbylene group containing an ether bond, in case of a1=1 in formula (A2), the ether bond may be incorporated at any position excluding the position between the α- and β-carbon atoms relative to the ester oxygen atom. In case of a1=0, the atom bonding to the backbone becomes an ether oxygen atom, and a second ether bond may be incorporated at any position excluding the position between the α- and β-carbon atoms relative to the ether oxygen atom. Saturated hydrocarbylene groups having 10 carbon atoms or less are preferred because of a sufficient solubility in alkaline developer.

Preferred examples of the repeat units A2 wherein a1=0 and A¹ is a single bond (meaning that the aromatic ring is directly bonded to the main chain of the polymer), that is, repeat units free of a linker: -C(=O)-O-A¹- include units derived from 3-hydroxystyrene, 4-hydroxystyrene, 5-hydroxy-2-vinylnaphthalene, and 6-hydroxy-2-vinylnaphthalene. More preferred repeat units are repeat units having the formula (A2-1).

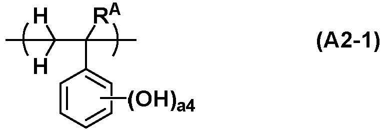

Herein R^{A} and a4 are as defined above.

Preferred examples of the repeat units A2 wherein a1=1, that is, having a linker: -C(=O)-O-A¹- are shown below, but are not limited thereto. Herein R^{A} is as defined above.

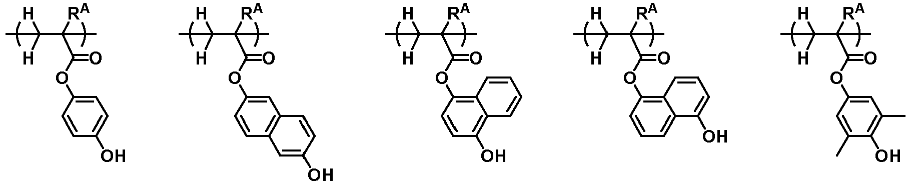

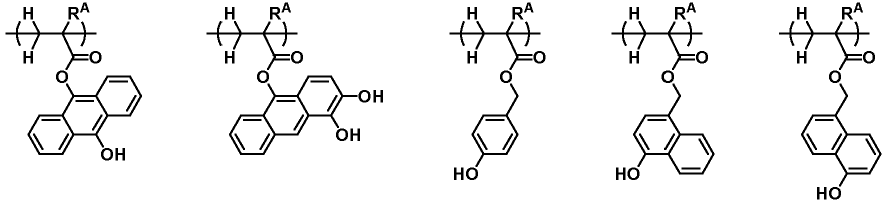

The repeat units A2 may be of one type or a combination of plural types.

The polymer A may comprise repeat units of at least one type selected from repeat units having the formula (A3) (hereinafter, referred to as repeat units A3) and repeat units having the formula (A4) (hereinafter, referred to as repeat units A4) (hereinafter, among the polymers A, a polymer further comprising repeat units at least one type selected from the repeat units A3 and the repeat units A4 is referred to as polymer A').

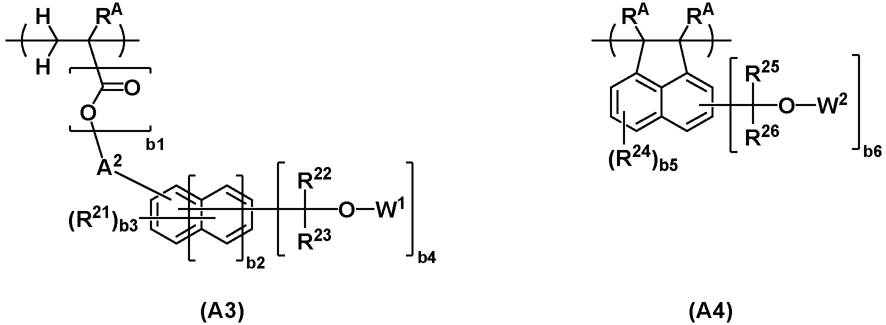

Upon exposure to high-energy radiation, the repeat units A3 and A4 function such that -O-W¹ or -O-W² undergoes elimination reaction under the action of an acid which is generated by the acid generator. That is, the repeat units A3 and A4 induce insolubilization in alkaline developer and crosslinking reaction between polymer molecules. The repeat units A3 and A4 provide for efficient progress of negative-working reaction, leading to an improvement in resolution performance.

In formula (A3), b1 is 0 or 1, b2 is 0, 1, or 2, b3 is an integer satisfying 0 ≤ b3 ≤ 5+2(b2)-b4, and b4 is 1, 2, or 3.

In formula (A4), b5 is 0, 1, or 2, and is preferably 0 or 1, and b6 is 1 or 2, and is preferably 1.

In formulae (A3) and (A4), R^{A} is each independently a hydrogen atom, a fluorine atom, a methyl group, or a trifluoromethyl group. In formula (A4), a hydrogen atom or a methyl group is preferred, and a hydrogen atom is more preferred.

In formula (A3), R²¹ is a C₁-C₂₀ hydrocarbyl group which may contain a halogen atom or a heteroatom. Specific examples of the halogen atom represented by R²¹ include a fluorine atom, a chlorine atom, a bromine atom, and an iodine atom. The C₁-C₂₀ hydrocarbyl group R²¹ may be saturated or unsaturated, and may be straight, branched, or cyclic. Specific examples thereof are as exemplified above for the hydrocarbyl group R¹ in formula (A1). When b3 is 2 or more, each R²¹ may be identical or different.

In formula (A3), R²² and R²³ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group. Provided that R²² and R²³ are not hydrogen atoms at the same time. R²² and R²³ may bond together to form a ring with the carbon atoms to which they are attached, and some -CH₂- in the ring may be replaced by -O- or -S-. Preferred examples of R²² and R²³ include alkyl groups such as a methyl group, an ethyl group, a propyl group, a butyl group, and structural isomers thereof, and substituted forms of the foregoing alkyl groups in which some hydrogen atoms are substituted by a hydroxy group or a saturated hydrocarbyloxy group.

In formula (A4), R²⁴ is a C₁-C₂₀ hydrocarbyl group which may contain a halogen atom or a heteroatom. Specific examples of the halogen atom represented by R²⁴ include a fluorine atom, a chlorine atom, a bromine atom, and an iodine atom. The C₁-C₂₀ hydrocarbyl group R²⁴ may be saturated or unsaturated, and may be straight, branched, or cyclic. Specific examples thereof are as exemplified above for the hydrocarbyl group R¹ in formula (A1). When b5 is 2 or more, each R²⁴ may be identical or different.

In formula (A4), R²⁵ and R²⁶ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group.

The C₁-C₁₅ saturated hydrocarbyl groups R²⁵ and R²⁶ may be straight, branched, or cyclic. Specific examples thereof are as exemplified above for the saturated hydrocarbyl groups R²² and R²³.

Specific examples of the C₆-C₁₅ aryl groups R²⁵ and R²⁶ include a phenyl group, a naphthyl group, and an anthryl group, and among them, a phenyl group is preferred. The aryl group may have a substituent, and specific examples of the substituent include a halogen atom, an optionally halogenated C₁-C₆ saturated hydrocarbyl group, and an optionally halogenated C₁-C₆ saturated hydrocarbyloxy group.

R²⁵ and R²⁶ are not hydrogen atoms at the same time. When one of R²⁵ and R²⁶ is an optionally substituted aryl group, the preferred substituent on the other of R²⁵ and R²⁶ is a hydrogen atom.

Preferably, R²⁵ and R²⁶ are identical, more preferably, both are methyl groups.

R²⁵ and R²⁶ may bond together to form a ring with the carbon atoms to which they are attached, and some -CH₂- in the ring may be replaced by -O- or -S-. Examples of the ring include a cyclopropane ring, a cyclobutene ring, a cyclopentane ring, a cyclohexane ring, a norbornane ring, an adamantane ring, a tricyclo[5.2.1.0^{2,6}]decane ring, a tetracyclo[6.2.1.1^{3,6}.0^{2,7}]dodecane ring, an oxanorbornane ring, and a thianorbornane ring, but are not limited thereto.

In formula (A3), A² is a single bond or a C₁-C₁₀ saturated hydrocarbylene group in which some -CH₂- may be replaced by -O-. The saturated hydrocarbylene group may be straight, branched, or cyclic, and examples thereof include alkanediyl groups such as a methylene group, an ethane-1,2-diyl group, a propane-1,3-diyl group, a butane-1,4-diyl group, a pentane-1,5-diyl group, a hexane-1,6-diyl group, and structural isomers thereof; cyclic saturated hydrocarbylene groups such as a cyclopropanediyl group, a cyclobutanediyl group, a cyclopentanediyl group, and a cyclohexanediyl group; and combinations thereof. For the saturated hydrocarbylene group containing an ether bond, in case of b1=1 in formula (A3), the ether bond may be incorporated at any position excluding the position between the α- and β-carbon atoms relative to the ester oxygen atom. In case of b1=0, the atom bonding to the backbone becomes an ether oxygen atom, and a second ether bond may be incorporated at any position excluding the position between the α- and β-carbon atoms relative to the ether oxygen atom.

In formulae (A3) and (A4), W¹ and W² are each independently a hydrogen atom, a C₁-C₁₀ aliphatic hydrocarbyl group, a C₂-C₁₀ aliphatic hydrocarbylcarbonyl group, or an optionally substituted C₆-C₁₅ aryl group.

The C₁-C₁₀ aliphatic hydrocarbyl groups W¹ and W² may be saturated or unsaturated, and may be straight, branched, or cyclic. Specific examples thereof include C₁-C₁₀ alkyl groups such as a methyl group, an ethyl group, an n-propyl group, an isopropyl group, an n-butyl group, an isobutyl group, a sec-butyl group, a tert-butyl group, an n-pentyl group, a neopentyl group, an n-hexyl group, an n-heptyl group, an n-octyl group, a 2-ethylhexyl group, an n-nonyl group, and an n-decyl group; C₃-C₁₀ cyclic saturated hydrocarbyl groups such as a cyclopentyl group and a cyclohexyl group; C₂-C₁₀ alkenyl groups such as a vinyl group, a 1-propenyl group, a 2-propenyl group, a butenyl group, and a hexenyl group; and C₃-C₁₀ cyclic unsaturated hydrocarbyl groups such as a cyclohexenyl group. The hydrocarbyl moiety in the C₂-C₁₀ aliphatic hydrocarbylcarbonyl groups W¹ and W² may be saturated or unsaturated, and may be straight, branched, or cyclic. Specific examples thereof include C₁₋C₉ aliphatic hydrocarbyl groups among the specific examples of the C₁-C₁₀ aliphatic hydrocarbyl groups described above. Specific examples of the C₆-C₁₅ aryl groups W¹ and W² include a phenyl group, a naphthyl group, and an anthryl group, and among them, a phenyl group is preferred. The aryl group may have a substituent, and specific examples of the substituent include a halogen atom, an optionally halogenated C₁-C₆ saturated hydrocarbyl group, and an optionally halogenated C₁-C₆ saturated hydrocarbyloxy group.

Of the repeat units A3, repeat units having the formula (A3-1) or (A3-2) are preferred.

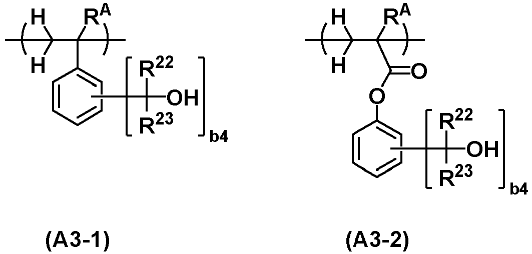

Herein b4, R^{A}, R²², and R²³ are as defined above.

Preferred examples of the repeat units A3 include those shown below, but are not limited thereto. In the formula below, R^{A} is as defined above, Me is a methyl group, and Ac is an acetyl group.

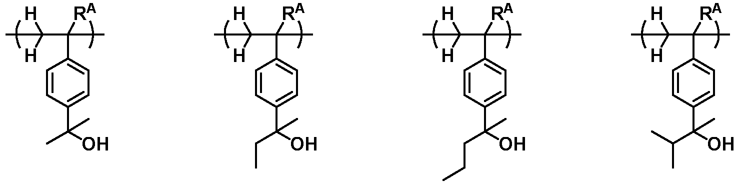

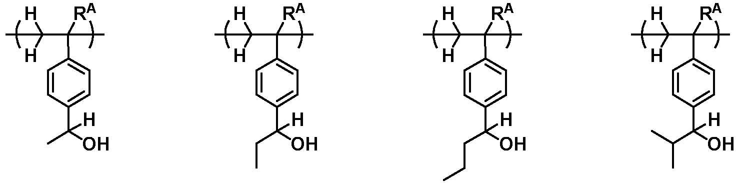

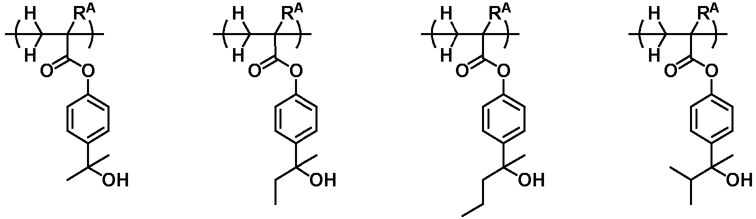

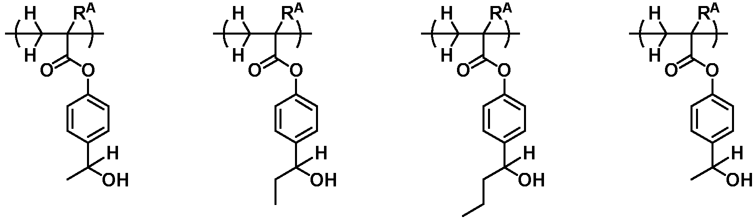

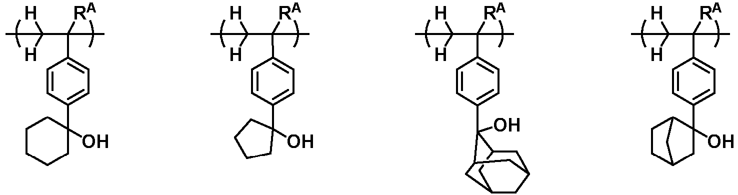

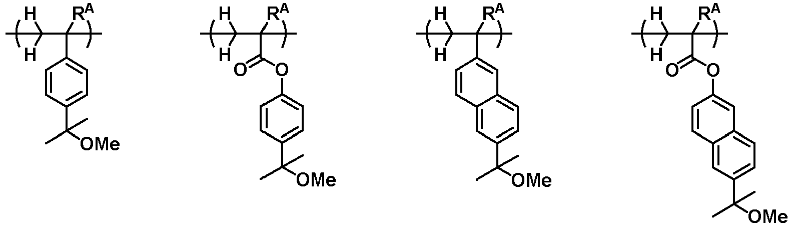

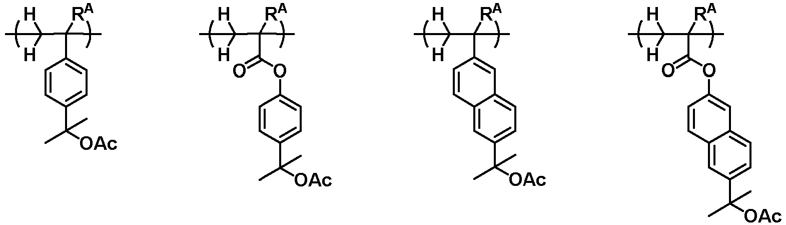

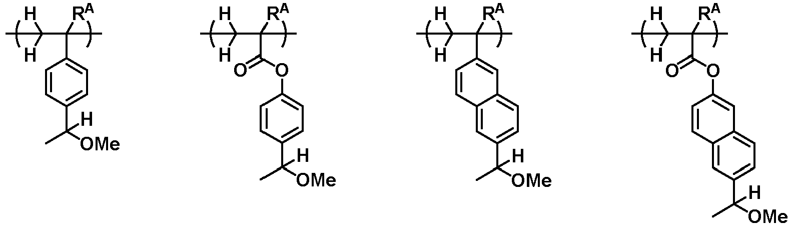

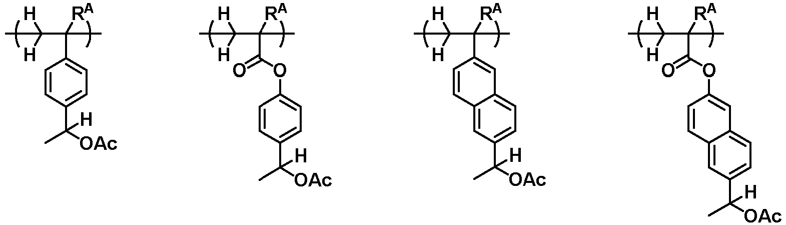

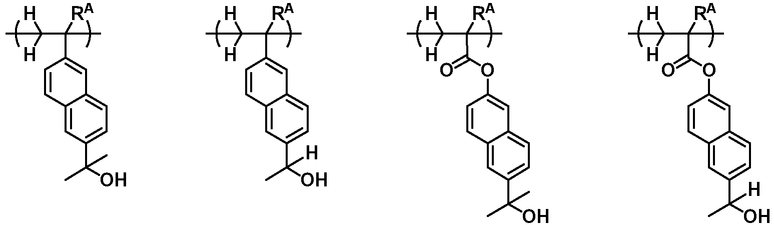

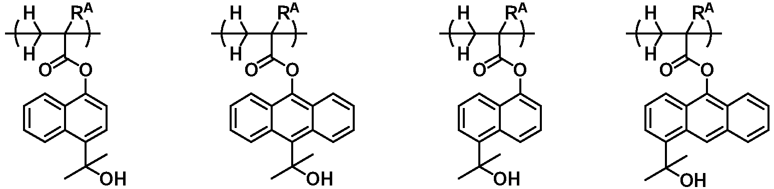

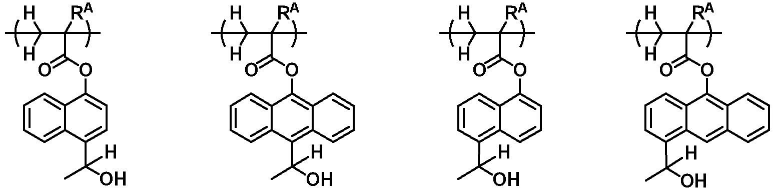

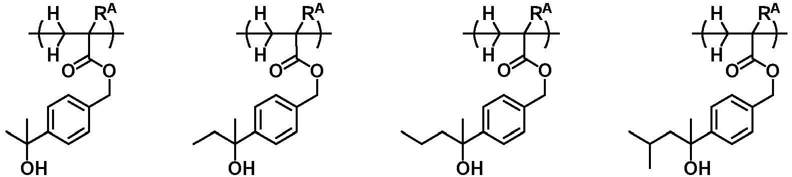

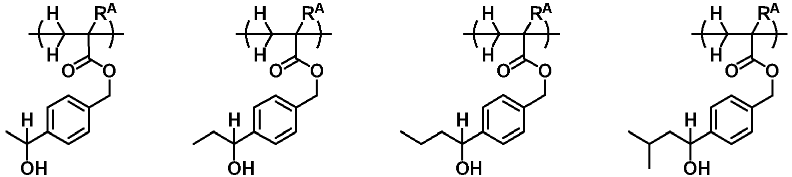

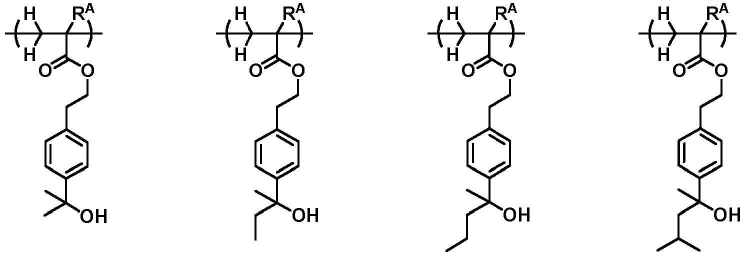

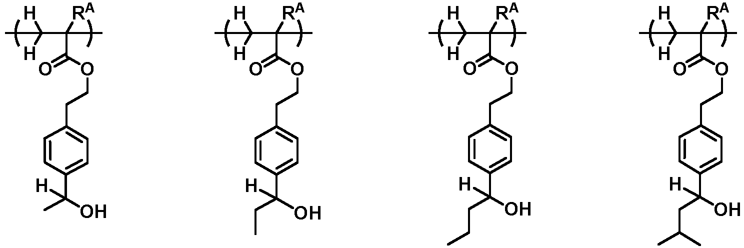

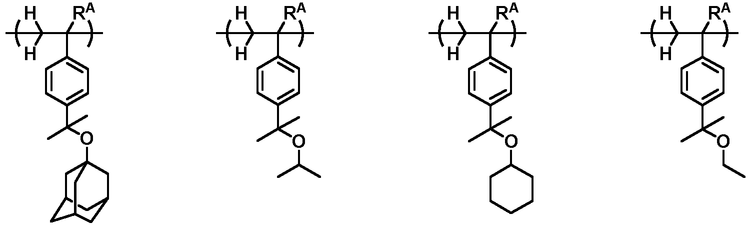

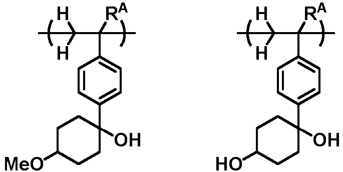

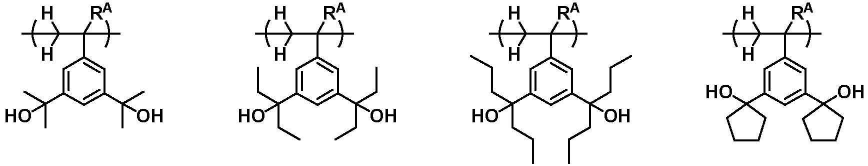

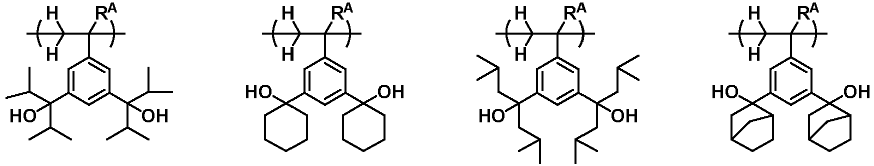

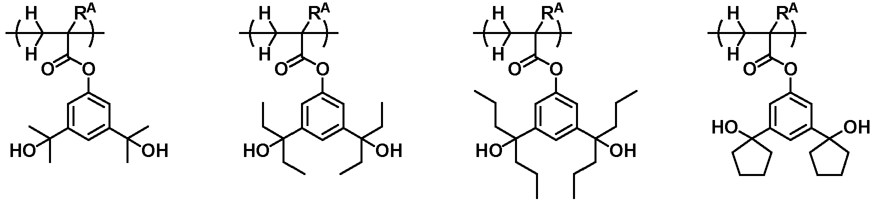

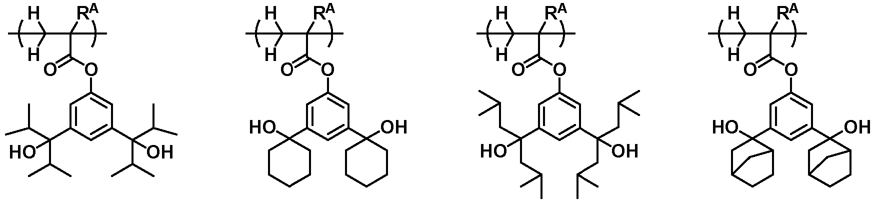

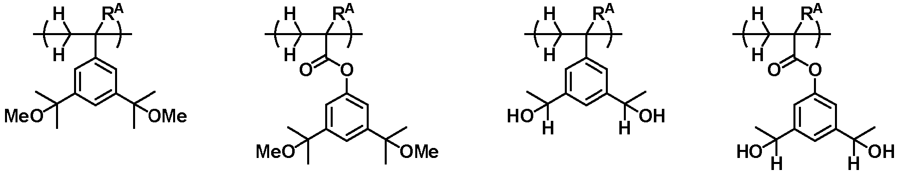

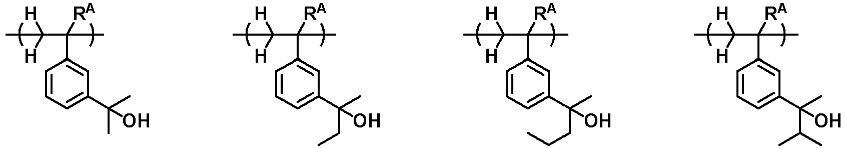

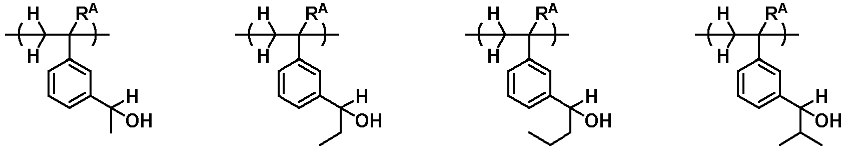

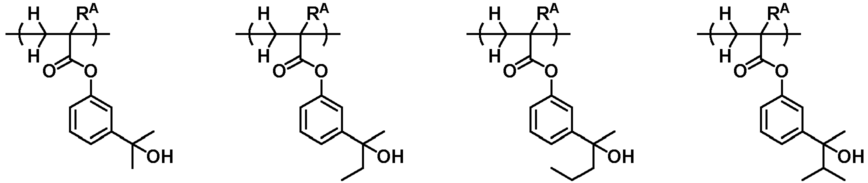

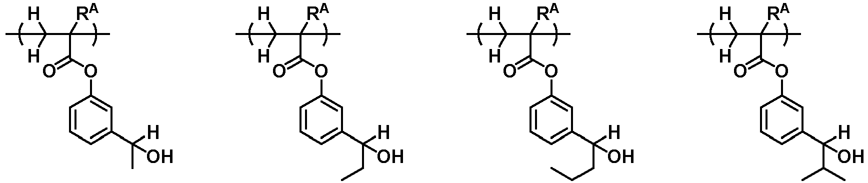

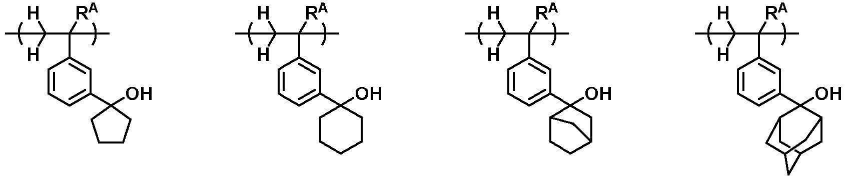

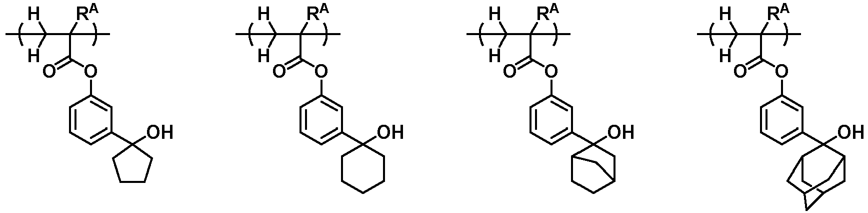

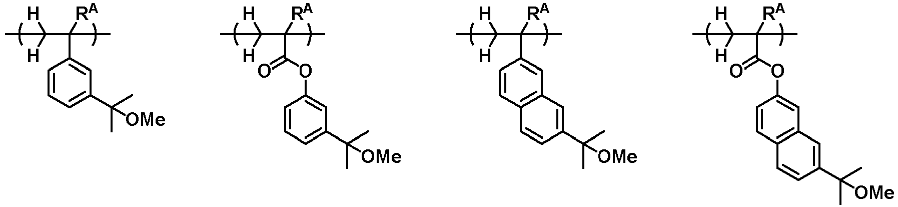

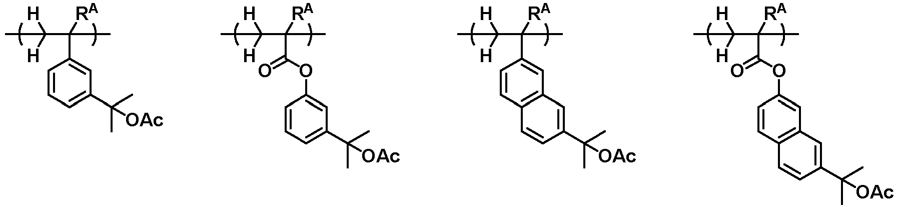

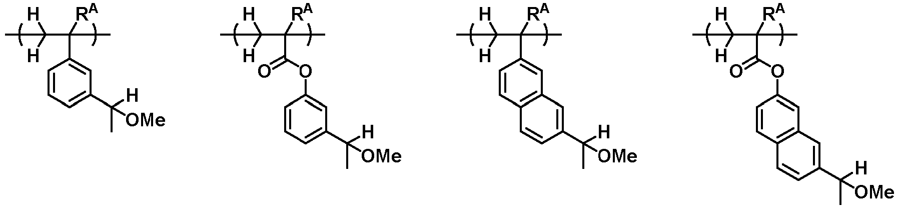

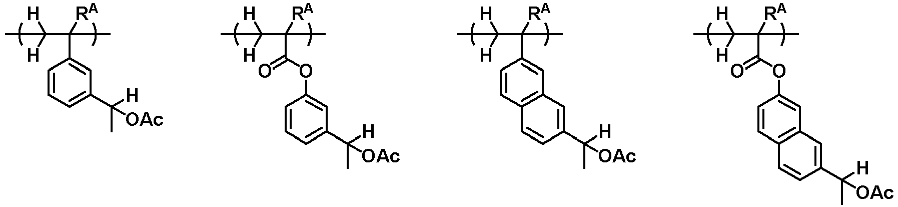

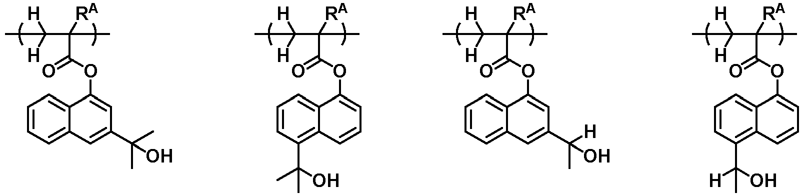

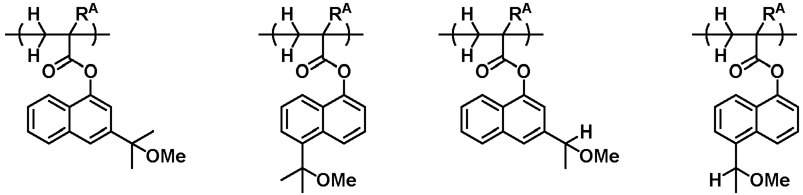

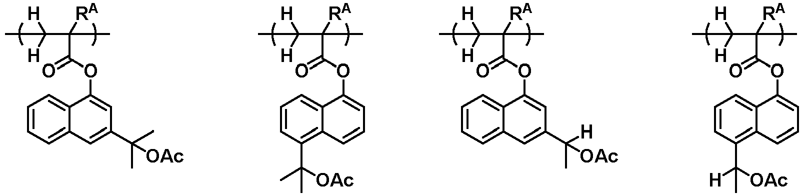

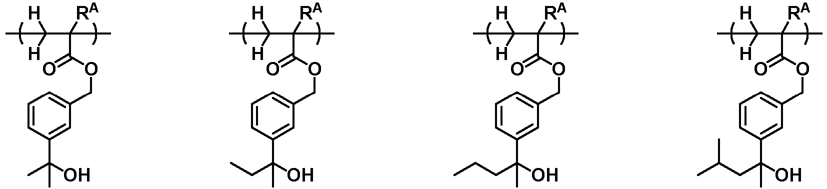

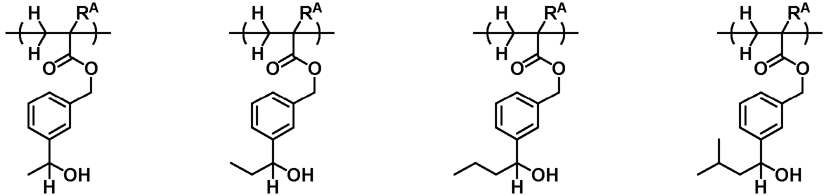

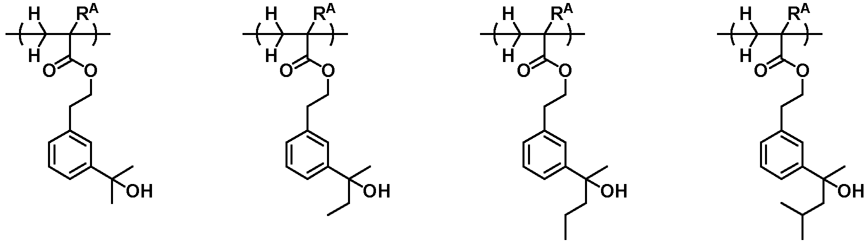

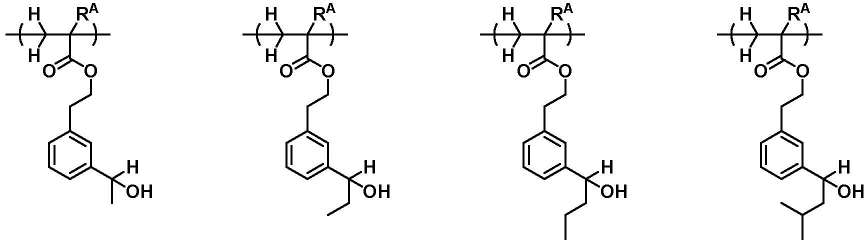

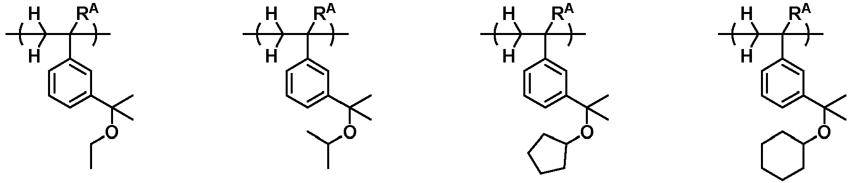

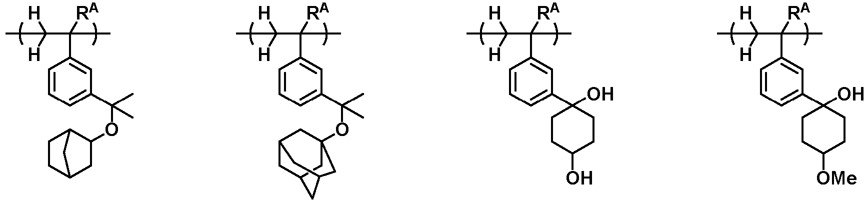

The repeat units A3 may be of one type or a combination of plural types.

Of the repeat units A4, repeat units having the formula (A4-1) are preferred.

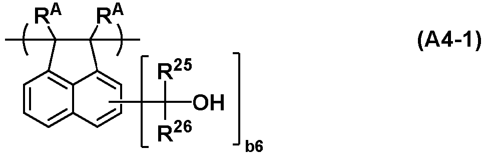

Herein b6, R^{A}, R²⁵, and R²⁶ are as defined above.

Preferred examples of the repeat units A4 include those shown below, but are not limited thereto. In the formula below, R^{A} is as defined above, Me is a methyl group, and Ac is an acetyl group.

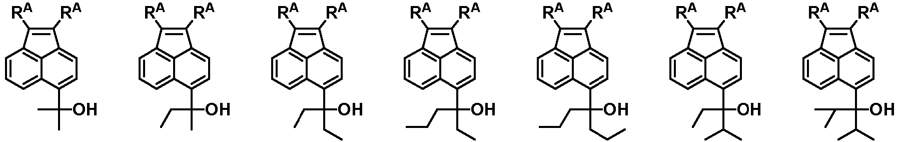

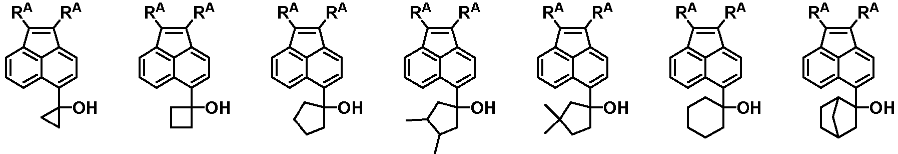

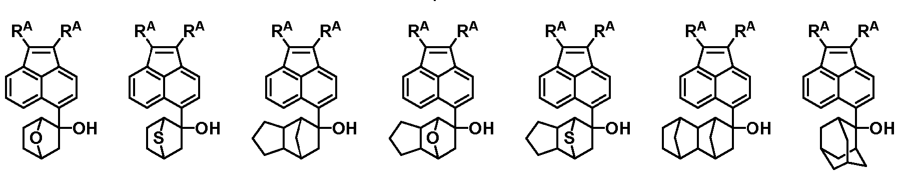

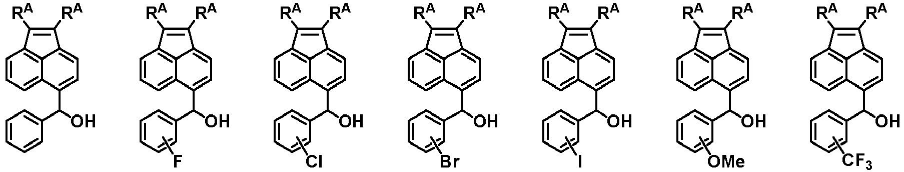

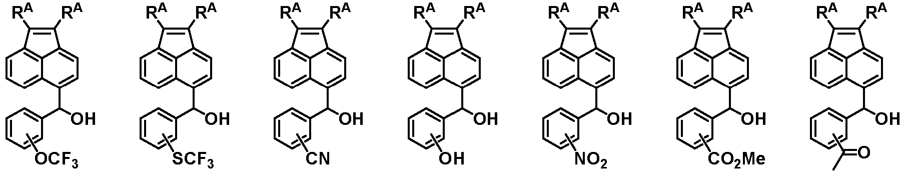

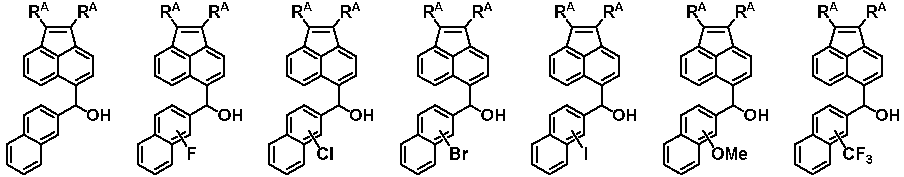

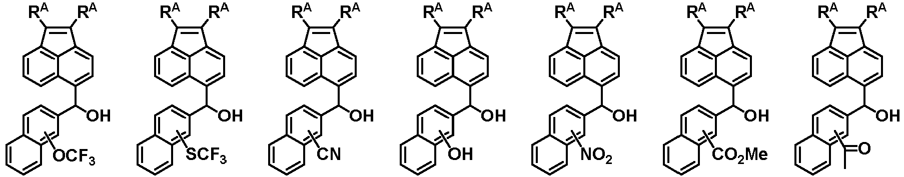

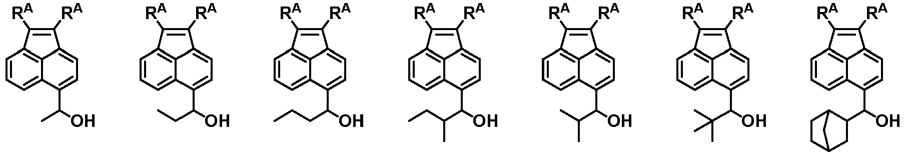

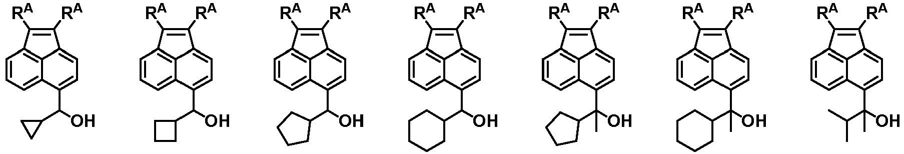

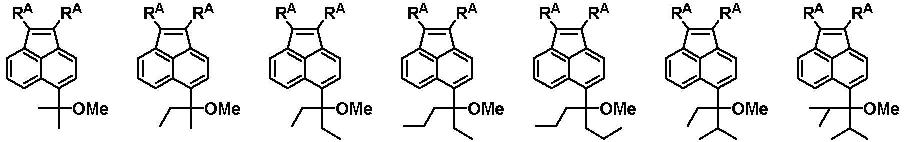

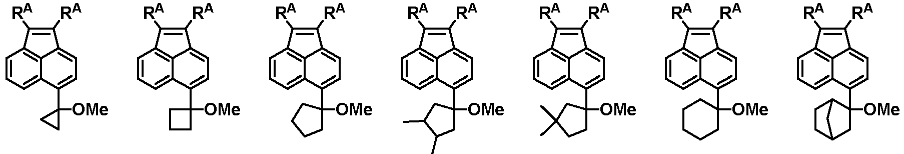

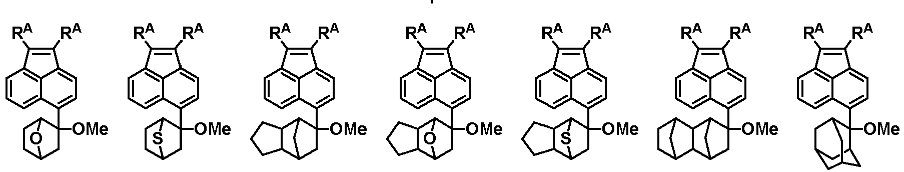

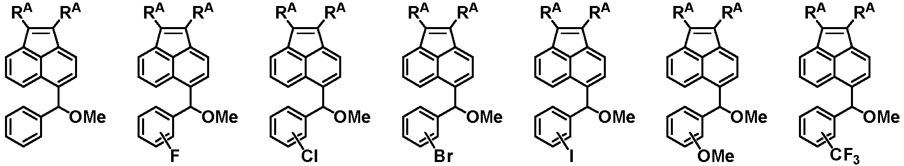

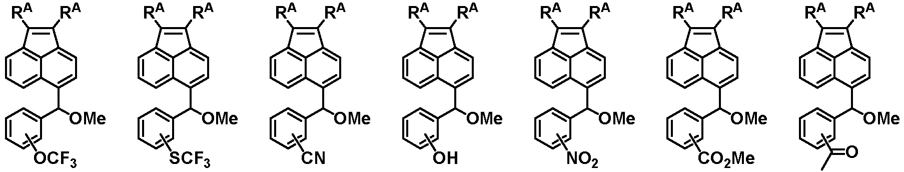

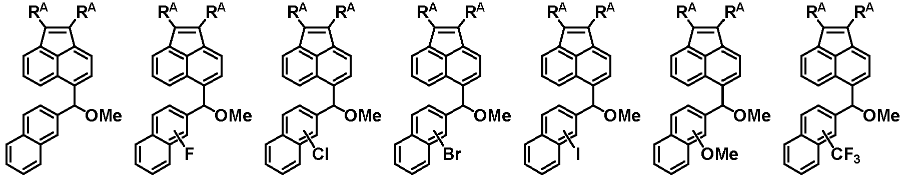

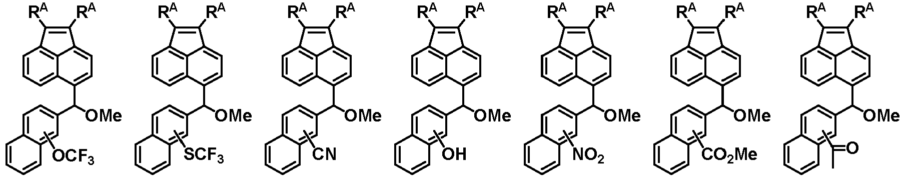

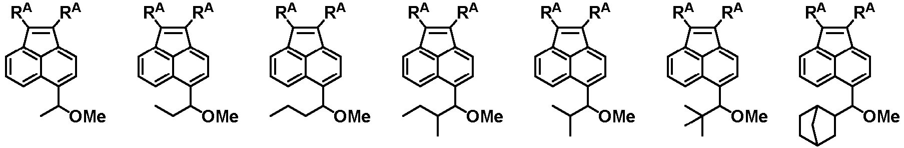

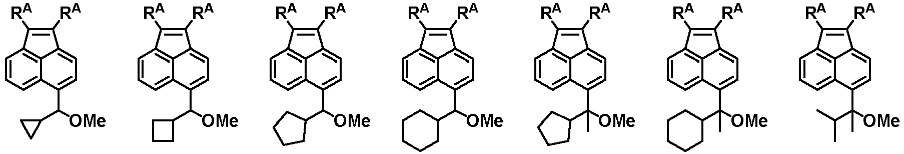

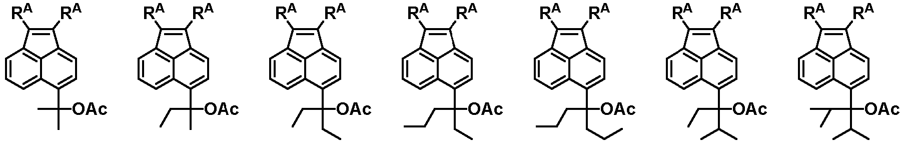

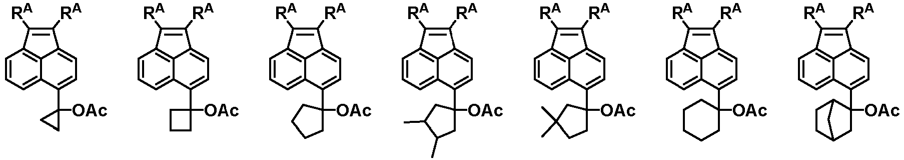

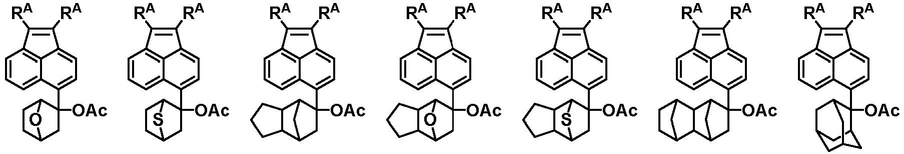

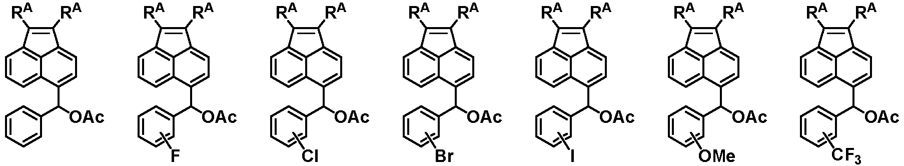

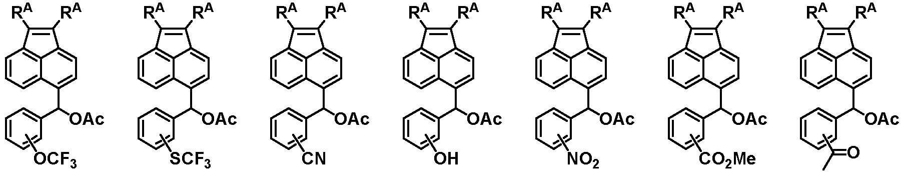

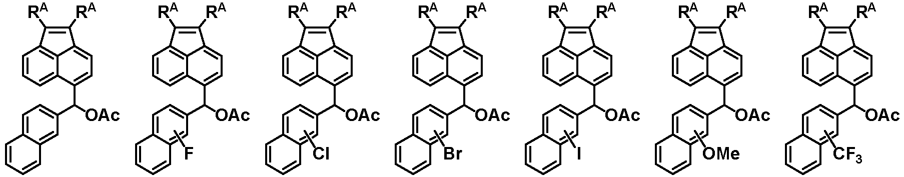

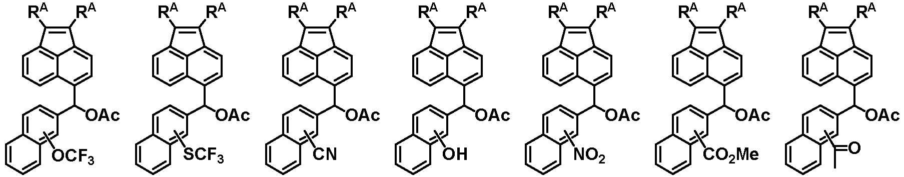

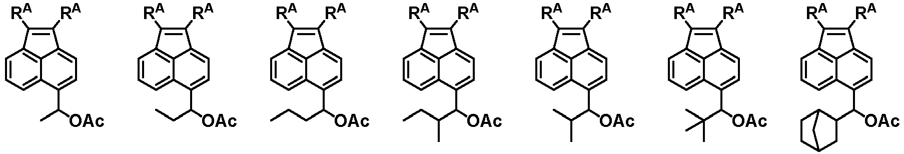

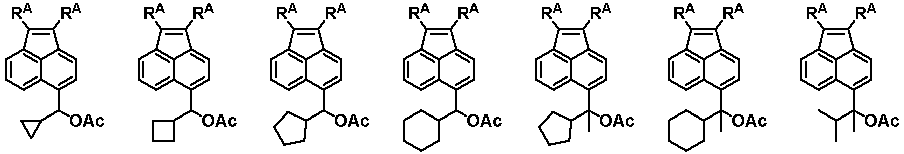

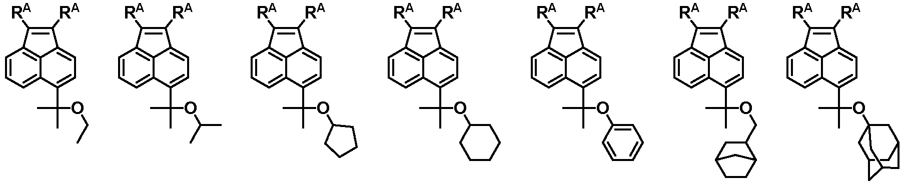

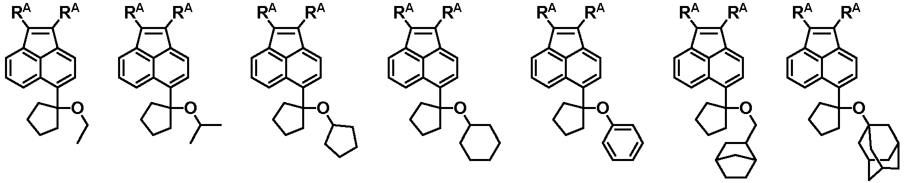

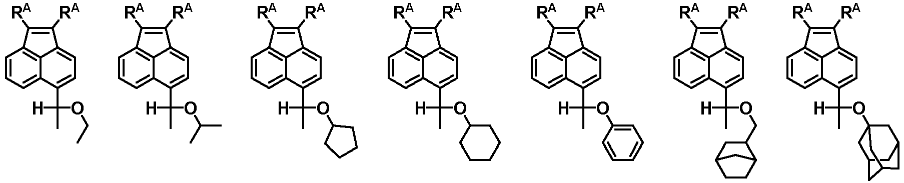

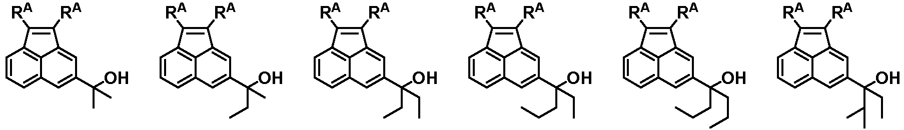

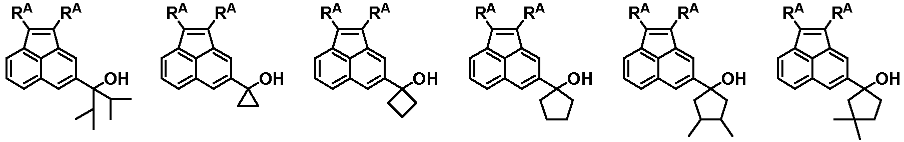

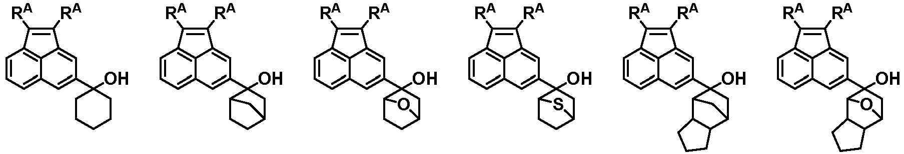

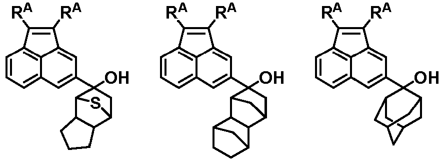

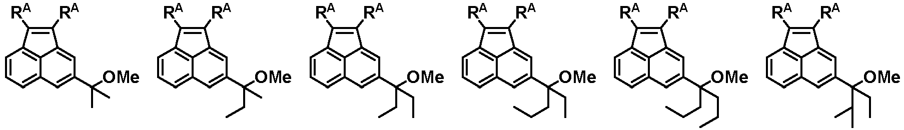

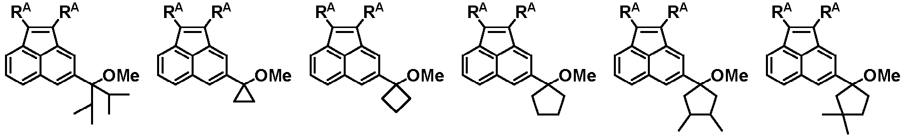

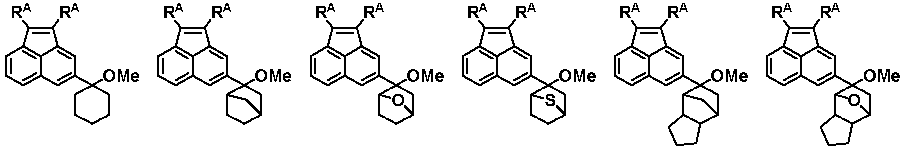

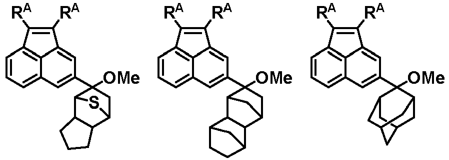

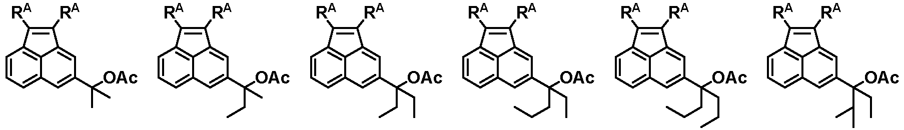

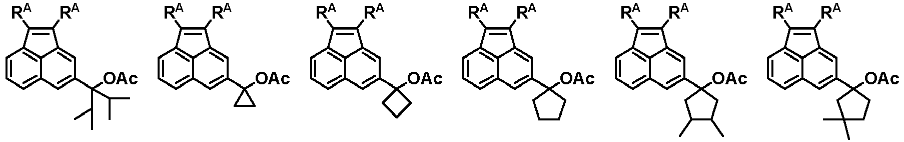

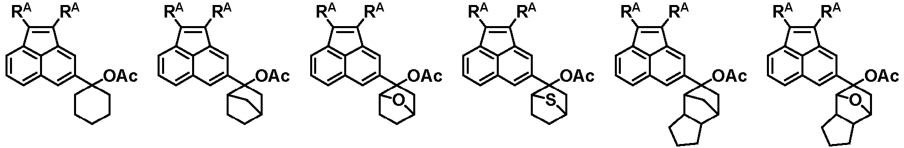

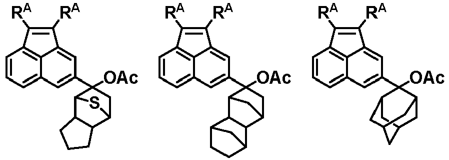

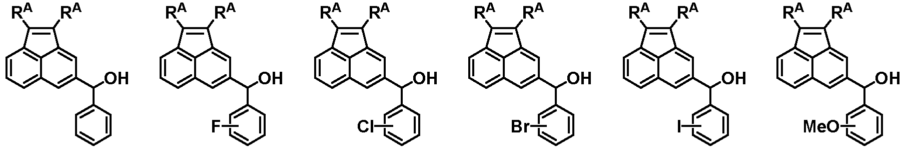

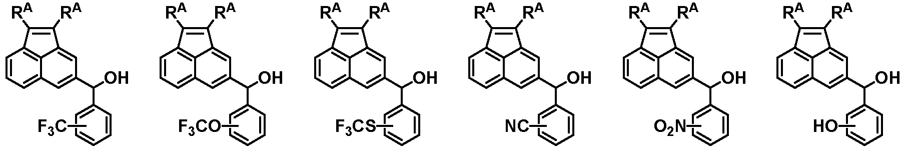

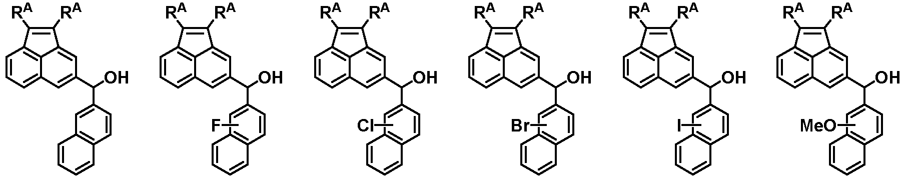

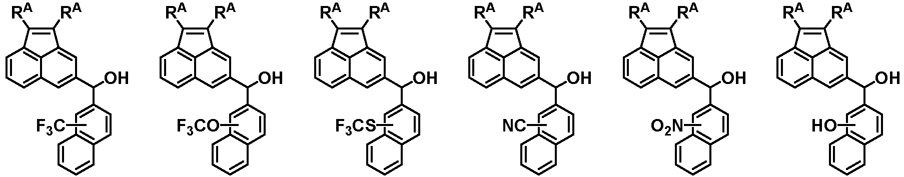

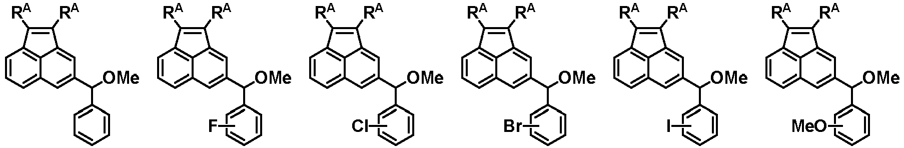

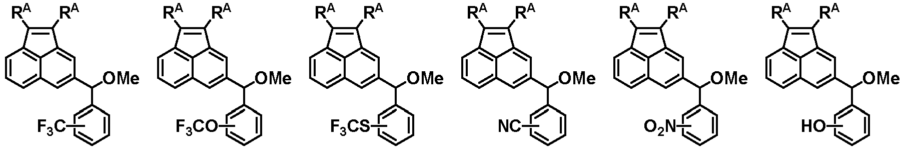

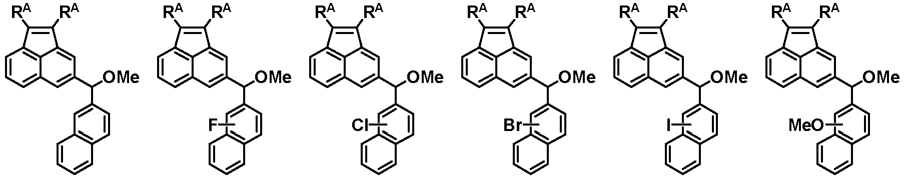

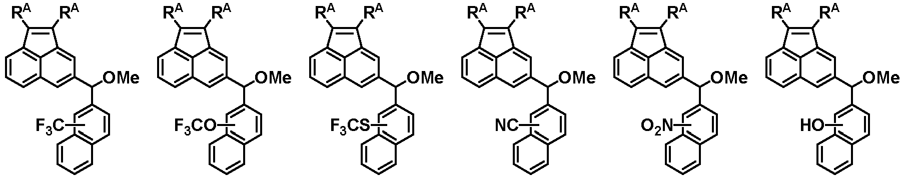

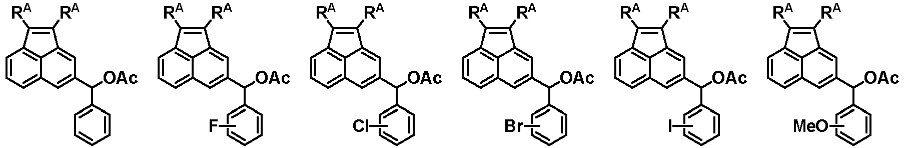

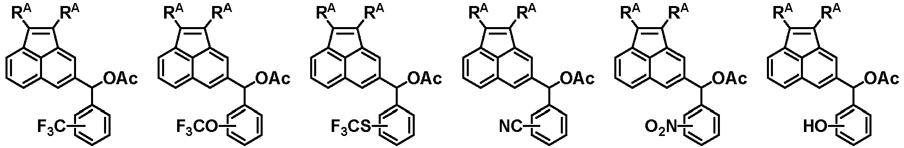

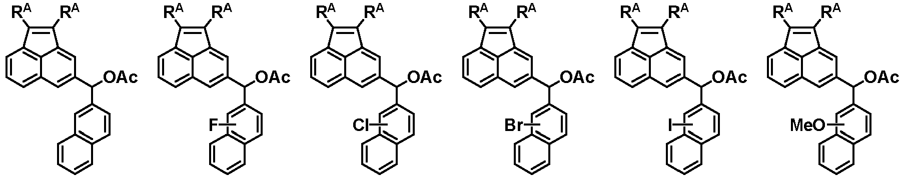

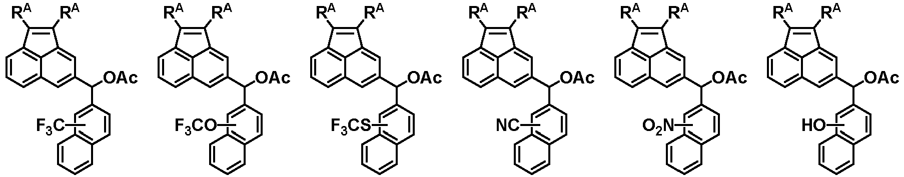

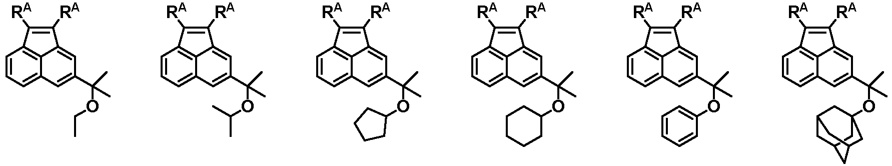

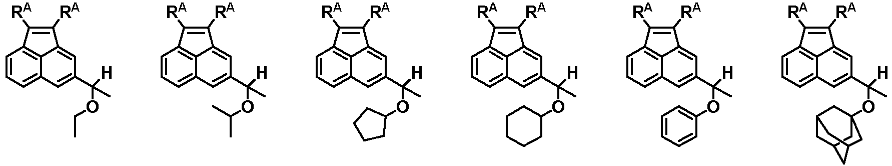

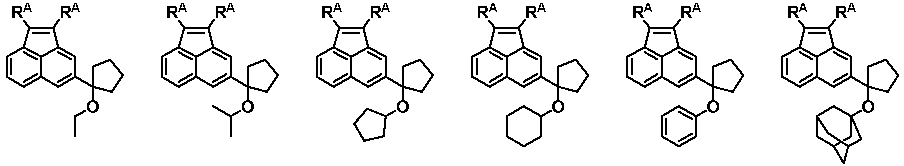

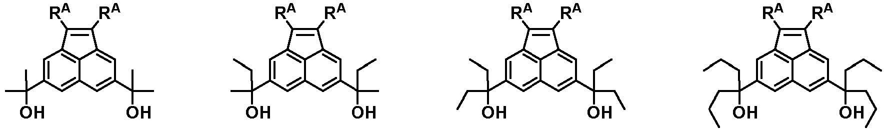

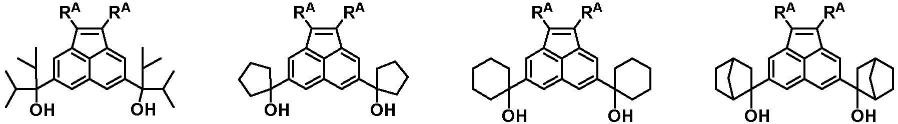

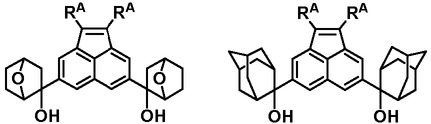

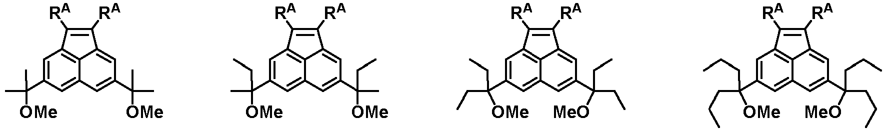

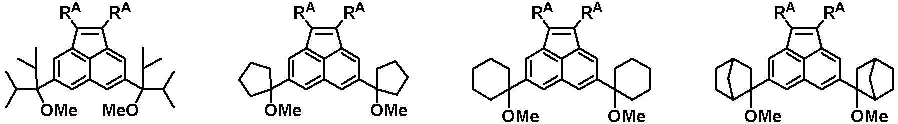

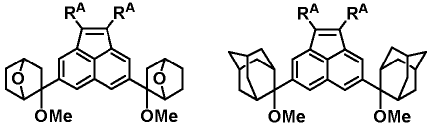

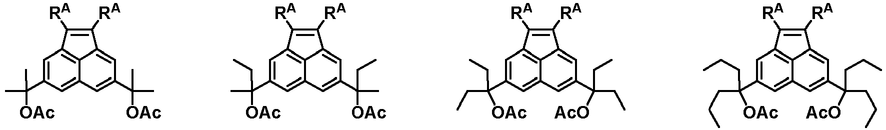

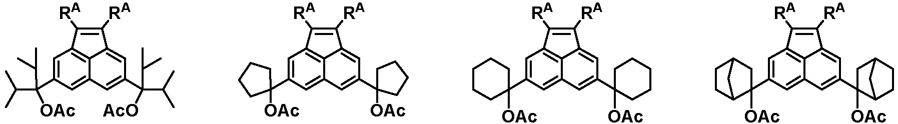

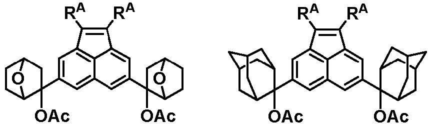

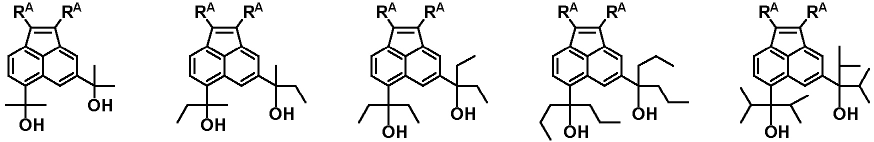

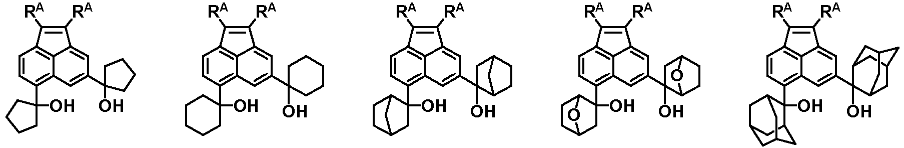

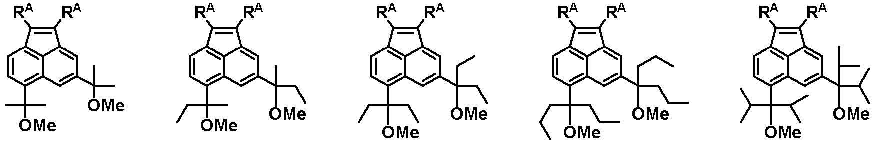

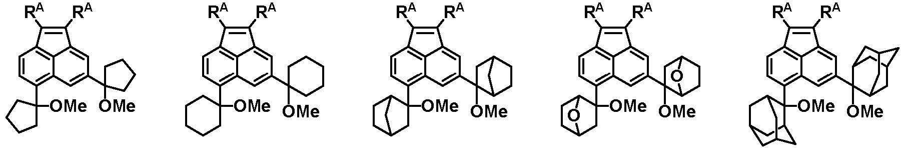

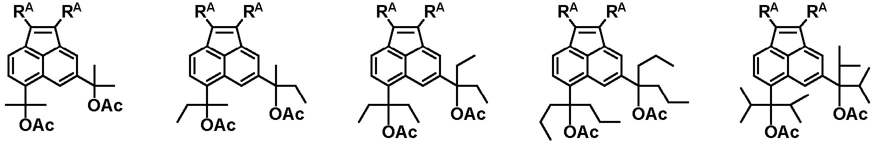

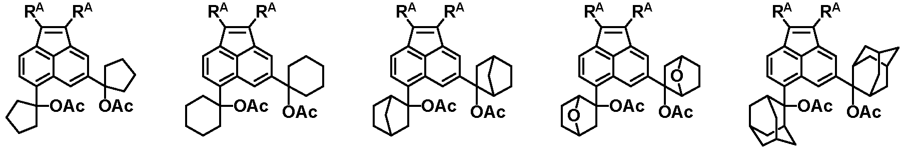

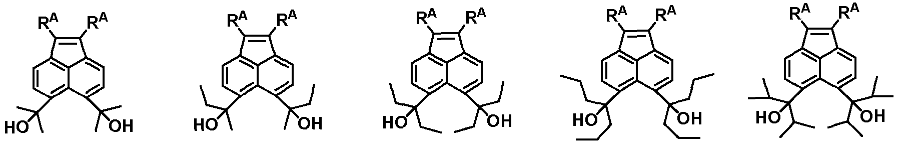

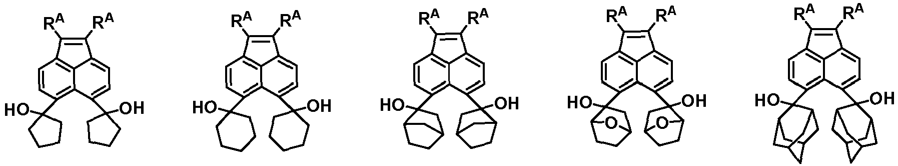

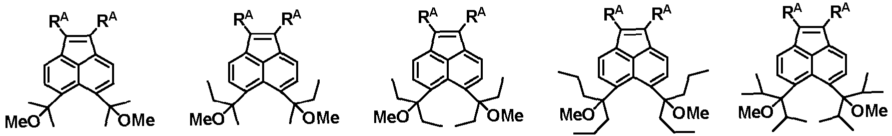

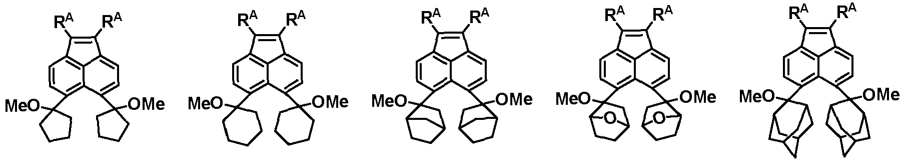

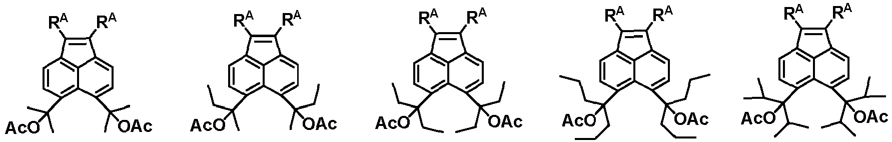

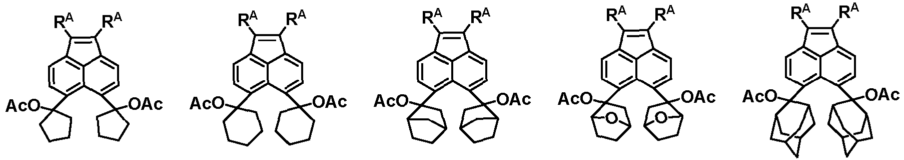

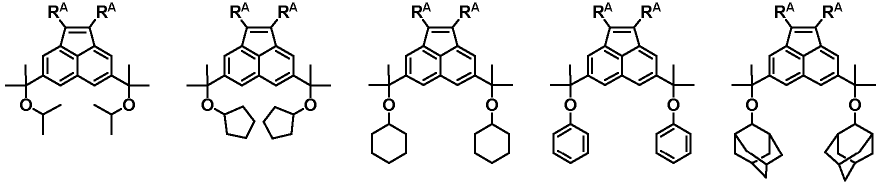

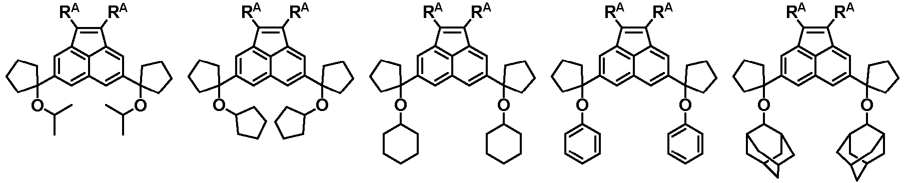

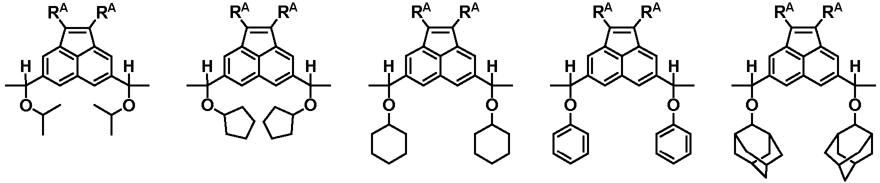

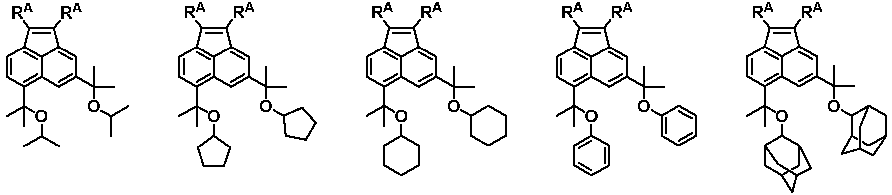

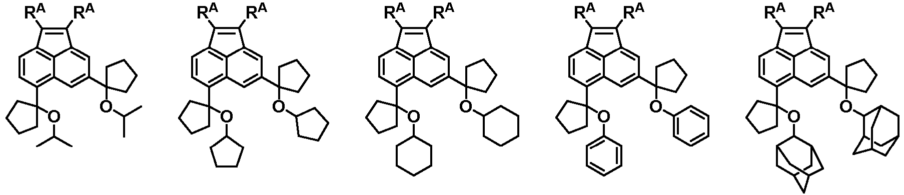

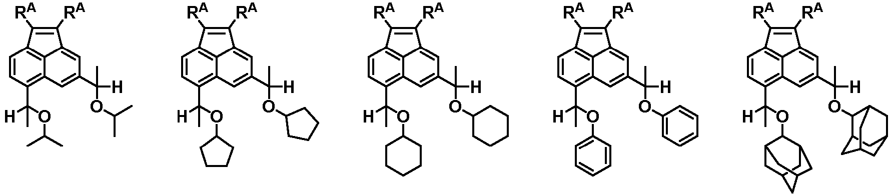

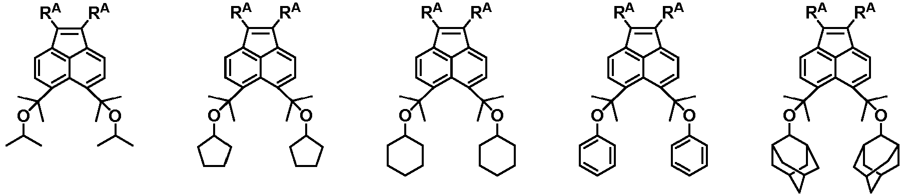

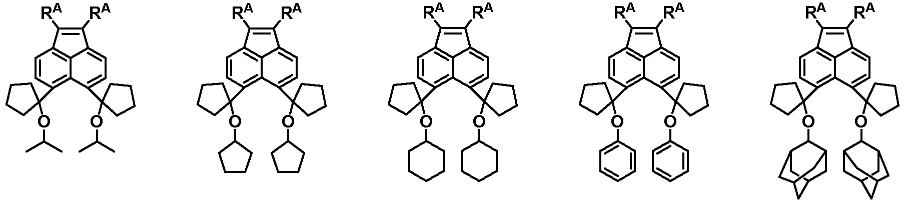

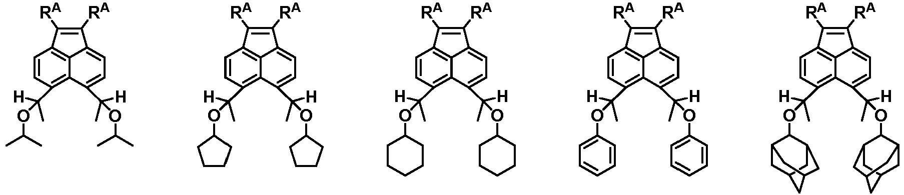

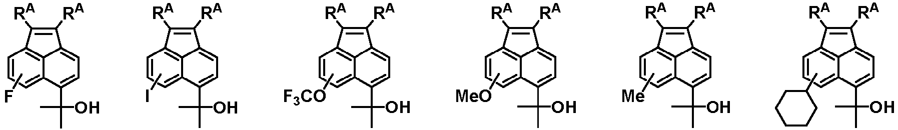

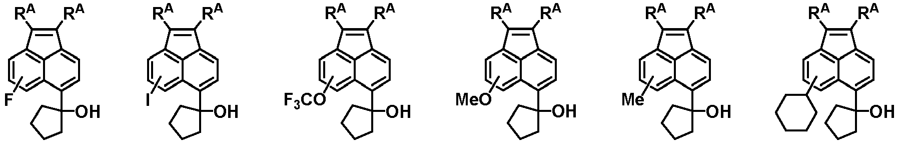

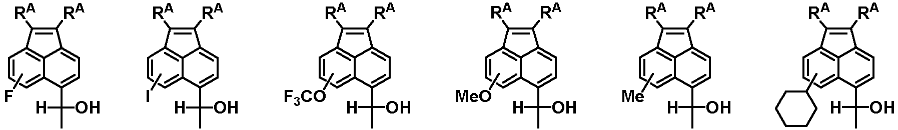

The repeat units A4 may be of one type or a combination of plural types.

For the purpose of improving etching resistance, the polymers A and A' may comprise repeat units of at least one type selected from repeat units having the formula (A5) (hereinafter, referred to as repeat units A5), repeat units having the formula (A6) (hereinafter, referred to as repeat units A6), and repeat units having the formula (A7) (hereinafter, referred to as repeat units A7).

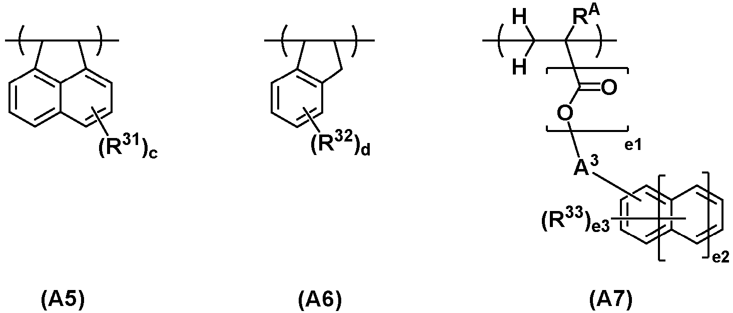

In formulae (A5) and (A6), c and d are each independently 0, 1, 2, 3, or 4.

In formulae (A5) and (A6), R³¹ and R³² are each independently a hydroxy group, a halogen atom, an optionally halogenated C₁-C₈ saturated hydrocarbyl group, an optionally halogenated C₁-C₈ saturated hydrocarbyloxy group, or an optionally halogenated C₂-C₈ saturated hydrocarbylcarbonyloxy group. The saturated hydrocarbyl group, the saturated hydrocarbyloxy group, and the saturated hydrocarbylcarbonyloxy group may be straight, branched, or cyclic. When c is 2 or more, each R³¹ may be identical or different. When d is 2 or more, each R³² may be identical or different.

In formula (A7), e1 is 0 or 1, e2 is 0, 1, or 2, when e2 is 0, e2 represents a benzene structure, when e2 is 1, e2 represents a naphthalene structure, and when e2 is 2, e2 represents an anthracene structure, e3 is 0, 1, 2, 3, 4, or 5, and when e2 is 0, preferably, e3 is 0, 1, 2, or 3, and when e2 is 1 or 2, preferably, e3 is 0, 1, 2, 3, or 4.

In formula (A7), R^{A} is a hydrogen atom, a fluorine atom, a methyl group, or a trifluoromethyl group.

In formula (A7), R³³ is a C₁-C₂₀ saturated hydrocarbyl group, a C₁-C₂₀ saturated hydrocarbyloxy group, a C₂-C₂₀ saturated hydrocarbylcarbonyloxy group, a C₂-C₂₀ saturated hydrocarbyloxyhydrocarbyl group, a C₂-C₂₀ saturated hydrocarbylthiohydrocarbyl group, a halogen atom, a nitro group, a cyano group, a C₁-C₂₀ saturated hydrocarbylsulfinyl group, or a C₁-C₂₀ saturated hydrocarbylsulfonyl group. The saturated hydrocarbyl group, the saturated hydrocarbyloxy group, the saturated hydrocarbylcarbonyloxy group, the saturated hydrocarbyloxyhydrocarbyl group, the saturated hydrocarbylthiohydrocarbyl group, the saturated hydrocarbylsulfinyl group, and the saturated hydrocarbylsulfonyl group may be straight, branched, or cyclic. When e3 is 2 or more, each R³³ may be identical or different.

R³³ is preferably halogen atoms such as a chlorine atom, a bromine atom, and an iodine atom; saturated hydrocarbyl groups such as a methyl group, an ethyl group, a propyl group, a butyl group, a pentyl group, a hexyl group, a cyclopentyl group, a cyclohexyl group, and structural isomers thereof; and saturated hydrocarbyloxy groups such as a methoxy group, an ethoxy group, a propoxy group, a butoxy group, a pentyloxy group, a hexyloxy group, a cyclopentyloxy group, a cyclohexyloxy group, and structural isomers of their hydrocarbon moiety. Inter alia, methoxy groups and ethoxy groups are most useful.

The saturated hydrocarbylcarbonyloxy group may be readily introduced into a polymer even after polymerization by a chemical modification method, and is advantageously utilized for fine adjustment of the solubility of the base polymer in alkaline developer. Examples of the saturated hydrocarbylcarbonyloxy group include a methylcarbonyloxy group, an ethylcarbonyloxy group, a propylcarbonyloxy group, a butylcarbonyloxy group, a pentylcarbonyloxy group, a hexylcarbonyloxy group, a cyclopentylcarbonyloxy group, a cyclohexylcarbonyloxy group, a benzoyloxy group, and structural isomers of their hydrocarbon moiety. As long as the carbon count is 20 or less, an appropriate effect of controlling or adjusting (typically reducing) the solubility of the base polymer in alkaline developer can be obtained, and the generation of scum (development defects) can be suppressed.

Of the foregoing preferred substituents, examples of the substituents which are particularly easily prepared as monomers and are usefully used include a chlorine atom, a bromine atom, an iodine atom, a methyl group, an ethyl group, and a methoxy group.

In formula (A7), A³ is a single bond or a C₁-C₁₀ saturated hydrocarbylene group in which some -CH₂- may be replaced by -O-. The saturated hydrocarbylene group may be straight, branched, or cyclic, and examples thereof include C₁-C₁₀ alkanediyl groups such as a methylene group, an ethane-1,2-diyl group, a propane-1,3-diyl group, a butane-1,4-diyl group, a pentane-1,5-diyl group, a hexane-1,6-diyl group, and structural isomers thereof; C₃-C₁₀ cyclic saturated hydrocarbylene groups such as a cyclopropanediyl group, a cyclobutanediyl group, a cyclopentanediyl group, and a cyclohexanediyl group; and combinations thereof.

Preferred examples of the repeat units A7 wherein e1 is 0 and A³ is a single bond (meaning that the aromatic ring is directly bonded to the main chain of the polymer), that is, repeat units free of the linker: -C(=O)-O-A³- include units derived from styrene, 4-chlorostyrene, 4-methylstyrene, 4-methoxystyrene, 4-bromostyrene, 4-acetoxystyrene, 2-hydroxypropylstyrene, 2-vinylnaphthalene, and 3-vinylnaphthalene.

Preferred examples of the repeat units A7 wherein e1=1, that is, having a linker: -C(=O)-O-A³- are shown below, but are not limited thereto. Herein R^{A} is as defined above.

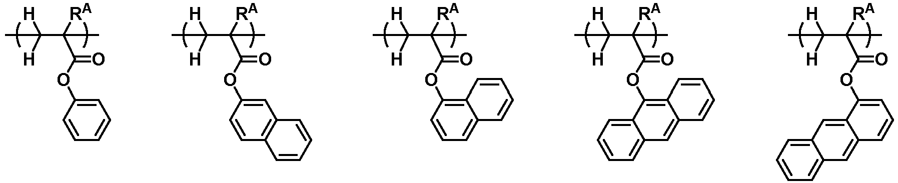

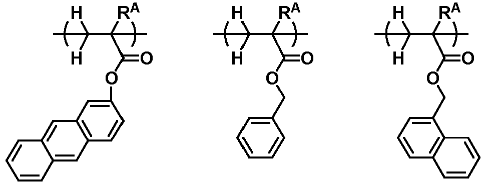

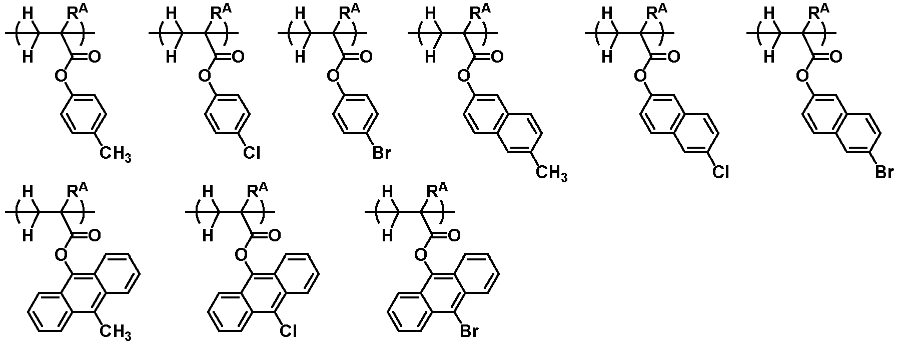

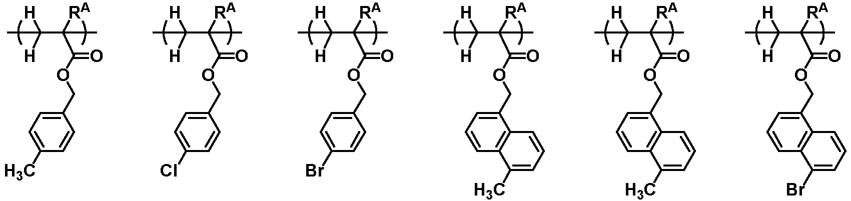

When repeat units of at least one type selected from repeat units A5 to A7 are incorporated, better performance is obtained because not only the aromatic ring possesses etching resistance, but the cyclic structure incorporated into the main chain also exerts the effect of improving resistance to etching and EB irradiation during pattern inspection step.

The repeat units A5 to A7 may be of one type or a combination of plural types.

In the polymers A and A', (meth)acrylate units and other repeat units having an adhesive group such as a lactone structure or a hydroxy group other than a phenolic hydroxy group may be incorporated for fine adjustment of properties of a resist film.

Examples of the (meth)acrylate unit having an adhesive group include repeat units having the formula (A8) (hereinafter, referred to as repeat units A8), repeat units having the formula (A9) (hereinafter, referred to as repeat units A9), and repeat units having the formula (A10) (hereinafter, referred to as repeat units A10). While these units do not exhibit acidity, they can be used as auxiliary units for providing adhesion to substrates or adjusting solubility.

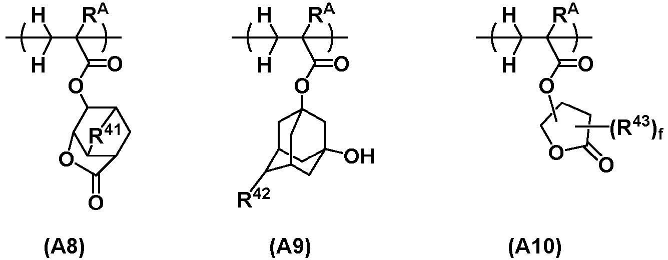

In formulae (A8) to (A10), R^{A} is each independently a hydrogen atom, a fluorine atom, a methyl group, or a trifluoromethyl group, R⁴¹ is -O- or a methylene group, R⁴² is a hydrogen atom or a hydroxy group, R⁴³ is a C₁-C₄ saturated hydrocarbyl group, and f is 0, 1, 2, or 3.

In polymer A, the content of repeat units A1 is preferably 1 to 20 mol%, more preferably 2 to 15 mol%, in order to obtain an effect of promoting negative-working reaction. The content of the repeat units A2 is preferably 30 to 95 mol%, more preferably 50 to 85 mol% for establishing a high contrast between a region which is exposed to high-energy radiation and turns negative and the unexposed region (which does not turn negative) for the purpose of achieving high resolution. The content of the repeat units A5 to A7 is preferably 0 to 30 mol%, more preferably 3 to 20 mol%, in order to obtain an effect of improving etching resistance. The other repeat units may be incorporated in a range of 0 to 30 mol%, and preferably 0 to 20 mol%.

In polymer A', the content of repeat units A1 is preferably 1 to 20 mol%, more preferably 2 to 15 mol%, in order to obtain an effect of promoting negative-working reaction. The content of the repeat units A2 is preferably 25 to 94.5 mol%, more preferably 36 to 85 mol%. The content of the repeat units A3 and A4 is preferably 5 to 70 mol%, more preferably 10 to 60 mol%. The content of the repeat units A5 to A7 is preferably 0 to 30 mol%, more preferably 3 to 20 mol%. The total content of the repeat units A2 to A7 is preferably 60 to 99.5 mol%. The content of the repeat units A1 is preferably 0.5 to 20 mol%, more preferably 1 to 10 mol%. The other repeat units may be incorporated in a range of 0 to 30 mol%, and preferably 0 to 20 mol%.

The polymer can be synthesized by combining suitable monomers optionally protected with a protective group, copolymerizing them in the standard way, and effecting deprotection reaction, if necessary. The copolymerization reaction is preferably radical polymerization or anionic polymerization though not limited thereto. For the polymerization reaction, reference can be made to WO 2006/121096, JP-A 2008-102383, JP-A 2008-304590, and JP-A 2004-115630.

The polymer preferably has a weight average molecular weight (Mw) of 1,000 to 50,000, more preferably 2,000 to 20,000. A Mw of 1,000 or more eliminates the risk that pattern features are rounded at their top, inviting degradations of resolution and LER. A Mw of 50,000 or less eliminates the risk that LER is increased when a pattern with a line width of 100 nm or less is formed. As used herein, Mw is measured by gel permeation chromatography (GPC) versus polystyrene standards using tetrahydrofuran (THF) or dimethylformamide (DMF) as a solvent.

The polymer preferably has a narrow molecular weight distribution or dispersity (Mw/Mn) of 1.0 to 2.0, more preferably 1.0 to 1.8. A polymer with such a narrow dispersity eliminates the risk that foreign matter is left on the pattern after development and the pattern profile is aggravated.

### Chemically amplified negative resist composition

The chemically amplified negative resist composition of the present invention comprises a base polymer containing the above-described polymer as the component (A). The polymer is decomposed by the action of the acid, and the solubility in alkaline developer is reduced.

The repeat units A2 to A7 preferably account for 60 mol% or more, more preferably 70 mol% or more, and still more preferably 80 mol% or more of the overall repeat units constituting the polymer. As a result, characteristics required for the chemically amplified negative resist composition of the present invention can be reliably obtained.

When the base polymer contains a polymer A', the polymer A' preferably comprises repeat units having formula (A1), repeat units having the formula (A2-1), and repeat units having the formula (A3-1), (A3-2), or (A4-1).

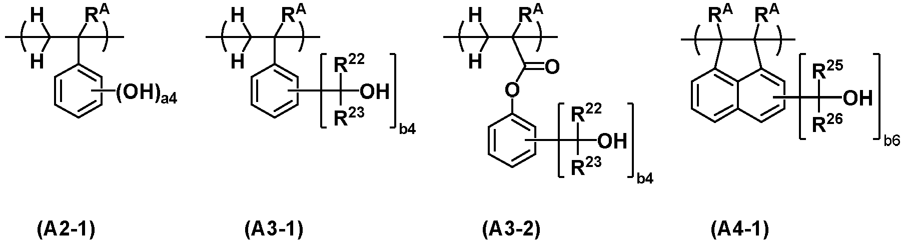

Herein a4, b4, b6, R^{A}, R²², R²³, R²⁵, and R²⁶ are as defined above.

When the polymer A' is used as the base polymer (A), the polymer A' and the polymer comprising repeat units A2 and repeat units A3 or A4, but not repeat units A1 (hereinafter, referred to as polymer A") can be used in combination. The content of the repeat units A2 in polymer A" is preferably 25 to 95 mol%, more preferably 40 to 85 mol%. The content of the repeat units A5 to A7 is preferably 0 to 30 mol%, more preferably 3 to 20 mol%. The content of the repeat units A3 and the repeat units A4 is preferably 5 to 70 mol%, more preferably 10 to 60 mol%. The other repeat units may be incorporated in a range of 0 to 30 mol%, and preferably 0 to 20 mol%. When the polymer A' and the polymer A" are used in combination, the content of the polymer A" in the chemically amplified negative resist composition of the present invention is preferably 2 to 5,000 parts by weight, and more preferably 10 to 1,000 parts by weight per 100 parts by weight of the polymer comprising the repeat units A1.

When the chemically amplified negative resist composition is used in the fabrication of masks, a coating film thickness in the advanced generation is 150 nm or less, preferably 100 nm or less. Since an intense development process is often employed to minimize defects resulting from resist residues, the base polymer constituting the chemically amplified negative resist composition should preferably have a dissolution rate in alkaline developer (typically 2.38 wt% tetramethylammonium hydroxide (TMAH) aqueous solution) of 80 nm/sec or less, more preferably 50 nm/sec or less in order to form a small size pattern. When the chemically amplified negative resist composition is used in the EUV lithography process for fabricating an LSI chip from a wafer, for example, the coating film often has a thickness of 100 nm or less, in view of the necessity of patterning narrow lines of 50 nm or less. In consideration of the risk that the pattern of such thin film can be degraded by development, the polymer preferably has a dissolution rate of 80 nm/sec or less, more preferably 50 nm/sec or less.

The base polymer (A) may contain two or more polymers having different composition ratios, Mw, and Mw/Mn.

### (B) Crosslinker

When the base polymer (A) does not comprise the repeat units A3 and A4, the chemically amplified negative resist composition of the present invention preferably comprises a crosslinker as the component (B). On the other hand, when the base polymer (A) comprises the repeat units A3 and A4, the base polymer (A) may or may not comprise a crosslinker.

Specific examples of the crosslinker which can be used in the present invention include epoxy compounds, melamine compounds, guanamine compounds, glycoluril compounds, and urea compounds substituted with at least one group selected from among a methylol group, an alkoxymethyl group, and an acyloxymethyl group, isocyanate compounds, azide compounds, and compounds having a double bond such as an alkenyloxy group. These compounds may be used as an additive or introduced into a polymer side chain as a pendant. Hydroxy-containing compounds may also be used as the crosslinker.

Examples of the epoxy compound include tris(2,3-epoxypropyl) isocyanurate, trimethylolmethanetriglycidyl ether, trimethylolpropane triglycidyl ether, and triethylolethanetriglycidyl ether.

Examples of the melamine compound include hexamethylol melamine, hexamethoxymethyl melamine, hexamethylol melamine compounds having 1 to 6 methylol groups methoxymethylated and mixtures thereof, hexamethoxyethyl melamine, hexaacyloxymethyl melamine, and hexamethylol melamine compounds having 1 to 6 methylol groups acyloxymethylated and mixtures thereof.

Examples of the guanamine compound include tetramethylol guanamine, tetramethoxymethyl guanamine, tetramethylol guanamine compounds having 1 to 4 methylol groups methoxymethylated and mixtures thereof, tetramethoxyethyl guanamine, tetraacyloxyguanamine, and tetramethylol guanamine compounds having 1 to 4 methylol groups acyloxymethylated and mixtures thereof.

Examples of the glycoluril compound include tetramethylol glycoluril, tetramethoxyglycoluril, tetramethoxymethyl glycoluril, tetramethylol glycoluril compounds having 1 to 4 methylol groups methoxymethylated and mixtures thereof, and tetramethylol glycoluril compounds having 1 to 4 methylol groups acyloxymethylated and mixtures thereof.

Examples of the urea compound include tetramethylol urea, tetramethoxymethyl urea, tetramethylol urea compounds having 1 to 4 methylol groups methoxymethylated and mixtures thereof, and tetramethoxyethyl urea.

Examples of the isocyanate compound include tolylene diisocyanate, diphenylmethane diisocyanate, hexamethylene diisocyanate and cyclohexane diisocyanate.

Examples of the azide compound include 1,1'-biphenyl-4,4'-bisazide, 4,4'-methylidenebisazide, and 4,4'-oxybisazide.

Examples of the alkenyloxy-containing compound include ethylene glycol divinyl ether, triethylene glycol divinyl ether, 1,2-propanediol divinyl ether, 1,4-butanediol divinyl ether, tetramethylene glycol divinyl ether, neopentyl glycol divinyl ether, trimethylol propane trivinyl ether, hexanediol divinyl ether, 1,4-cyclohexanediol divinyl ether, pentaerythritol trivinyl ether, pentaerythritol tetravinyl ether, sorbitol tetravinyl ether, sorbitol pentavinyl ether, and trimethylol propane trivinyl ether.

The content of the crosslinker (B) in the chemically amplified negative resist composition of the present invention is preferably 0.1 to 50 parts by weight, more preferably 1 to 30 parts by weight per 80 parts by weight of the base polymer (A). As long as the amount of the crosslinker is in the range, the risk of resolution being reduced by forming bridges between pattern features may be mitigated. The crosslinkers (B) may be of one type or a combination of plural types.

### (C) Fluorinated polymer

The chemically amplified negative resist composition of the present invention may comprise a fluorinated polymer which comprises, as the component (C), repeat units of at least one type selected from repeat units having the formula (C1) (hereinafter, referred to as repeat units C1), repeat units having the formula (C2) (hereinafter, referred to as repeat units C2), repeat units having the formula (C3) (hereinafter, referred to as repeat units C3), and repeat units having the formula (C4) (hereinafter, referred to as repeat units C4), and may further comprise repeat units of at least one type selected from repeat units having the formula (C5) (hereinafter, referred to as repeat units C5) and repeat units of at least one type selected from repeat units having the formula (C6) (hereinafter, referred to as repeat units C6), for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. Since the fluorinated polymer also has the function of a surfactant, the fluorinated polymer can prevent the insoluble matter that may be generated during the development process from being reattached to the substrate, so that the fluorinated polymer also exhibits an effect on development defects.

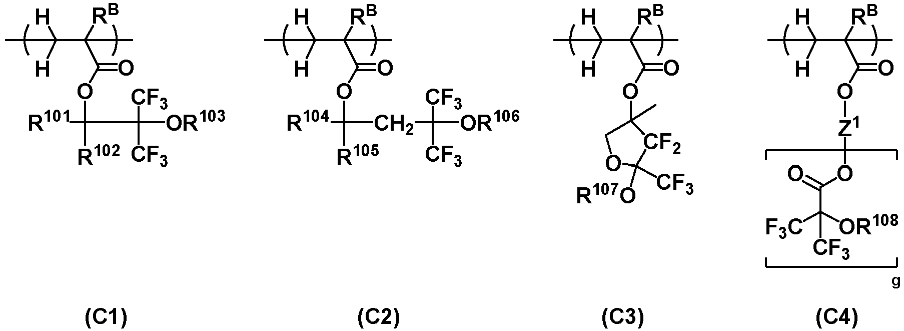

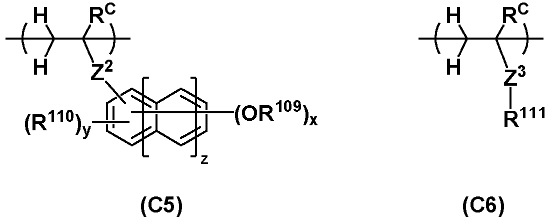

In formulae (C1) to (C6), x is 1, 2, or 3, y is an integer satisfying 0 ≤ y ≤ 5+2z-x, z is 0 or 1, g is 1, 2, or 3, R^{B} is each independently a hydrogen atom, a fluorine atom, a methyl group, or a trifluoromethyl group, R^{C} is each independently a hydrogen atom or a methyl group, and R¹⁰¹, R¹⁰², R¹⁰⁴, and R¹⁰⁵ are each independently a hydrogen atom or a C₁-C₁₀ saturated hydrocarbyl group, R¹⁰³, R¹⁰⁶, R¹⁰⁷, and R¹⁰⁸ are each independently a hydrogen atom, a C₁-C₁₅ hydrocarbyl group, a C₁-C₁₅ fluorinated hydrocarbyl group, or an acid labile group, and when R¹⁰³, R¹⁰⁶, R¹⁰⁷, and R¹⁰⁸ each are a hydrocarbyl group or a fluorinated hydrocarbyl group, an ether bond or a carbonyl group may intervene in a carbon-carbon bond, R¹⁰⁹ is a hydrogen atom or a C₁-C₅ straight or branched hydrocarbyl group in which a heteroatom-containing group may intervene in a carbon-carbon bond, R¹¹⁰ is a C₁-C₅ straight or branched hydrocarbyl group in which a heteroatom containing group may intervene in a carbon-carbon bond, R¹¹¹ is a C₁-C₂₀ saturated hydrocarbyl group in which at least one hydrogen atom is substituted by a fluorine atom, and in which some -CH₂- may be replaced by an ester bond or an ether bond, Z¹ is a C₁-C₂₀ (g+1)-valent hydrocarbon group or C₁-C₂₀ (g+1)-valent fluorinated hydrocarbon group, Z² is a single bond, *-C(=O)-O-, or *-C(=O)-NH-, * designates a point of attachment to the carbon atom in the backbone, Z³ is a single bond, -O-, *-C(=O)-O-Z³¹-Z³²-, or *-C(=O)-NH-Z³¹-Z³²-, Z³¹ is a single bond or a C₁-C₁₀ saturated hydrocarbylene group, Z³² is a single bond, an ester bond, an ether bond, or a sulfonamide bond, and * designates a point of attachment to the carbon atom in the backbone,

In formulae (C1) and (C2), the C₁-C₁₀ saturated hydrocarbyl groups R¹⁰¹, R¹⁰², R¹⁰⁴, and R¹⁰⁵ may be straight, branched, or cyclic, and specific examples thereof include C₁-C₁₀ alkyl groups such as a methyl group, an ethyl group, an n-propyl group, an isopropyl group, an n-butyl group, an isobutyl group, a sec-butyl group, a tert-butyl group, an n-pentyl group, an n-hexyl group, an n-heptyl group, an n-octyl group, an n-nonyl group, and an n-decyl group; and C₃-C₁₀ cyclic saturated hydrocarbyl groups such as a cyclopropyl group, a cyclobutyl group, a cyclopentyl group, a cyclohexyl group, an adamantyl group, and a norbornyl group. Inter alia, C₁-C₆ saturated hydrocarbyl groups are preferred.

In formulae (C1) to (C4), the C₁-C₁₅ hydrocarbyl groups R¹⁰³, R¹⁰⁶, R¹⁰⁷, and R¹⁰⁸ may be straight, branched, or cyclic, specific examples thereof include a C₁-C₁₅ alkyl group, a C₂-C₁₅ alkenyl group, and a C₂-C₁₅ alkynyl group, and the C₁-C₁₅ alkyl groups are preferred. Examples of the alkyl group include a methyl group, an ethyl group, an n-propyl group, an isopropyl group, an n-butyl group, an isobutyl group, a sec-butyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, and an n-pentadecyl group. The fluorinated hydrocarbyl groups correspond to the foregoing hydrocarbyl groups in which some or all carbon-bonded hydrogen atoms are substituted by fluorine atoms.

In formula (C4), examples of the C₁-C₂₀ (g+1)-valent hydrocarbon group Z¹ include the foregoing C₁-C₂₀ alkyl groups and C₃-C₂₀ cyclic saturated hydrocarbyl groups, with g number of hydrogen atoms being eliminated. Examples of the C₁-C₂₀ (g+1)-valent fluorinated hydrocarbon group Z¹ include the foregoing (g+1)-valent hydrocarbon groups in which at least one hydrogen atom is substituted by a fluorine atom.

Specific examples of the repeat units C1 to C4 are shown below, but are not limited thereto. Herein R^{B} is as defined above.

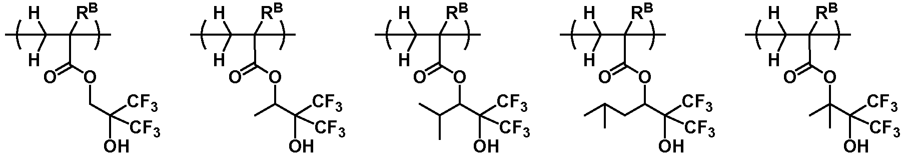

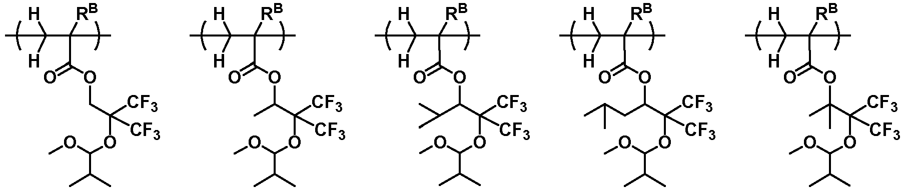

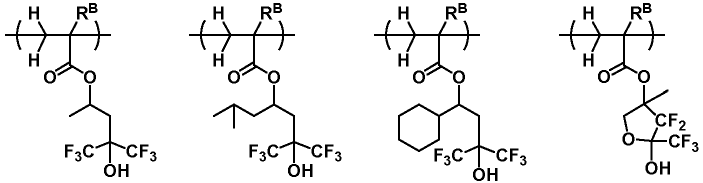

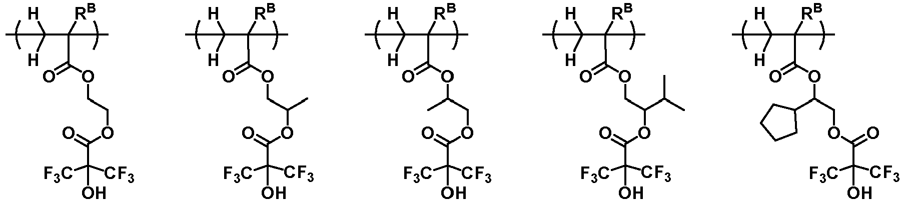

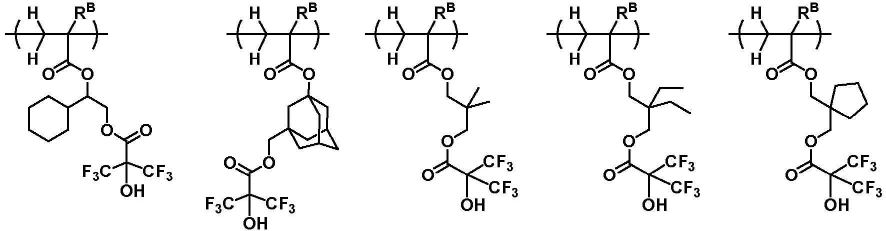

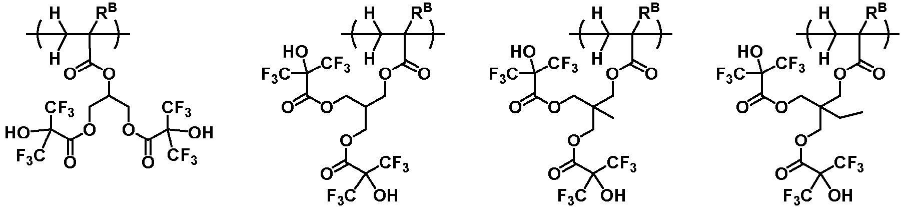

In formula (C5), examples of the C₁-C₅ hydrocarbyl groups R¹⁰⁹ and R¹¹⁰ include an alkyl group, an alkenyl group, and an alkynyl group, and the alkyl groups are preferred. Examples of the alkyl group include a methyl group, an ethyl group, an n-propyl group, an isopropyl group, an n-butyl group, an isobutyl group, a sec-butyl group, and an n-pentyl group. In these groups, a group containing a heteroatom such as an oxygen atom, a sulfur atom, or a nitrogen atom may intervene in a carbon-carbon bond of the hydrocarbyl group.

In formula (C5), -OR¹⁰⁹ is preferably a hydrophilic group. In this case, R¹⁰⁹ is preferably a hydrogen atom or a C₁-C₅ alkyl group in which an oxygen atom intervenes in a carbon-carbon bond.

In formula (C5), Z² is preferably *-C(=O)-O- or *-C(=O)-NH-. Also, R^{D} is preferably a methyl group. The inclusion of the carbonyl group in Z² enhances the ability to trap the acid originating from the anti-charging film. A polymer wherein R^{D} is a methyl group is a robust polymer having a high glass transition temperature which is effective for suppressing acid diffusion. As a result, the resist film is improved in stability with time, and neither resolution nor pattern profile is degraded.

Examples of the repeat unit C5 are shown below, but are not limited thereto. Herein R^{C} is as defined above.

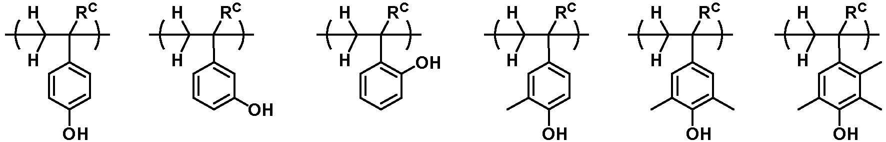

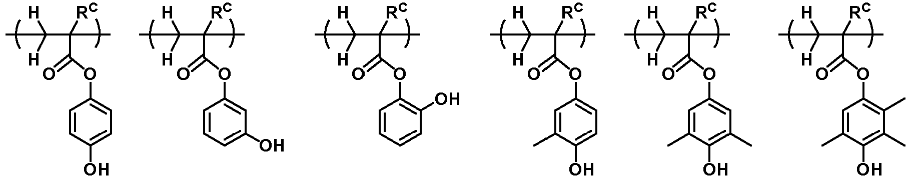

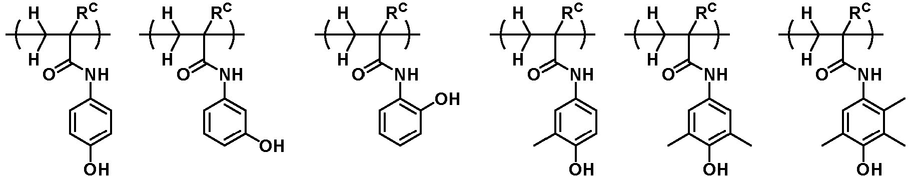

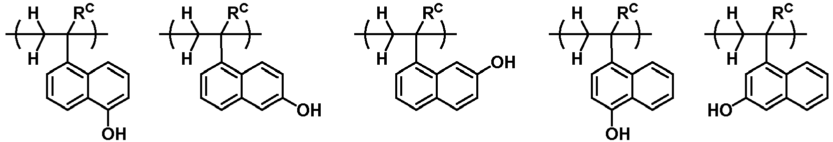

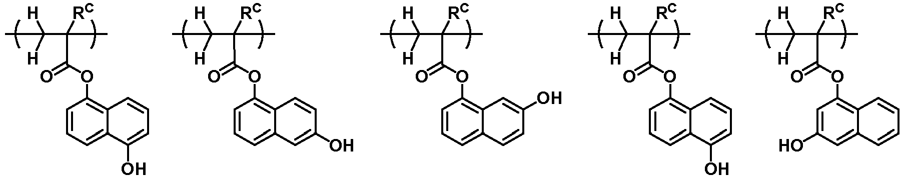

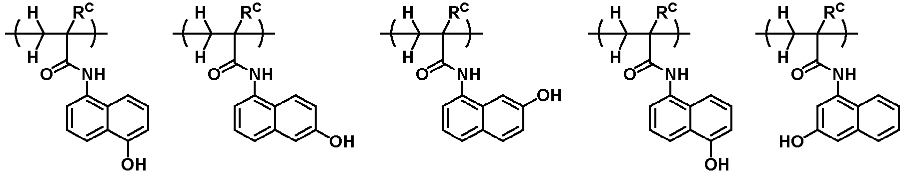

In formula (C6), the C₁-C₁₀ saturated hydrocarbylene group Z³ may be straight, branched, or cyclic, and specific examples thereof include a methanediyl group, an ethane-1,1-diyl group, an ethane-1,2-diyl group, a propane-1,1-diyl group, a propane-1,2-diyl group, a propane-1,3-diyl group, a propane-2,2-diyl group, a butane-1,1-diyl group, a butane-1,2-diyl group, a butane-1,3-diyl group, a butane-2,3-diyl group, a butane-1,4-diyl group, and a 1,1-dimethylethane-1,2-diyl group.

In formula (C6), the C₁-C₂₀ saturated hydrocarbyl group having at least one hydrogen atom substituted by a fluorine atom, represented by R¹¹¹, may be straight, branched, or cyclic, and specific examples thereof include C₁-C₂₀ alkyl groups and C₃-C₂₀ cyclic saturated hydrocarbyl groups in which at least one hydrogen atom is substituted by a fluorine atom.

Examples of the repeat unit C6 are shown below, but are not limited thereto. Herein R^{C} is as defined above.

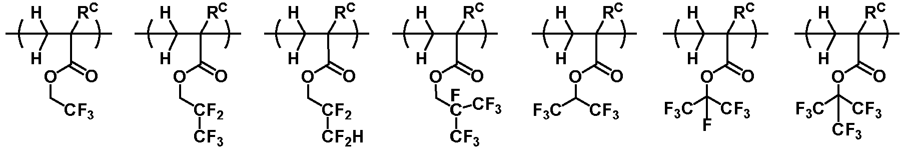

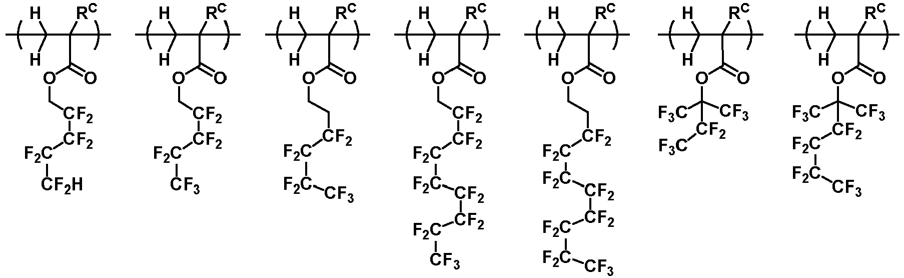

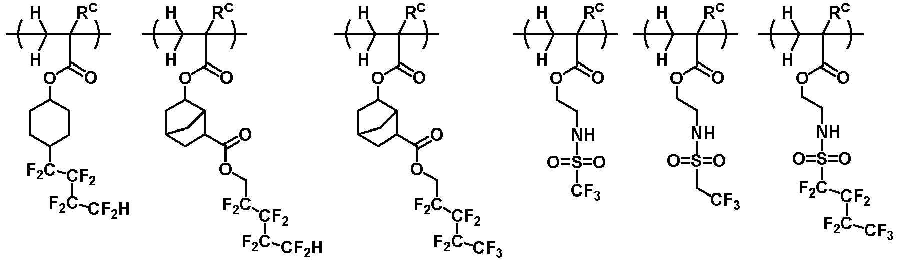

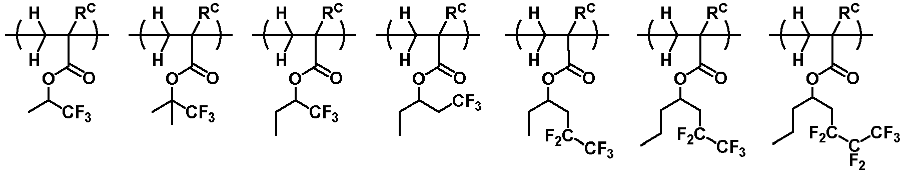

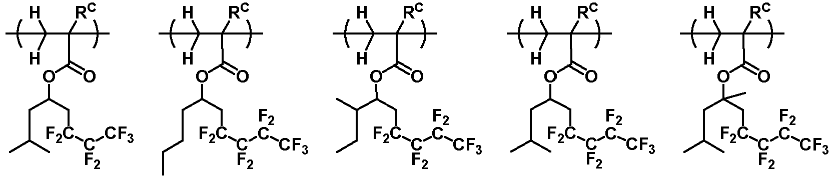

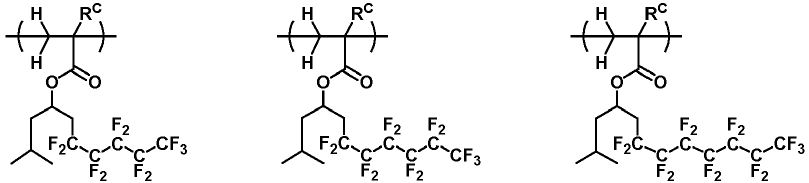

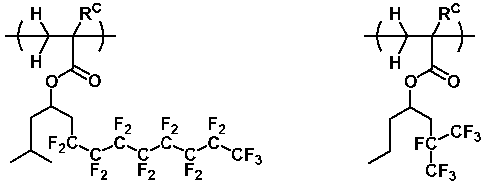

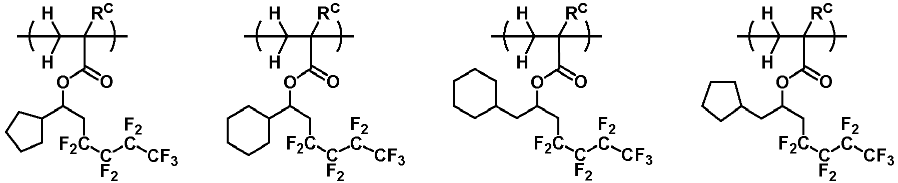

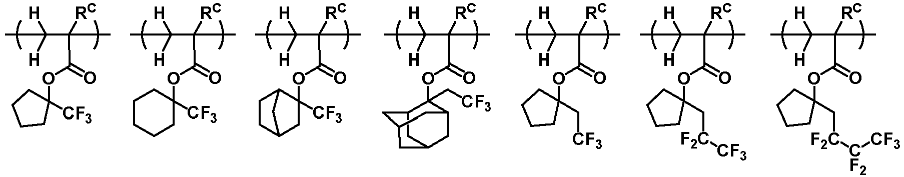

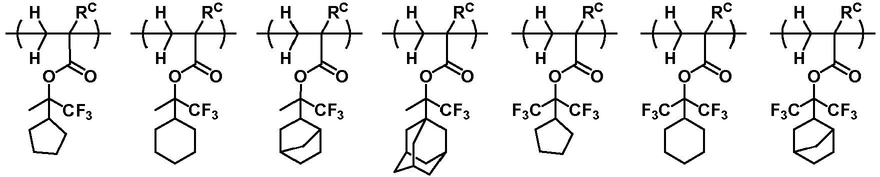

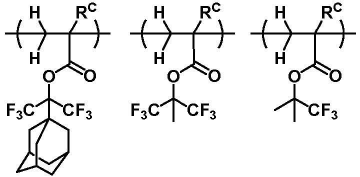

The content of the repeat units C1 to C4 is preferably 15 to 95 mol%, more preferably 20 to 85 mol% based on the overall repeat units of the fluorinated polymer. The content of repeat units C5 and/or C6 is preferably 5 to 85 mol%, more preferably 15 to 80 mol% based on the overall repeat units of the fluorinated polymer. Each of repeat units C1 to C6 may be of one type or a combination of plural types.

The fluorinated polymer may comprise additional repeat units as well as the repeat units described above. Examples of the additional repeat units include those described in paragraphs [0046]-[0078] of JP-A 2014-177407. When the fluorinated polymer comprises additional repeat units, the content thereof is preferably 50 mol% or less based on the overall repeat units of the fluorinated polymer.

The fluorinated polymer can be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

A Mw of the fluorinated polymer is preferably 2,000 to 50,000, more preferably 3,000 to 20,000. A fluorinated polymer with a Mw of less than 2,000 helps acid diffusion, degrading resolution and detracting from age stability. A polymer with too high Mw has a reduced solubility in solvent, with a risk of leaving coating defects. The fluorinated polymer preferably has a dispersity (Mw/Mn) of 1.0 to 2.2, more preferably 1.0 to 1.7.

In the chemically amplified negative resist composition of the present invention, the content of the fluorinated polymer (C) is preferably 0.01 to 30 parts by weight, more preferably 0.1 to 20 parts by weight, even more preferably 0.5 to 10 parts by weight per 80 parts by weight of the base polymer (A). The fluorinated polymer (C) may be of one type or a combination of plural types.

### (D) Quencher

The chemically amplified negative resist composition of the present invention preferably comprises a quencher as the component (D). As used herein, the quencher refers to a compound capable of trapping the acid generated by the photoacid generator in the chemically amplified resist composition to prevent the acid from diffusing to the unexposed region for thereby forming the desired pattern.

Examples of the quencher include conventional basic compounds. Examples of the conventional basic compound include primary, secondary, and tertiary aliphatic amines, mixed amines, aromatic amines, heterocyclic amines, nitrogen-containing compounds with a carboxy group, nitrogen-containing compounds with a sulfonyl group, nitrogen-containing compounds with a hydroxy group, nitrogen-containing compounds with a hydroxyphenyl group, alcoholic nitrogen-containing compounds, amide derivatives, imide derivatives, and carbamate derivatives. The primary, secondary, and tertiary amine compounds, specifically amine compounds having a hydroxy group, an ether bond, an ester bond, a lactone ring, a cyano group, or a sulfonate ester bond as described in paragraphs [0146]-[0164] of JP-A 2008-111103, and compounds having a carbamate group as described in JP 3790649, are preferred. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound is effective for further suppressing the diffusion rate of acid in the resist film or correcting the pattern profile.

Onium salts such as sulfonium, iodonium, and ammonium salts of carboxylic acids which are not fluorinated at α-position as described in JP-A 2008-158339 may also be used as the quencher. While an α-fluorinated sulfonic acid, imide acid, and methide acid are necessary to deprotect the acid labile group, an α-non-fluorinated carboxylic acid is released by salt exchange with an α-non-fluorinated onium salt. An α-non-fluorinated carboxylic acid functions as a quencher because it does not induce substantial deprotection reaction.

Examples of the onium salt of α-non-fluorinated carboxylic acid include compounds having the formula (D1).

R²⁰¹-CO₂⁻ Mq_{A}⁺ (D1)

In formula (D1), R²⁰¹ is a hydrogen atom or a C₁-C₄₀ hydrocarbyl group which may contain a heteroatom, exclusive of the hydrocarbyl group in which the hydrogen atom bonded to the carbon atom at α-position of the carboxy group is substituted by a fluorine atom or a fluoroalkyl group.

The hydrocarbyl group R²⁰¹ may be saturated or unsaturated, and may be straight, branched, or cyclic. Specific examples thereof include C₁-C₄₀ alkyl groups such as a methyl group, an ethyl group, an n-propyl group, an isopropyl group, an n-butyl group, a sec-butyl group, a tert-butyl group, an n-pentyl group, a tert-pentyl group, an n-hexyl group, an n-octyl group, a 2-ethylhexyl group, an n-nonyl group, an n-decyl group; C₃-C₄₀ cyclic saturated hydrocarbyl groups such as a cyclopentyl group, a cyclohexyl group, a cyclopentylmethyl group, a cyclopentylethyl group, a cyclopentylbutyl group, a cyclohexylmethyl group, a cyclohexylethyl group, a cyclohexylbutyl group, a norbornyl group, a tricyclo[5.2.1.0^{2,6}]decyl group, an adamantyl group, and an adamantylmethyl group; C₂-C₄₀ alkenyl groups such as a vinyl group, an allyl group, a propenyl group, a butenyl group, and a hexenyl group; C₃-C₄₀ cyclic unsaturated aliphatic hydrocarbyl groups such as a cyclohexenyl group; C₆-C₄₀ aryl groups such as a phenyl group, a naphthyl group, alkylphenyl groups (a 2-methylphenyl group, a 3-methylphenyl group, a 4-methylphenyl group, a 4-ethylphenyl group, a 4-tert-butylphenyl group, a 4-n-butylphenyl group, and the like), dialkylphenyl groups (a 2,4-dimethylphenyl group, a 2,4,6-triisopropylphenyl group, and the like), alkylnaphthyl groups (a methylnaphthyl group, an ethylnaphthyl group, and the like), dialkylnaphthyl groups (a dimethylnaphthyl group, a diethylnaphthyl group, and the like); and C₇-C₄₀ aralkyl groups such as a benzyl group, a 1-phenylethyl group, and a 2-phenylethyl group.

In the hydrocarbyl group, some or all of the hydrogen atoms may be substituted by a group containing a heteroatom such as an oxygen atom, a sulfur atom, a nitrogen atom, or a halogen atom, and some -CH₂- may be replaced by a group containing a heteroatom such as an oxygen atom, a sulfur atom, or a nitrogen atom, so that the group may contain a hydroxy group, a cyano group, a carbonyl group, an ether bond, a thioether bond, an ester bond, a sulfonate ester bond, a carbonate bond, a lactone ring, a sultone ring, a carboxylic anhydride (-C(=O)-O-C(=O)-), or a haloalkyl group. Examples of the heteroatom-containing hydrocarbyl groups include heteroaryl groups such as a thienyl group; alkoxyphenyl groups such as a 4-hydroxyphenyl group, a 4-methoxyphenyl group, a 3-methoxyphenyl group, a 2-methoxyphenyl group, a 4-ethoxyphenyl group, a 4-tert-butoxyphenyl group, a 3-tert-butoxyphenyl group; alkoxynaphthyl groups such as a methoxynaphthyl group, an ethoxynaphthyl group, an n-propoxynaphthyl group, and an n-butoxynaphthyl group; dialkoxynaphthyl groups such as a dimethoxynaphthyl group and a diethoxynaphthyl group; and aryloxoalkyl groups, typically 2-aryl-2-oxoethyl groups such as a 2-phenyl-2-oxoethyl group, a 2-(1-naphthyl)-2-oxoethyl group, and a 2-(2-naphthyl)-2-oxoethyl group.

In formula (D1), Mq_{A}⁺ is an onium cation. The onium cation is preferably a sulfonium cation, an iodonium cation, and an ammonium cation, more preferably a sulfonium cation and an iodonium cation. Specific examples of the sulfonium cation are as exemplified above for the sulfonium cation having formula (cation-1). Specific examples of the iodonium cation are as exemplified above for the iodonium cation having formula (cation-2).

Examples of the anion in the onium salt having formula (D1) are shown below, but are not limited thereto.

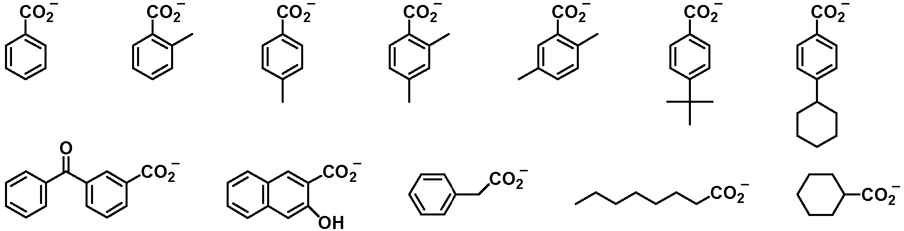

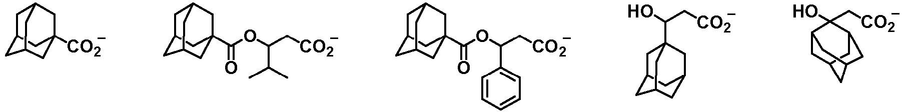

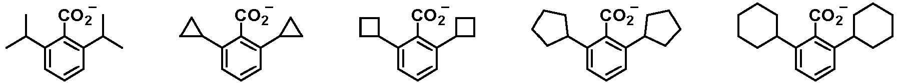

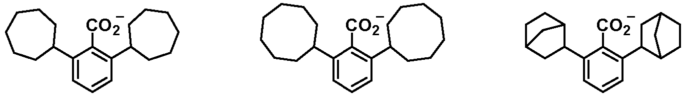

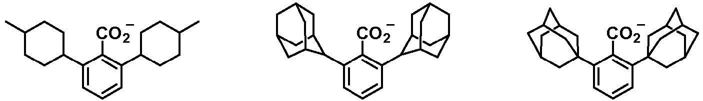

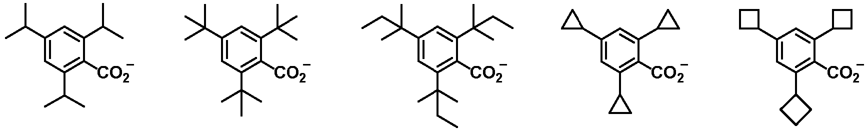

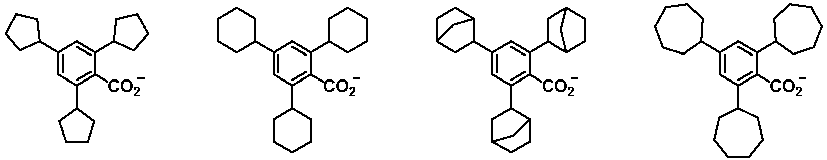

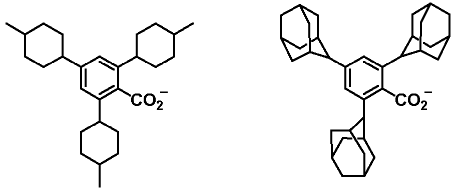

A sulfonium salt of iodized benzene ring-containing carboxylic acid having the formula (D2) is also useful as the quencher.

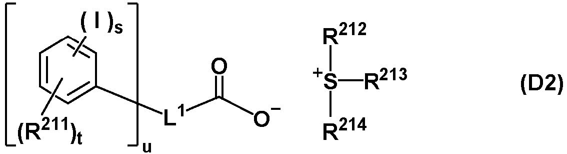

In formula (D2), s is 1, 2, 3, 4, or 5, t is 0, 1, 2, or 3, provided that 1 ≤ s+t ≤ 5, and u is 1, 2, or 3.

In formula (D2), R²¹¹ is a hydroxy group, a fluorine atom, a chlorine atom, a bromine atom, an amino group, a nitro group, a cyano group, or a C₁-C₆ saturated hydrocarbyl group, a C₁-C₆ saturated hydrocarbyloxy group, a C₂-C₆ saturated hydrocarbylcarbonyloxy group, or a C₁-C₄ saturated hydrocarbylsulfonyloxy group, in which some or all hydrogen atoms may be substituted by halogen atoms, or -N(R^{211A})-C(=O)-R^{211B}, or -N(R^{211A})-C(=O)-O-R^{211B}. R^{211A} is a hydrogen atom or a C₁-C₆ saturated hydrocarbyl group. R^{211B} is a C₁-C₆ saturated hydrocarbyl group or a C₂-C₈ unsaturated aliphatic hydrocarbyl group. When t and/or u is 2 or more, each R²¹¹ may be the same or different.

In formula (D2), L¹ is a single bond, or a C₁-C₂₀ (u+1)-valent linking group which may contain at least one moiety selected from an ether bond, a carbonyl group, an ester bond, an amide bond, a sultone ring, a lactam ring, a carbonate bond, a halogen atom, a hydroxy group, and a carboxy group. The saturated hydrocarbyl group, the saturated hydrocarbyloxy group, the saturated hydrocarbylcarbonyloxy group, and the saturated hydrocarbylsulfonyloxy group may be straight, branched, or cyclic.

In formula (D2), R²¹², R²¹³, and R²¹⁴ are each independently a halogen atom, or a C₁-C₂₀ hydrocarbyl group which may contain a heteroatom. The hydrocarbyl group may be saturated or unsaturated, and may be straight, branched, or cyclic. Specific examples thereof include a C₁-C₂₀ alkyl group, a C₂-C₂₀ alkenyl group, a C₆-C₂₀ aryl group, and a C₇-C₂₀ aralkyl group. In the hydrocarbyl group, some or all hydrogen atoms may be substituted by a hydroxy group, a carboxy group, a halogen atom, an oxo group, a cyano group, a nitro group, a sultone ring, a sulfo group, or a sulfonium salt-containing group, or some constituent -CH₂- may be replaced by an ether bond, an ester bond, a carbonyl group, an amide bond, a carbonate bond, or a sulfonate ester bond. R²¹² and R²¹³ may bond together to form a ring with the sulfur atom to which they are attached.

Specific examples of the compound having formula (D2) include compounds described in JP-A 2017-219836. The compound having formula (D2) exerts a sensitizing effect due to remarkable absorption and an acid diffusion-controlling effect.

A nitrogen-containing carboxylic acid salt compound having the formula (D3) is also useful as the quencher.

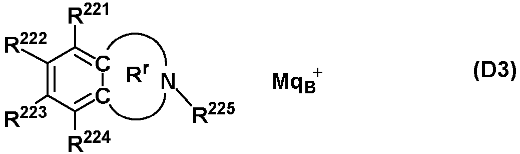

In formula (D3), R²²¹ to R²²⁴ are each independently a hydrogen atom, -L²-CO₂⁻, or a C₁-C₂₀ hydrocarbyl group which may contain a heteroatom. R²²¹ and R²²², R²²² and R²²³, or R²²³ and R²²⁴ may bond together to form a ring with the carbon atom to which they are attached. L² is a single bond or a C₁-C₂₀ hydrocarbylene group which may contain a heteroatom. R²²⁵ is a hydrogen atom or a C₁-C₂₀ hydrocarbyl group which may contain a heteroatom.

In formula (D3), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L²-CO₂⁻ and in which some -CH₂- may be replaced by a sulfur atom, an oxygen atom, or a nitrogen atom. The ring may be alicyclic or aromatic and is preferably a 5- or 6-membered ring. Specific examples of the rings include a pyridine ring, a pyrrole ring, a pyrrolidine ring, a piperidine ring, a pyrazole ring, an imidazoline ring, a pyridazine ring, a pyrimidine ring, a pyrazine ring, an oxazole ring, a thiazole ring, a morpholine ring, a thiazine ring, and a triazole ring.

The carboxylic onium salt having formula (D3) has at least one -L²-CO₂⁻ group. That is, at least one of R²²¹ to R²²⁴ is -L²-CO₂-, and/or at least one of hydrogen atoms bonded to carbon atoms in the ring R^{r} is substituted by -L²-CO₂⁻.

In formula (D3), Mq_{B}⁺ is a sulfonium cation, an iodonium cation, or an ammonium cation, and the sulfonium cation is preferred. Specific examples of the sulfonium cation are as exemplified above for the sulfonium cation having formula (cation-1).

Examples of the anion in the compound having formula (D3) are shown below, but are not limited thereto.

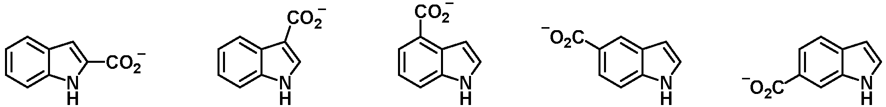

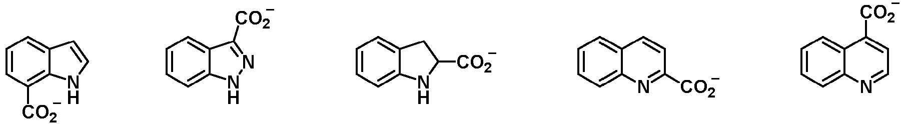

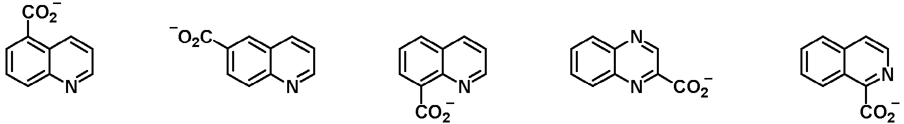

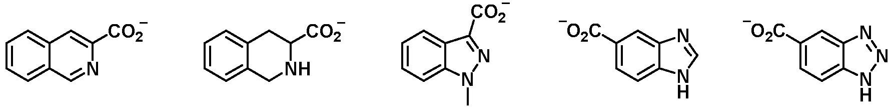

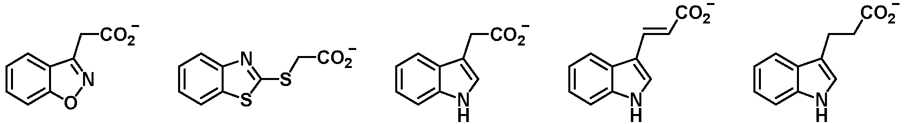

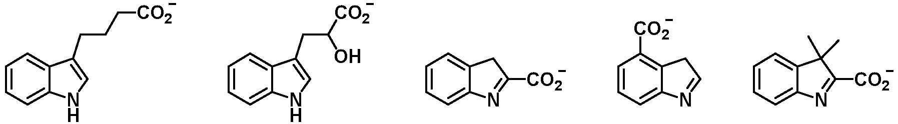

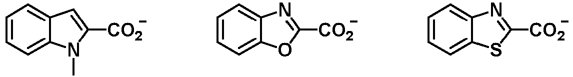

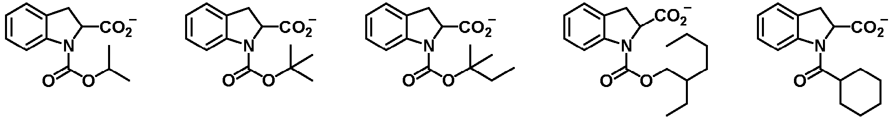

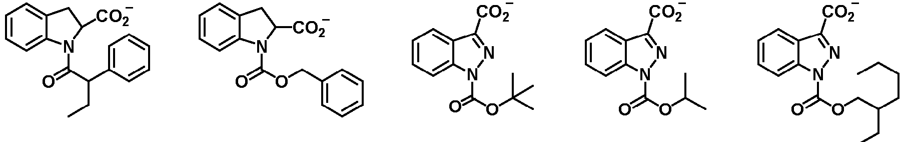

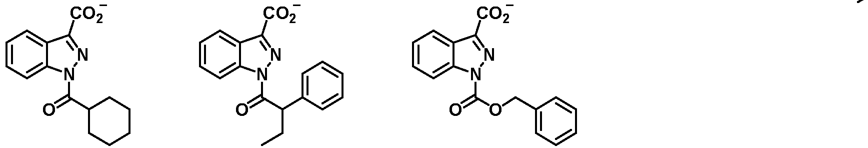

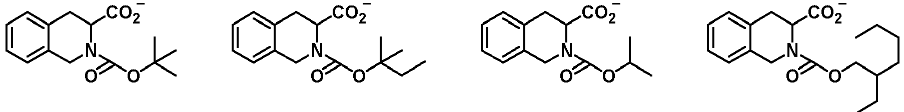

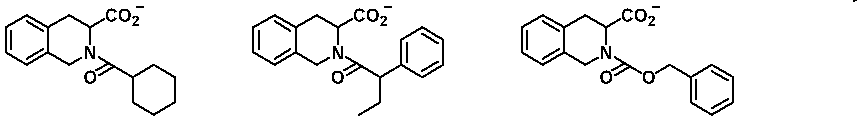

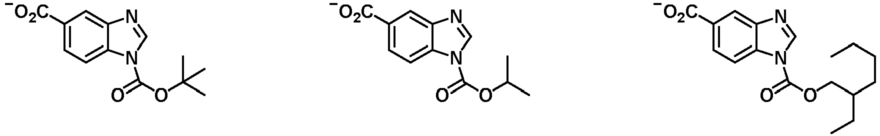

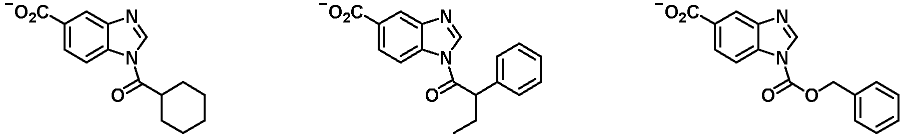

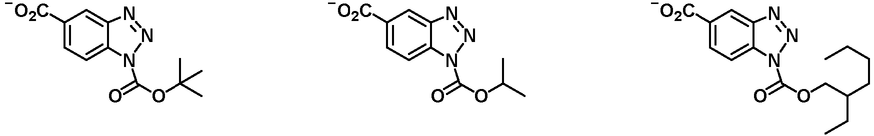

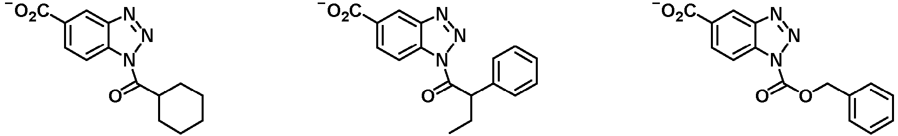

Weak acid betaine compounds are also useful as the quencher. Non-limiting specific examples thereof are shown below, but are not limited thereto.

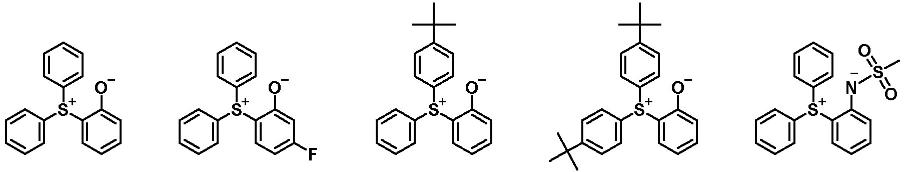

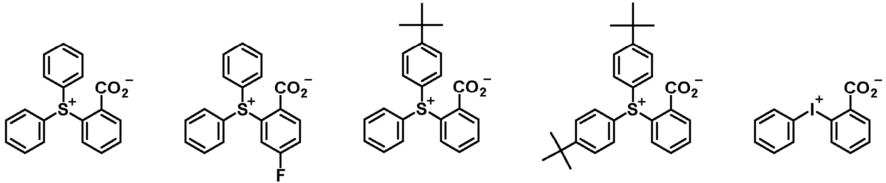

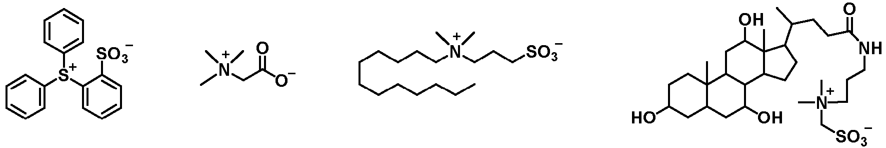

Quenchers of polymer type as described in JP-A 2008-239918 are also useful as the quencher. The polymeric quencher segregates at the surface of the resist film and thus enhances the rectangularity of resist pattern. When a protective film is applied as is often the case in the immersion lithography, the polymeric quencher is also effective for preventing a film thickness loss of resist pattern or rounding of pattern top.

The content of the quencher (D) in the chemically amplified negative resist composition of the present invention is preferably 0 to 50 parts by weight, more preferably 0.1 to 40 parts by weight per 80 parts by weight of the base polymer (A). The quencher (D) may be of one type or a combination of plural types.

In the embodiment wherein the chemically amplified negative resist composition comprises a photoacid generator as the component (F) described below and the quencher as the component (D), the photoacid generator (F) and the quencher (E) are preferably present in a weight ratio (F/E) of less than 6/1, more preferably less than 5/1, even more preferably less than 4/1. As long as the ratio of photoacid generator to the quencher contained in the chemically amplified negative resist composition is in the range, it is possible to fully suppress acid diffusion, leading to improved resolution and dimensional uniformity.

### (E) Organic solvent

The chemically amplified negative resist composition of the present invention may further comprise an organic solvent as the component (E). The organic solvent used herein is not particularly limited as long as the components are soluble therein. Examples of the organic solvent are described in paragraphs [0144]-[0145] of JP-A 2008-111103. Examples of the organic solvent include ketones such as cyclohexanone, cyclopentanone, methyl-2-n-pentyl ketone, and 2-heptanone; alcohols such as 3-methoxybutanol, 3-methyl-3-methoxybutanol, 1-methoxy-2-propanol, 1-ethoxy-2-propanol, and diacetone alcohol; ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate (EL), ethyl pyruvate, butyl acetate, methyl 3-methoxypropionate, ethyl 3-ethoxypropionate, t-butyl acetate, t-butyl propionate, and propylene glycol mono t-butyl ether acetate; and lactones such as γ-butyrolactone (GBL), and mixtures thereof.

Of the above organic solvents, 1-ethoxy-2-propanol, PGMEA, PGME, cyclohexanone, EL, γ-butyrolactone, and mixtures thereof are preferred.

In the chemically amplified negative resist composition of the present invention, the content of the organic solvent (E) is preferably 200 to 10,000 parts by weight, more preferably 400 to 6,000 parts by weight per 80 parts by weight of the base polymer (A). The organic solvent (E) may be used alone or in admixture.

### (F) Photoacid generator

The chemically amplified negative resist composition of the present invention may further comprise a photoacid generator as the component (F). The photoacid generator is not particularly limited as long as it is a compound that generates an acid by irradiation with high-energy radiation. Suitable photoacid generators include a sulfonium salt, an iodonium salt, sulfonyldiazomethane, N-sulfonyloxyimide, an oxime-O-sulfonate acid generator, and the like.

Specific examples of the photoacid generator include nonafluorobutane sulfonate, the partially fluorinated sulfonate described in paragraphs [0247]-[0251] of JP-A 2012-189977, the partially fluorinated sulfonate described in paragraphs [0261]-[0265] of JP-A 2013-101271, the partially fluorinated sulfonate described in paragraphs [0122]-[0142] of JP-A 2008-111103, and the partially fluorinated sulfonate described in paragraphs [0080]-[0081] of JP-A 2010-215608. Among the above specific examples, an arenesulfonate type or alkanesulfonate type photoacid generator is preferred because it generates an acid having an appropriate strength for deprotecting the acid labile group of the repeat unit A3.

As such a photoacid generator, a salt compound containing the following anion is preferred.

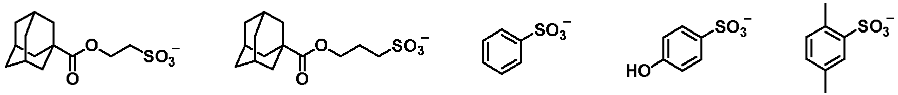

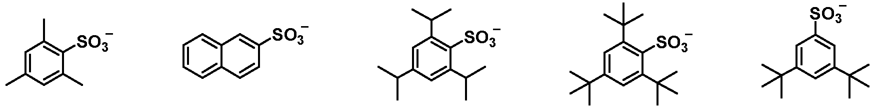

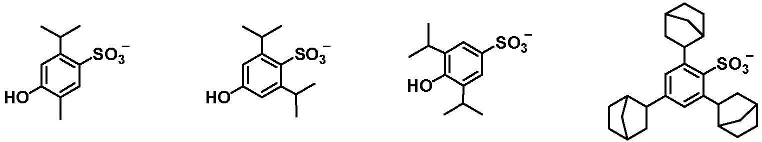

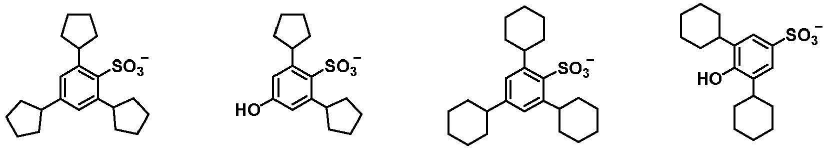

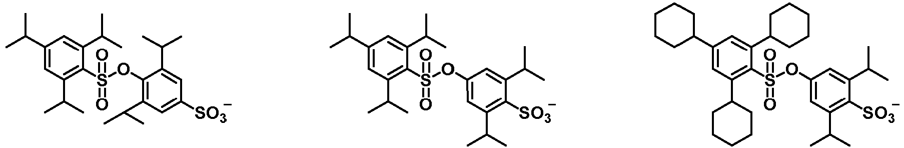

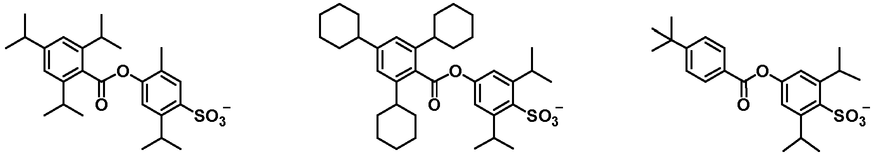

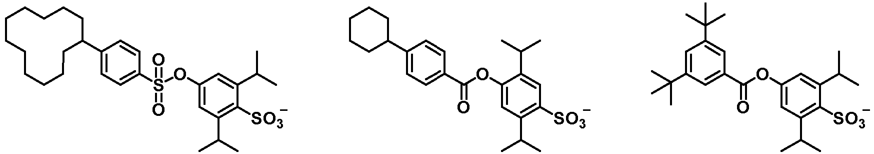

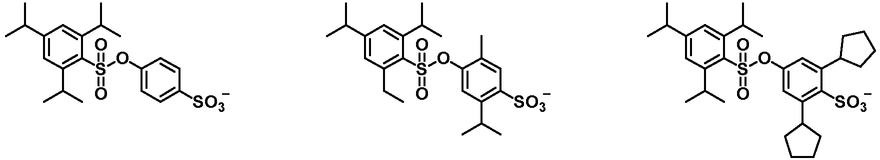

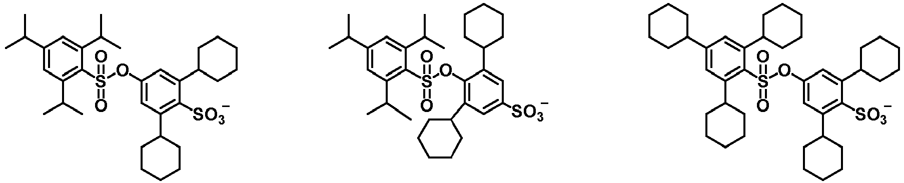

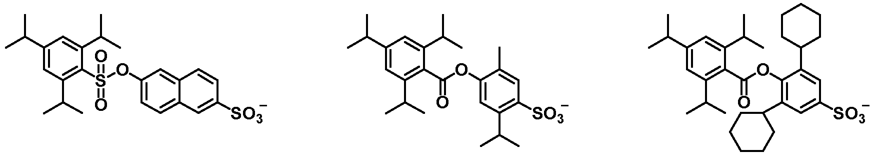

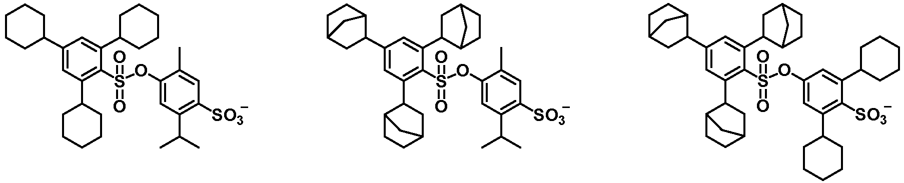

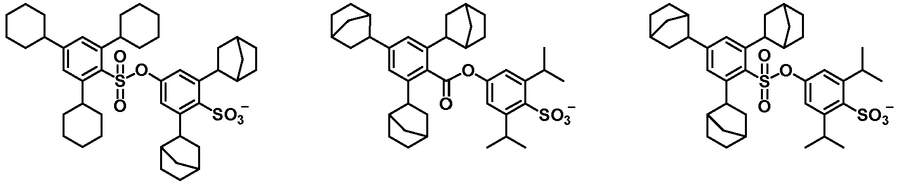

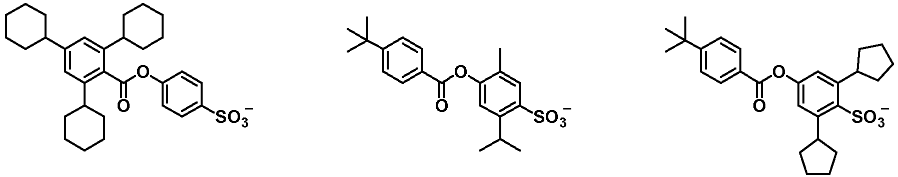

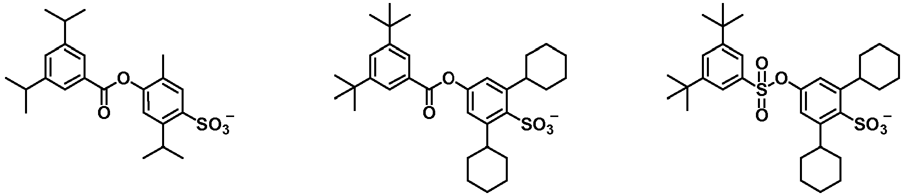

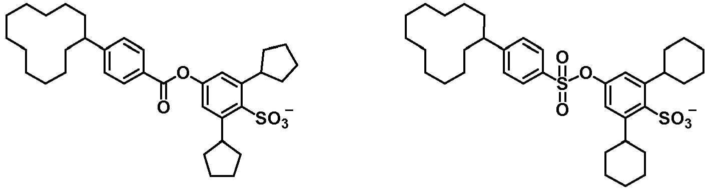

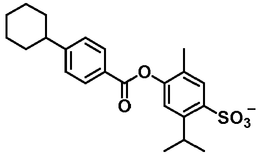

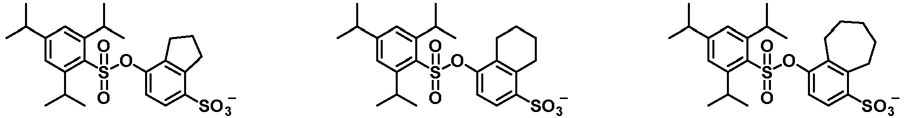

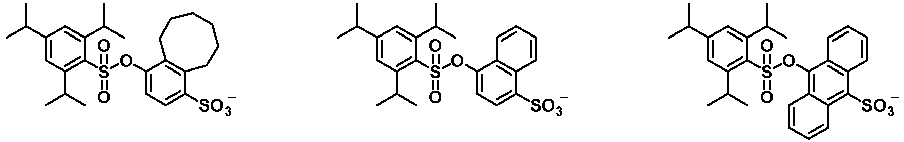

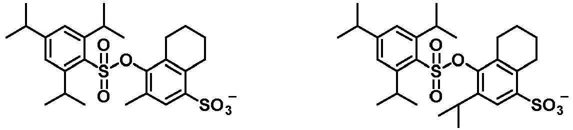

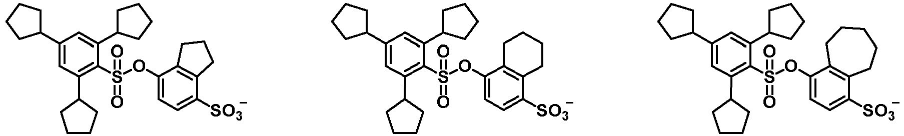

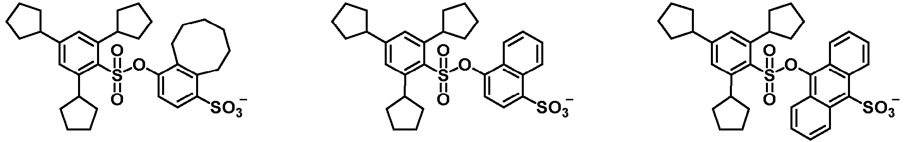

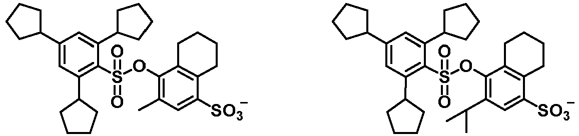

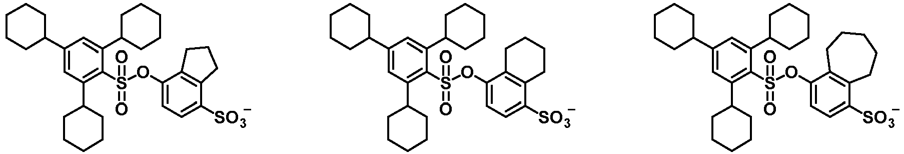

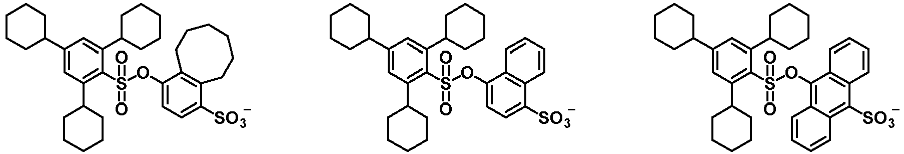

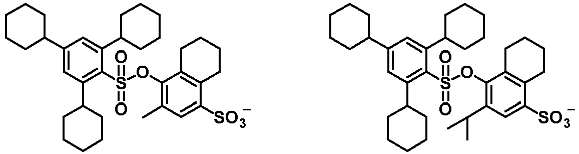

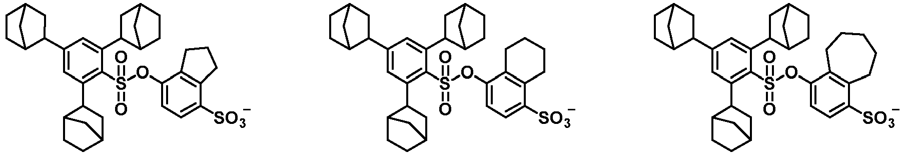

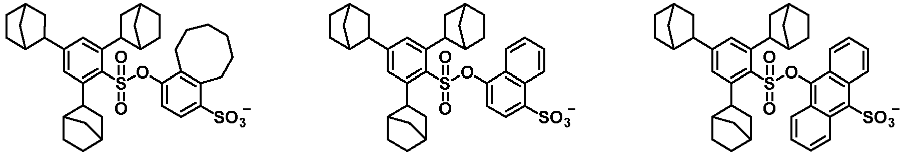

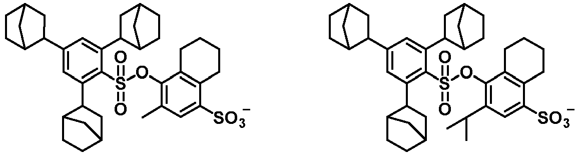

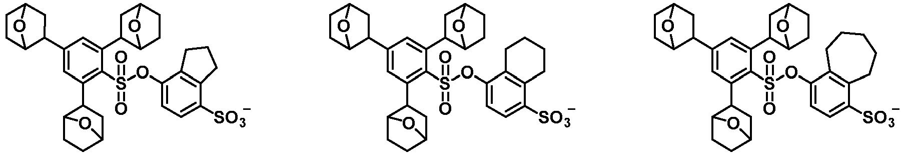

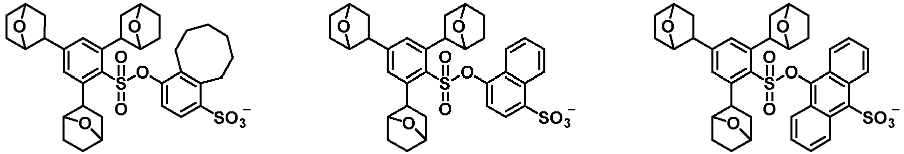

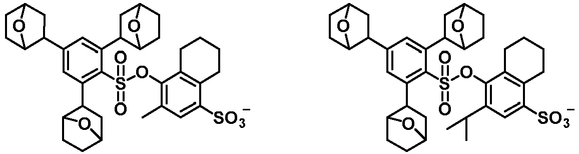

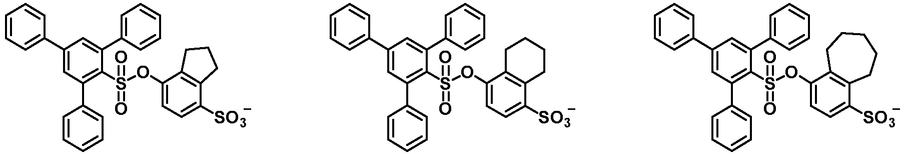

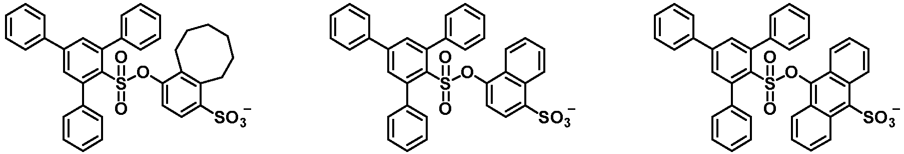

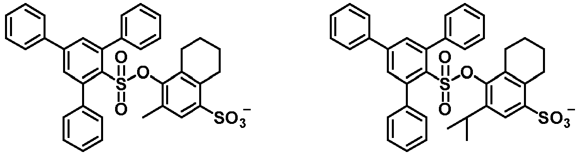

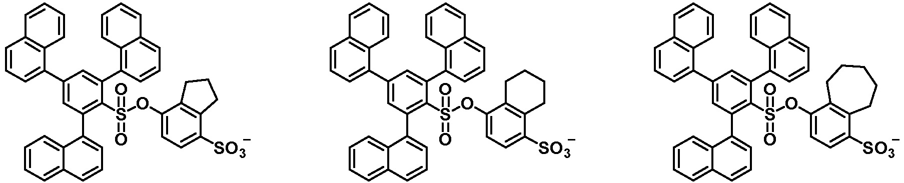

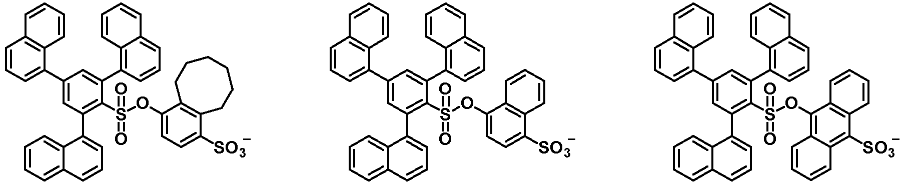

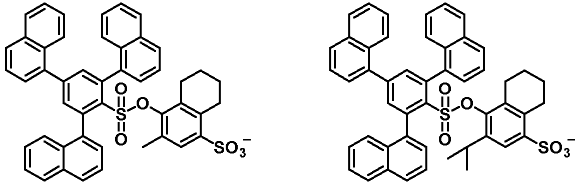

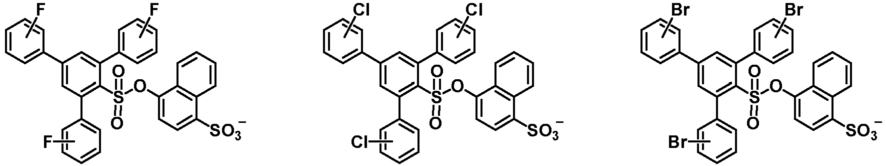

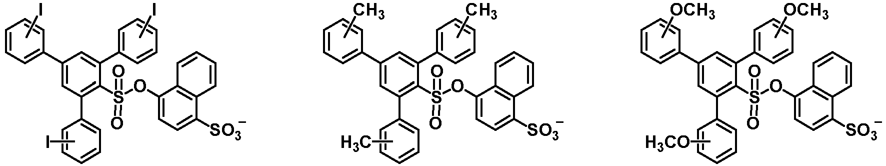

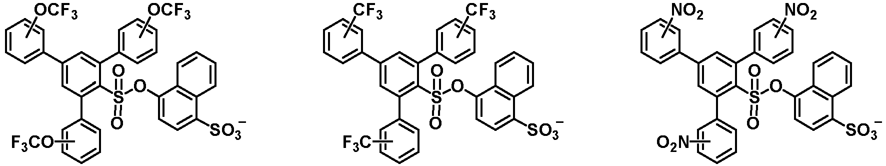

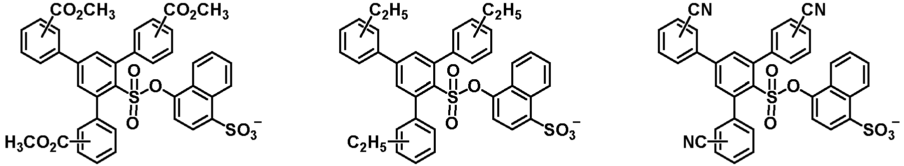

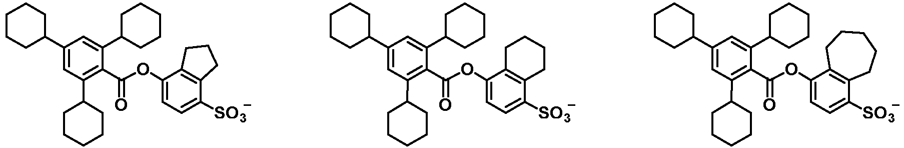

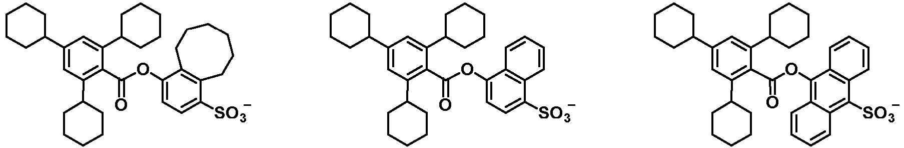

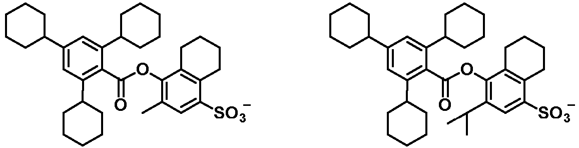

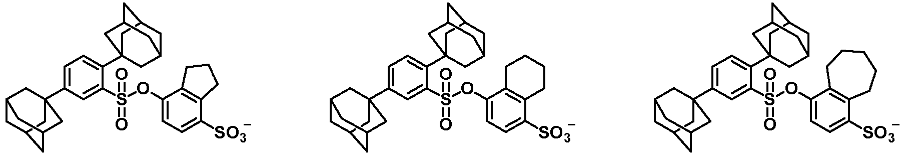

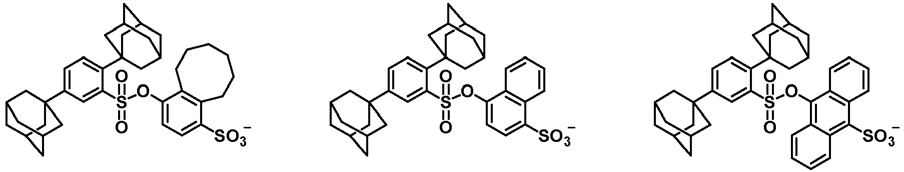

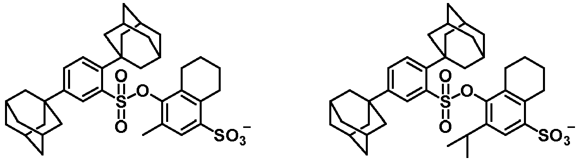

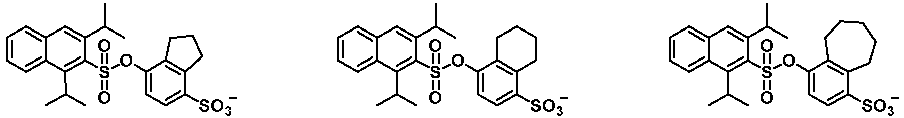

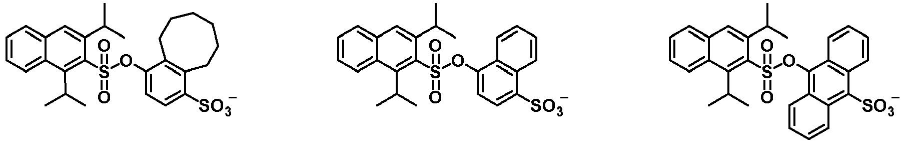

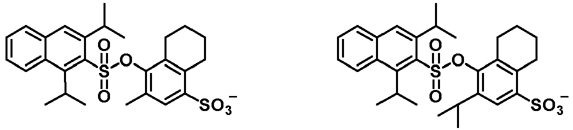

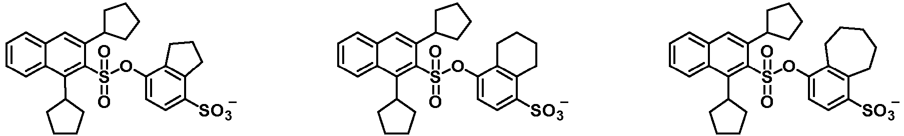

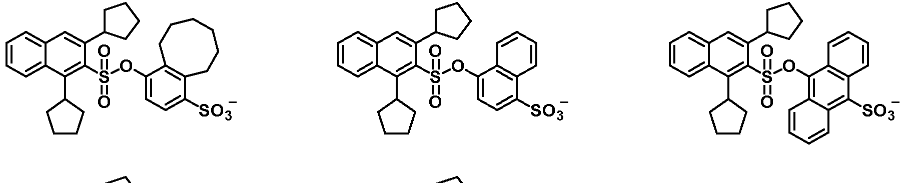

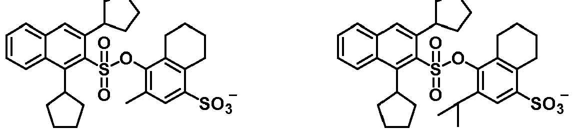

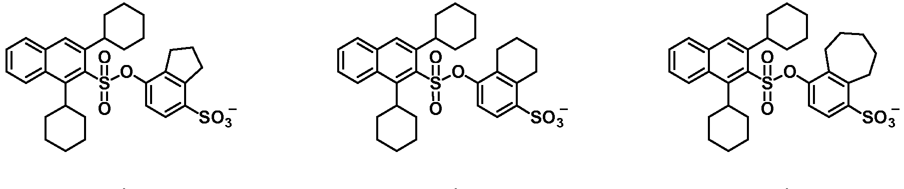

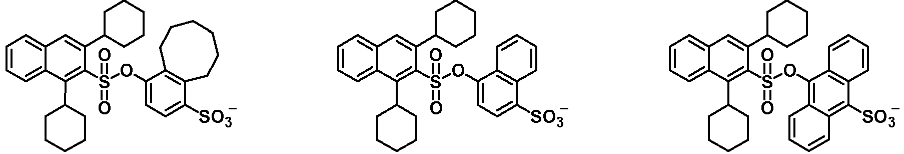

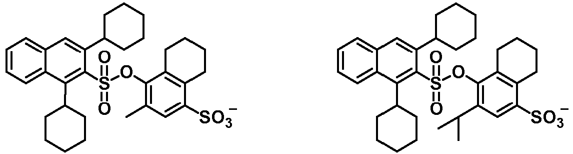

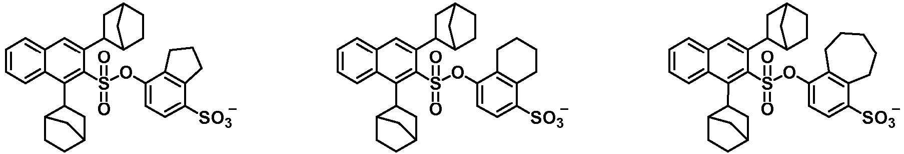

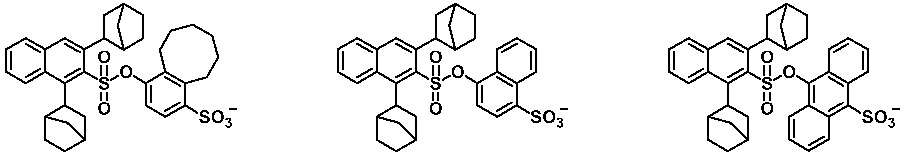

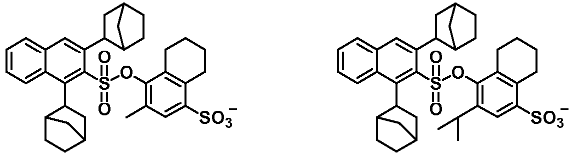

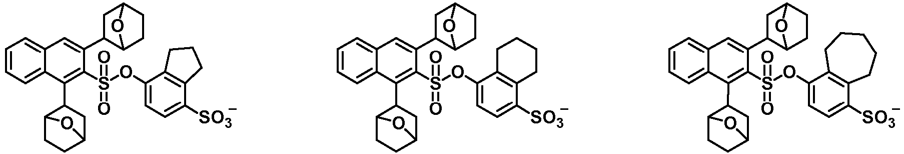

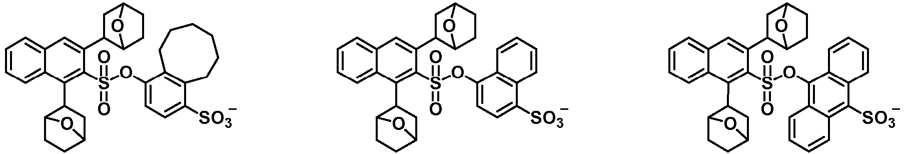

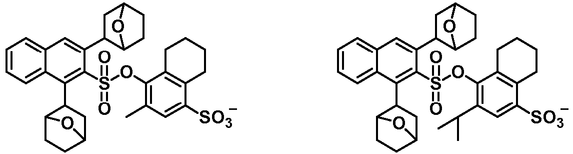

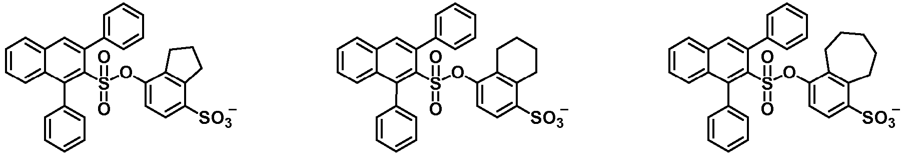

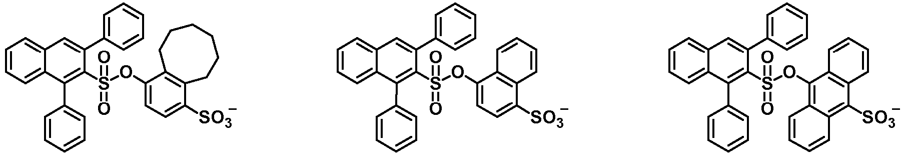

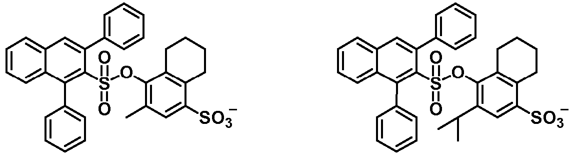

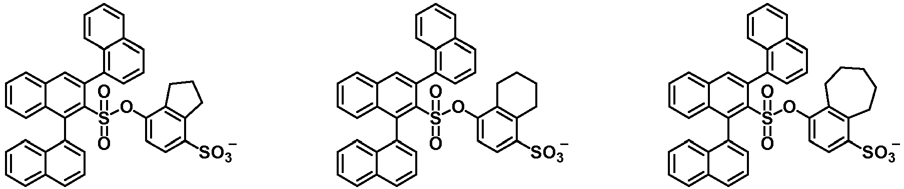

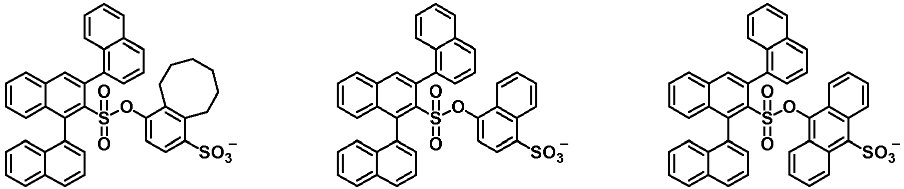

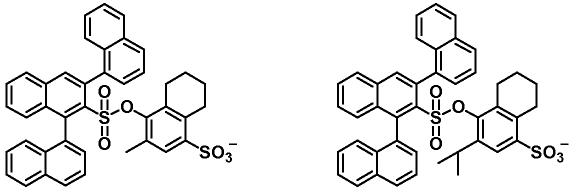

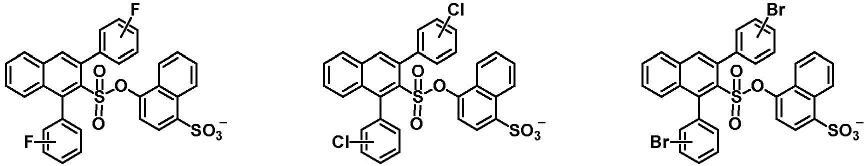

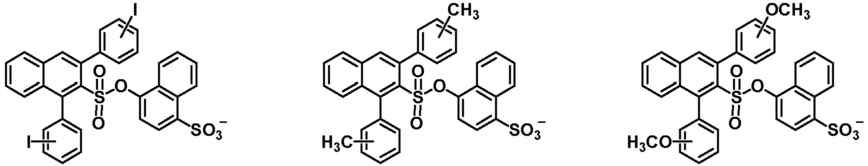

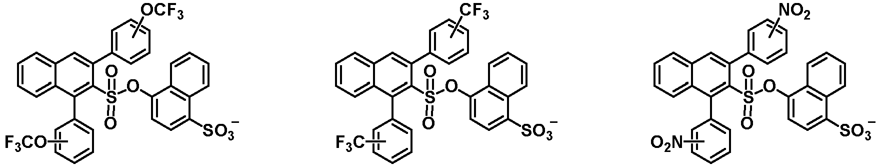

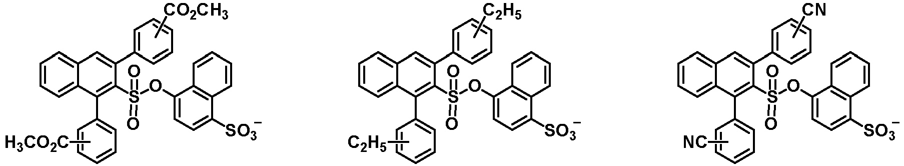

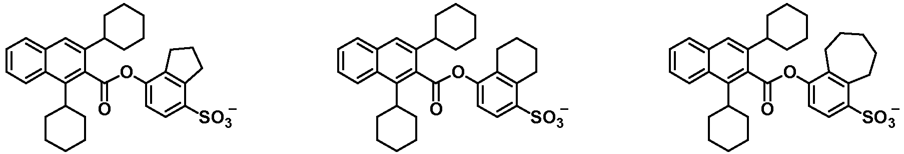

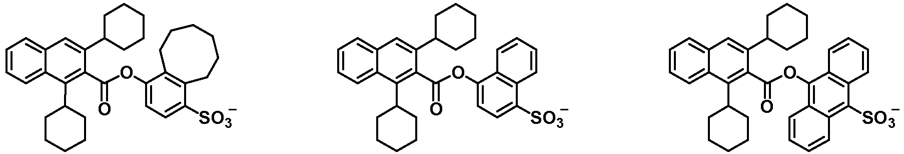

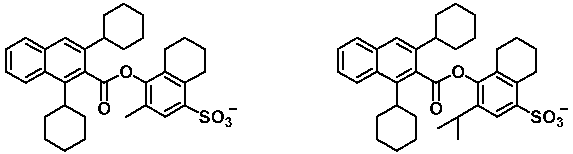

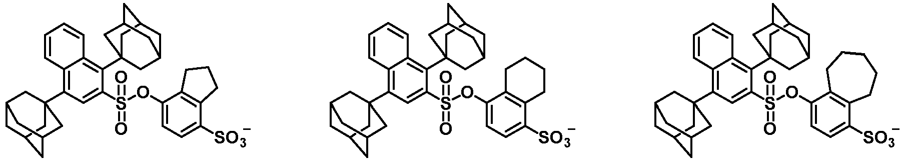

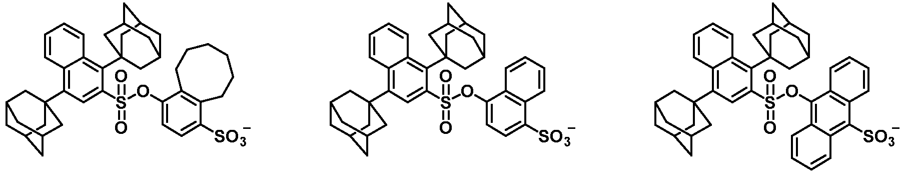

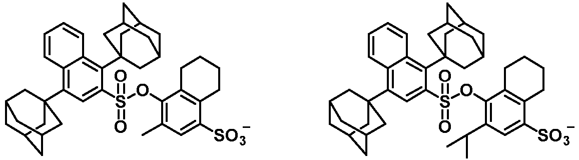

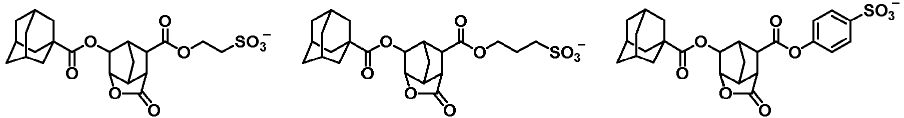

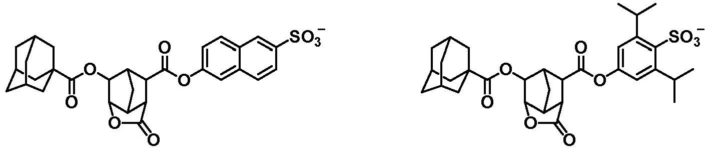

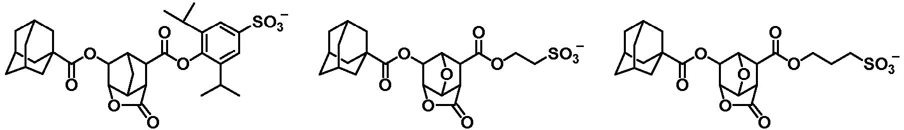

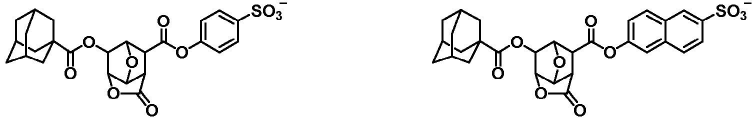

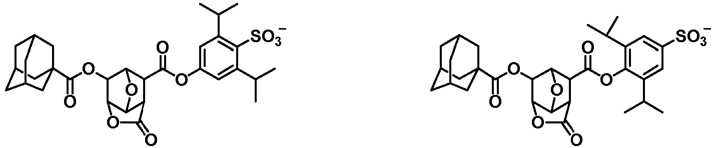

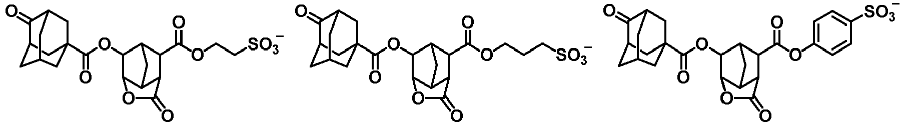

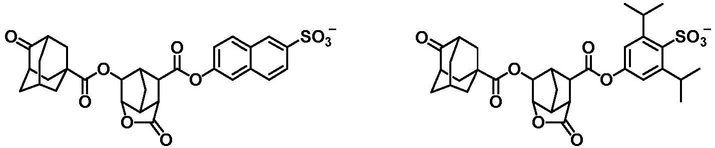

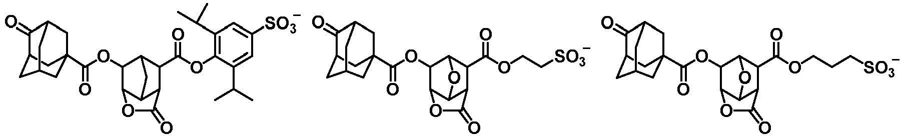

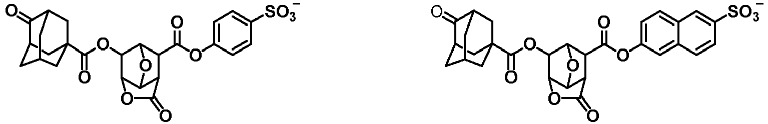

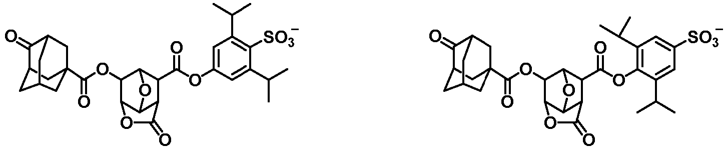

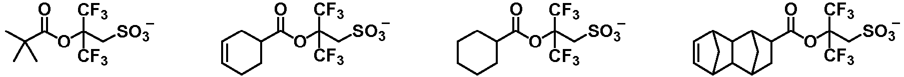

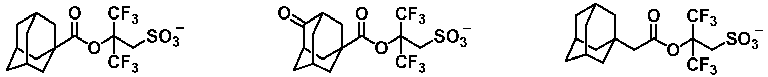

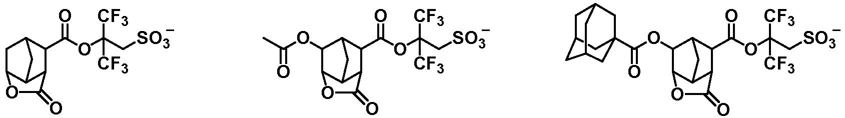

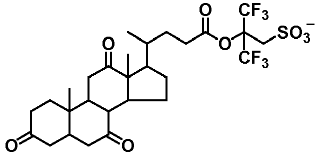

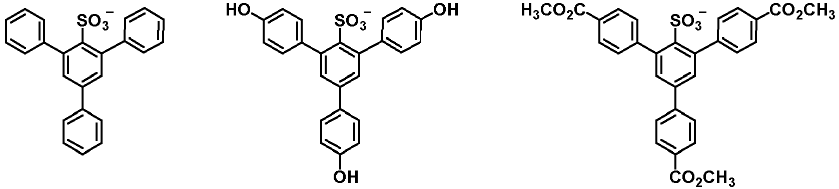

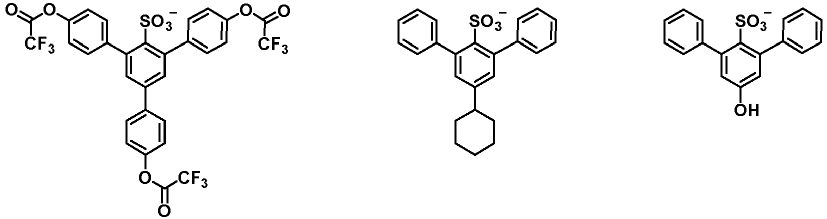

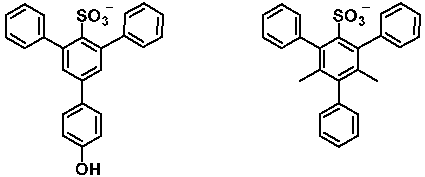

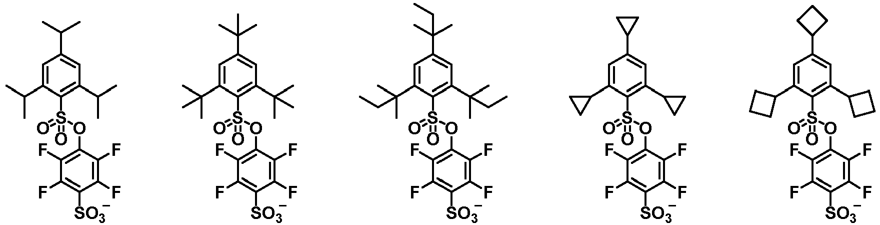

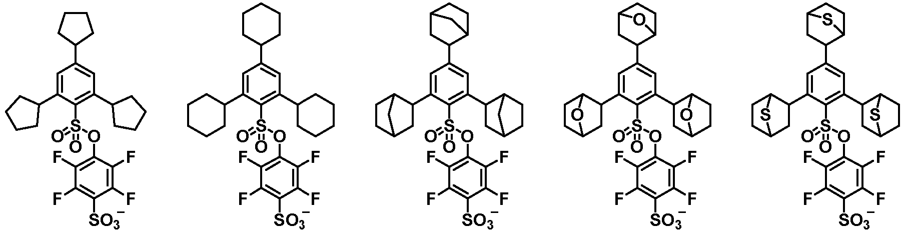

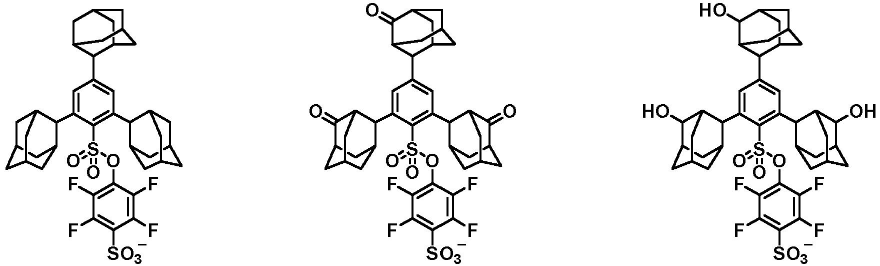

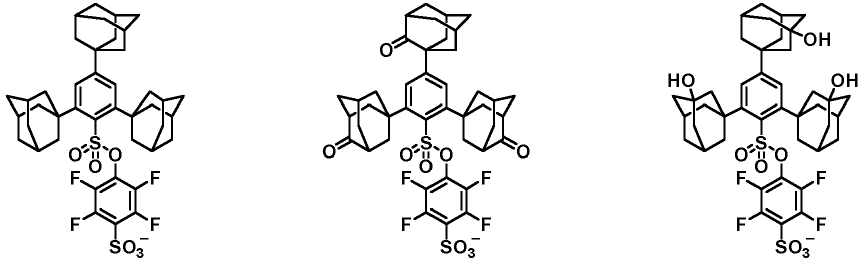

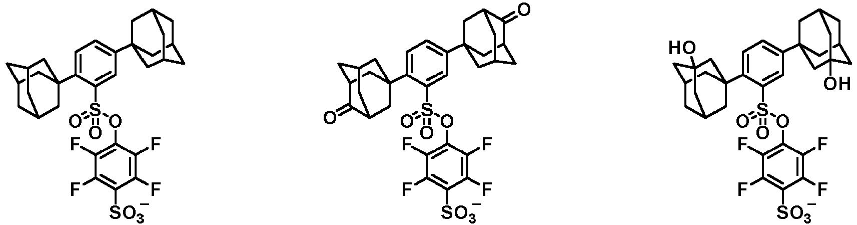

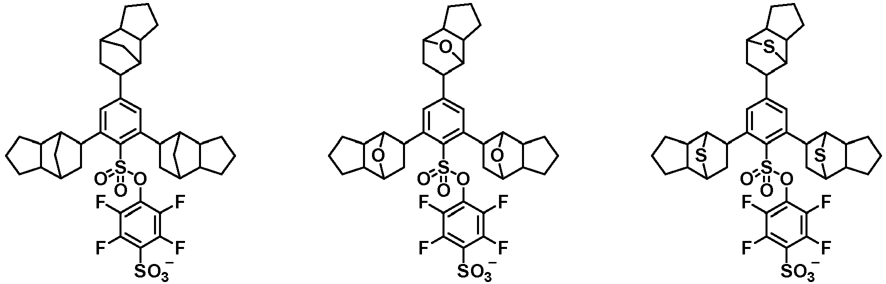

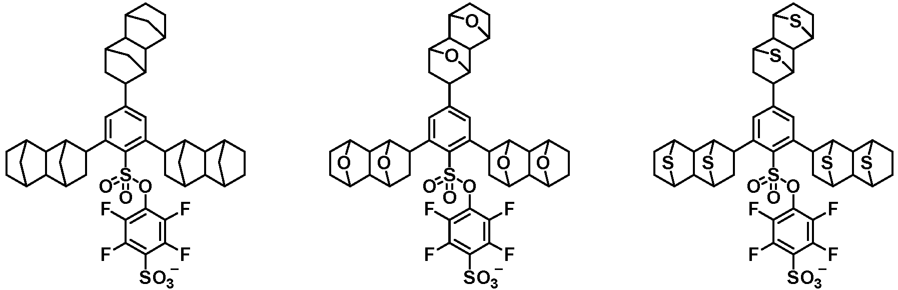

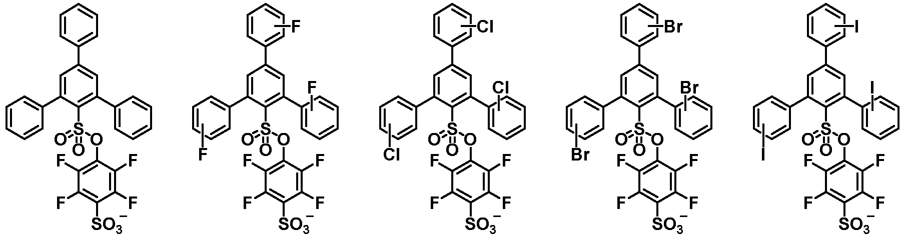

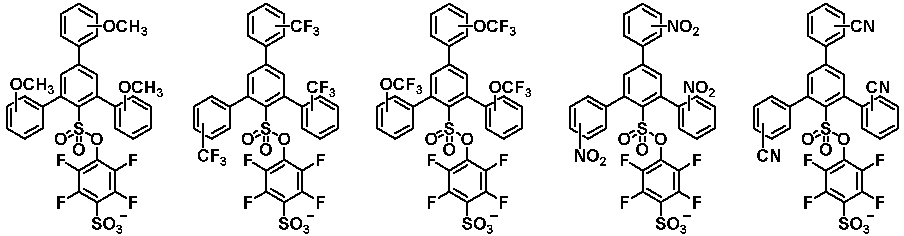

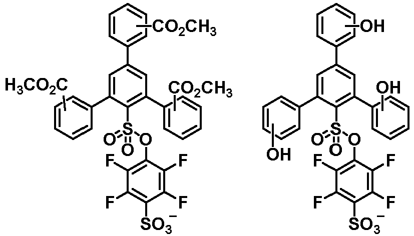

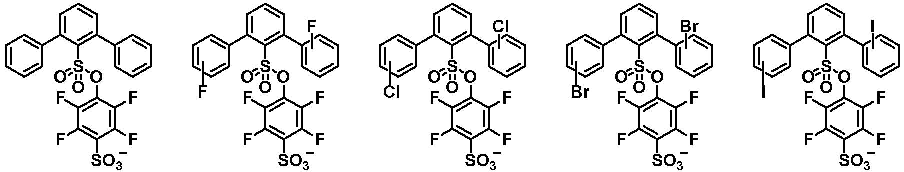

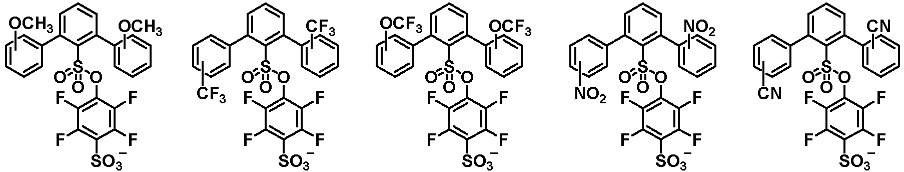

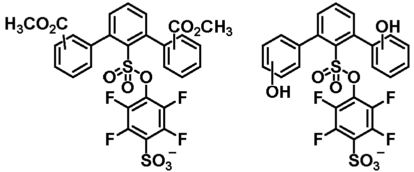

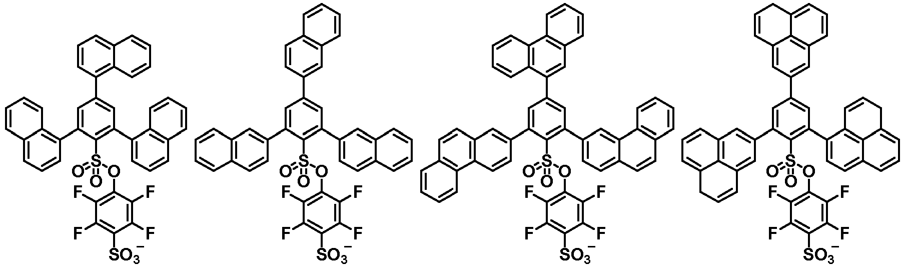

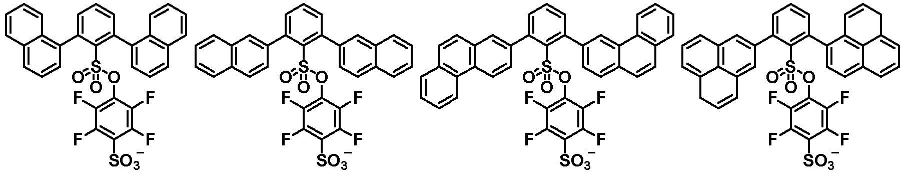

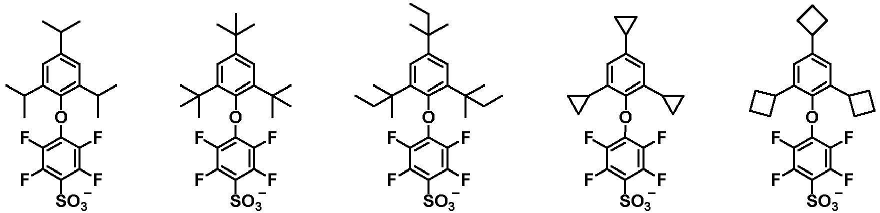

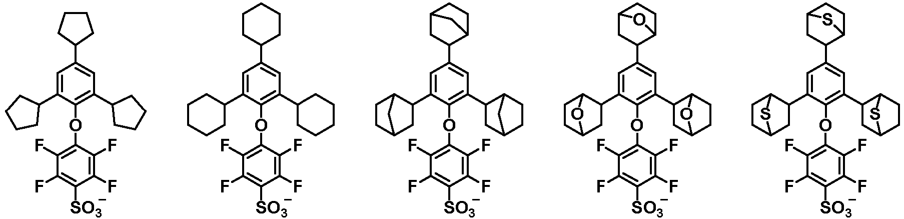

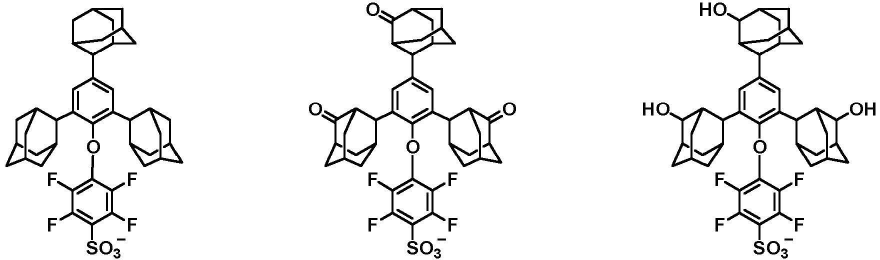

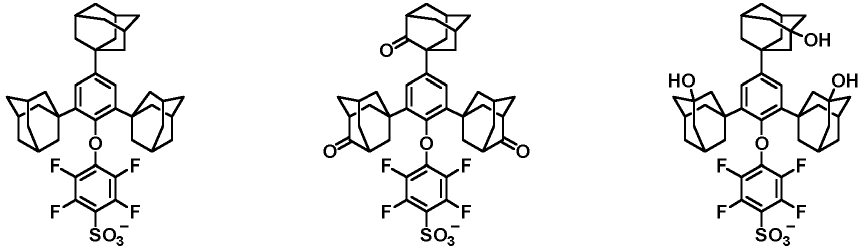

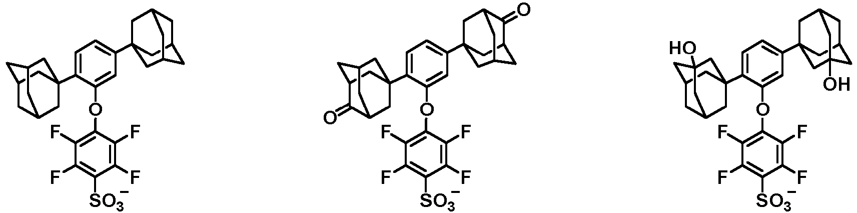

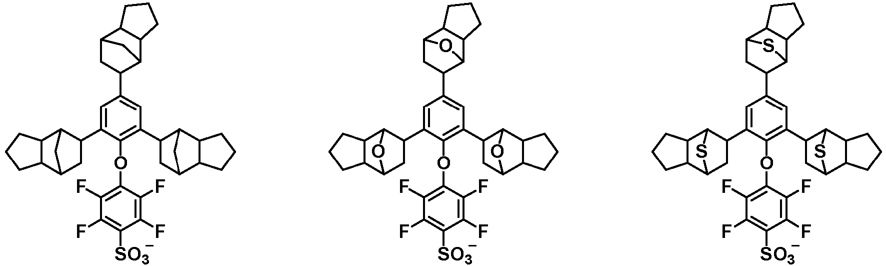

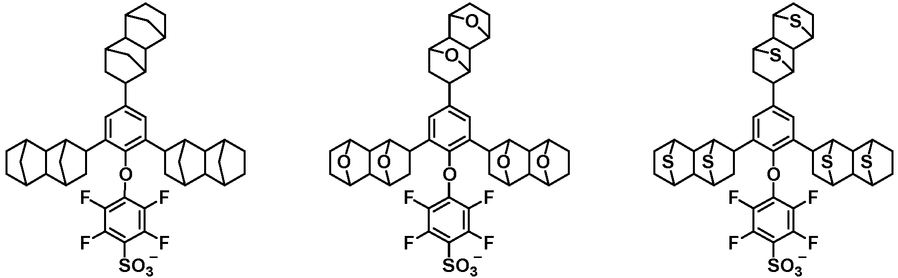

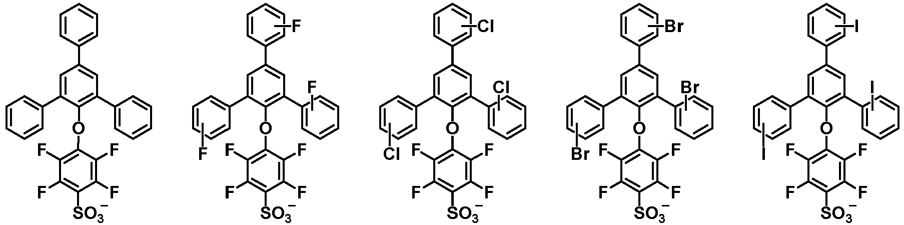

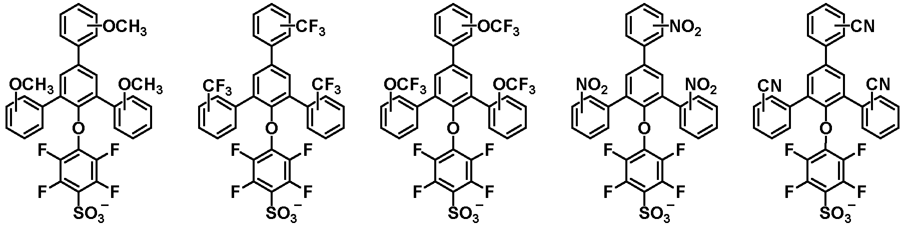

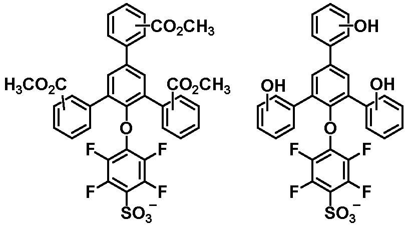

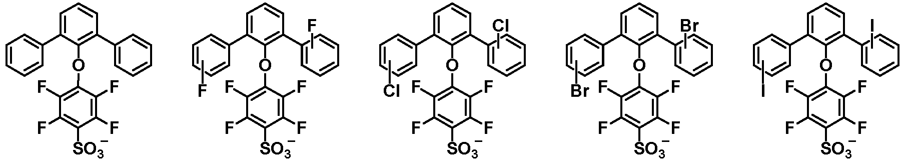

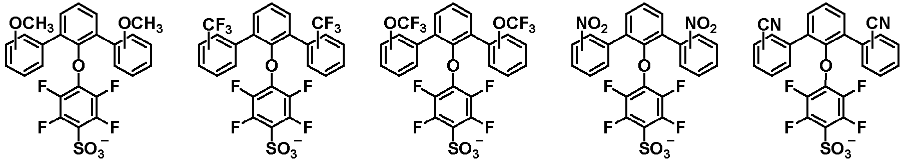

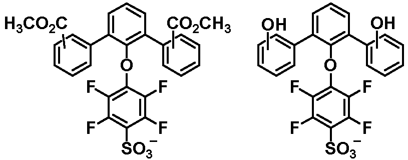

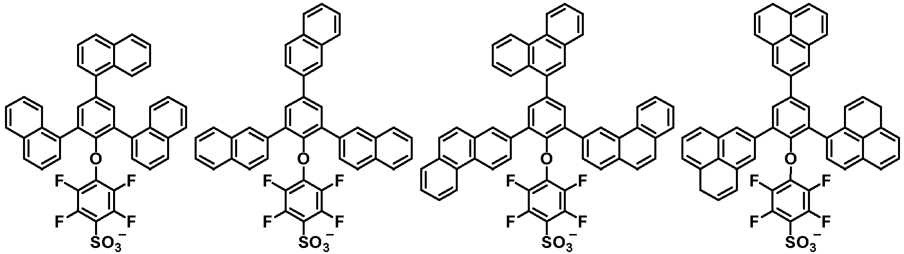

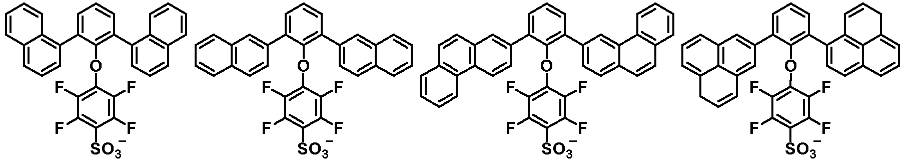

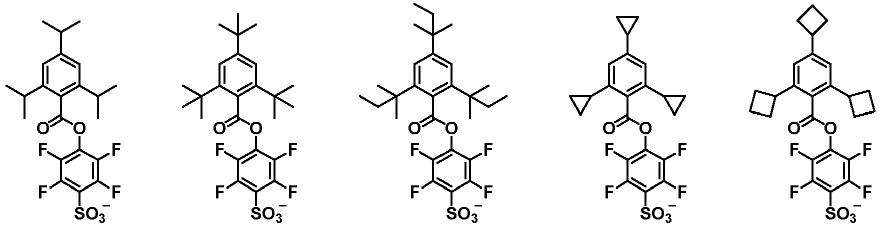

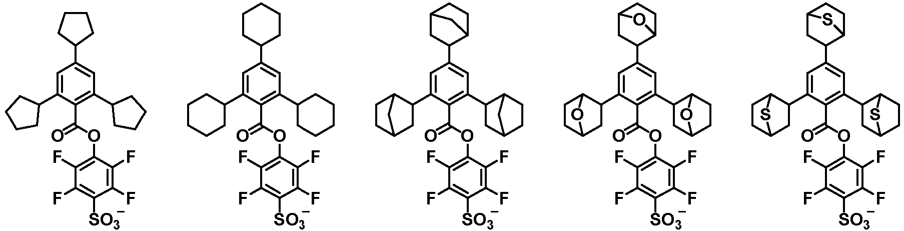

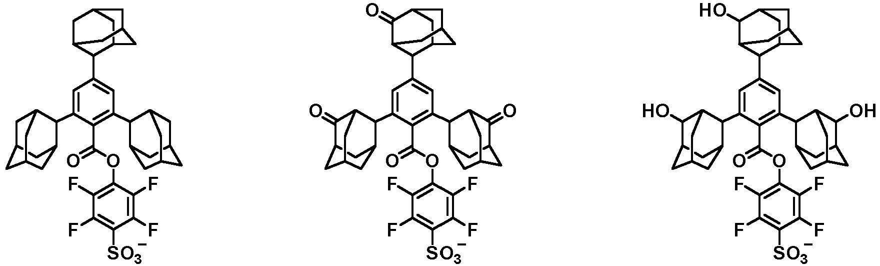

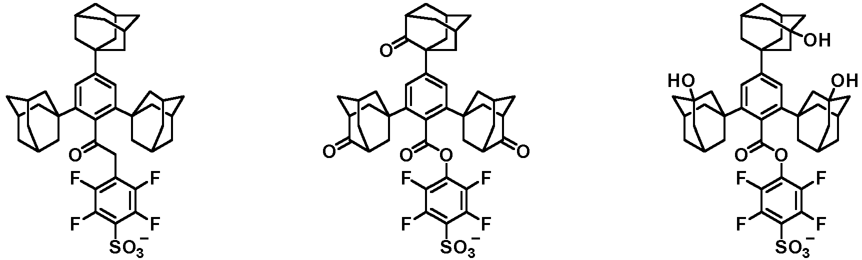

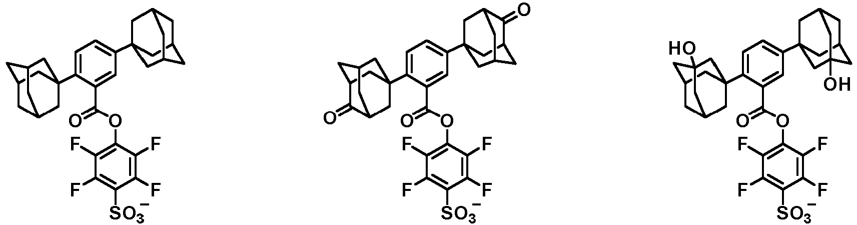

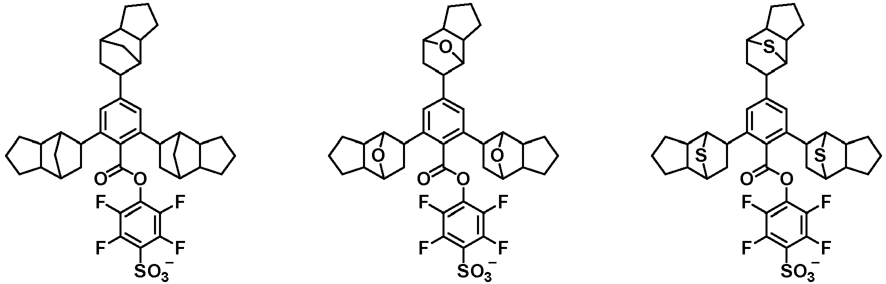

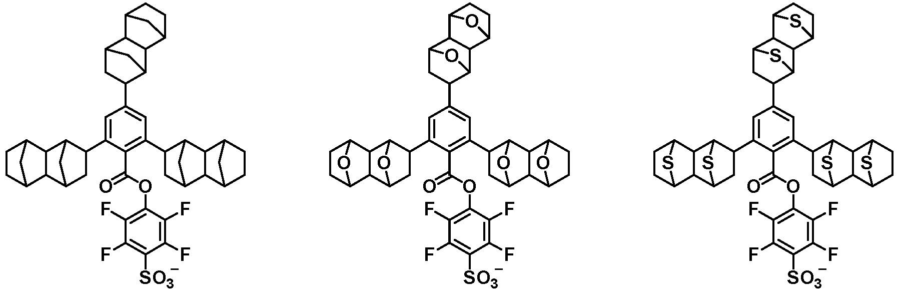

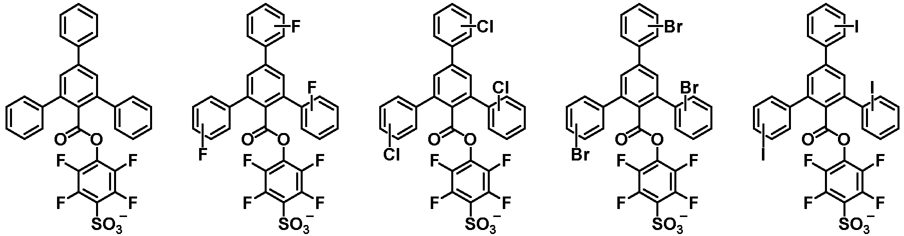

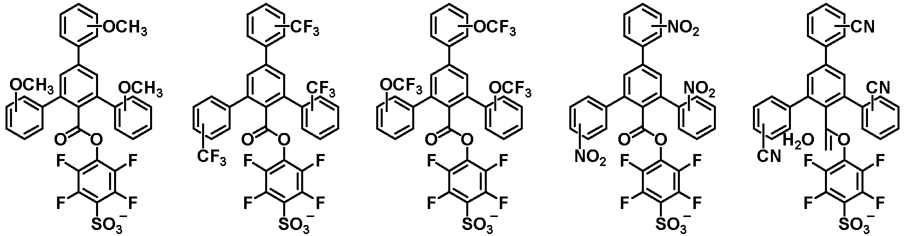

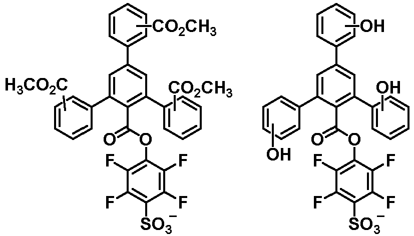

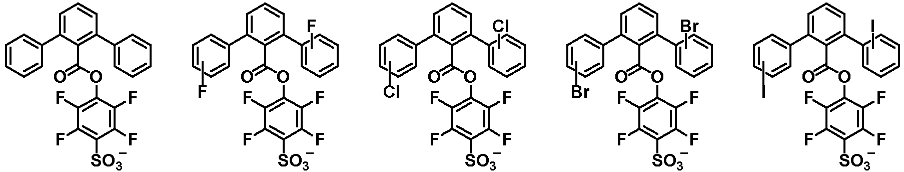

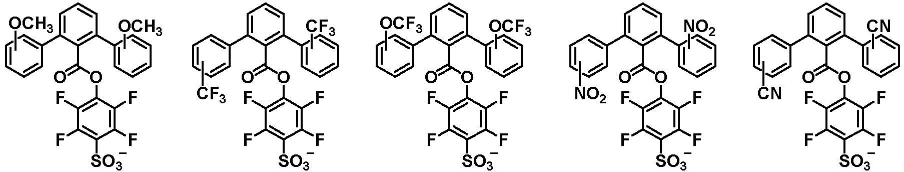

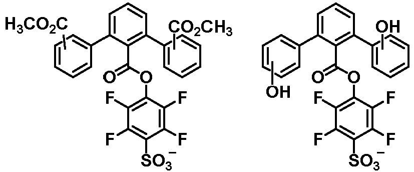

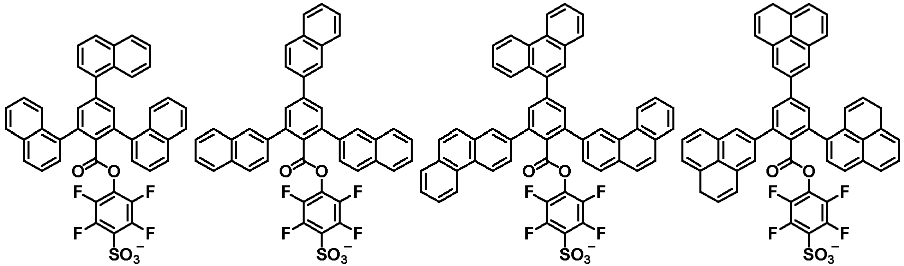

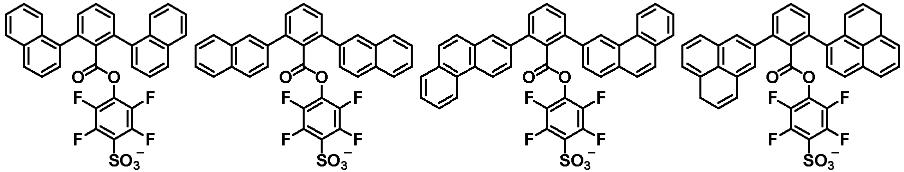

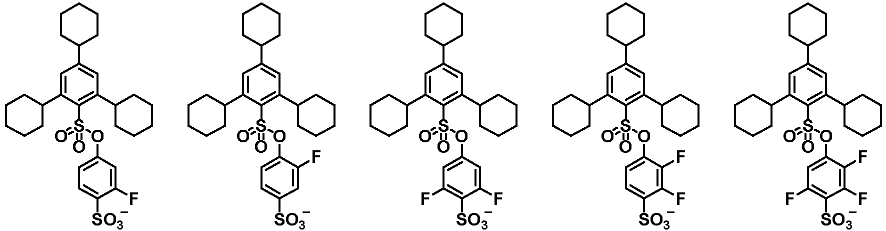

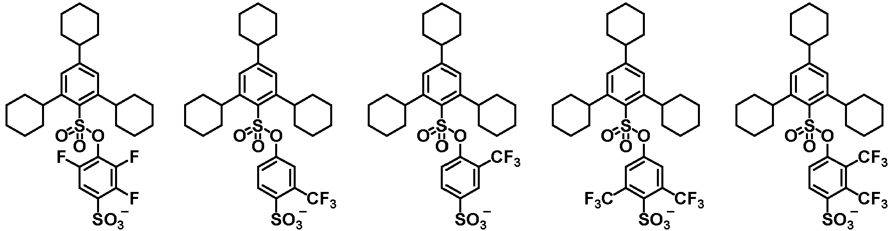

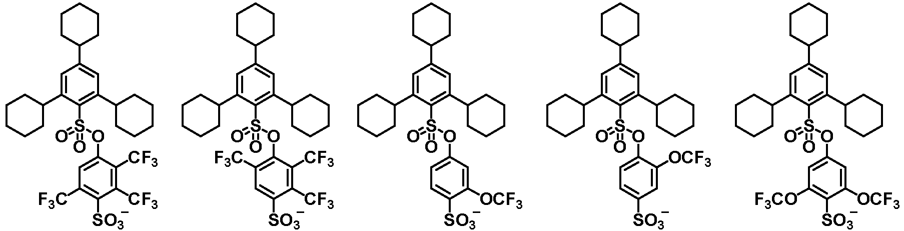

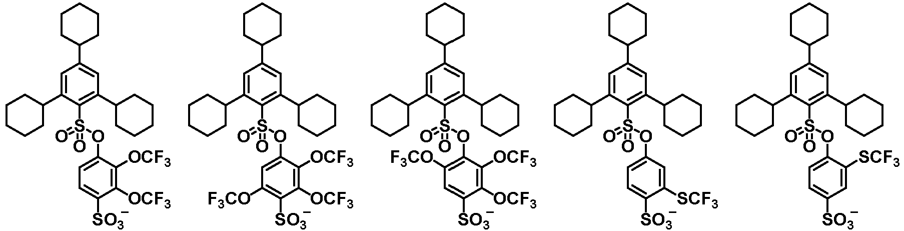

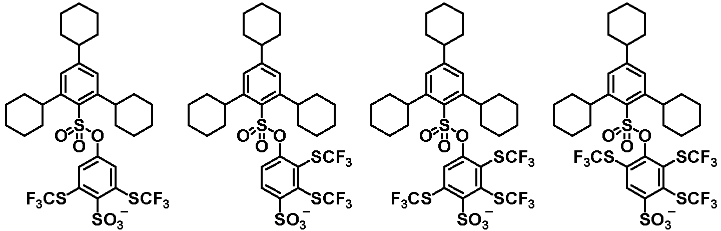

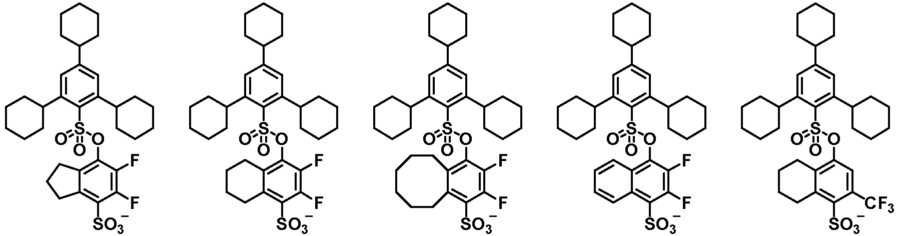

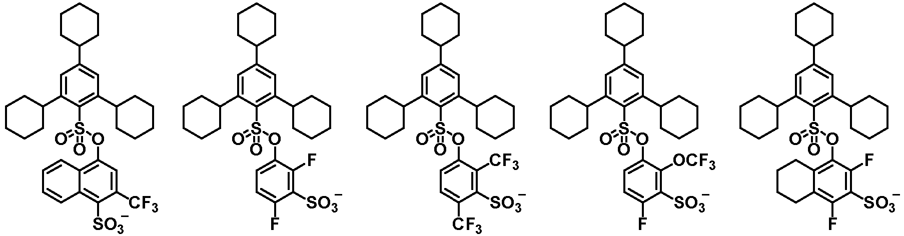

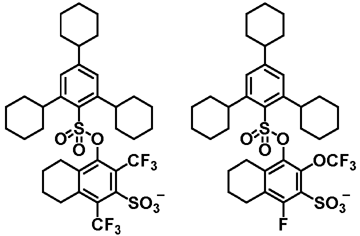

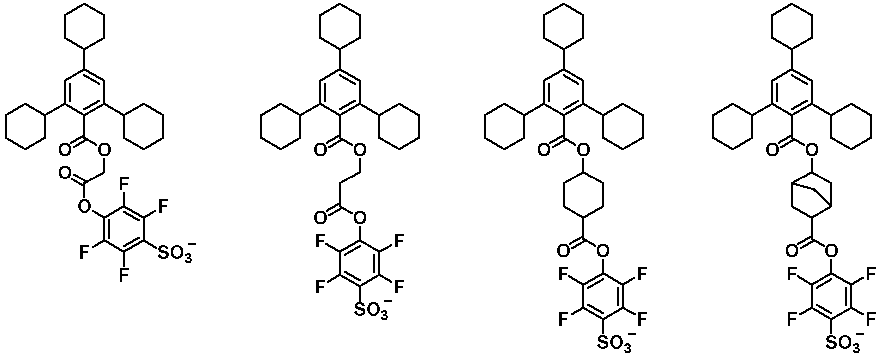

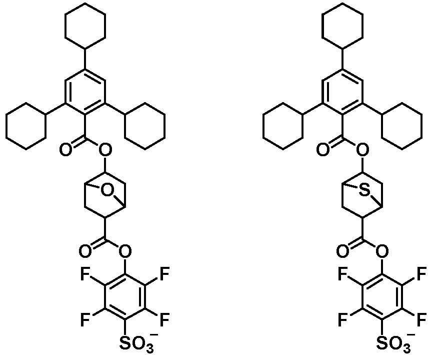

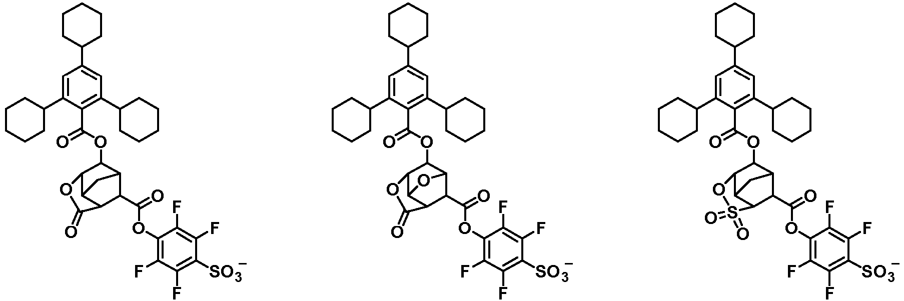

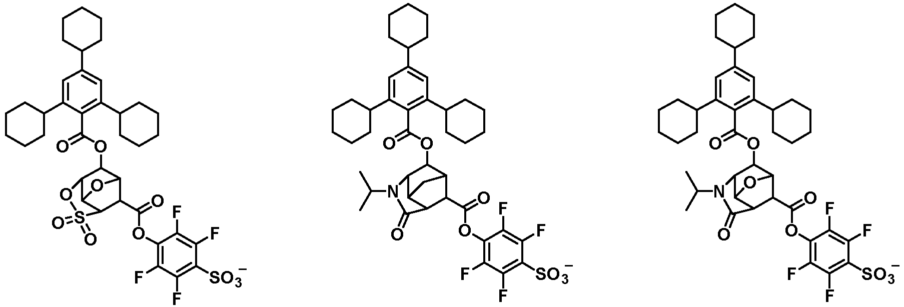

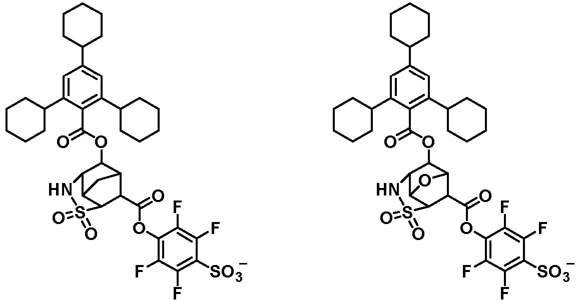

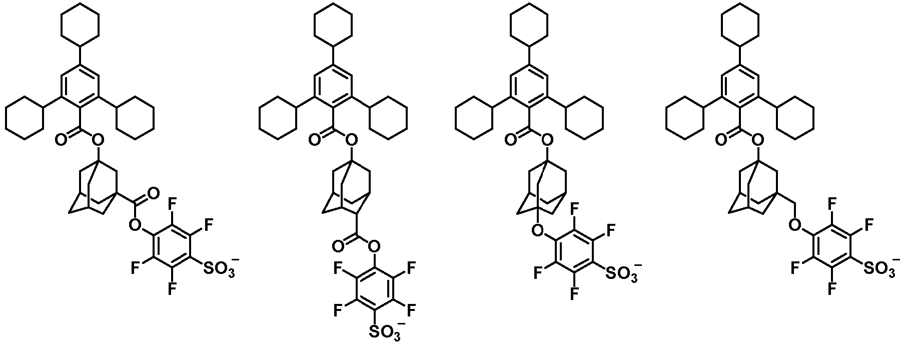

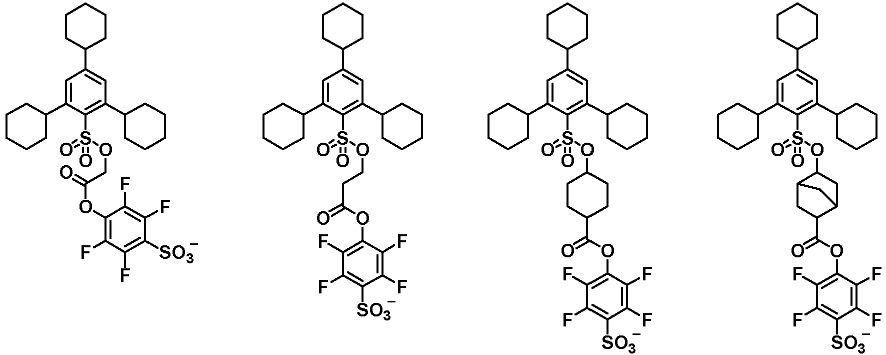

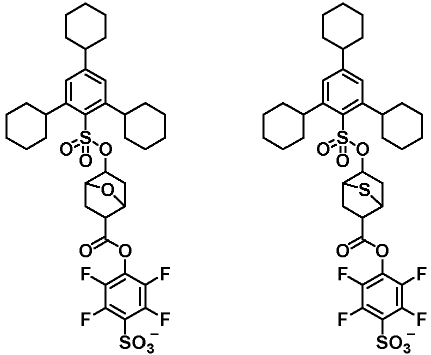

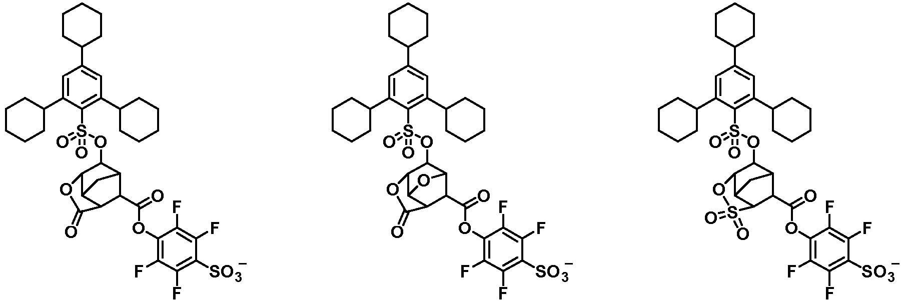

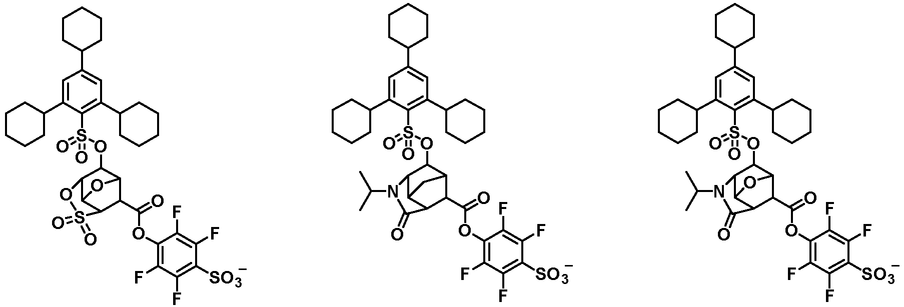

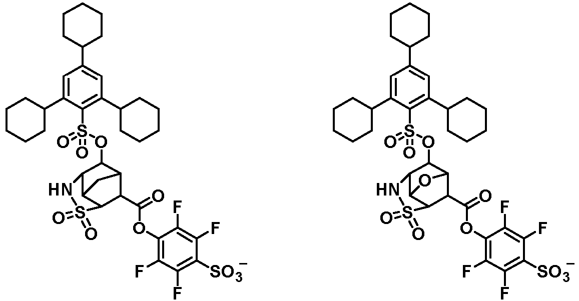

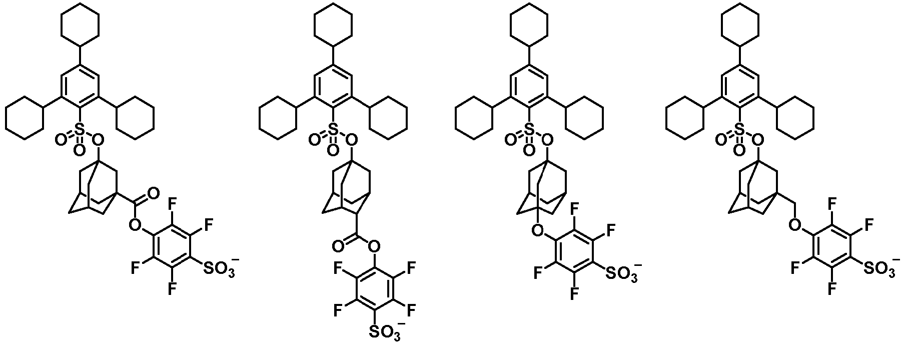

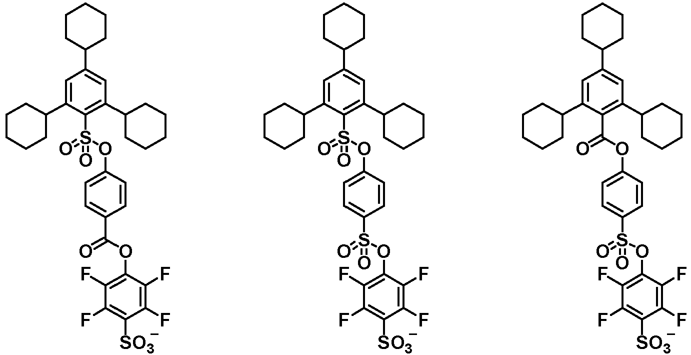

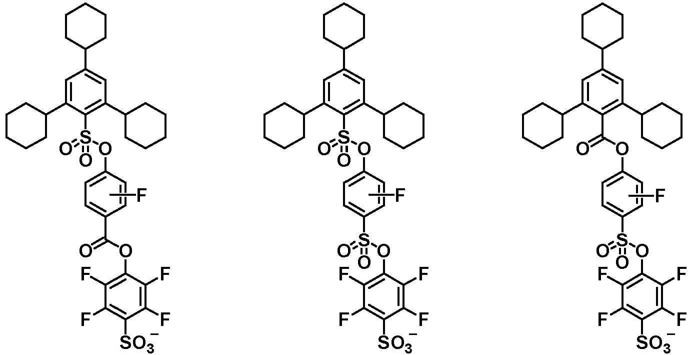

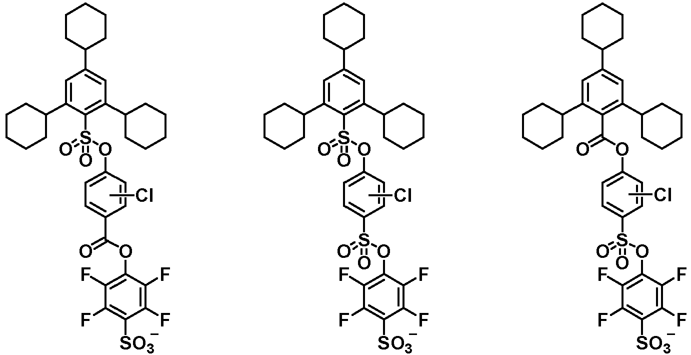

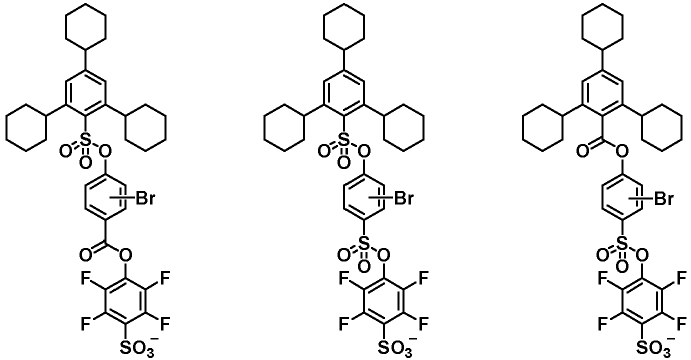

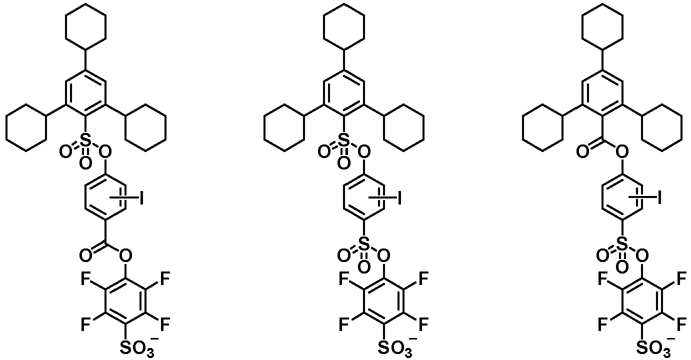

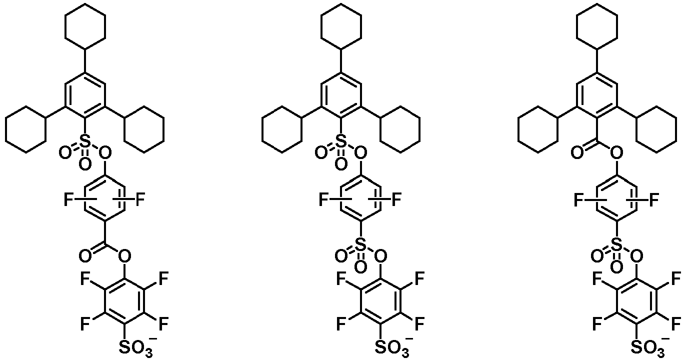

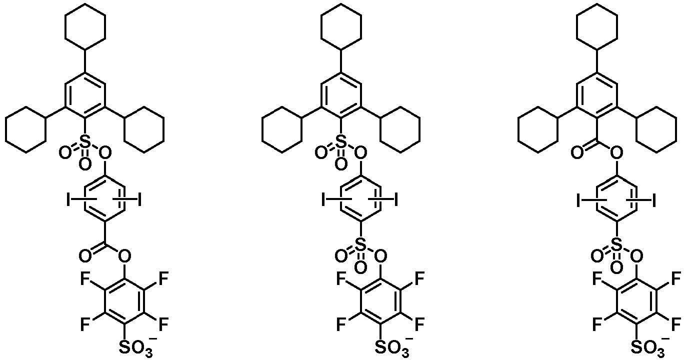

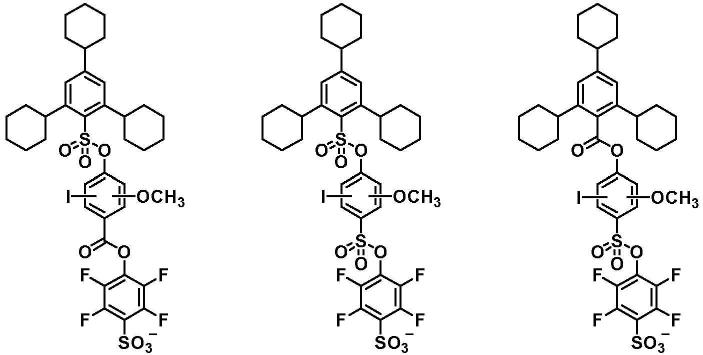

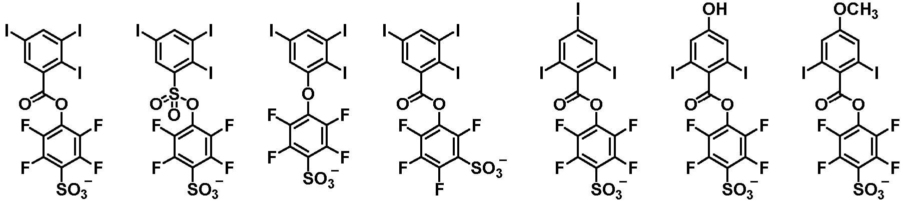

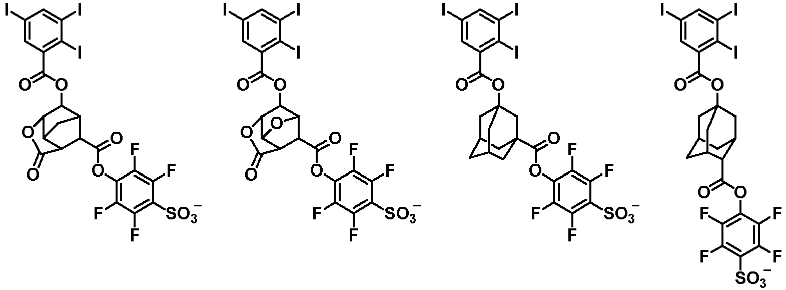

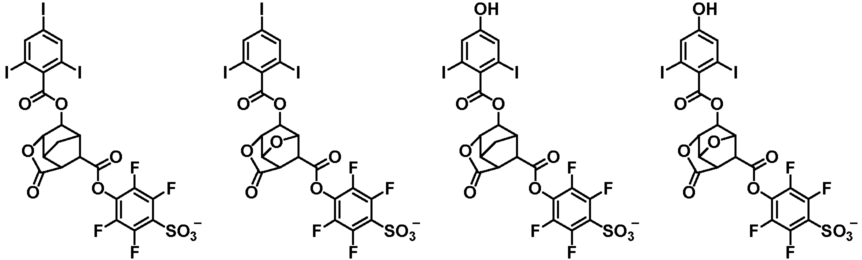

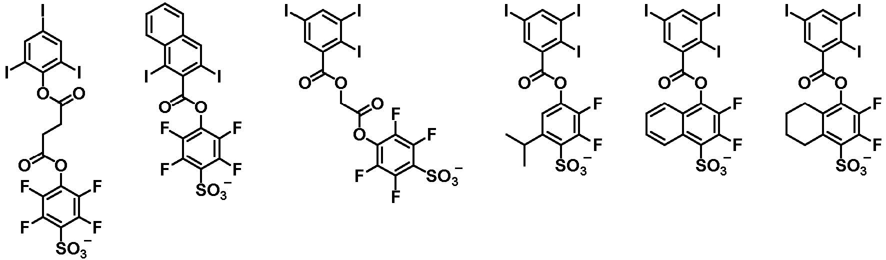

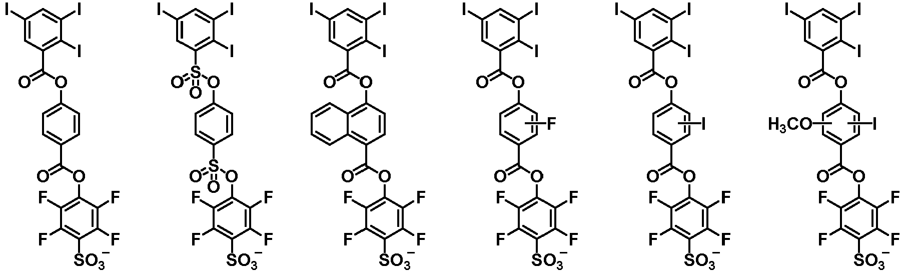

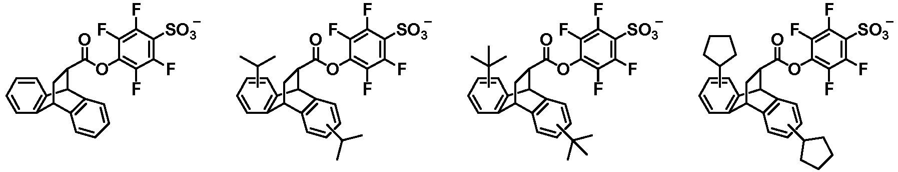

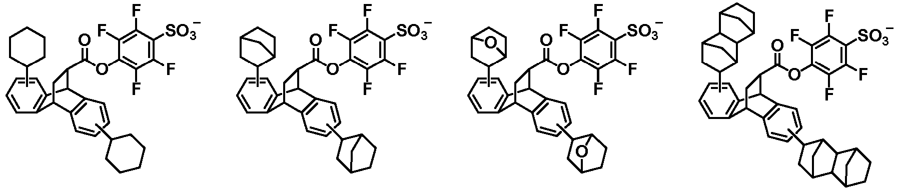

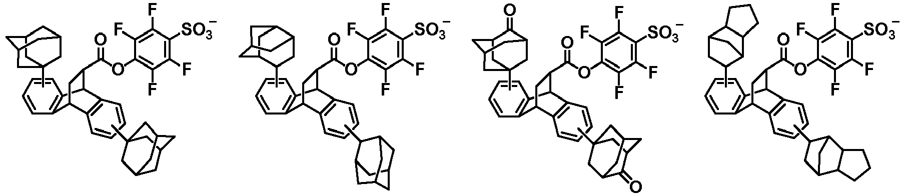

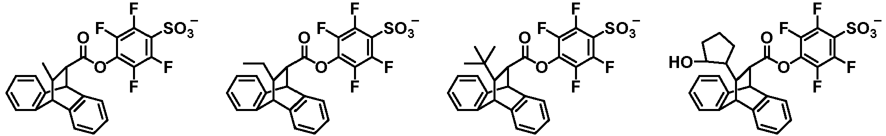

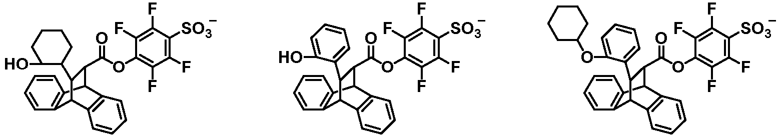

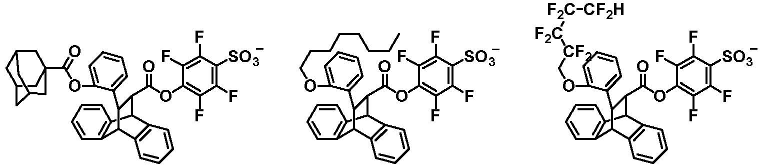

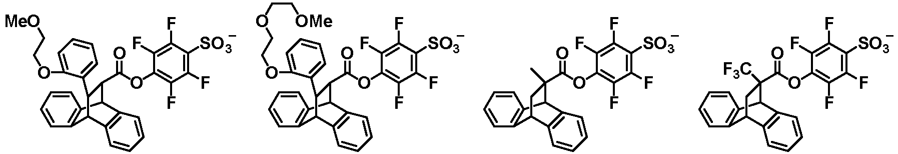

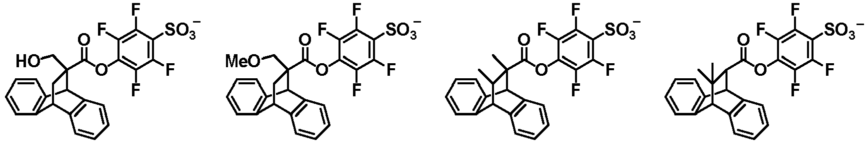

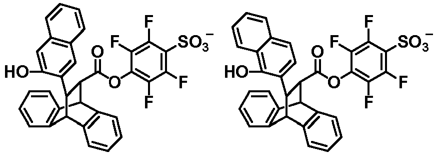

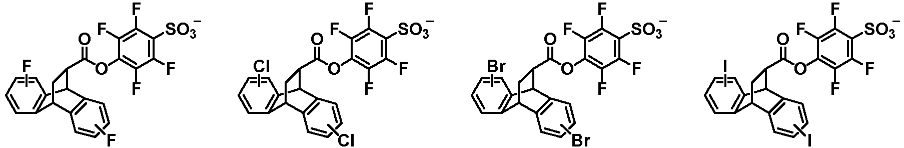

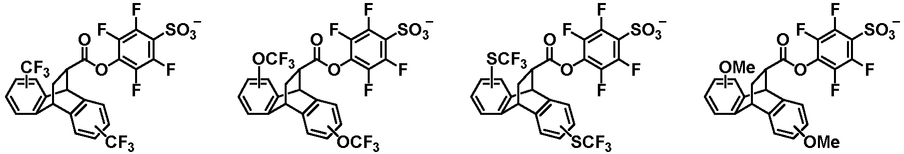

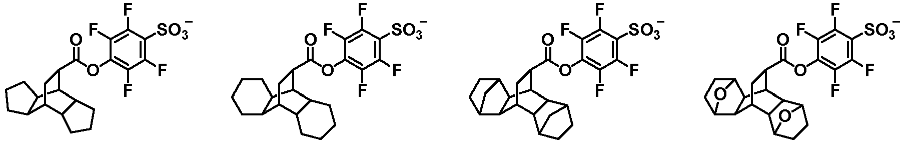

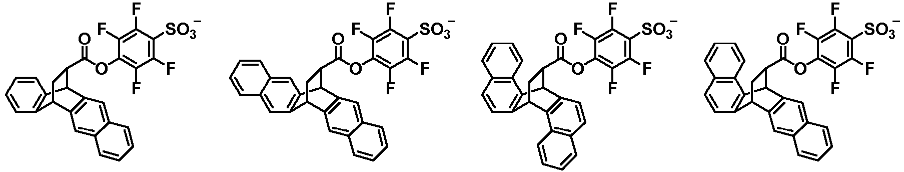

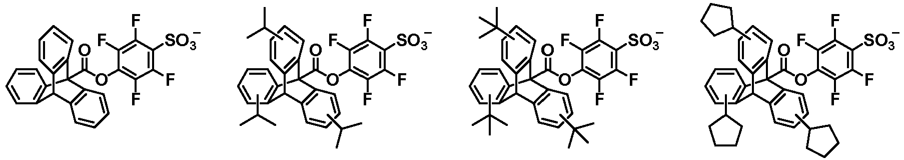

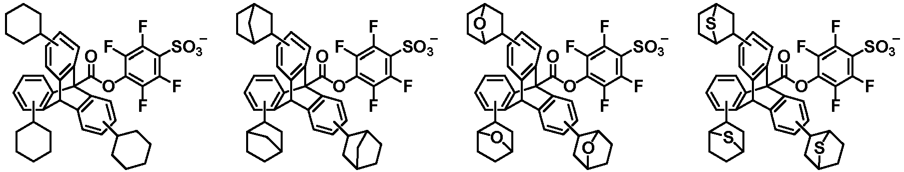

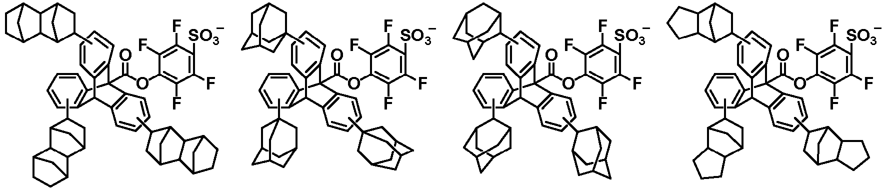

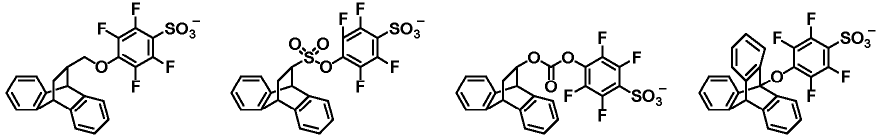

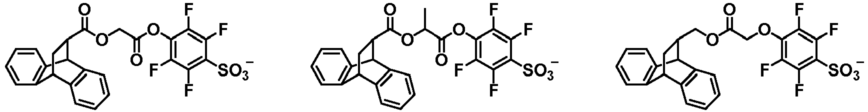

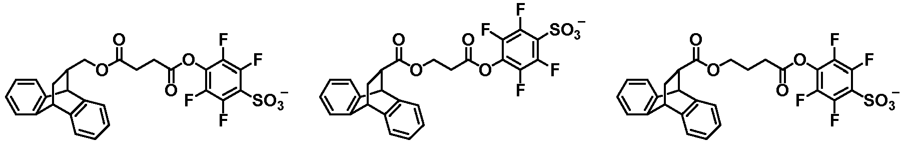

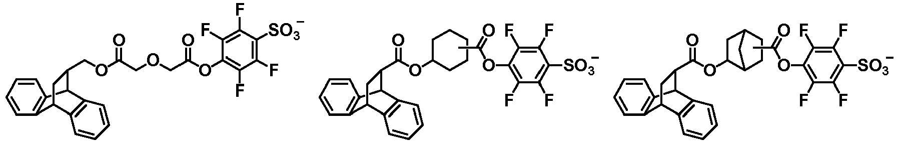

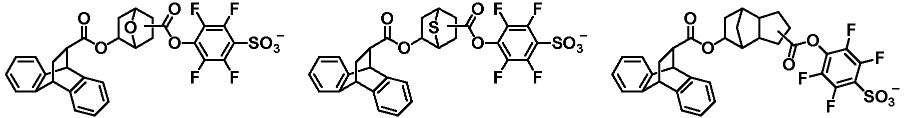

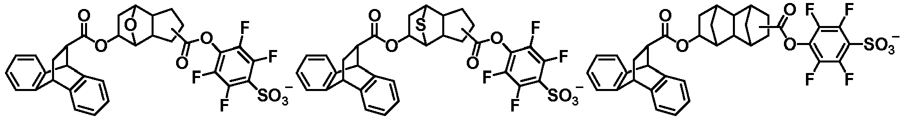

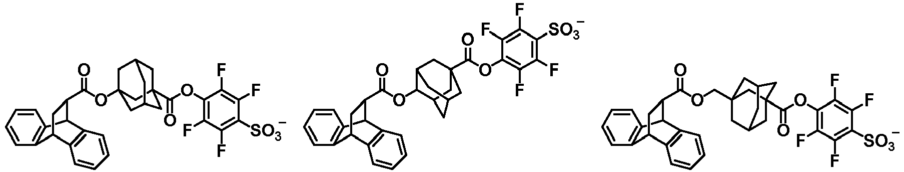

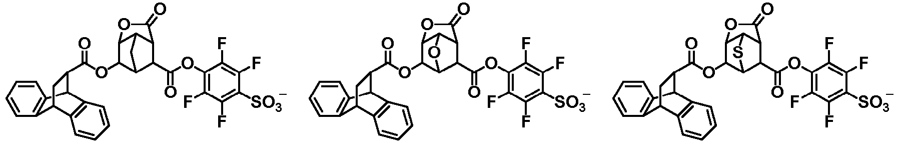

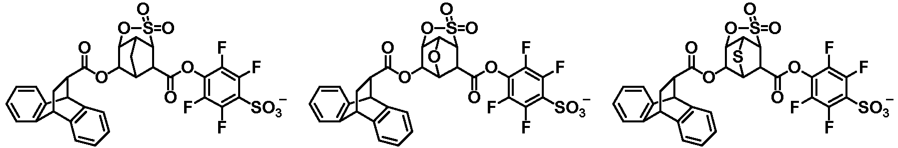

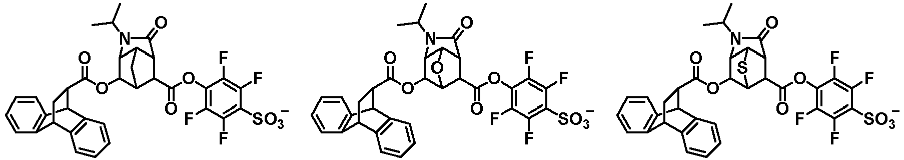

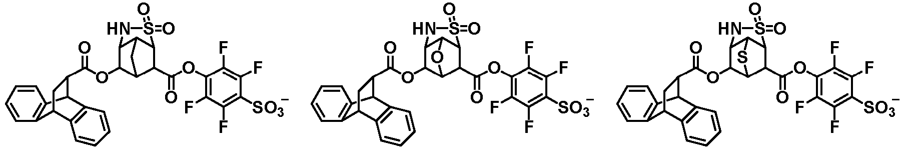

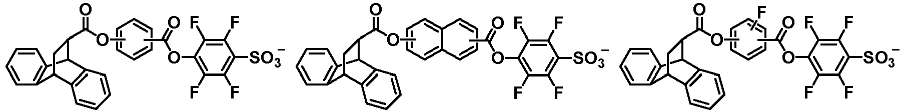

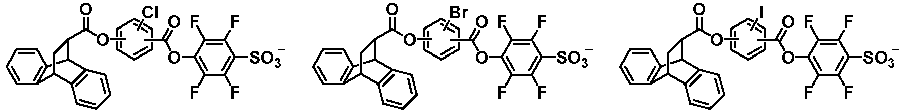

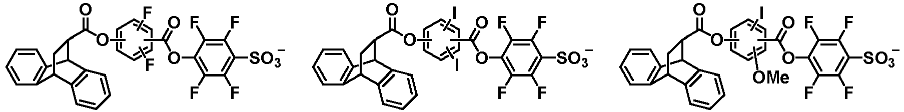

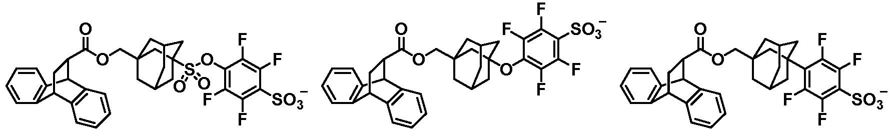

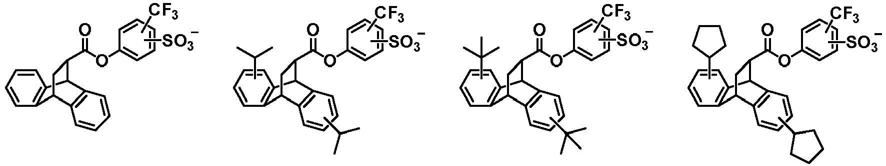

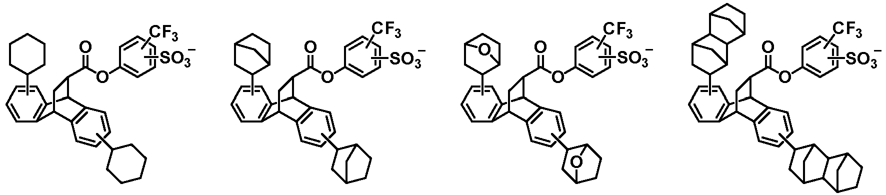

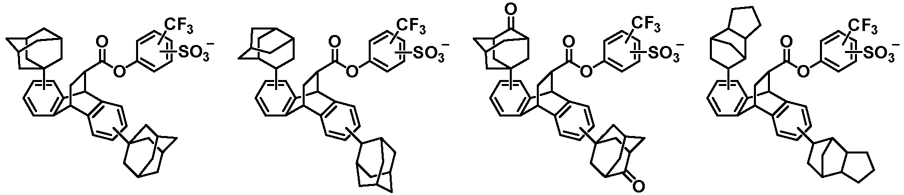

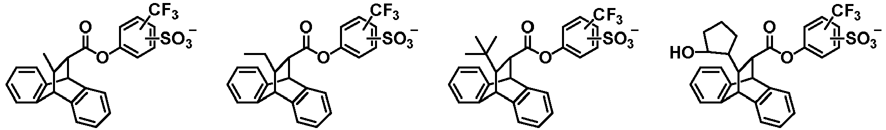

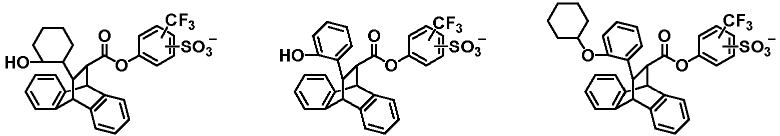

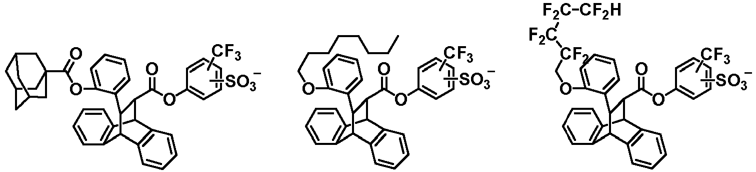

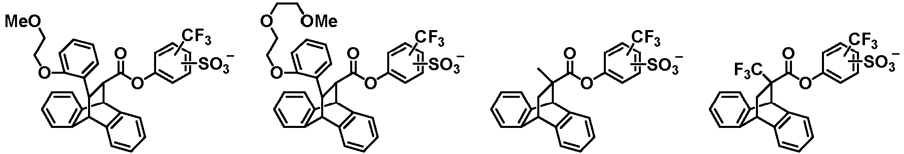

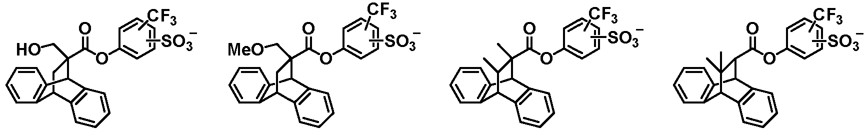

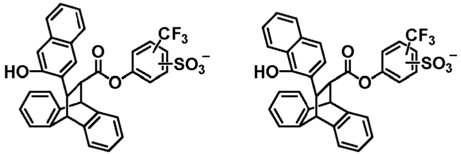

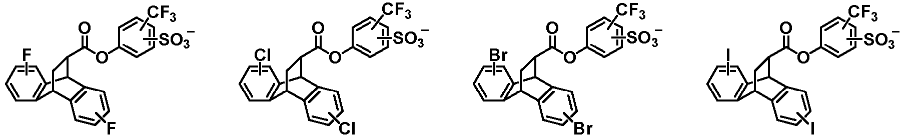

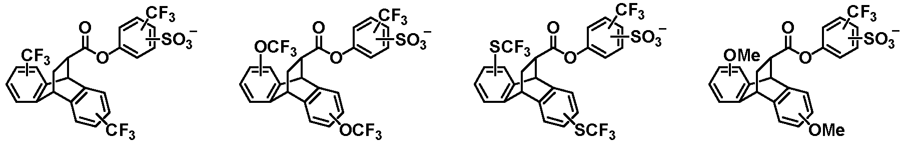

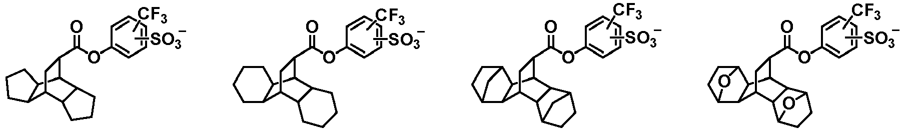

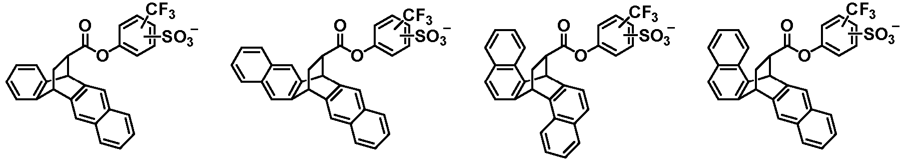

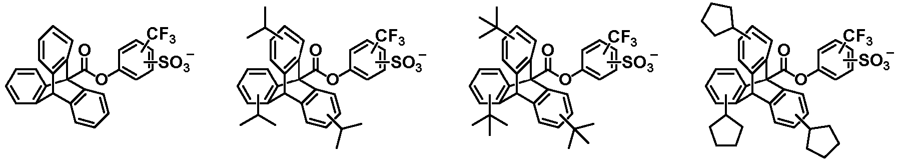

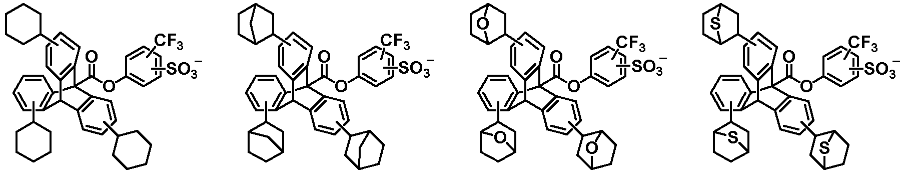

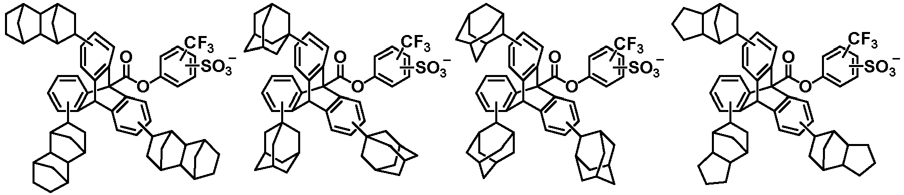

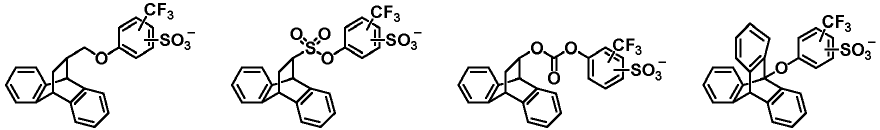

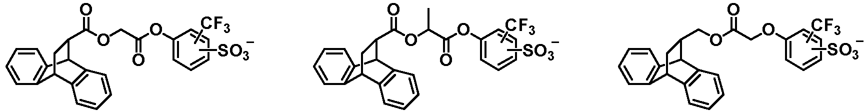

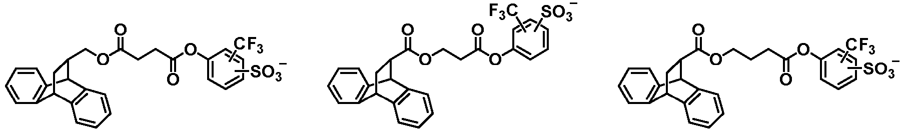

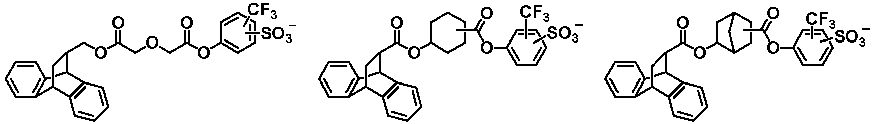

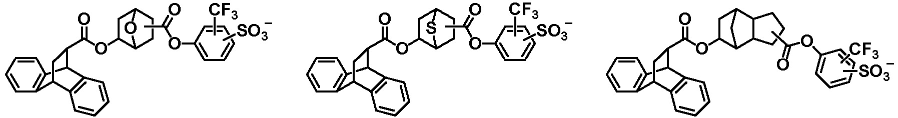

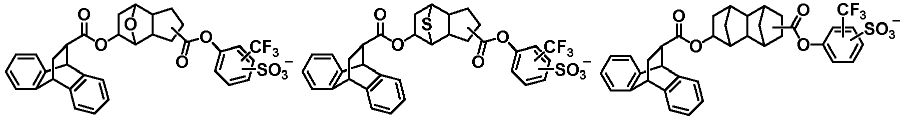

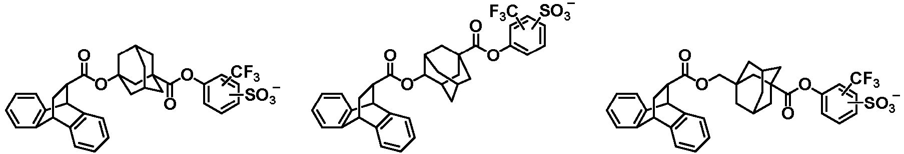

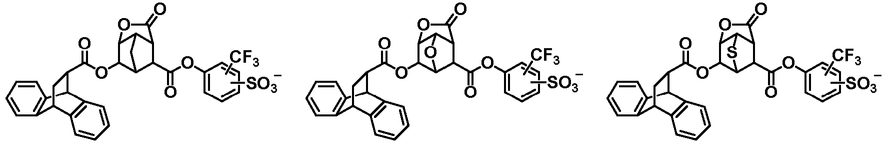

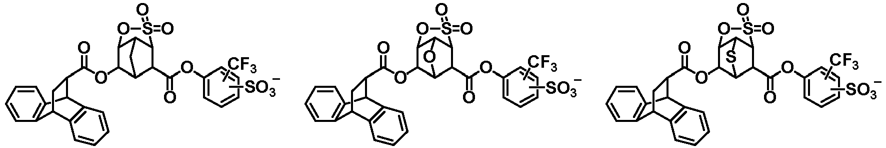

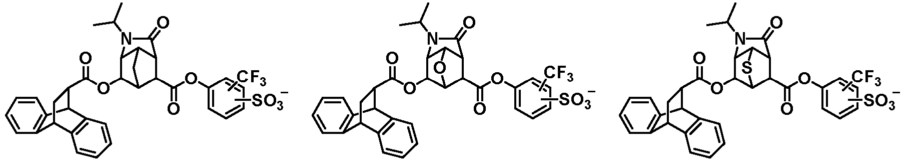

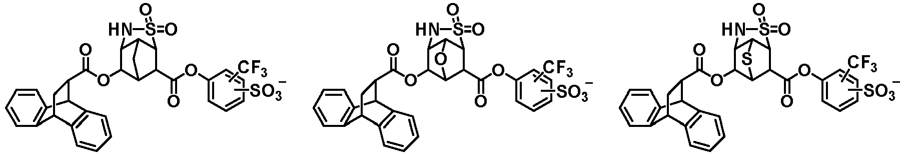

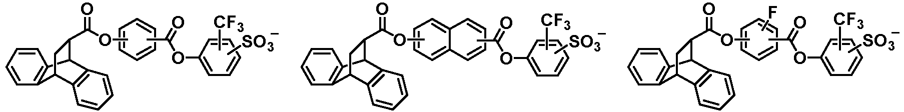

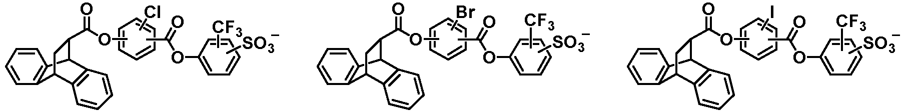

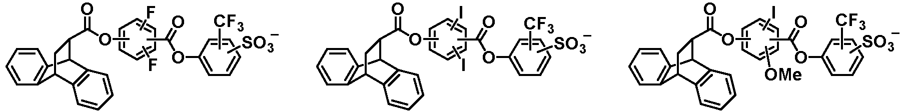

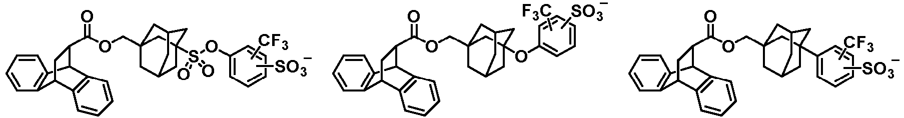

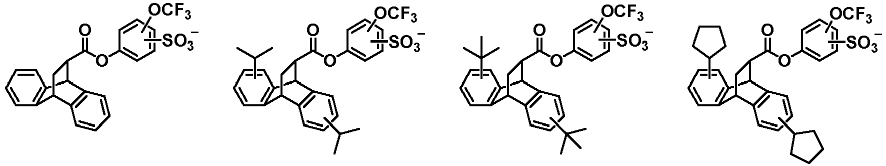

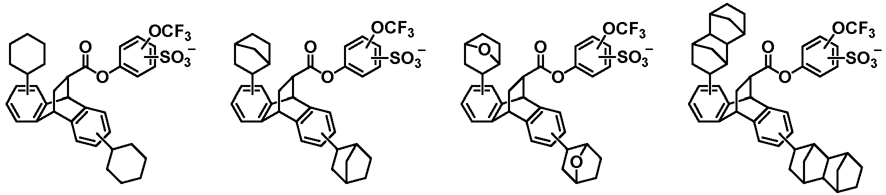

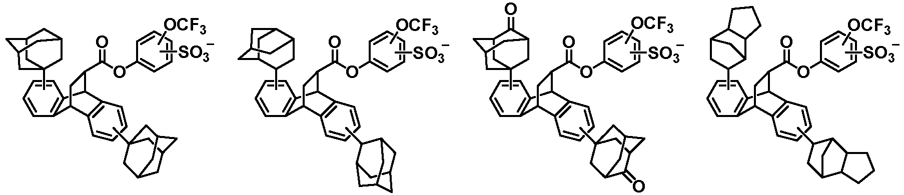

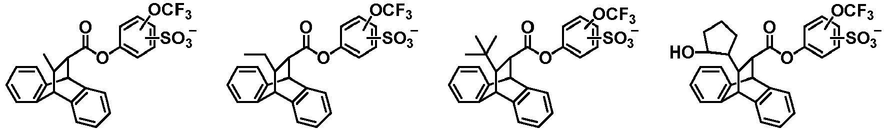

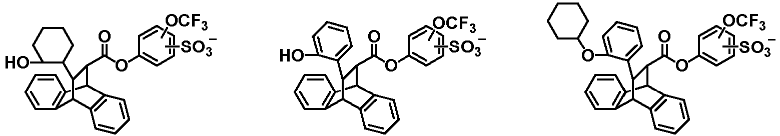

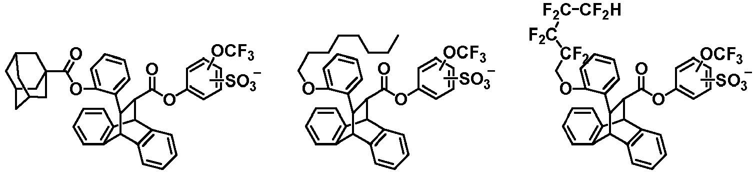

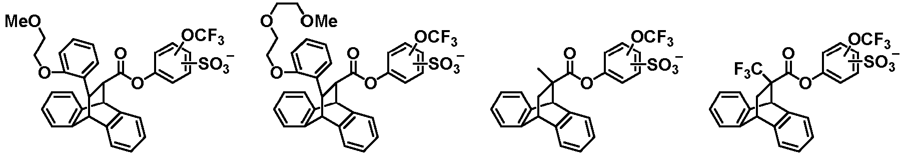

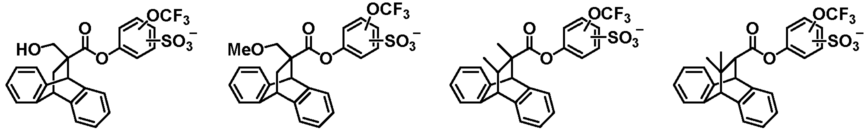

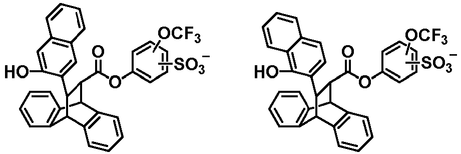

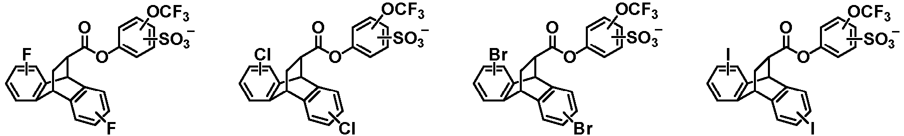

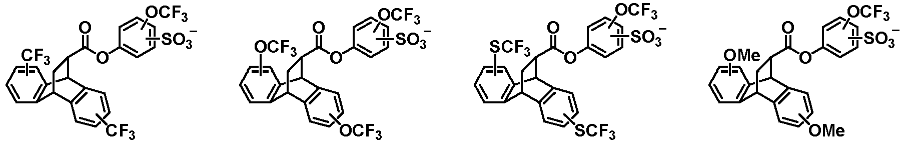

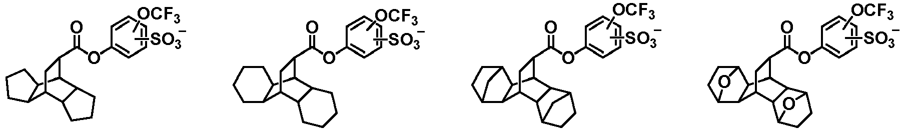

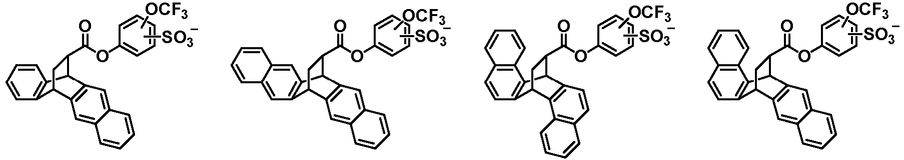

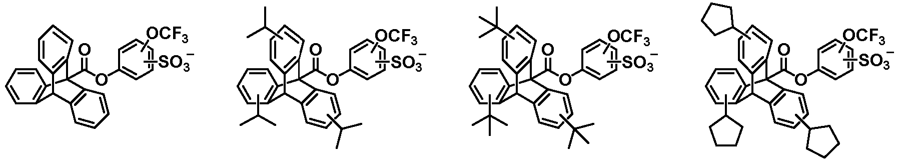

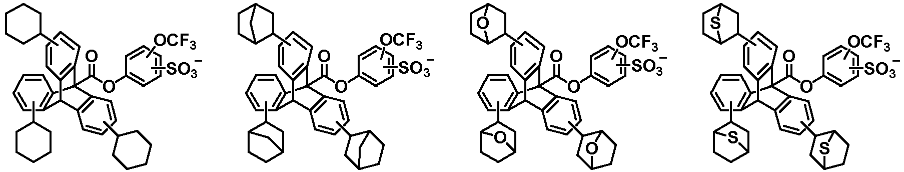

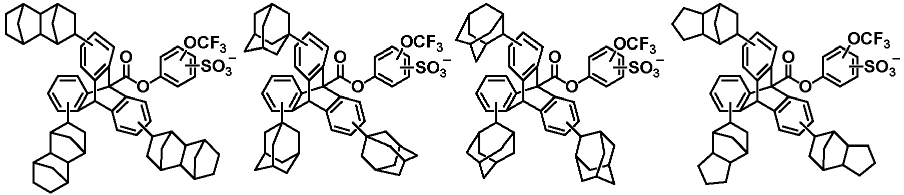

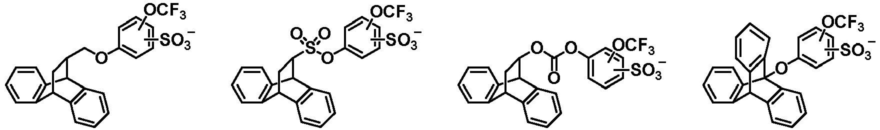

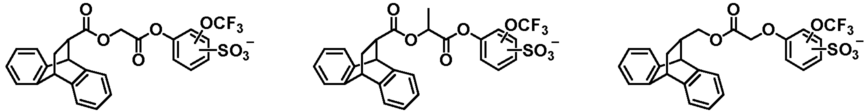

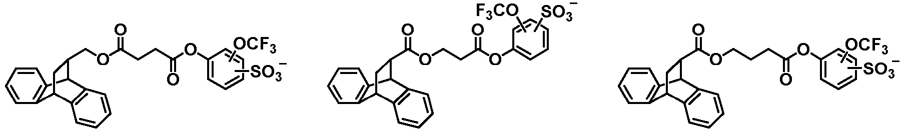

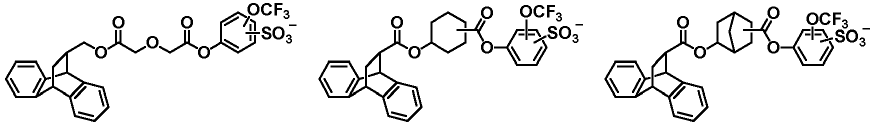

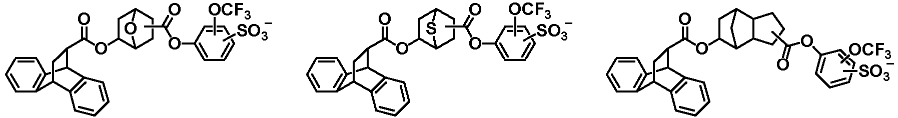

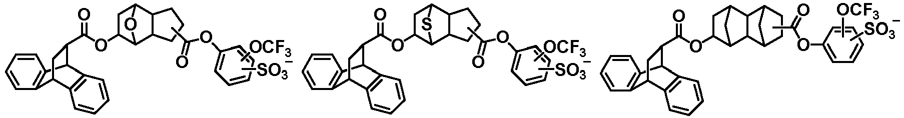

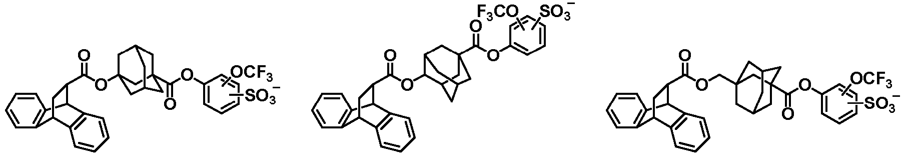

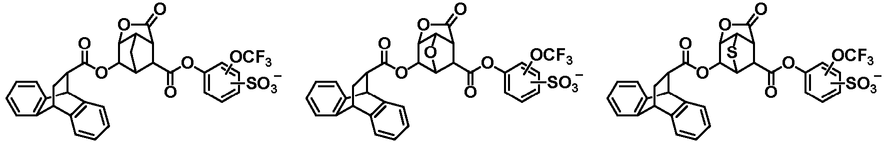

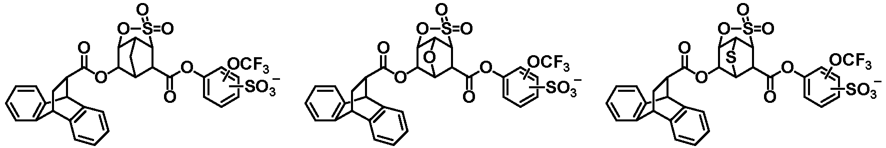

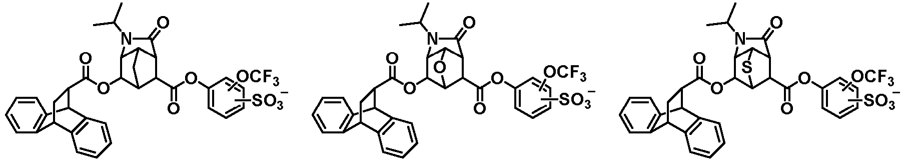

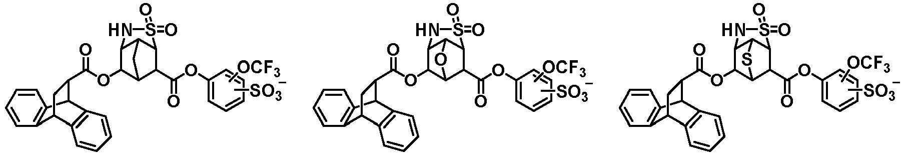

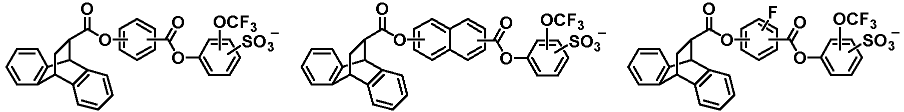

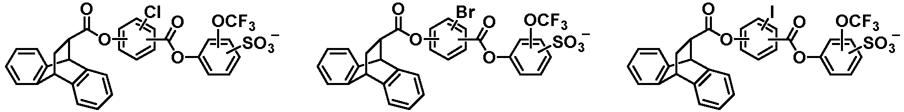

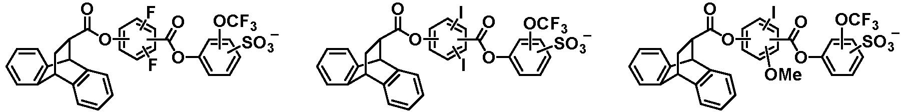

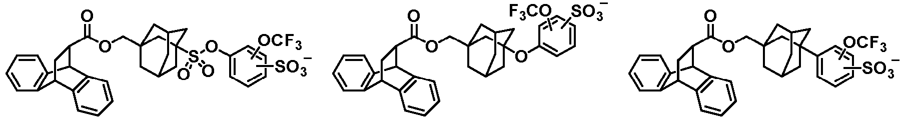

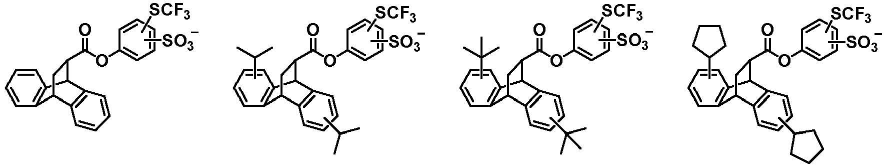

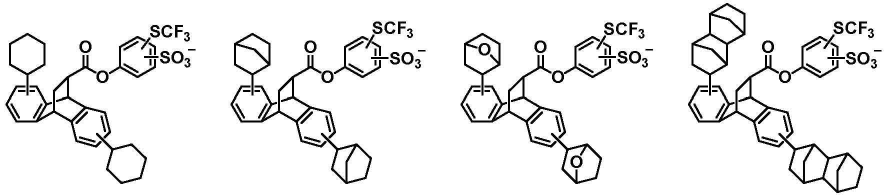

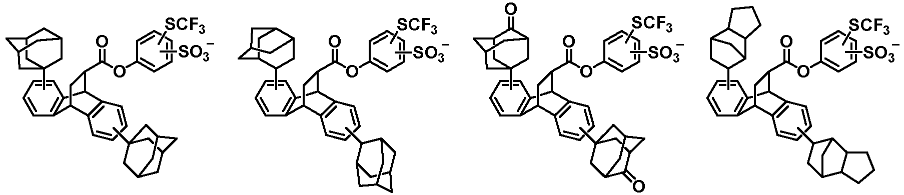

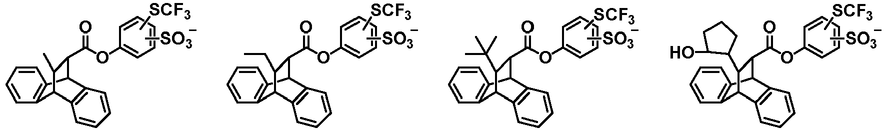

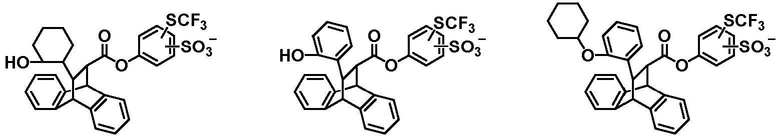

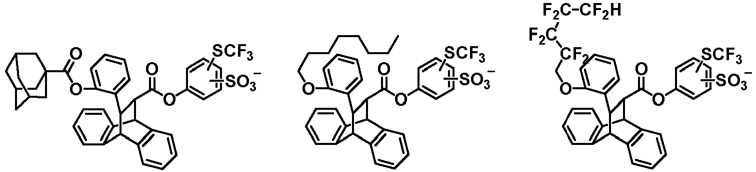

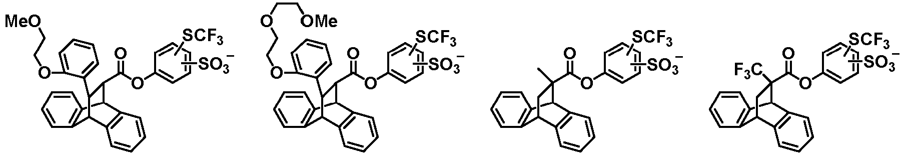

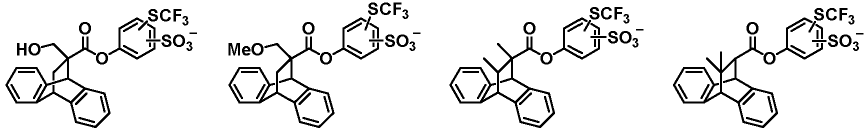

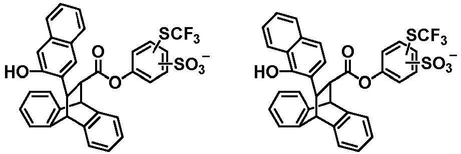

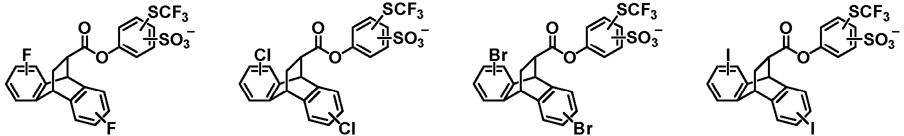

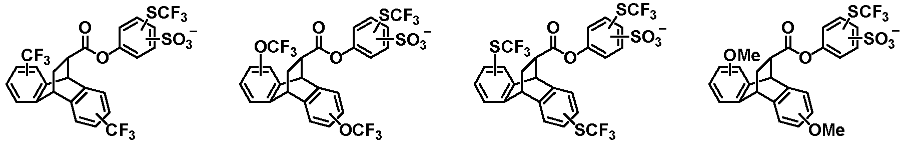

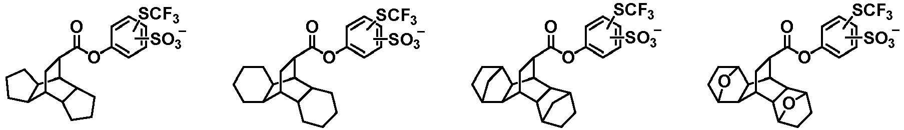

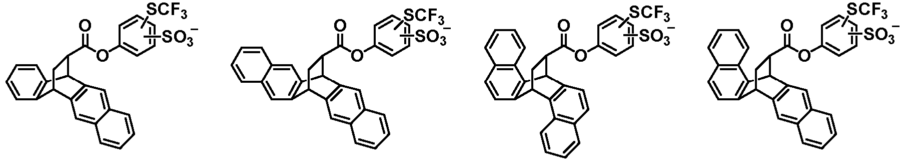

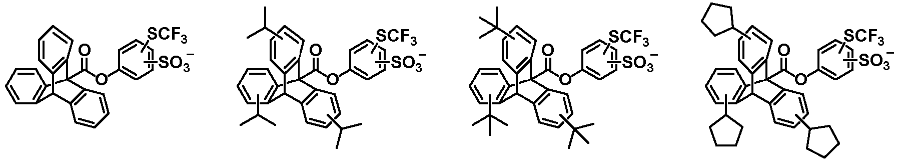

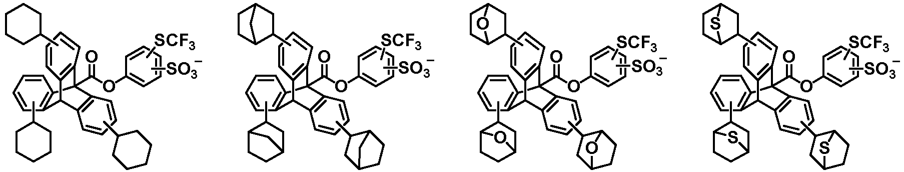

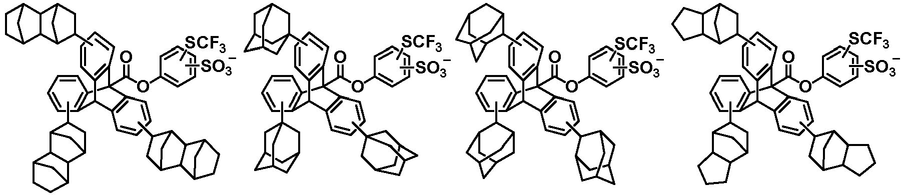

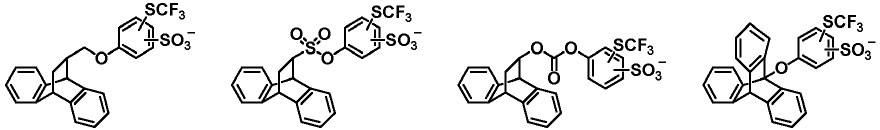

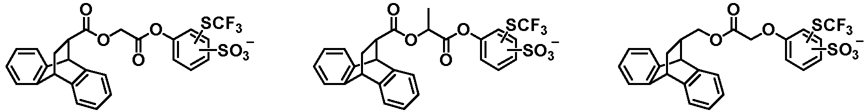

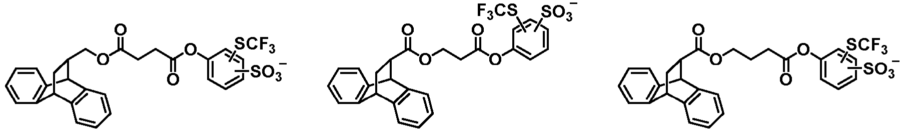

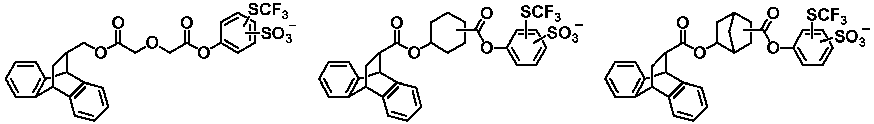

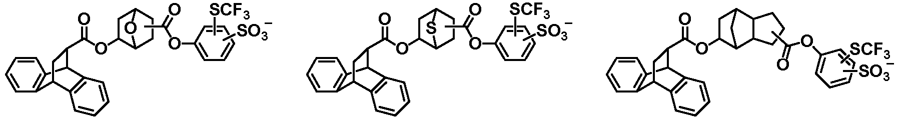

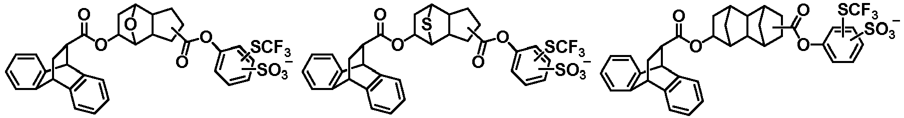

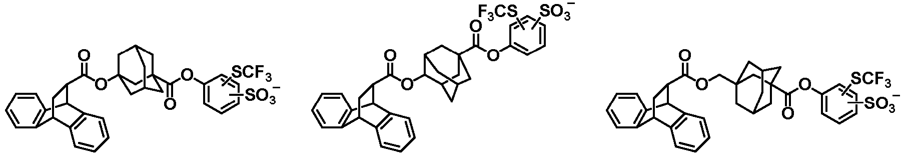

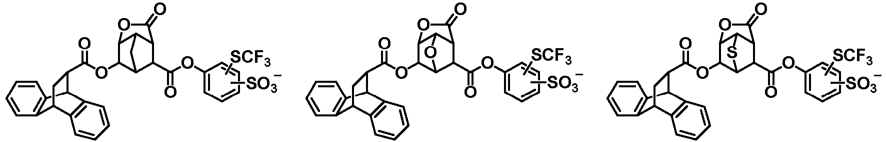

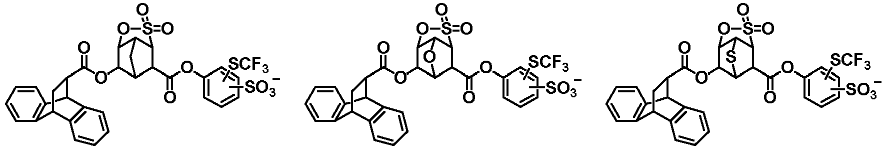

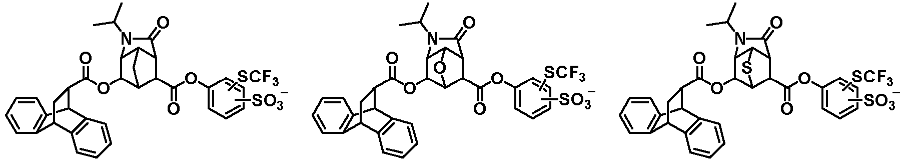

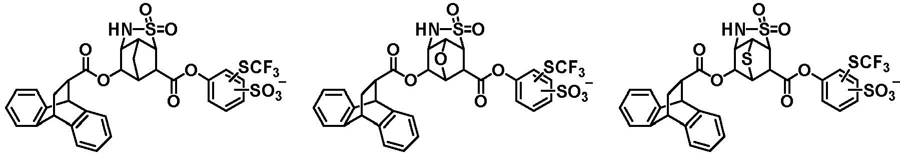

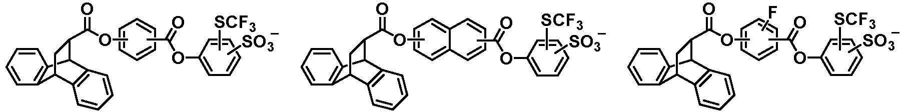

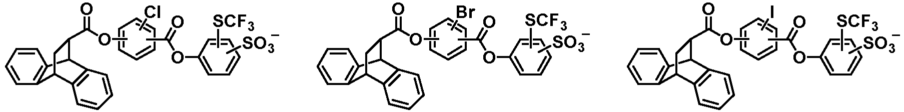

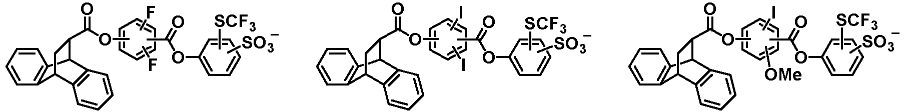

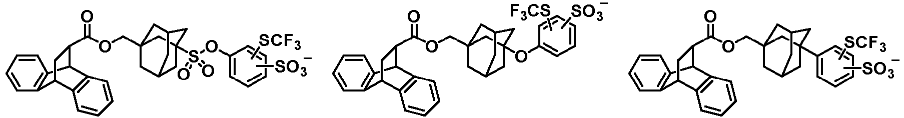

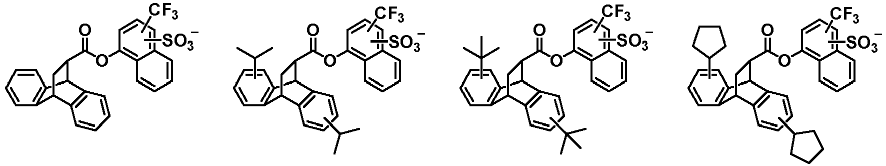

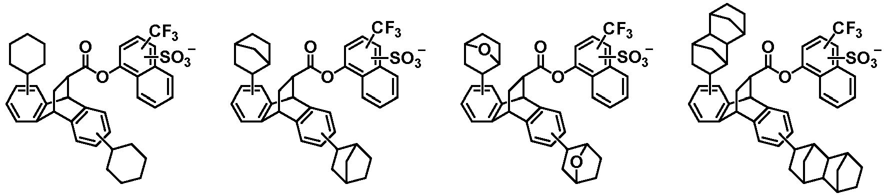

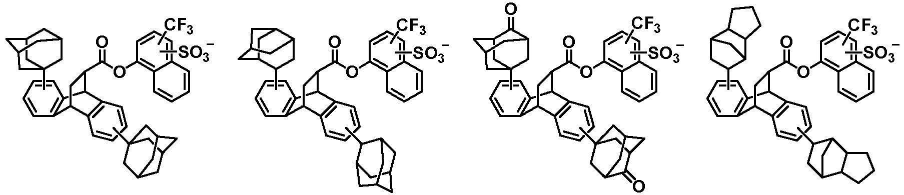

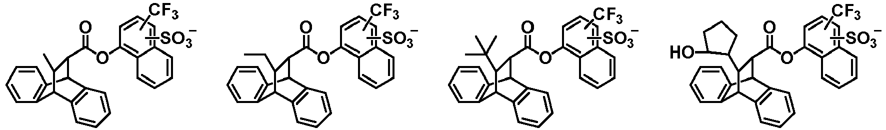

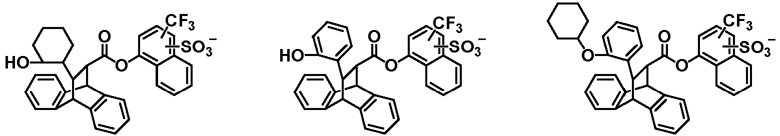

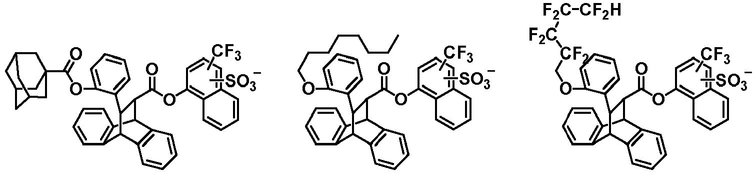

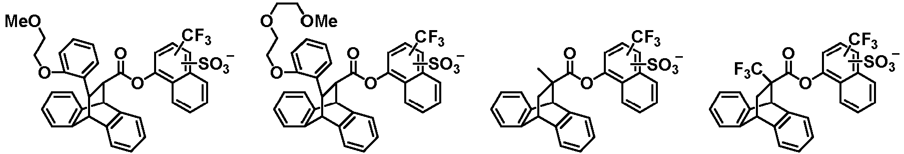

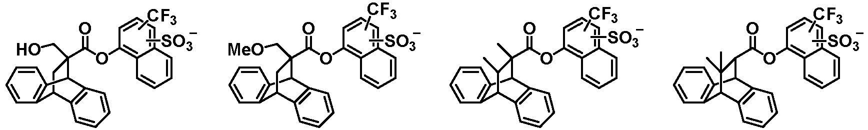

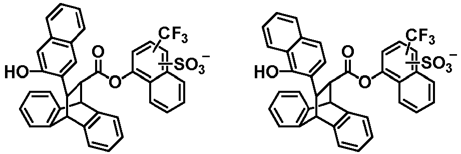

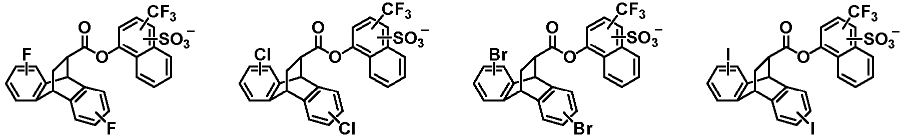

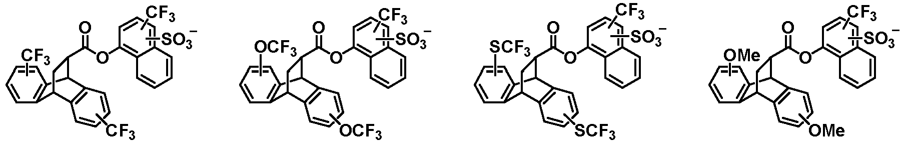

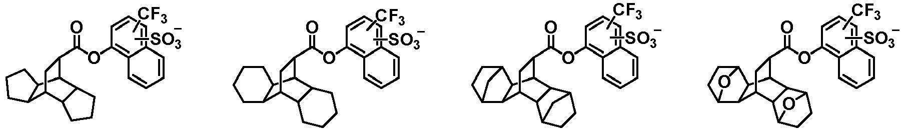

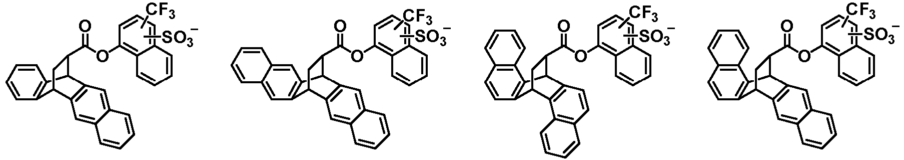

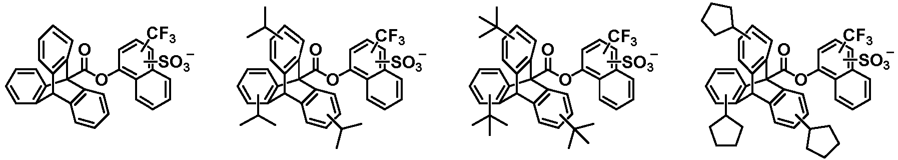

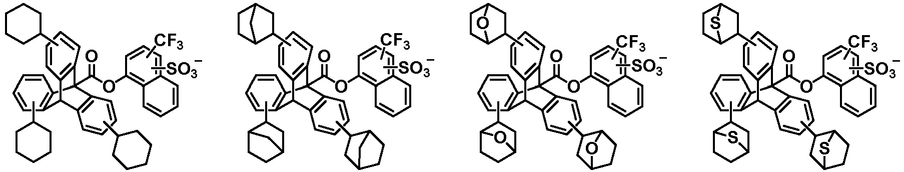

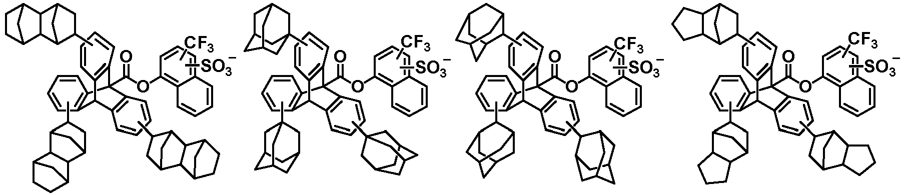

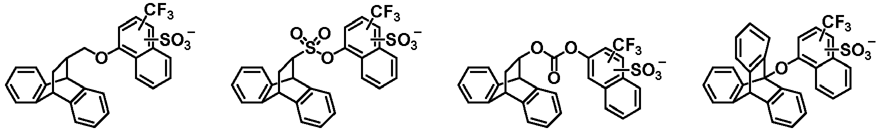

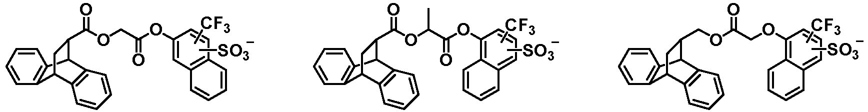

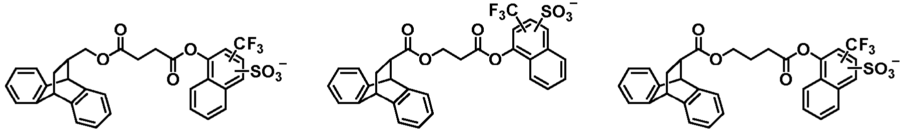

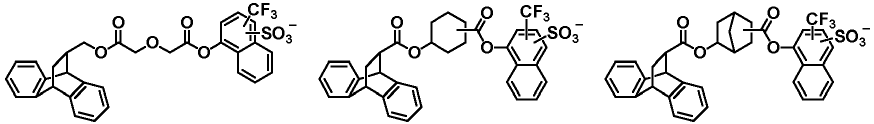

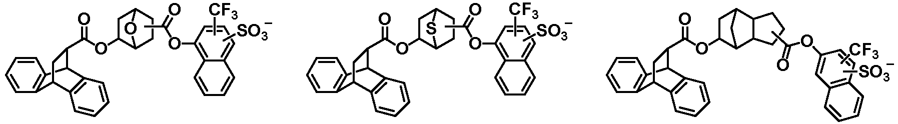

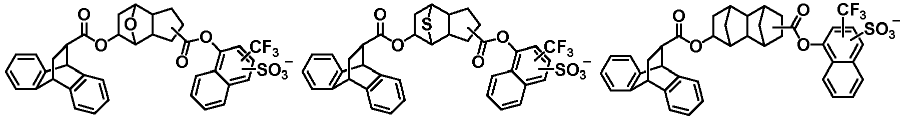

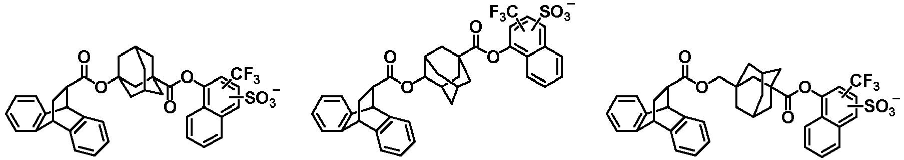

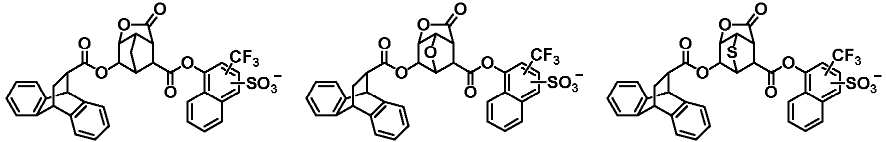

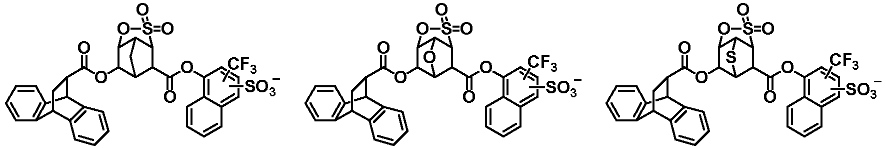

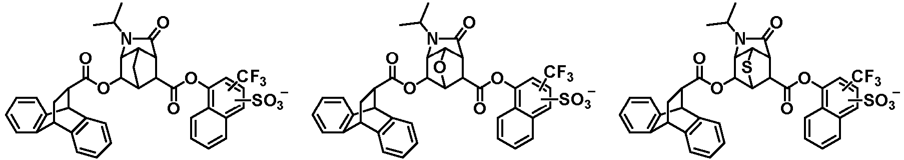

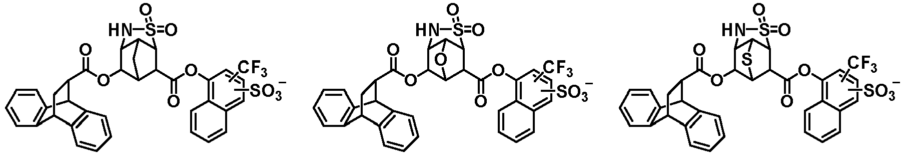

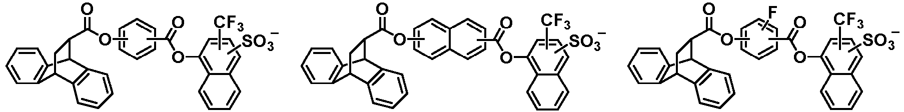

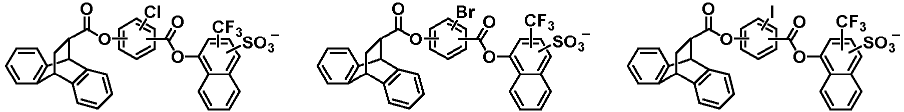

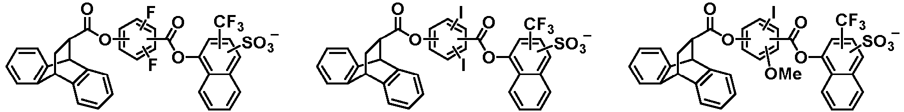

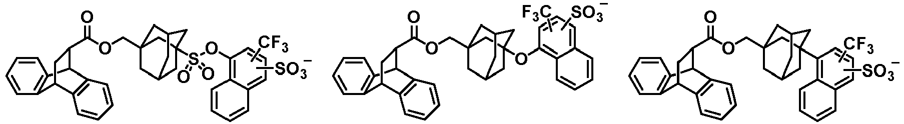

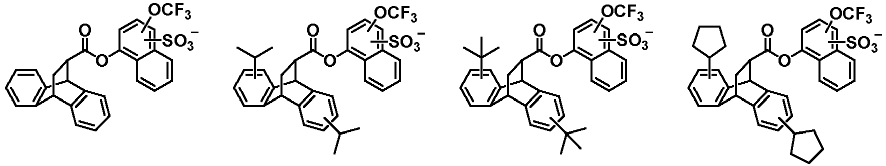

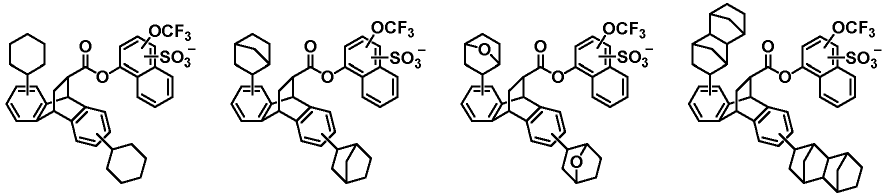

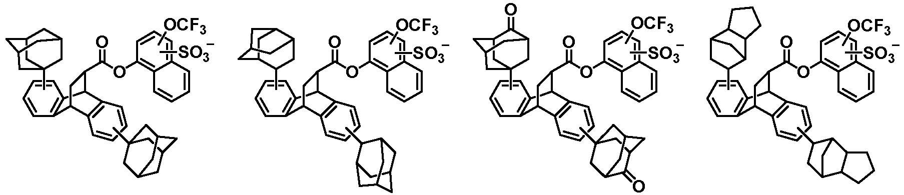

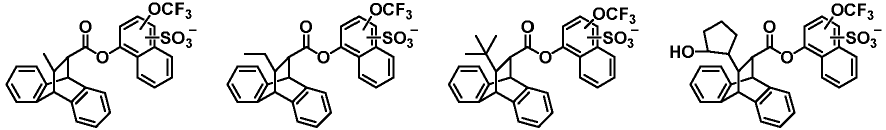

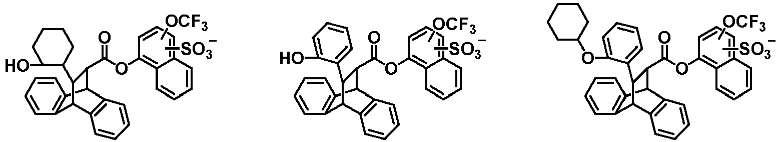

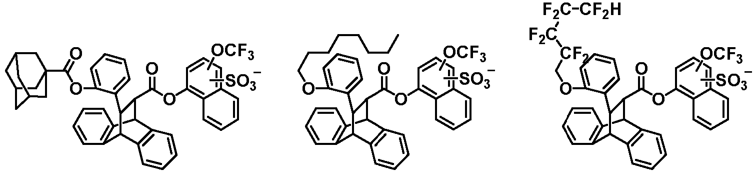

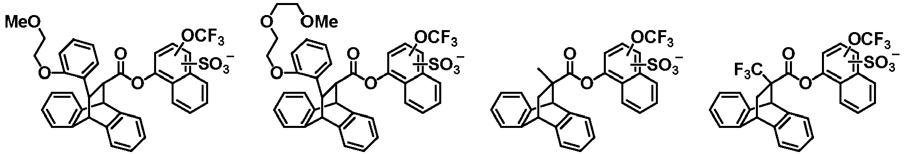

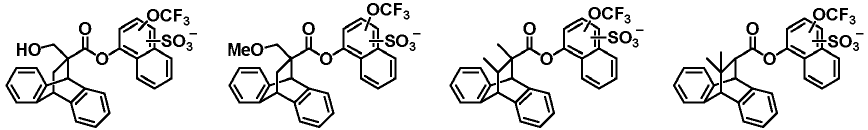

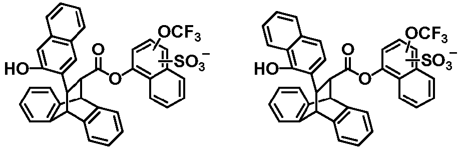

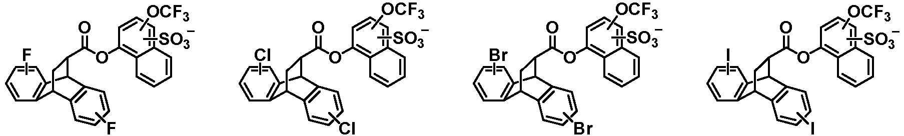

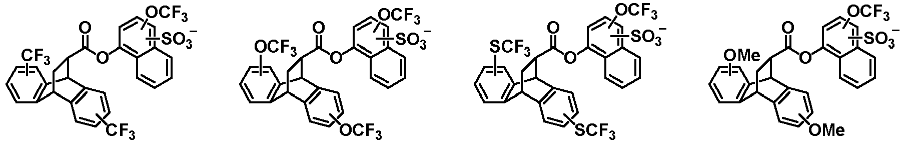

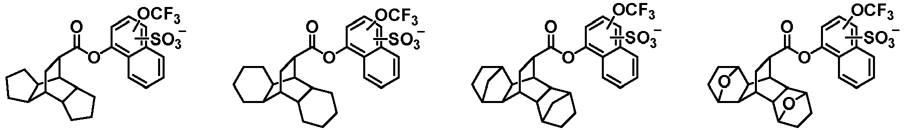

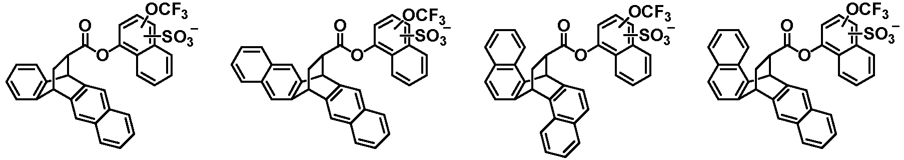

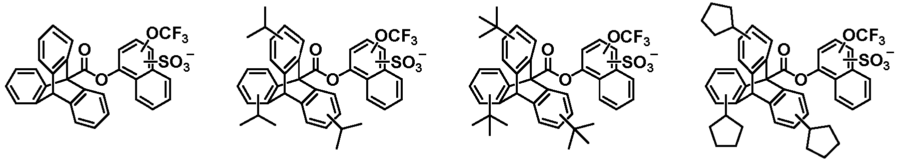

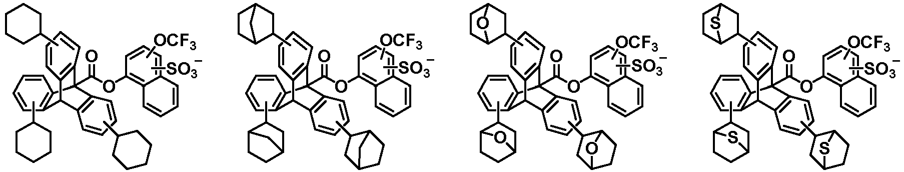

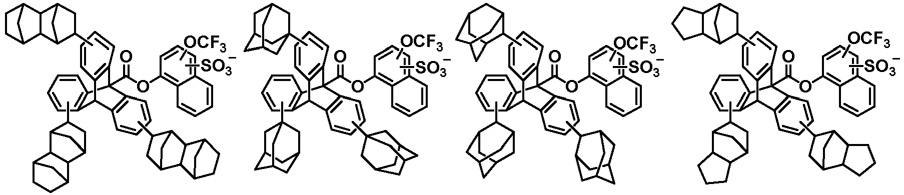

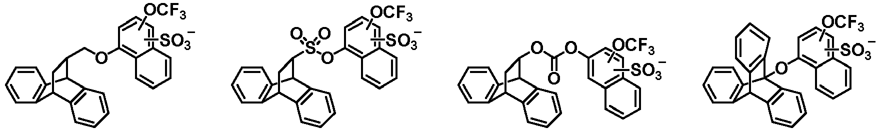

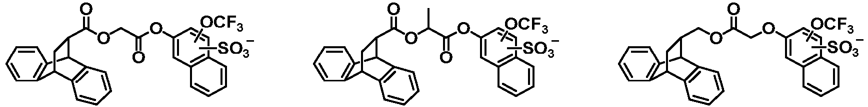

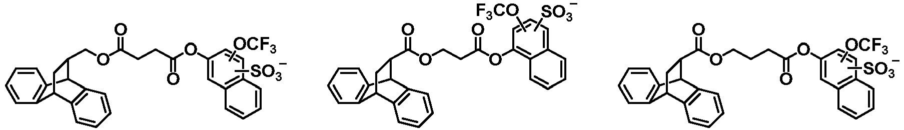

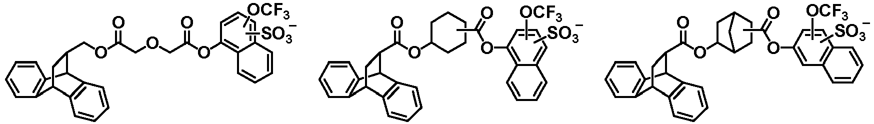

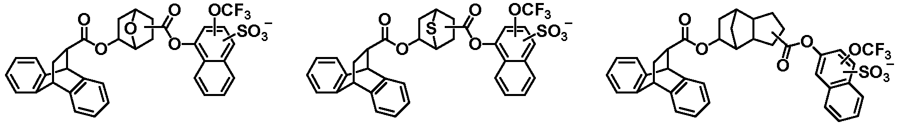

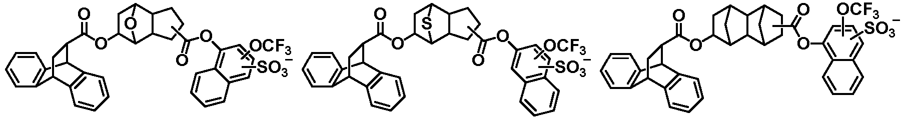

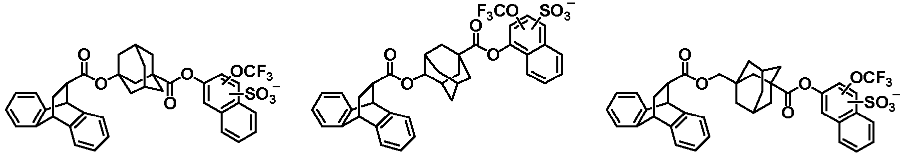

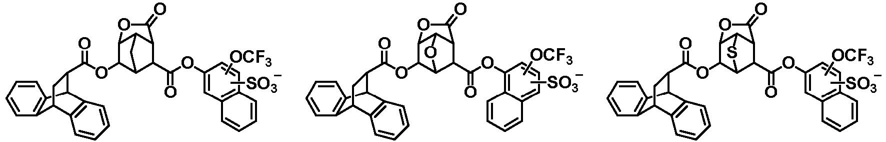

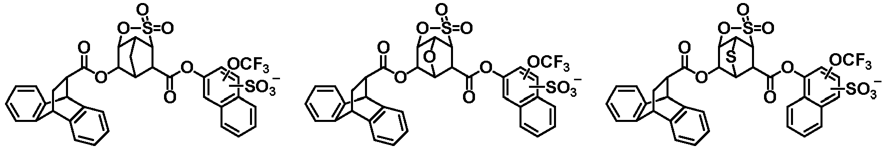

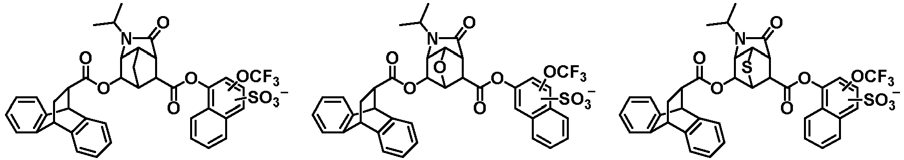

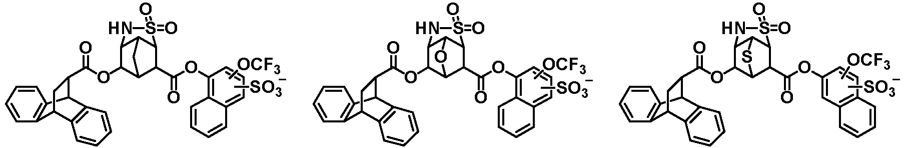

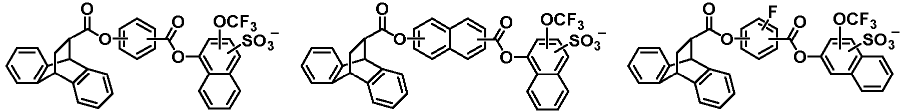

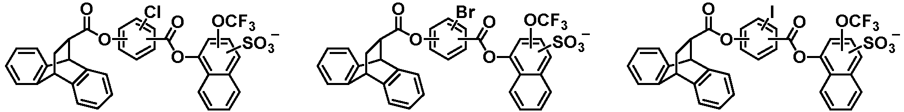

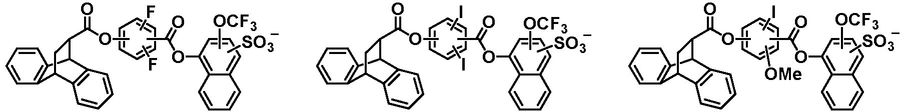

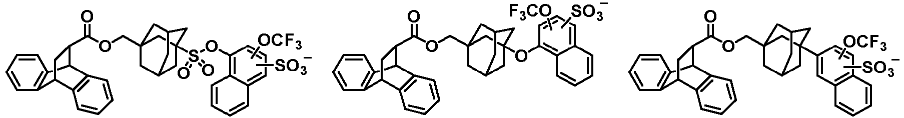

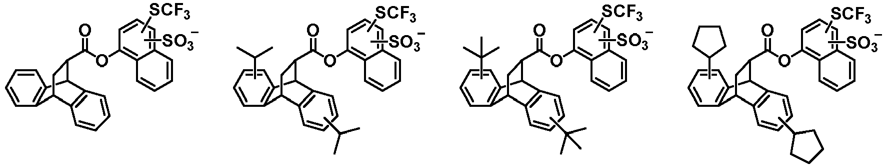

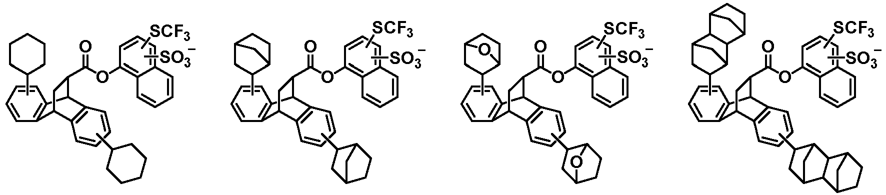

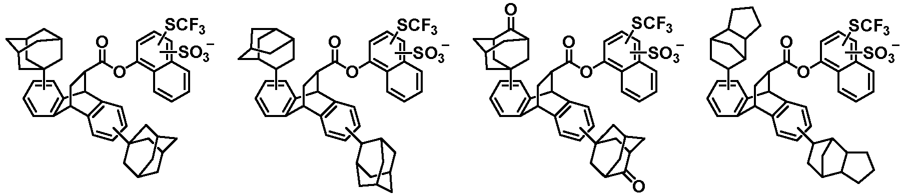

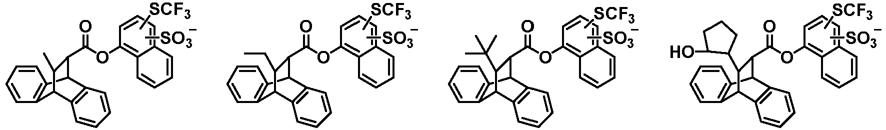

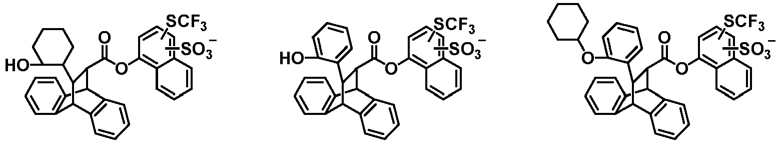

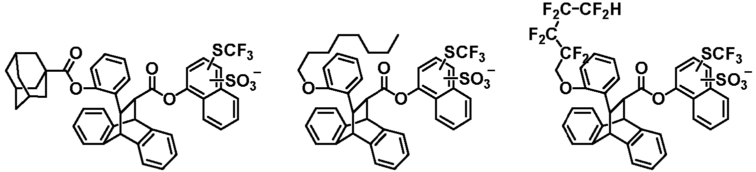

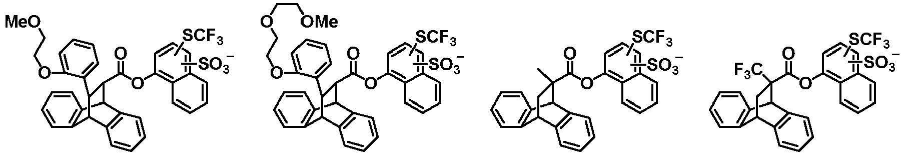

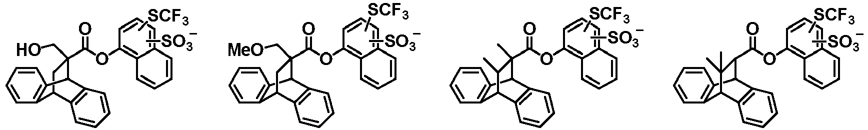

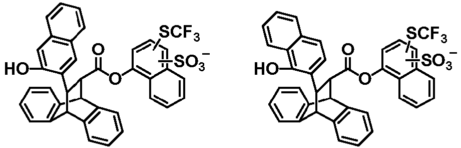

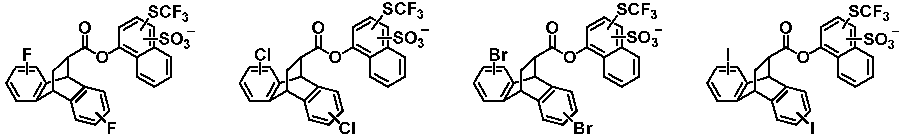

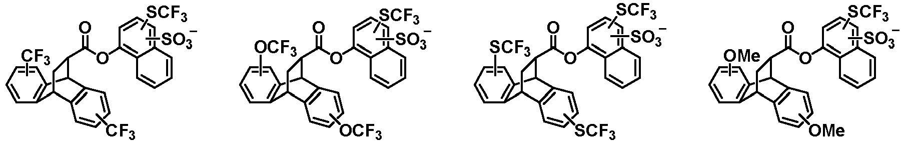

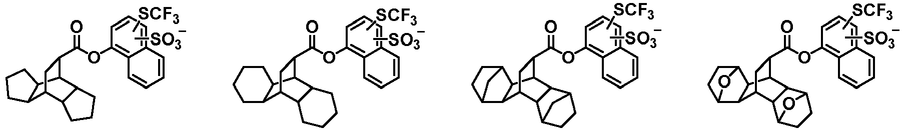

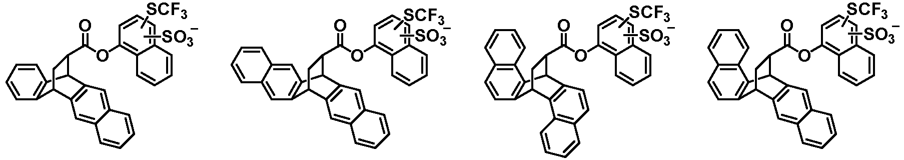

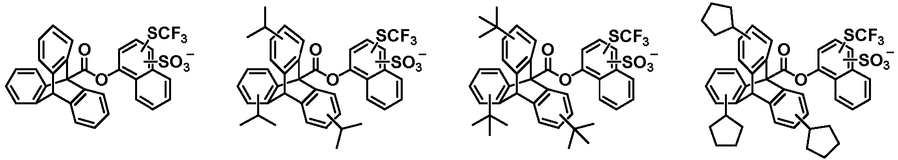

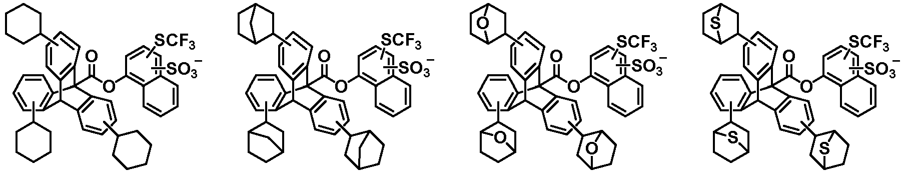

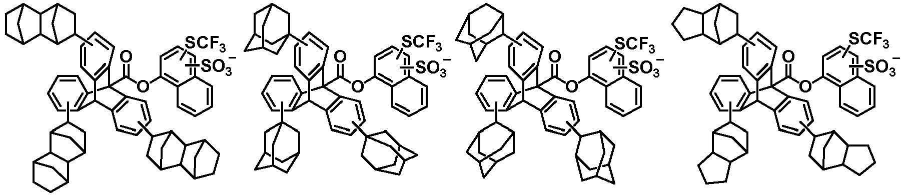

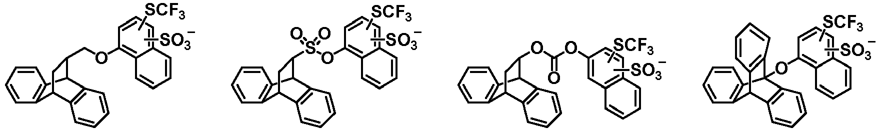

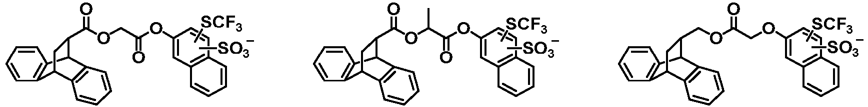

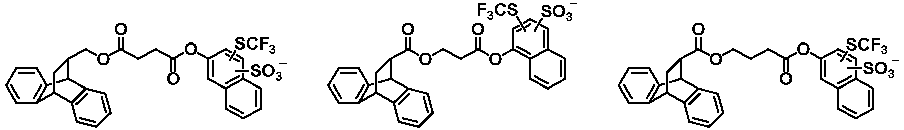

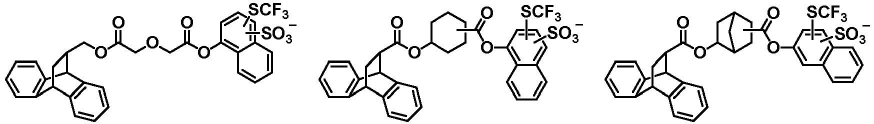

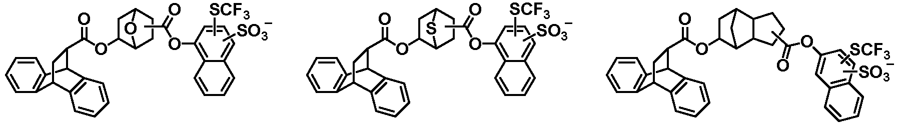

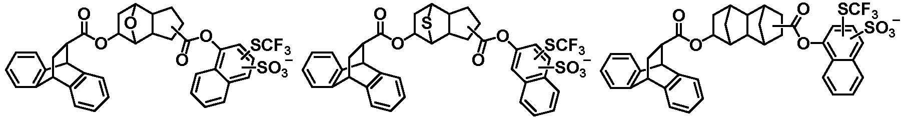

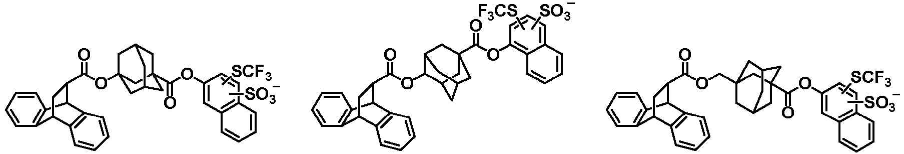

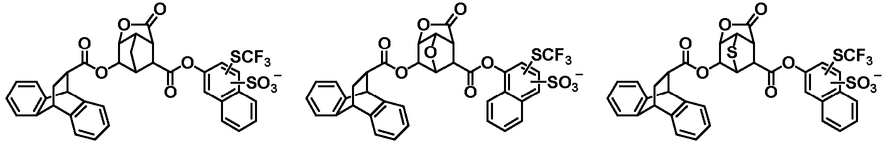

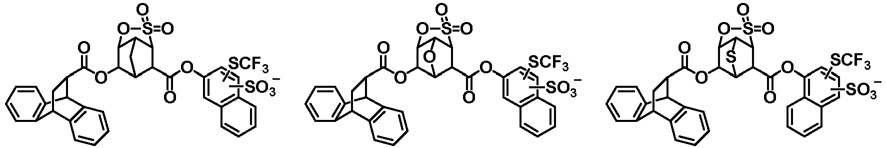

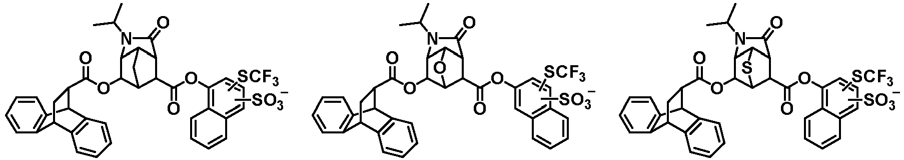

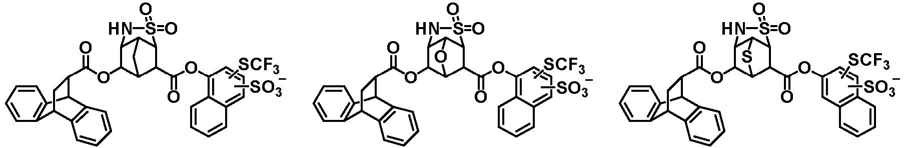

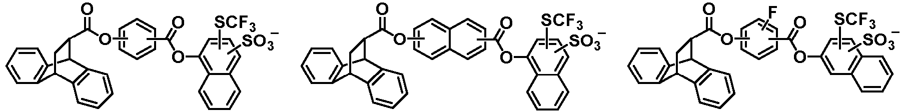

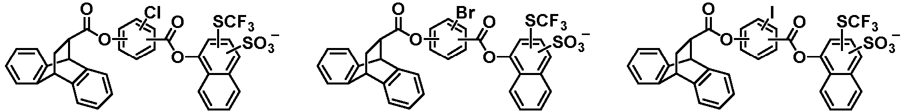

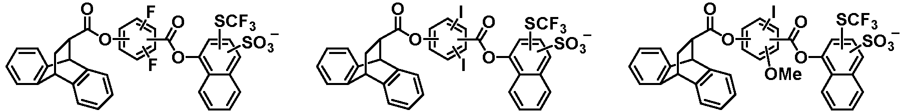

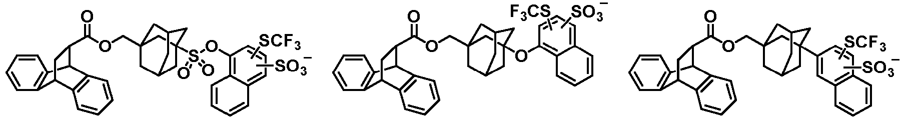

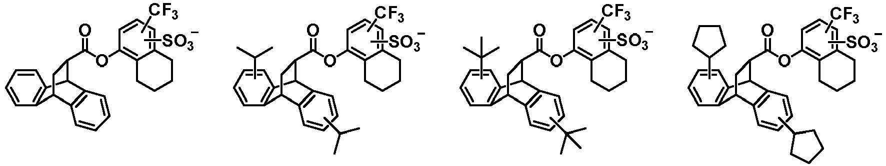

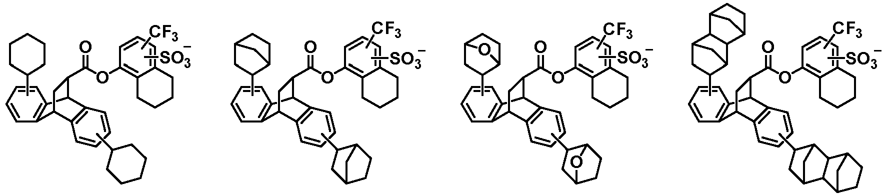

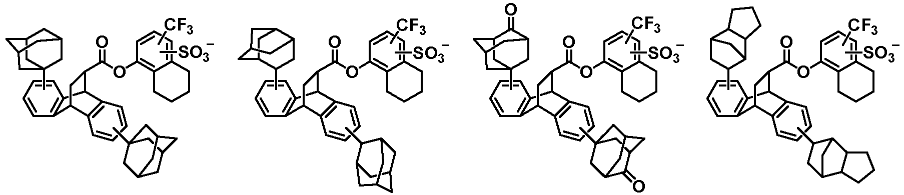

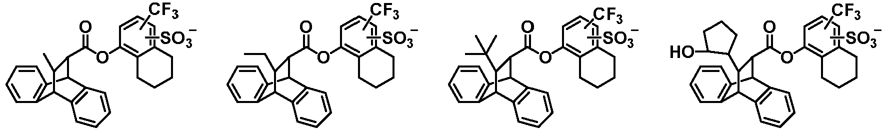

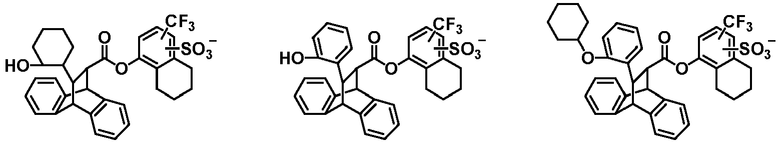

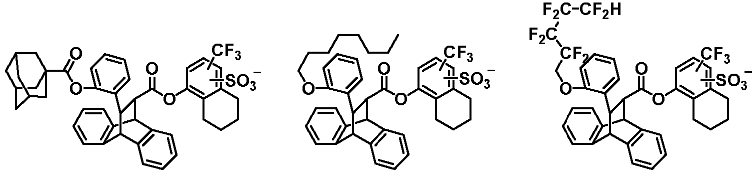

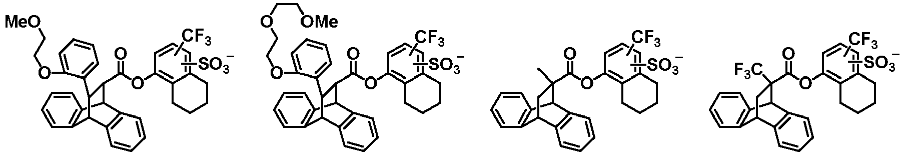

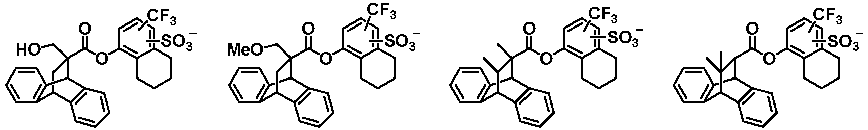

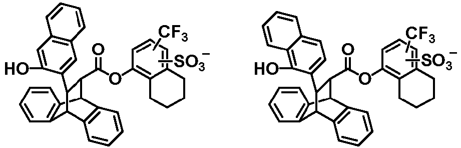

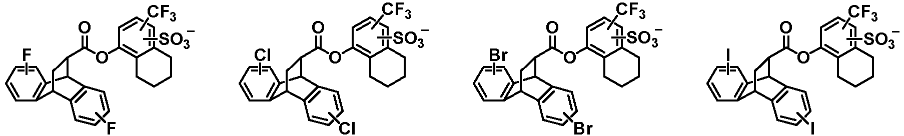

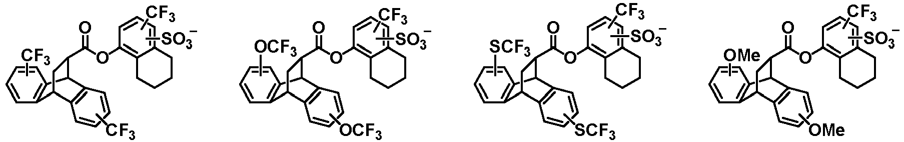

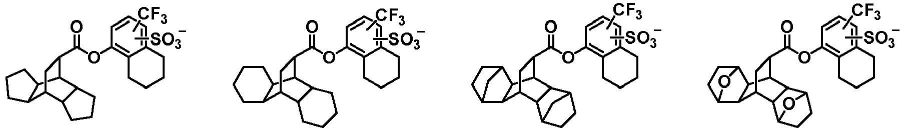

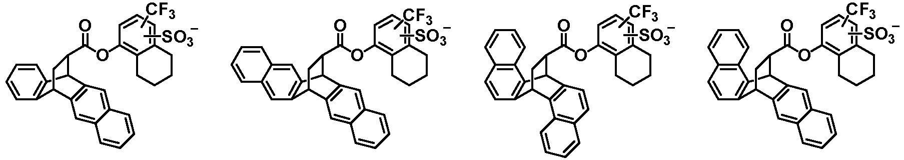

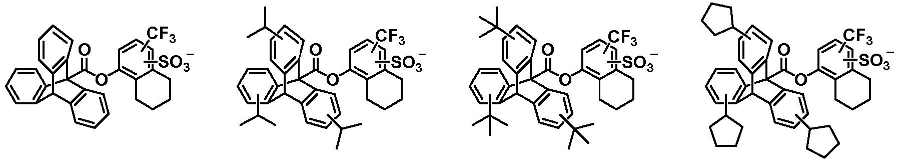

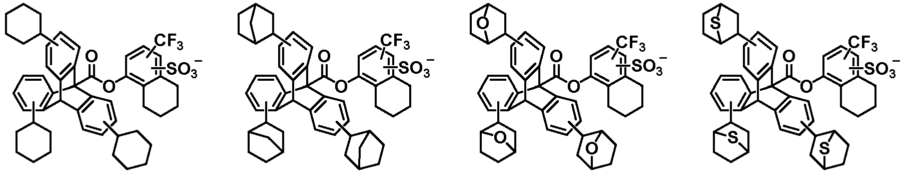

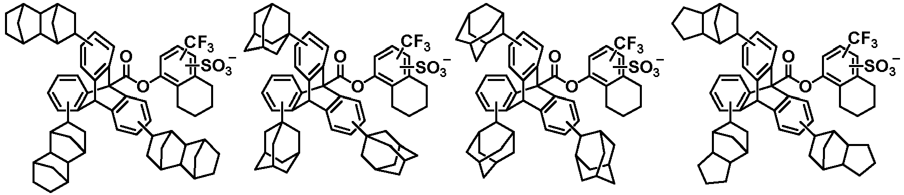

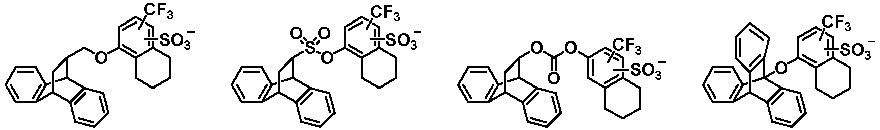

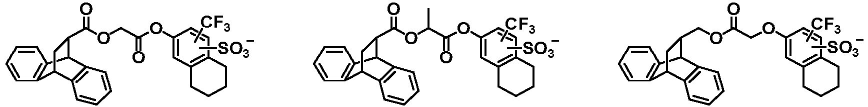

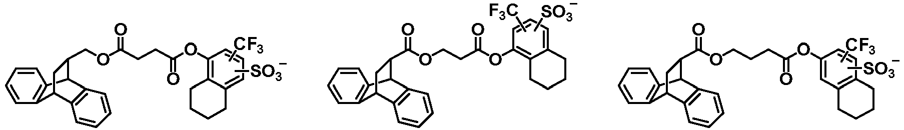

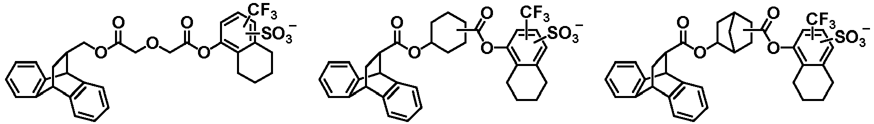

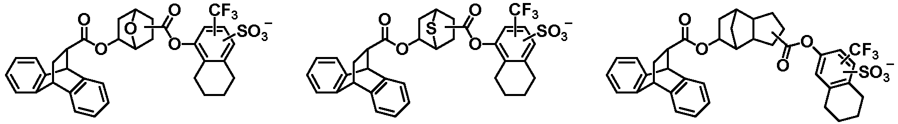

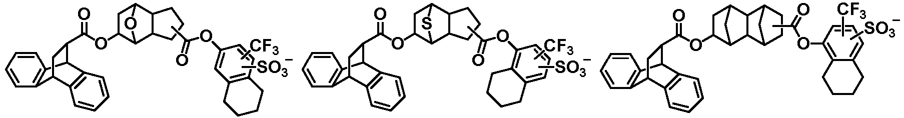

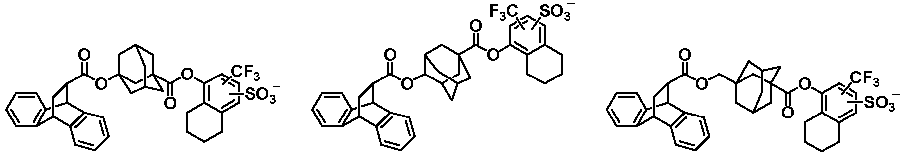

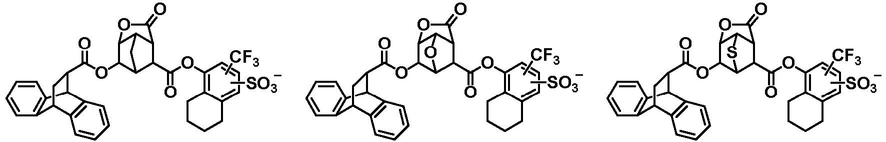

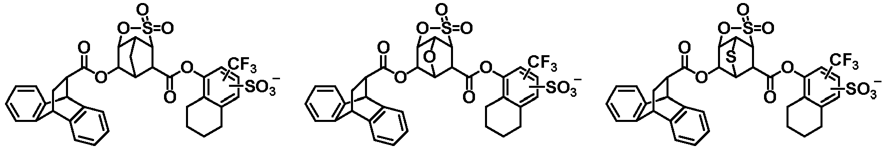

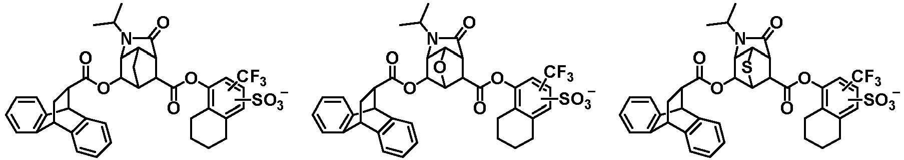

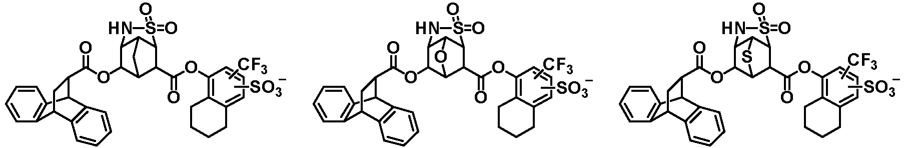

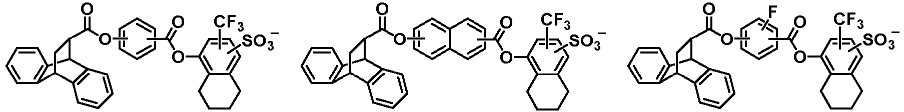

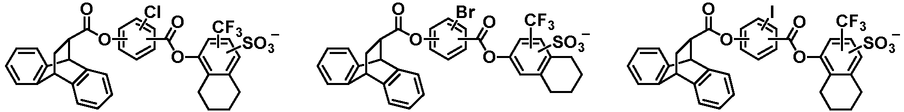

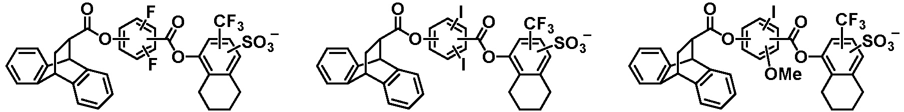

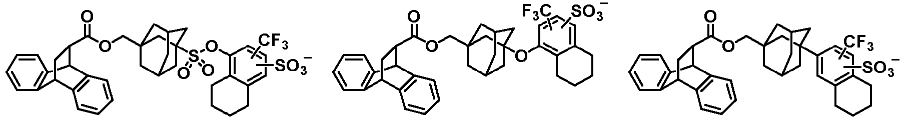

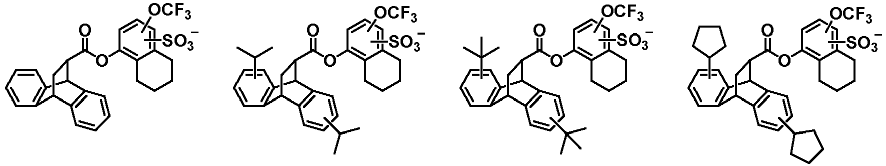

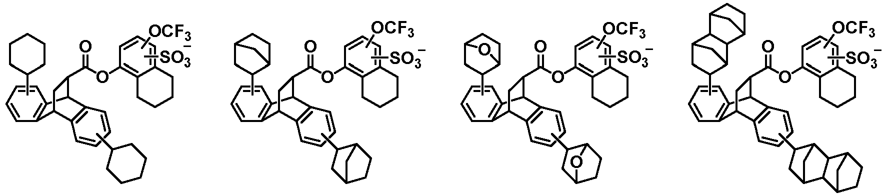

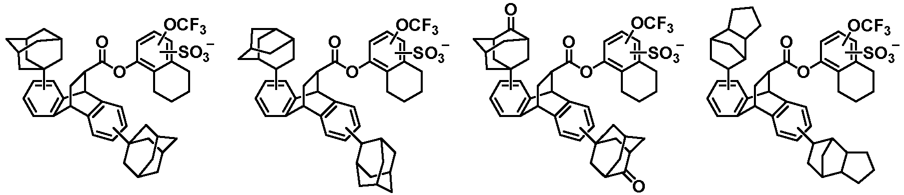

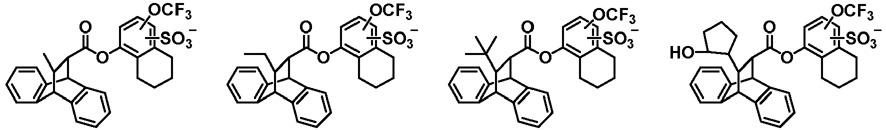

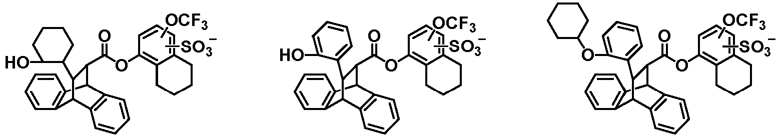

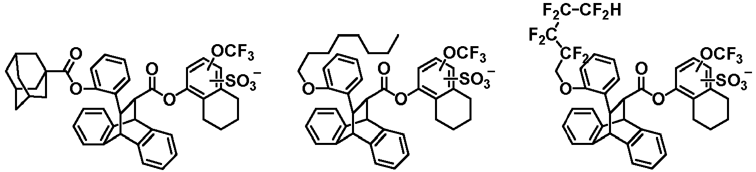

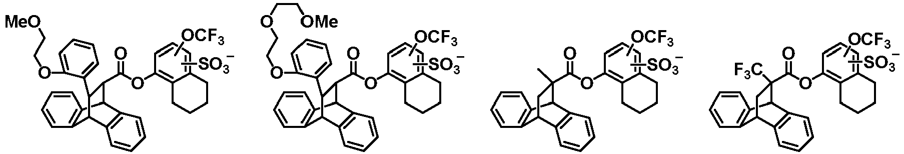

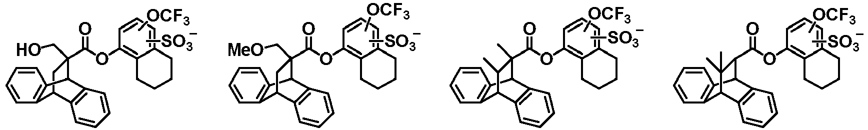

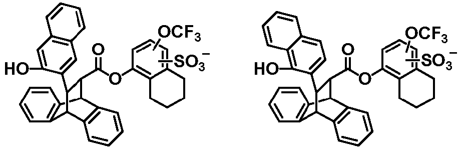

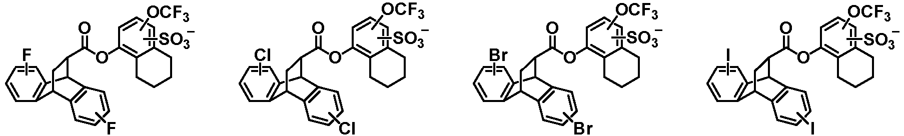

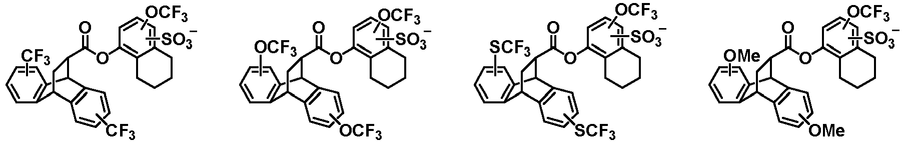

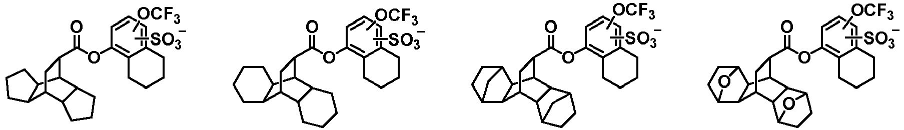

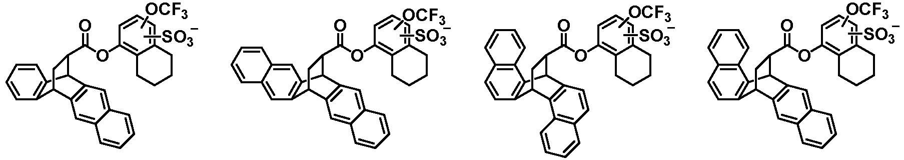

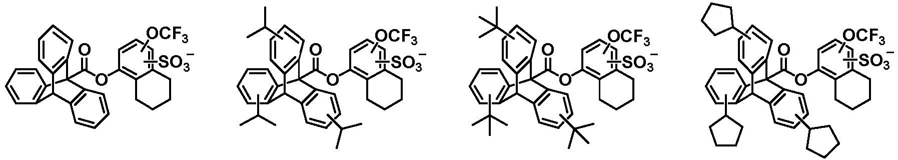

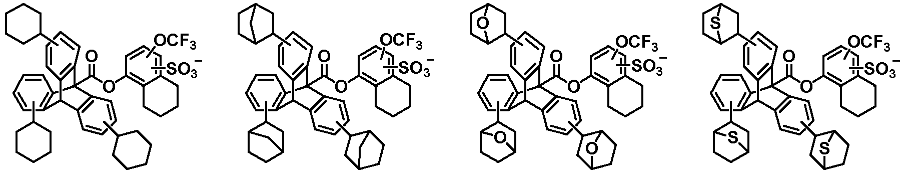

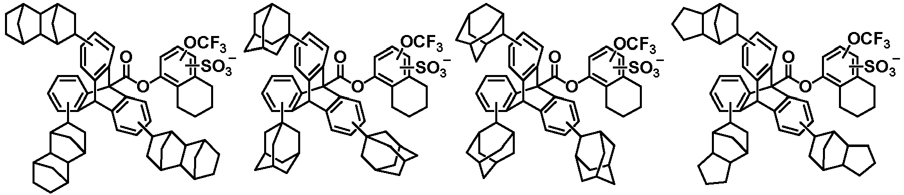

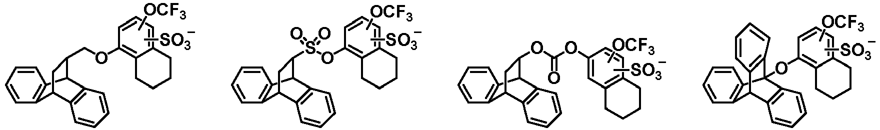

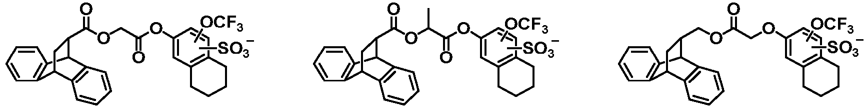

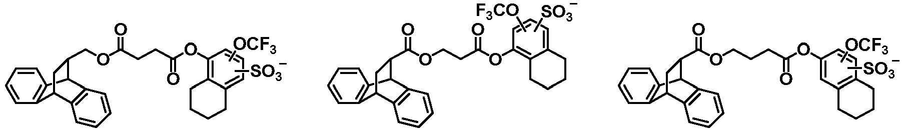

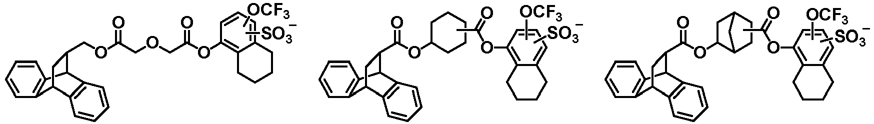

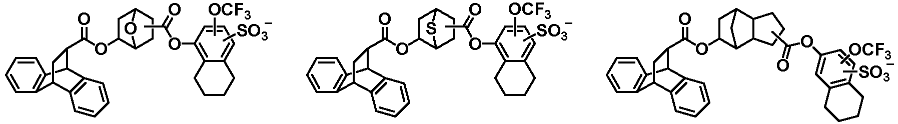

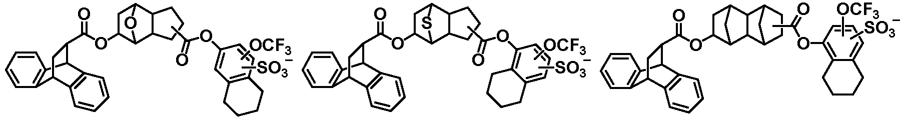

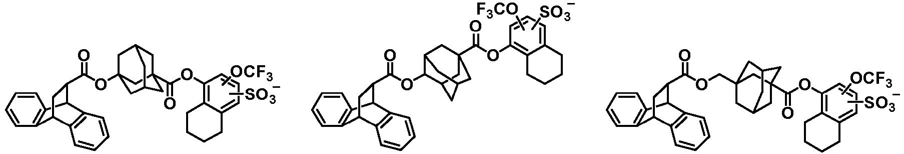

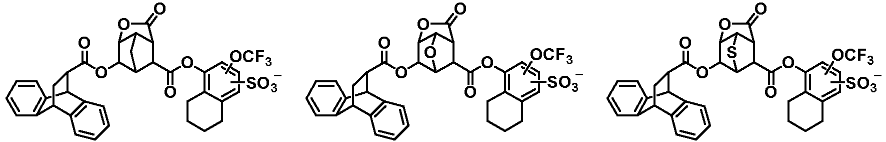

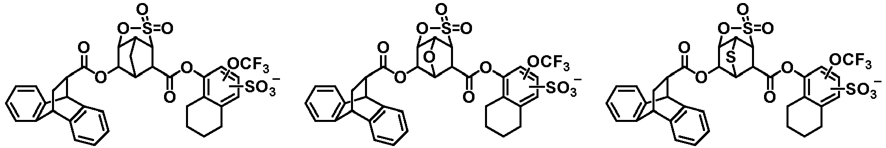

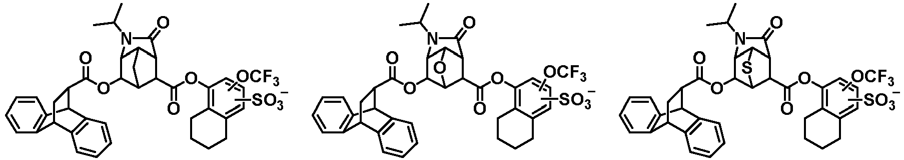

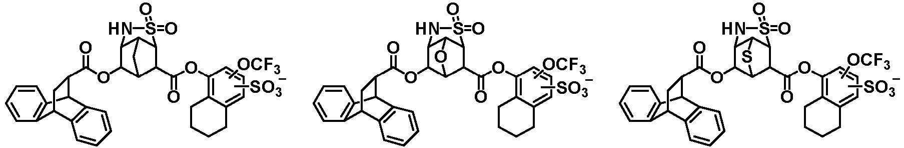

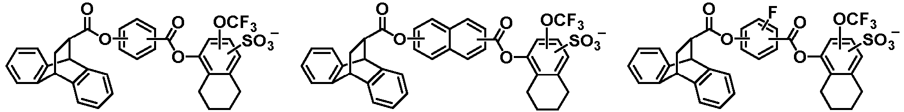

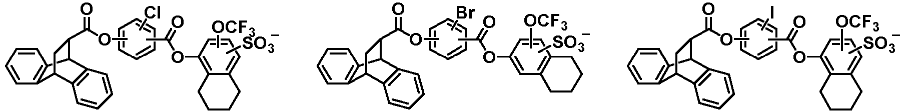

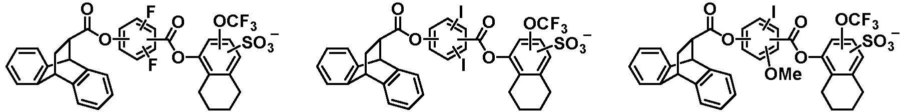

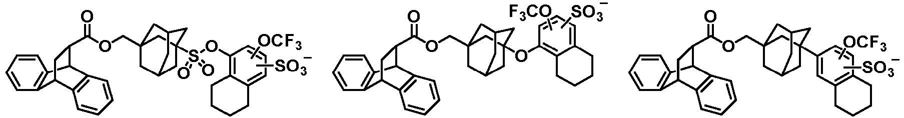

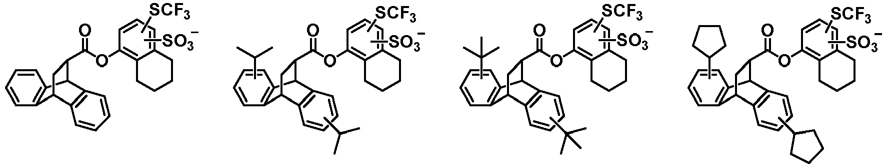

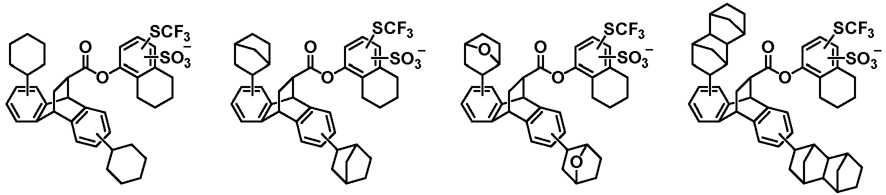

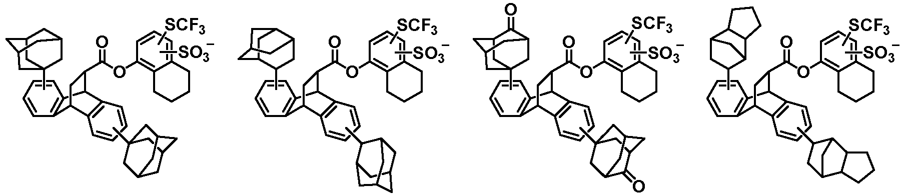

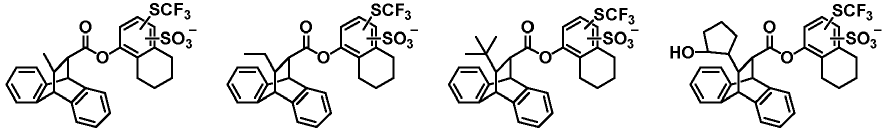

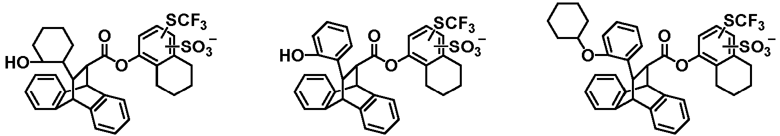

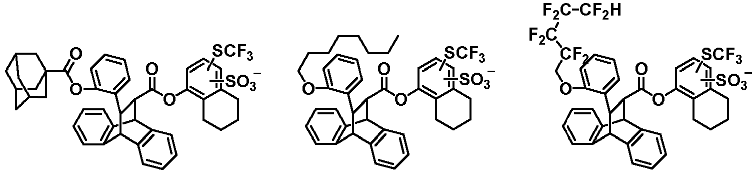

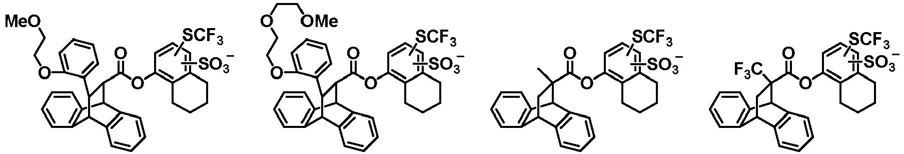

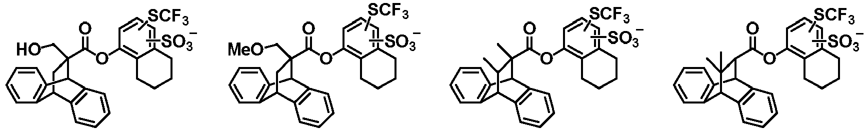

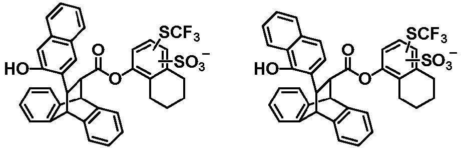

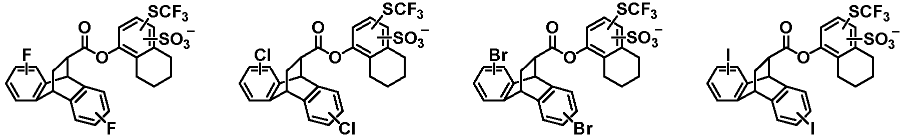

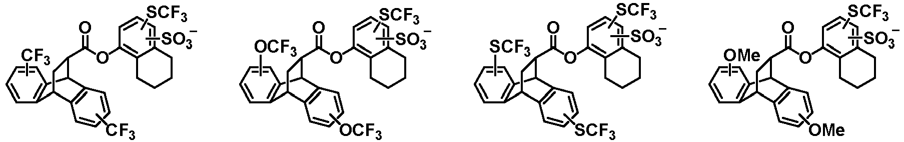

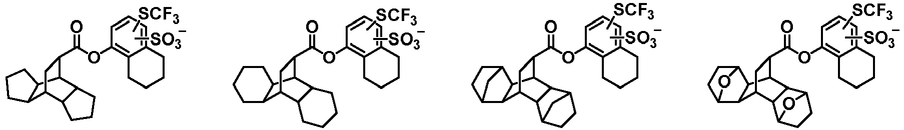

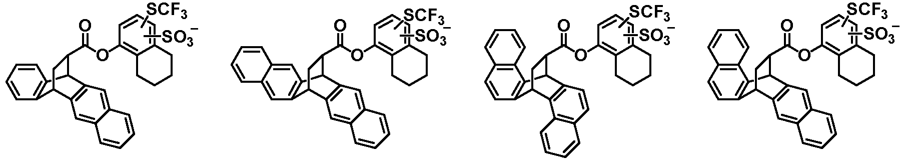

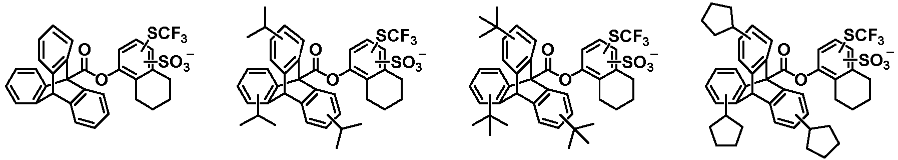

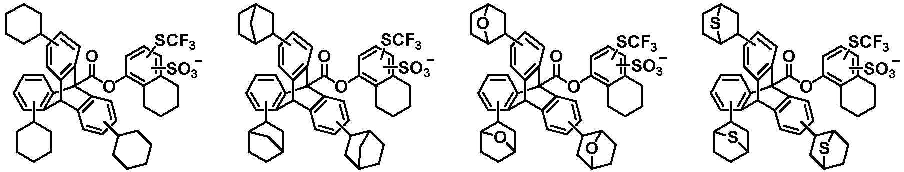

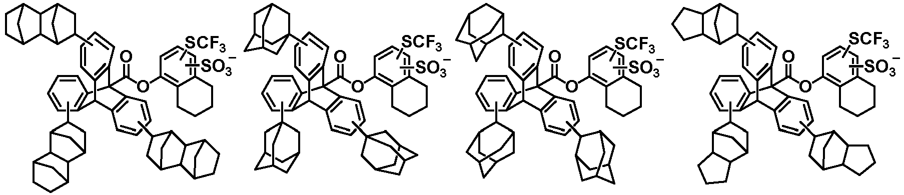

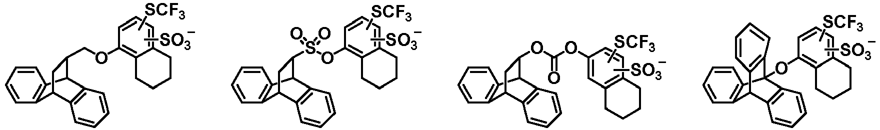

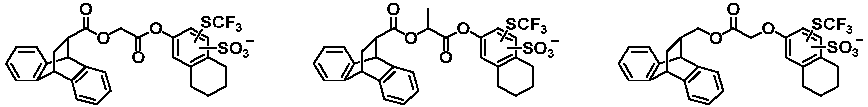

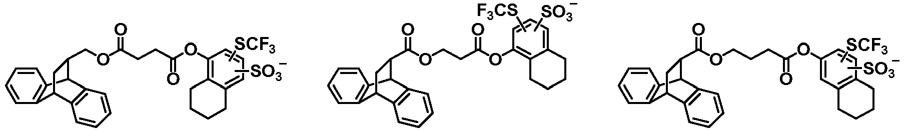

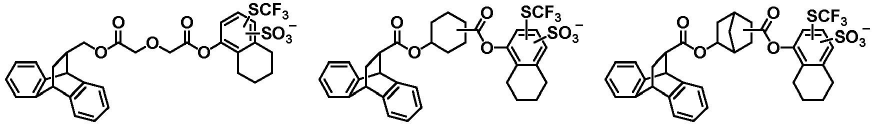

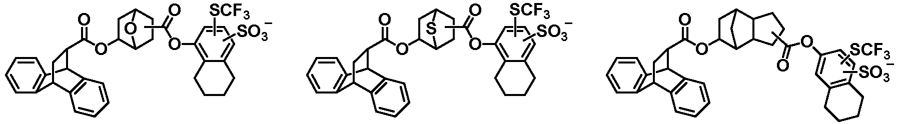

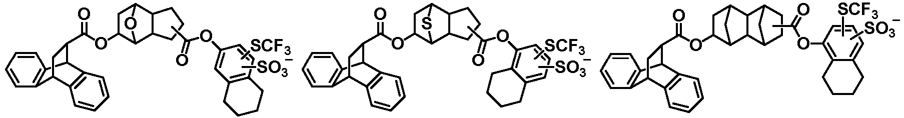

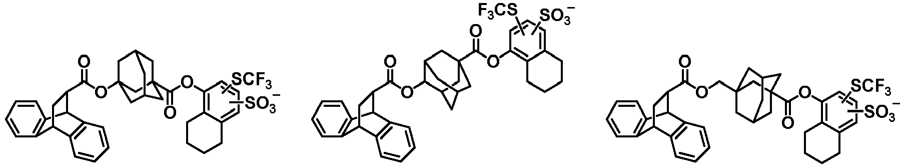

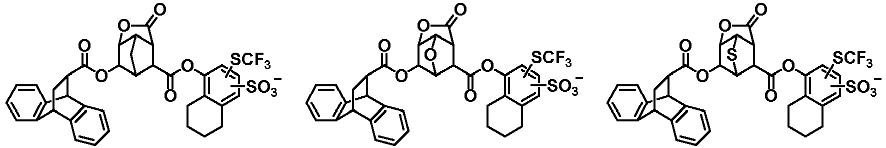

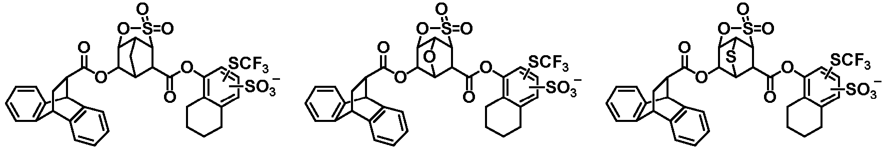

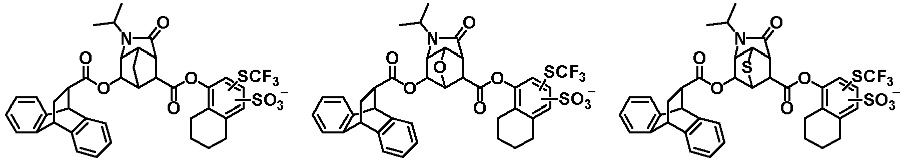

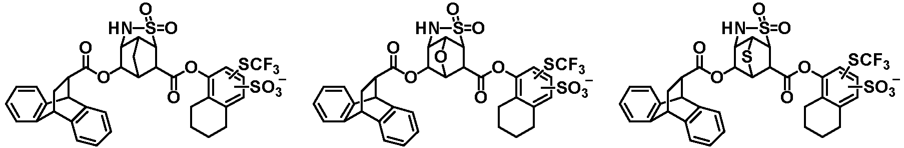

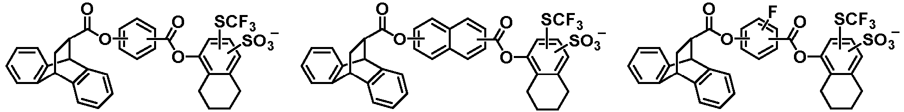

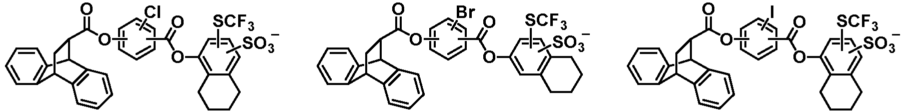

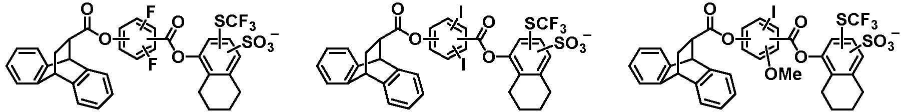

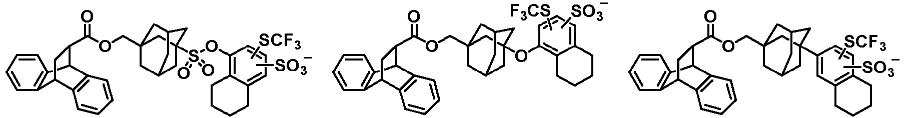

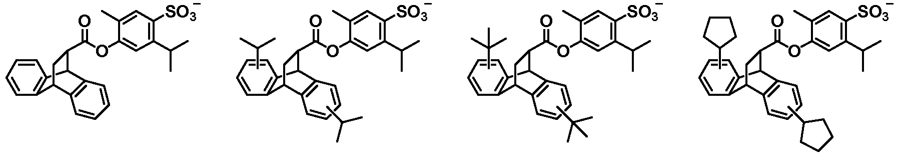

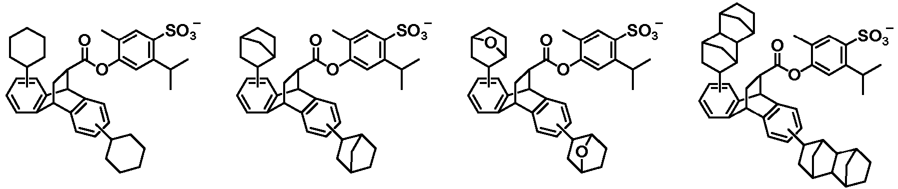

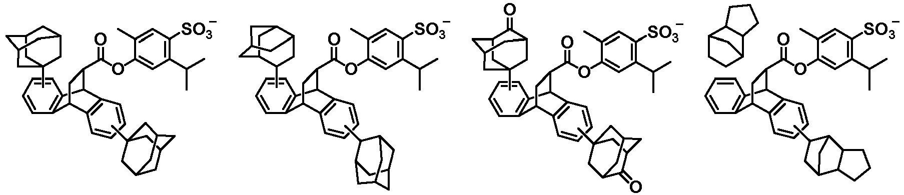

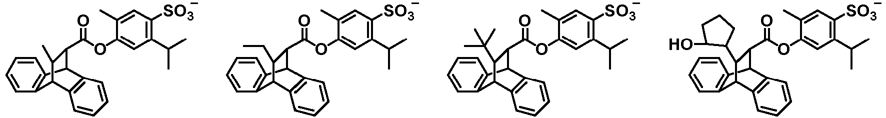

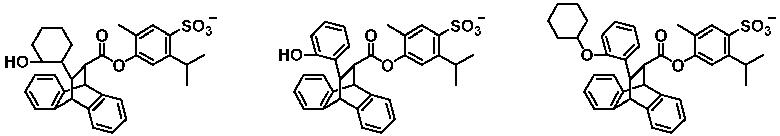

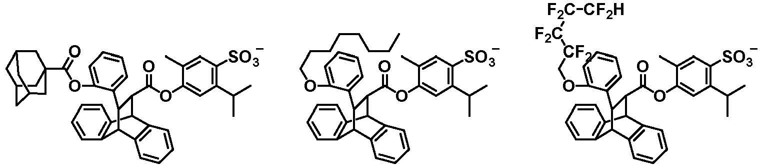

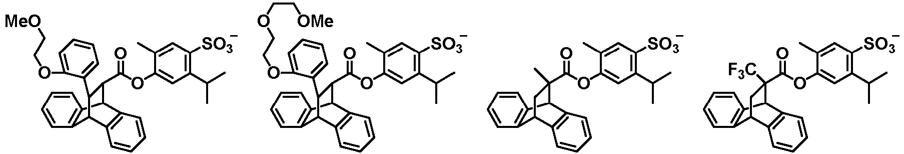

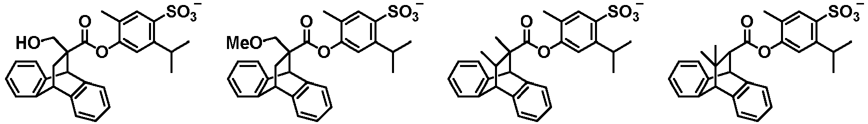

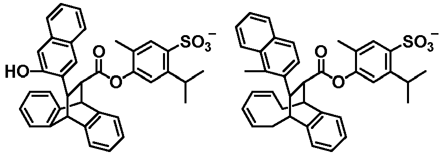

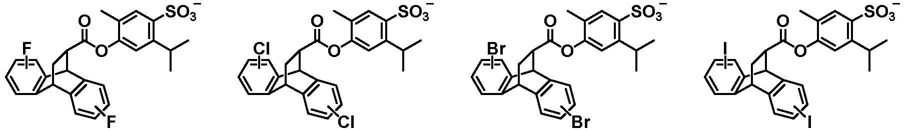

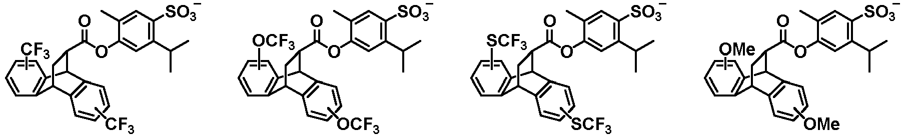

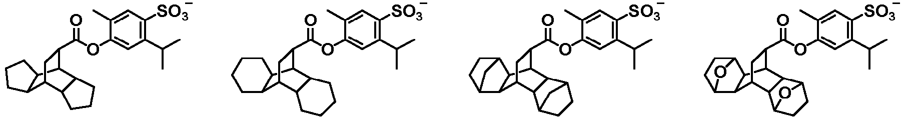

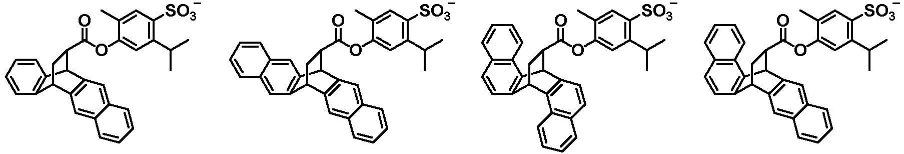

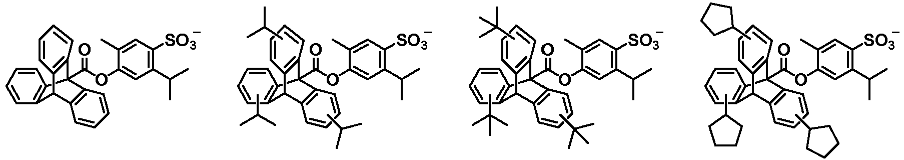

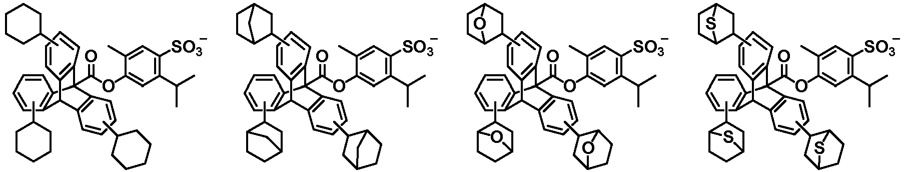

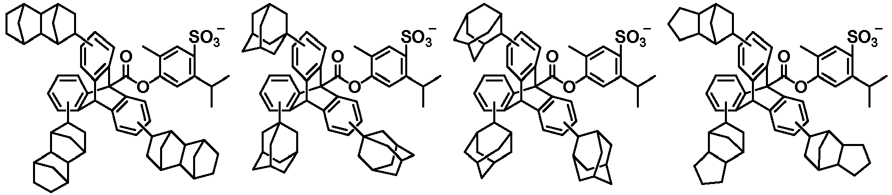

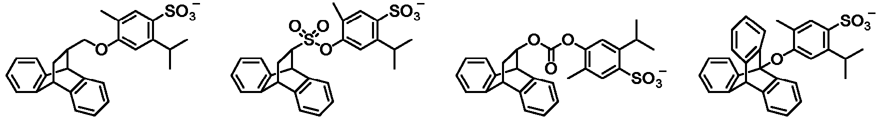

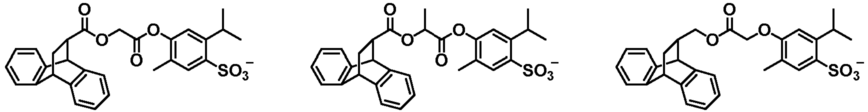

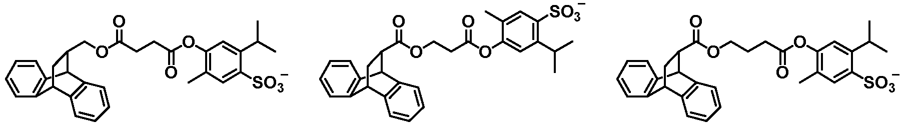

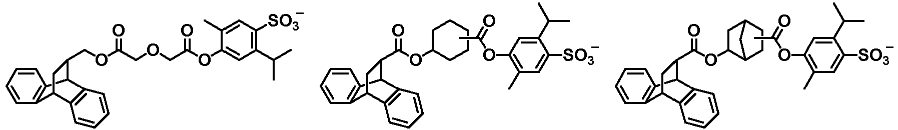

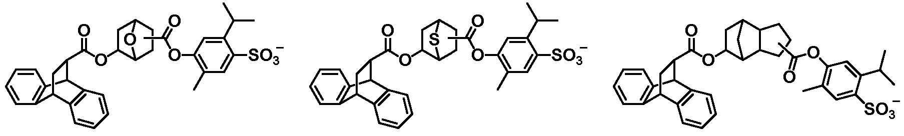

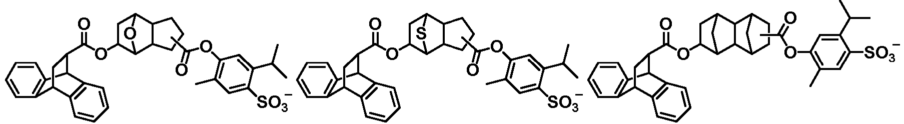

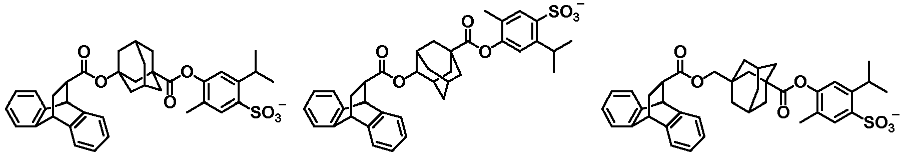

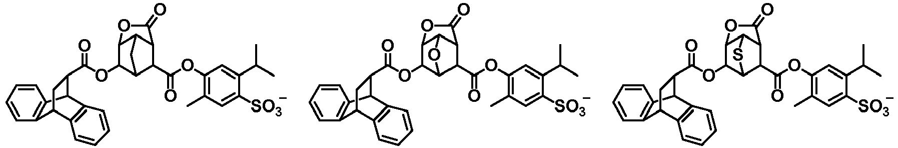

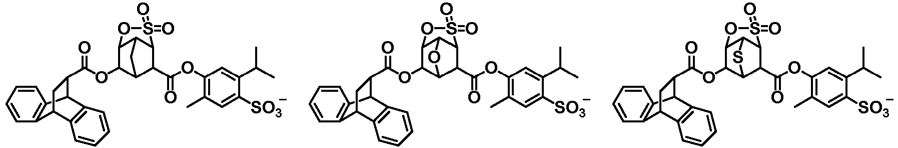

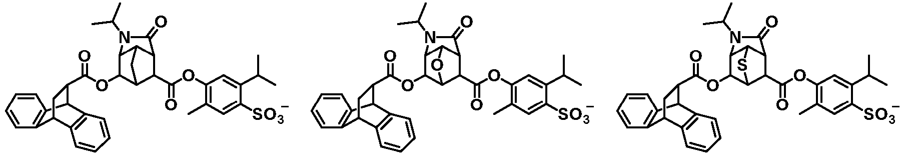

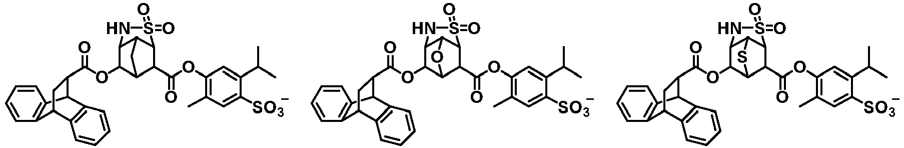

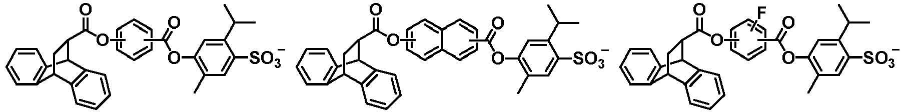

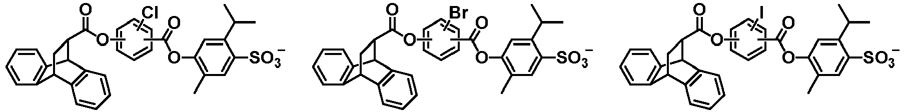

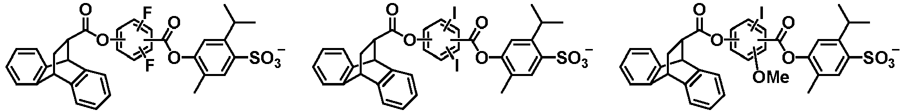

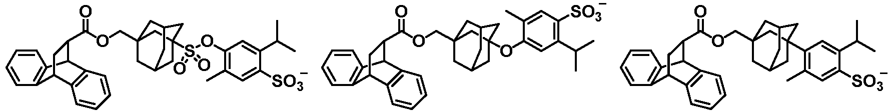

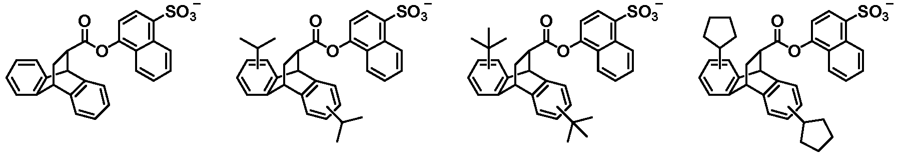

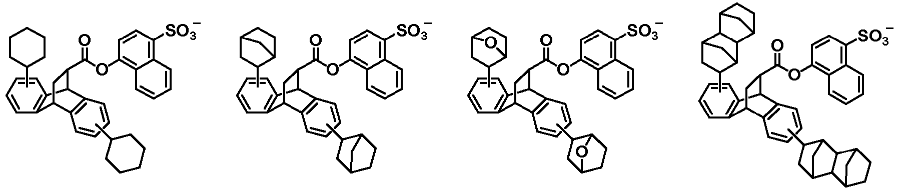

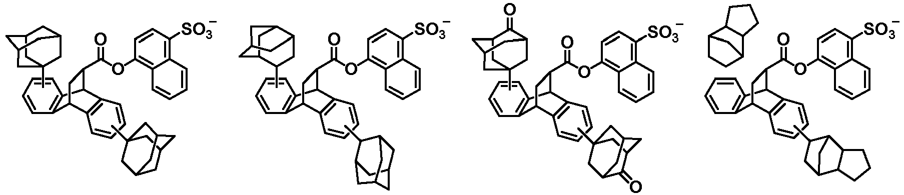

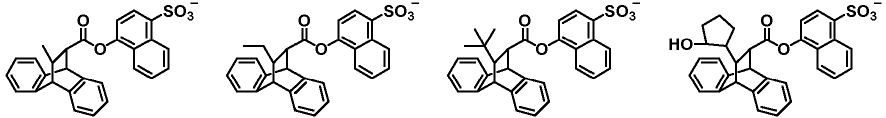

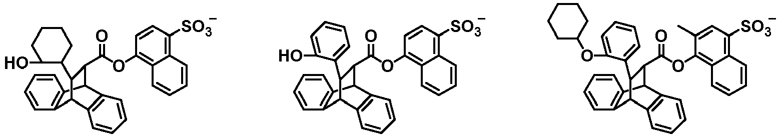

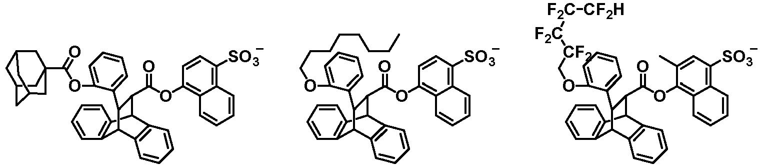

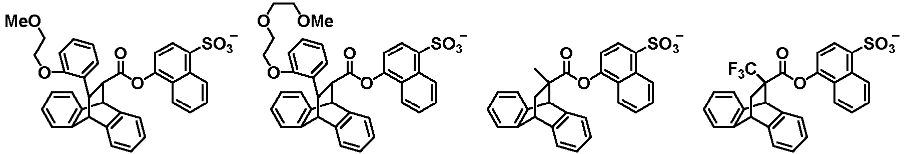

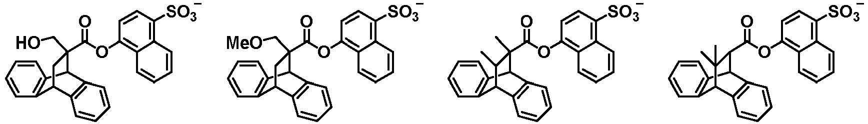

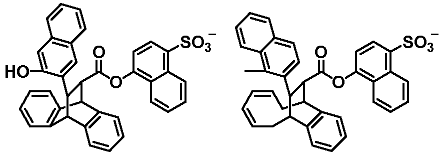

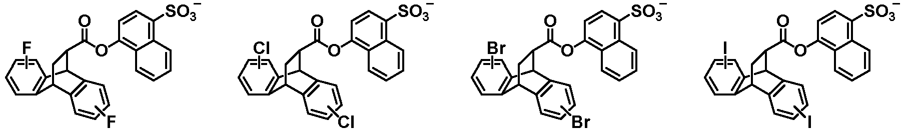

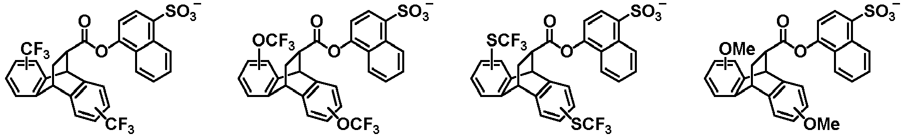

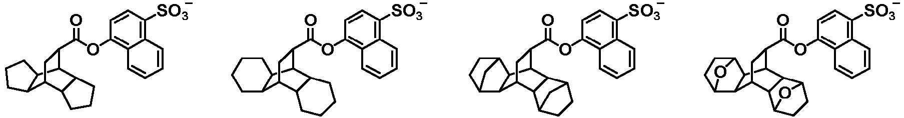

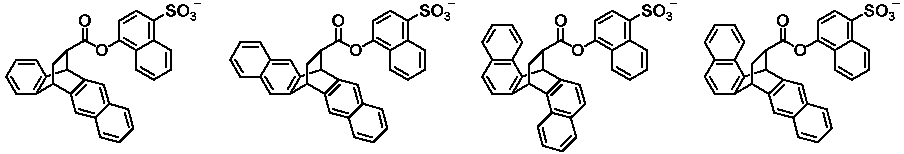

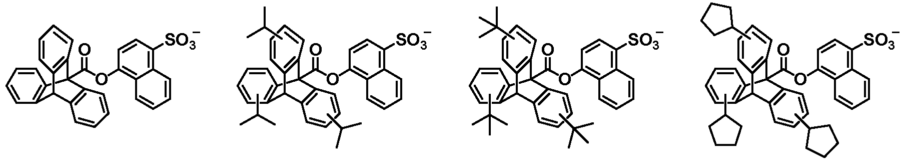

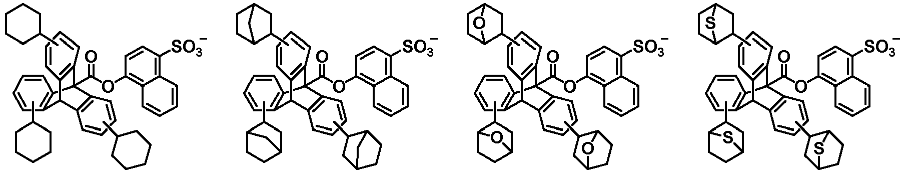

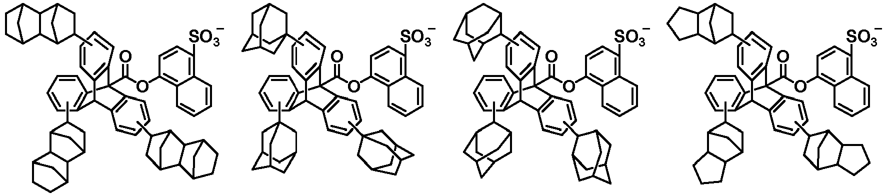

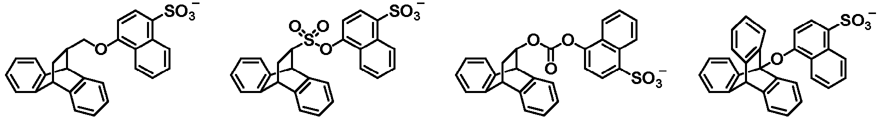

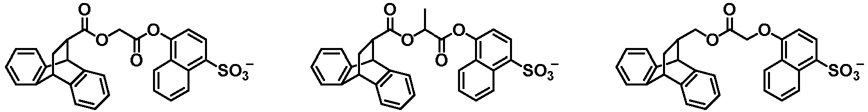

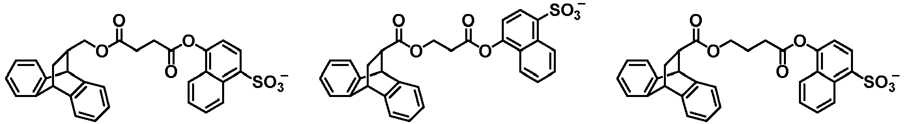

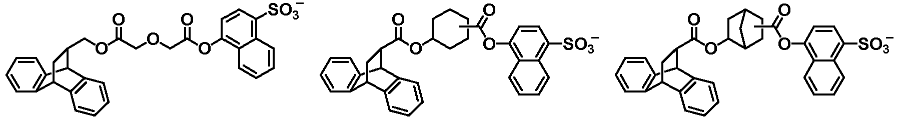

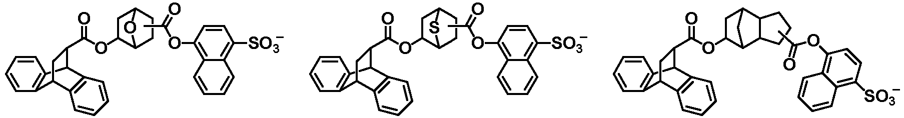

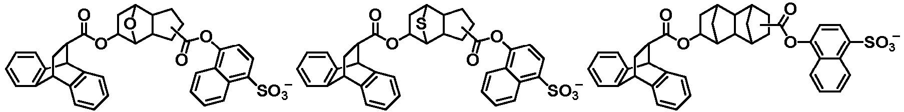

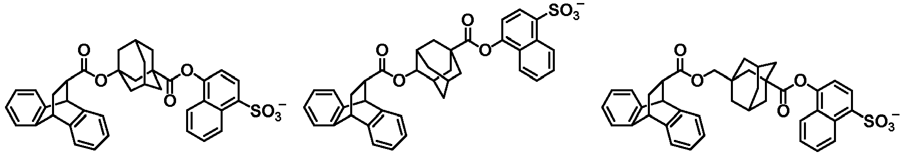

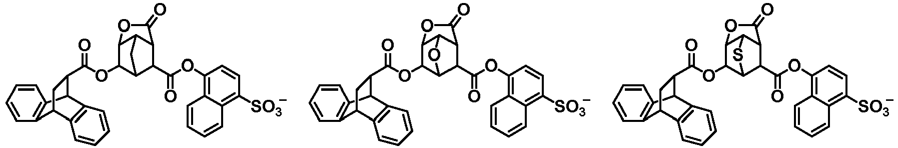

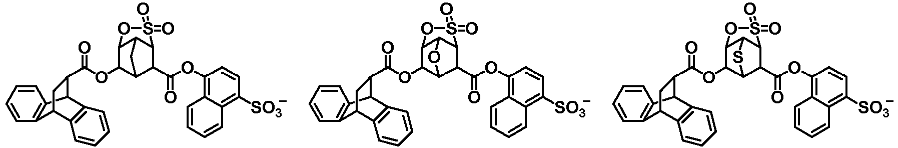

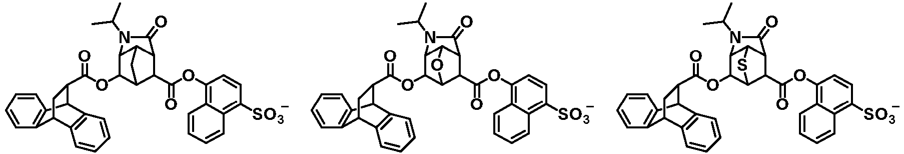

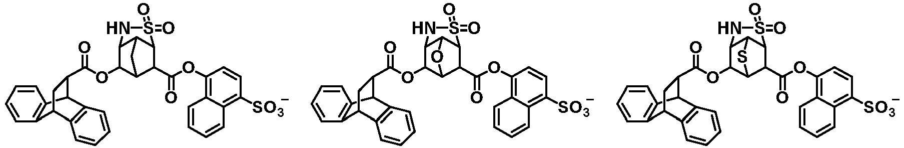

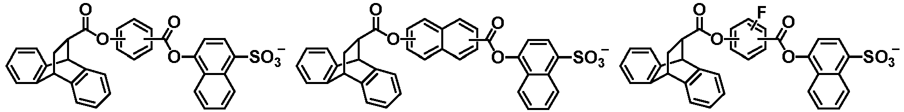

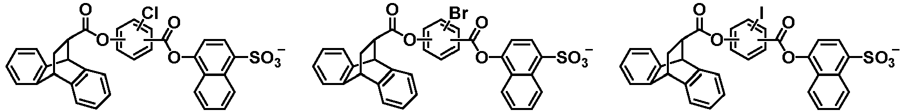

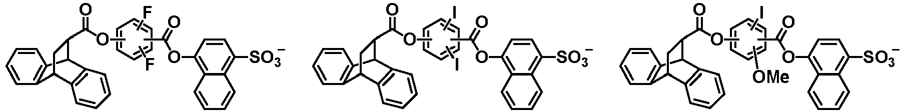

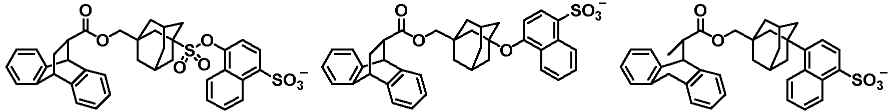

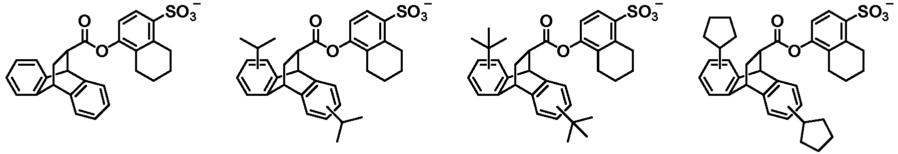

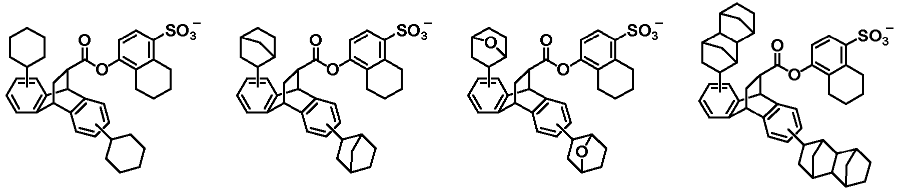

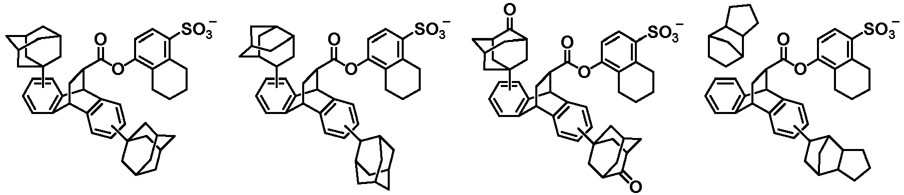

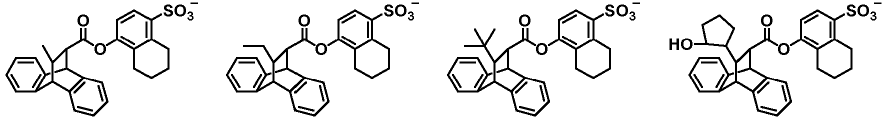

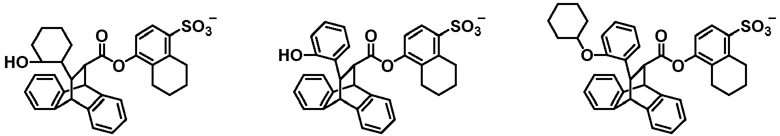

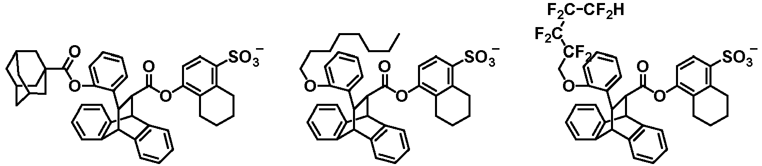

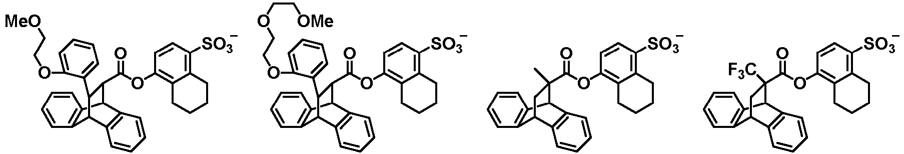

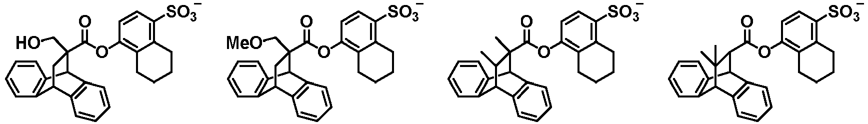

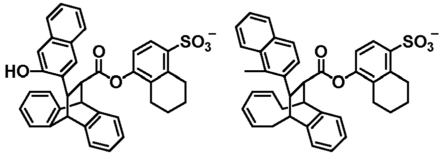

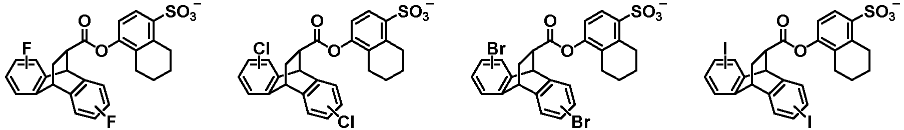

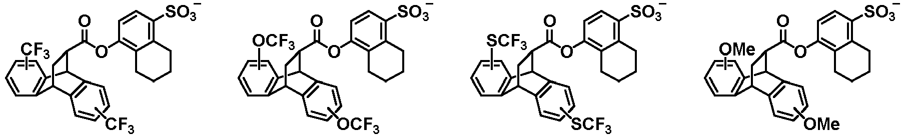

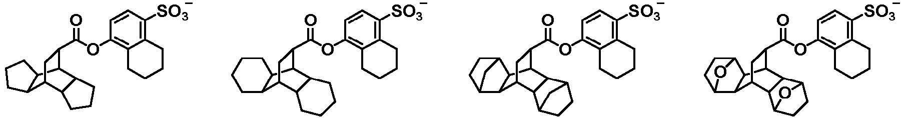

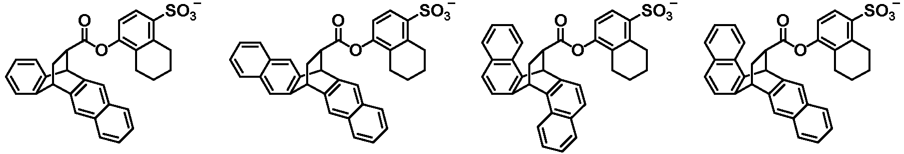

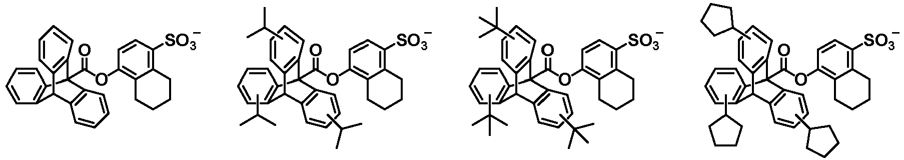

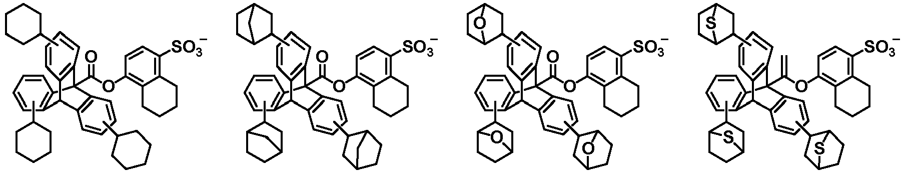

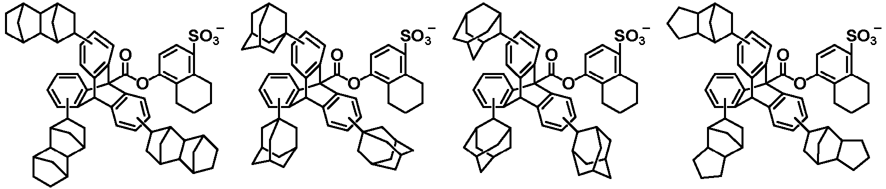

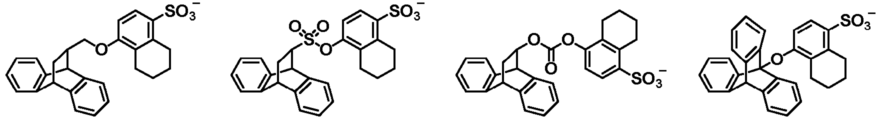

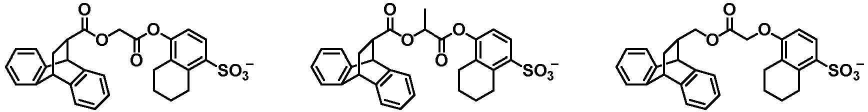

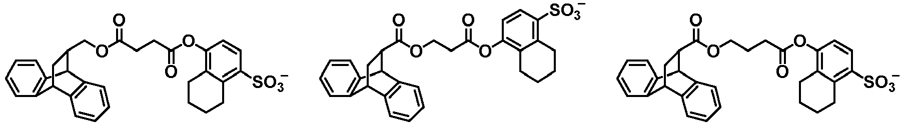

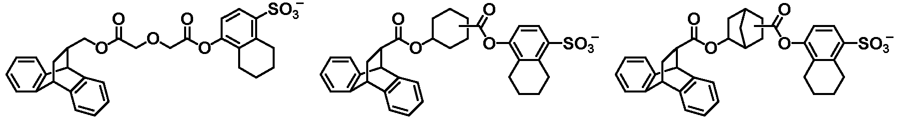

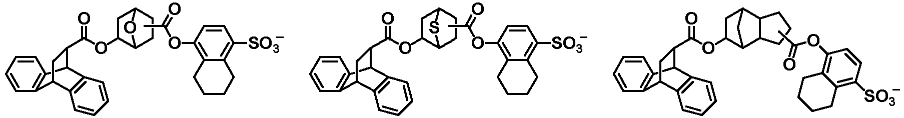

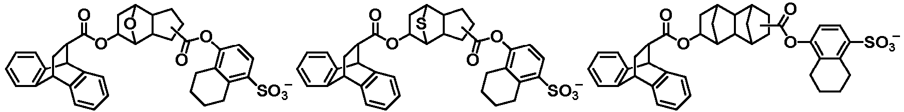

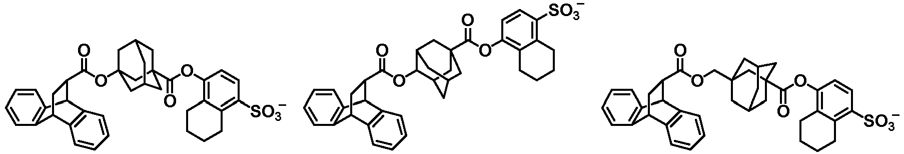

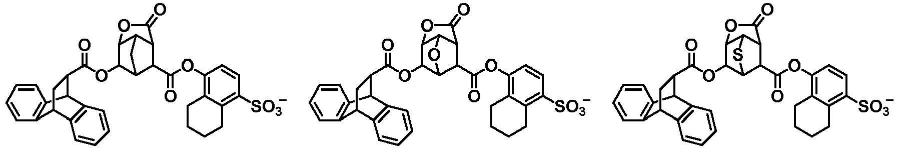

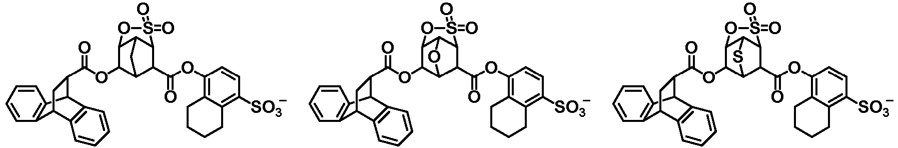

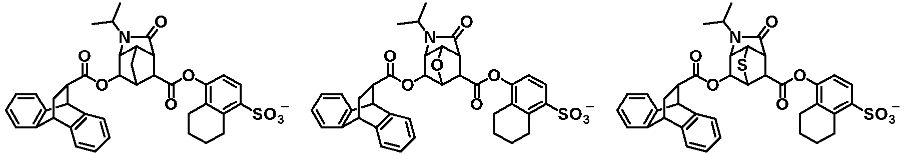

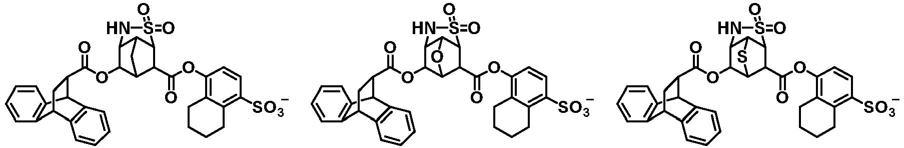

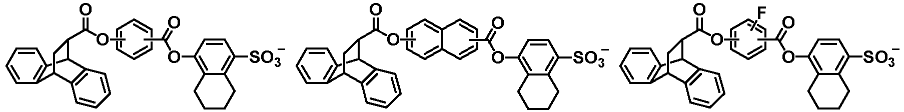

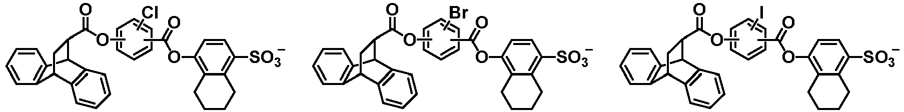

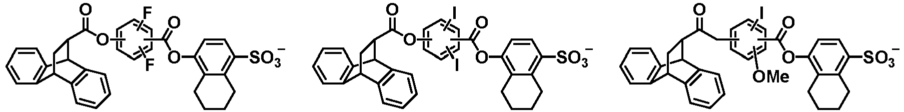

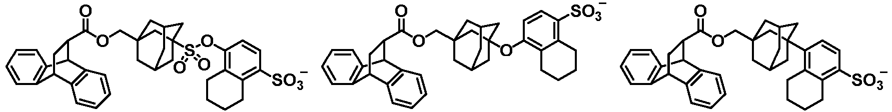

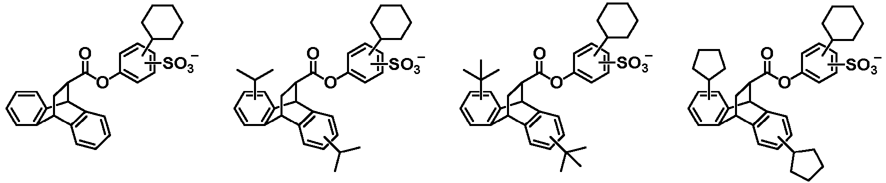

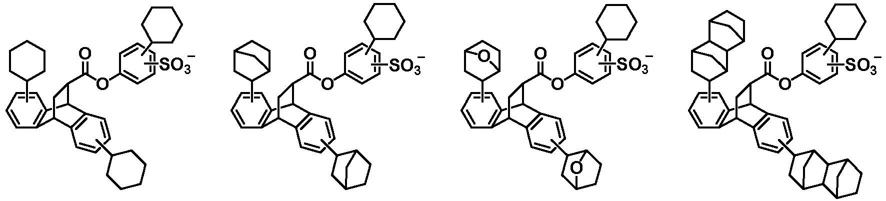

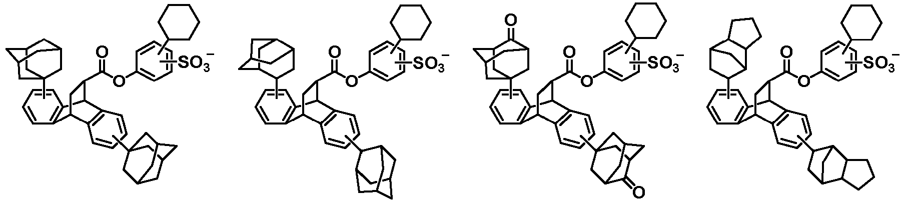

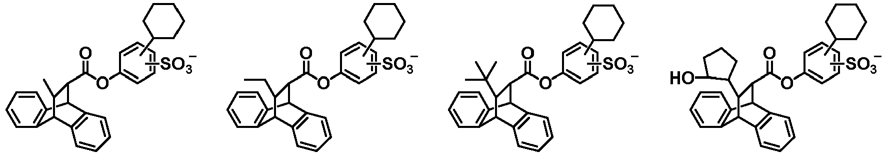

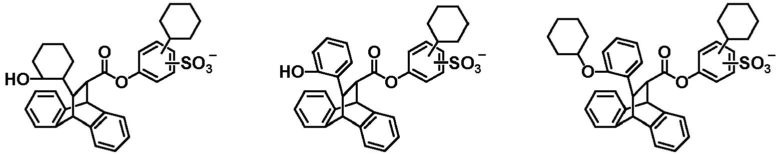

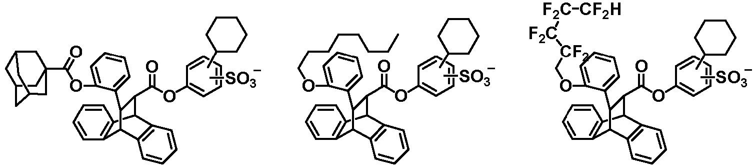

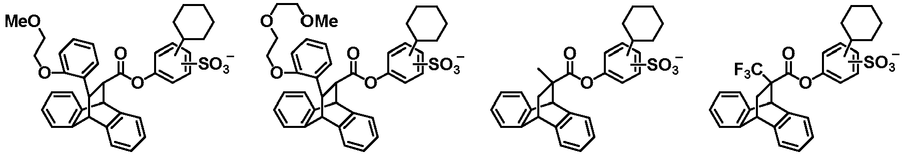

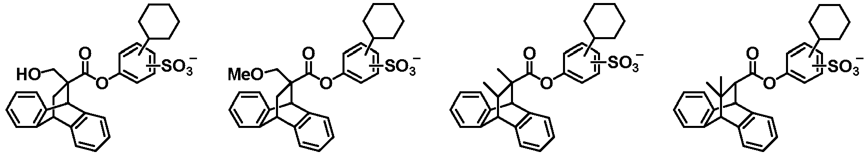

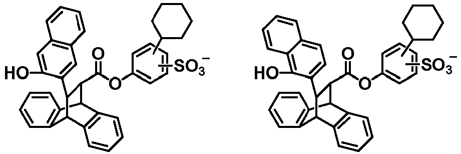

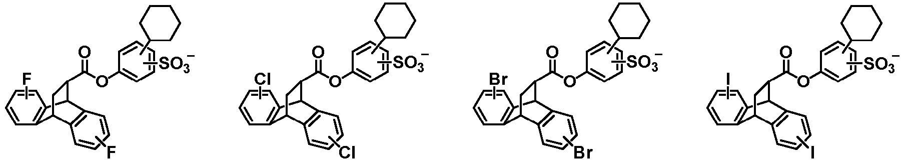

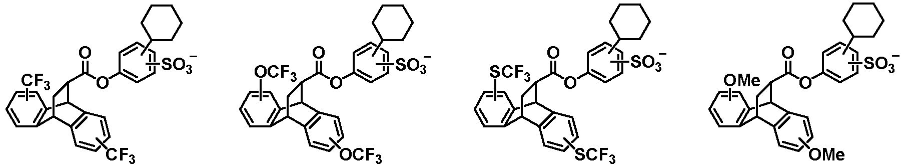

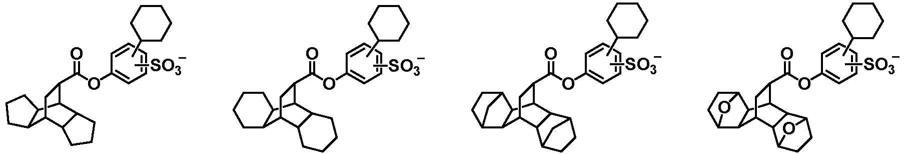

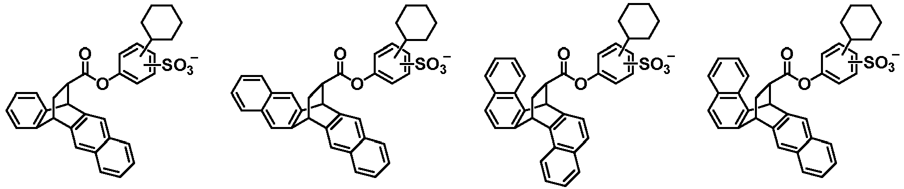

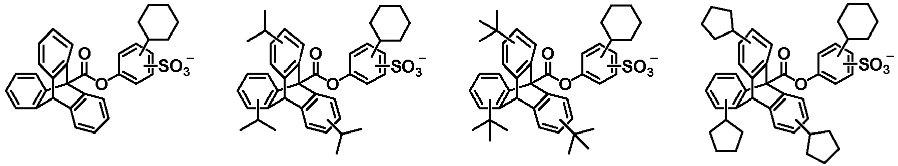

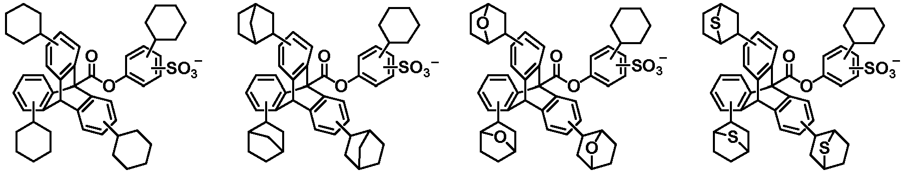

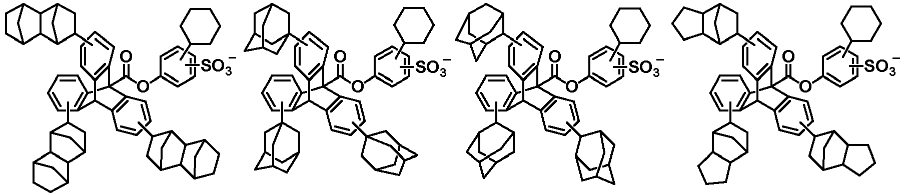

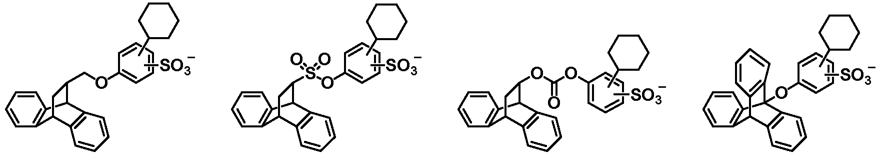

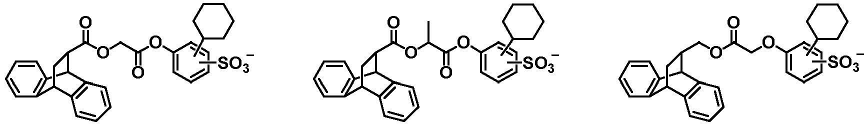

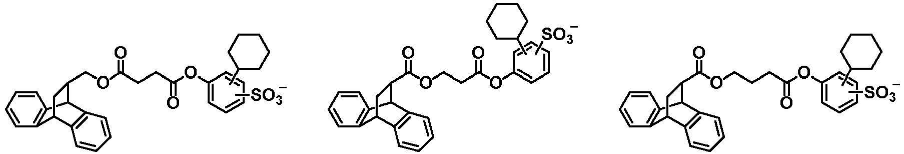

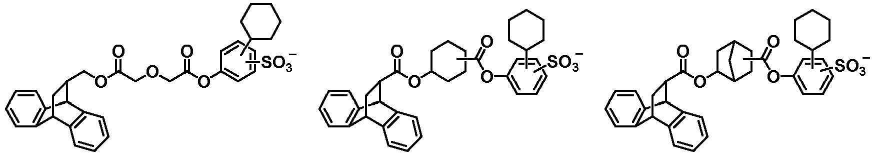

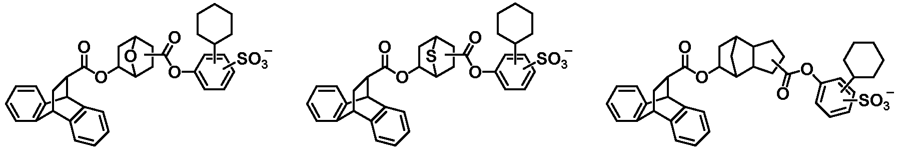

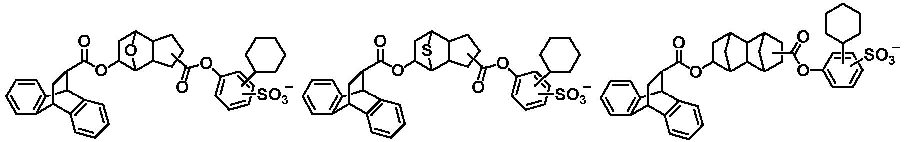

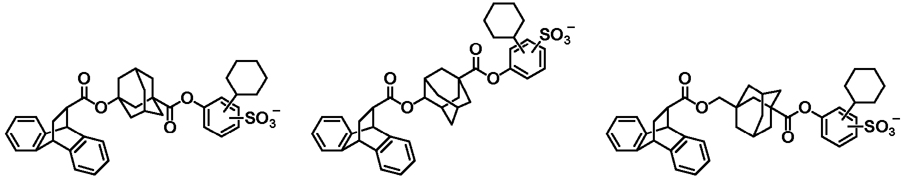

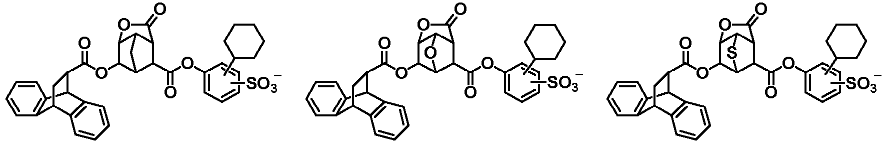

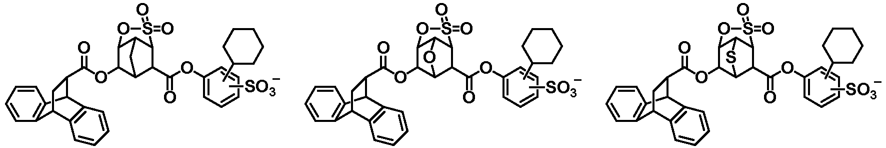

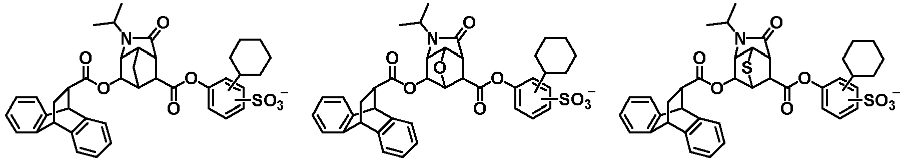

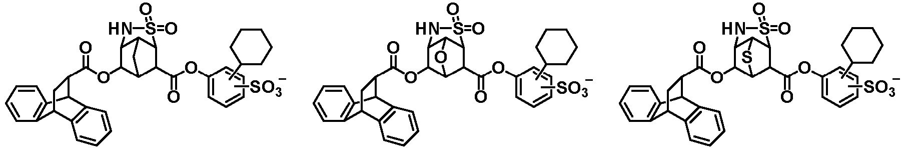

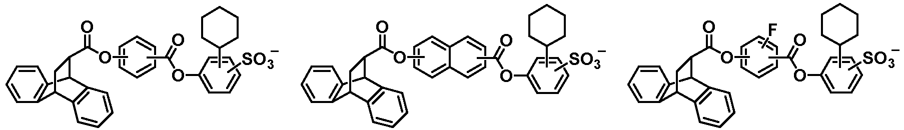

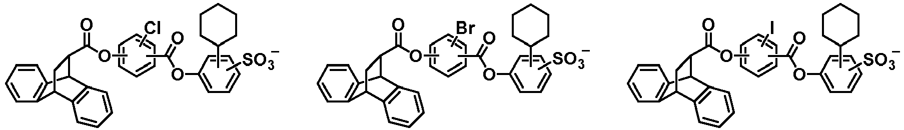

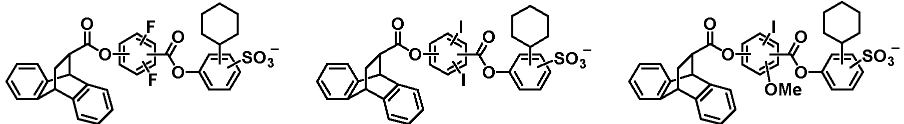

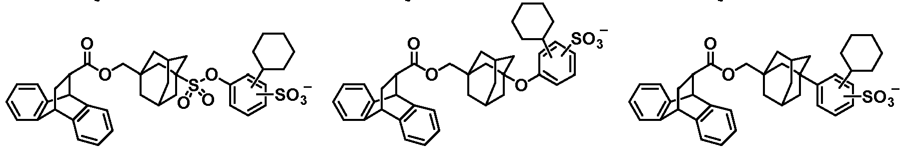

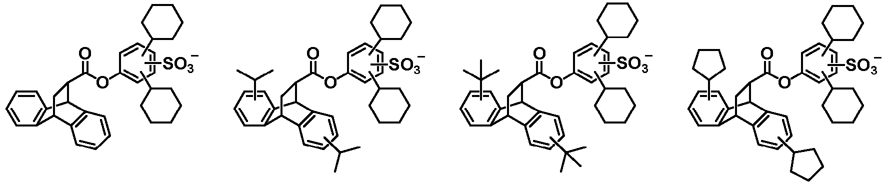

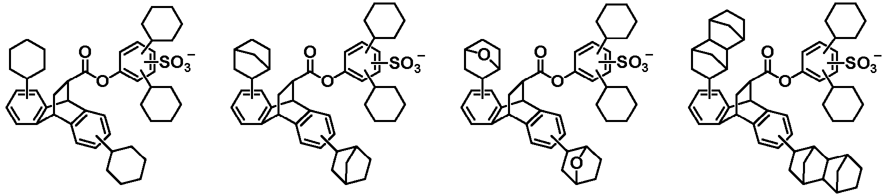

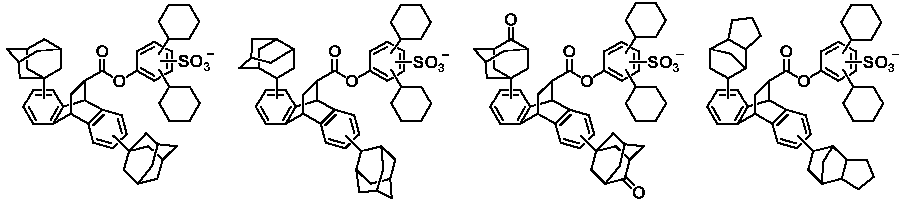

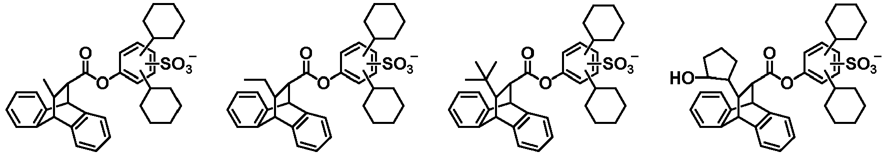

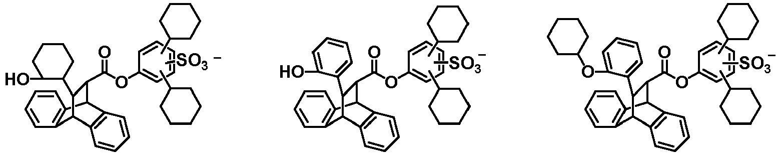

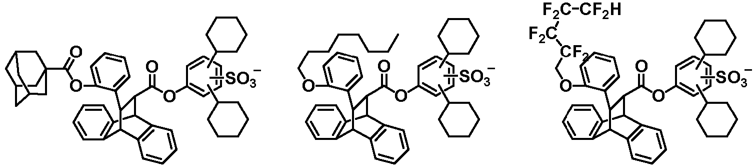

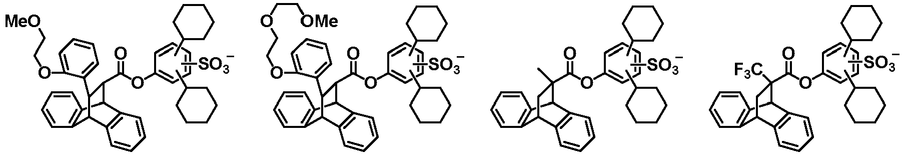

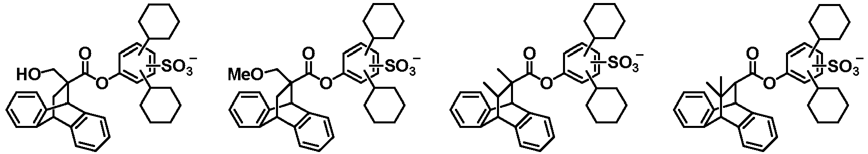

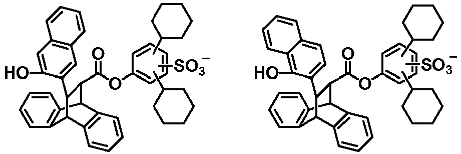

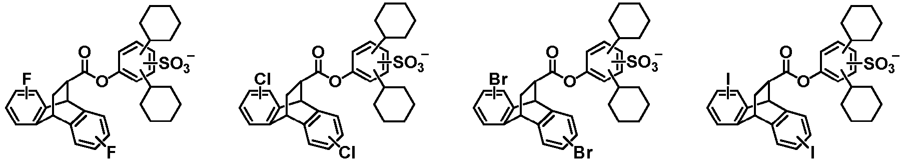

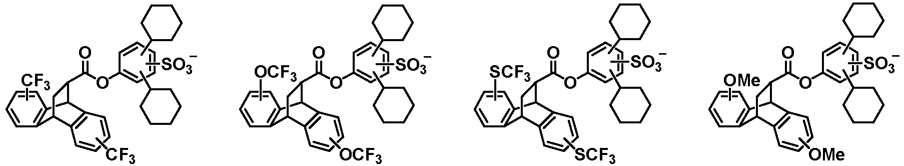

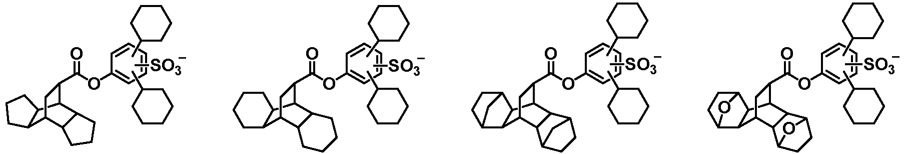

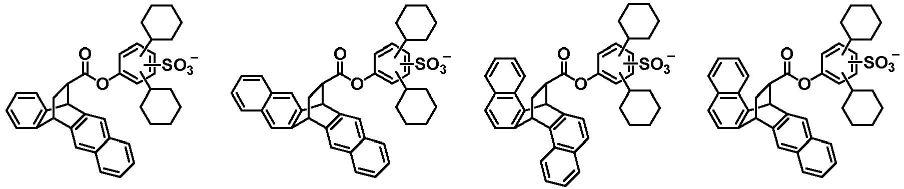

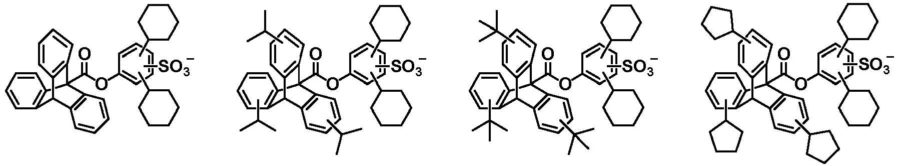

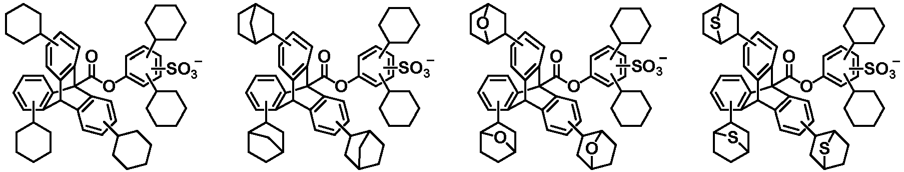

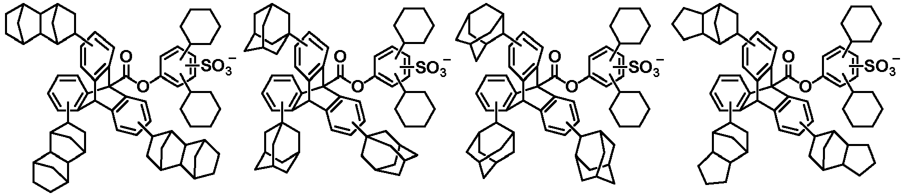

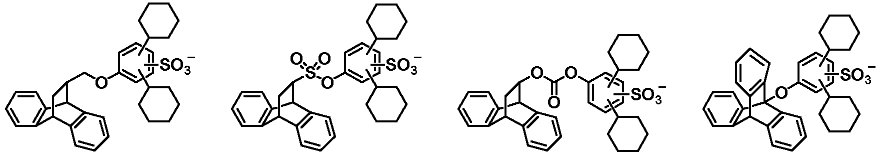

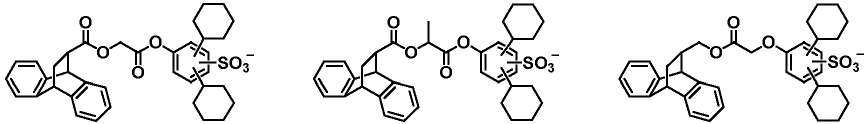

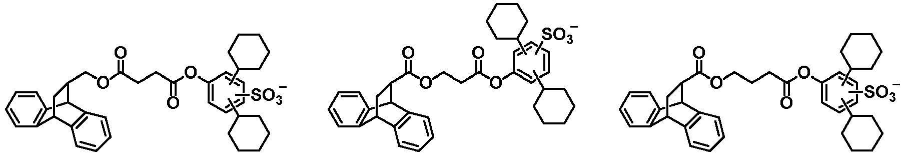

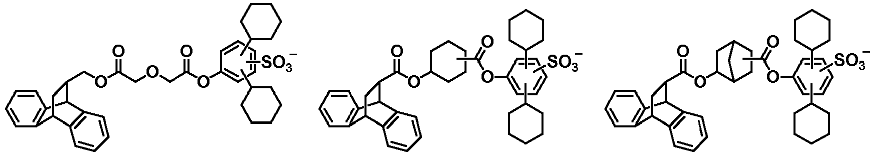

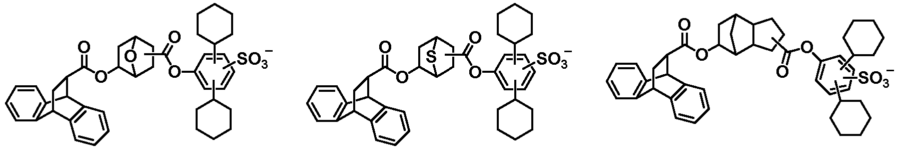

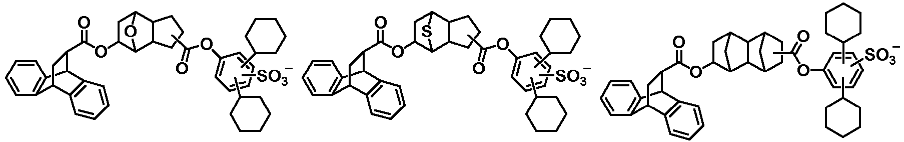

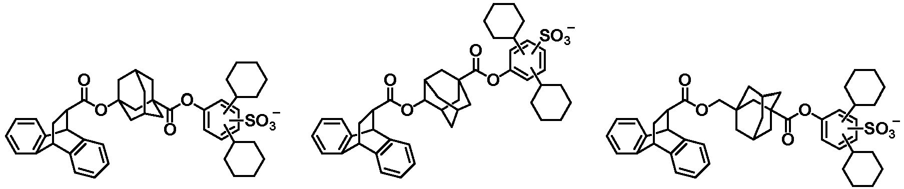

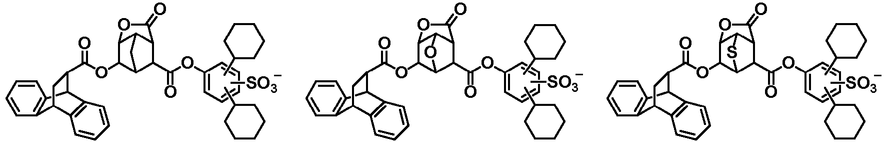

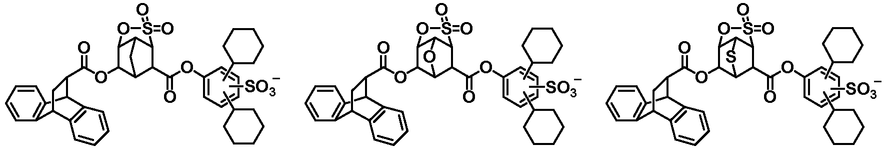

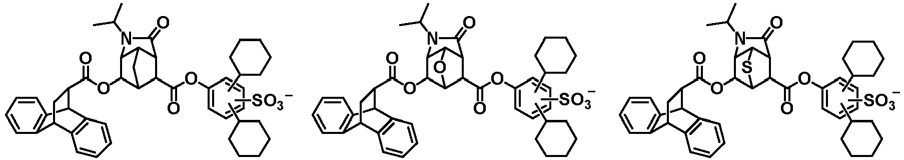

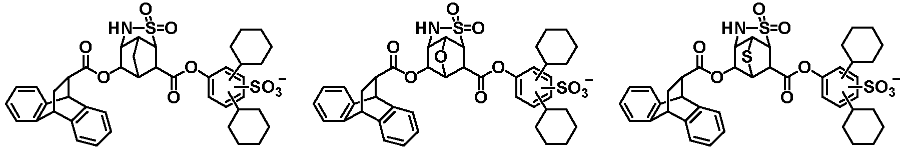

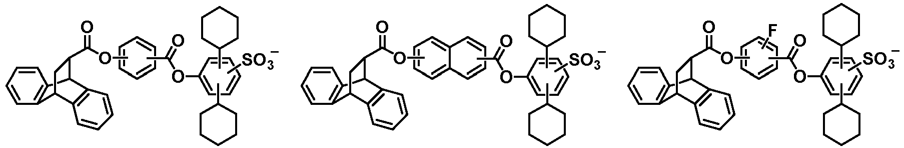

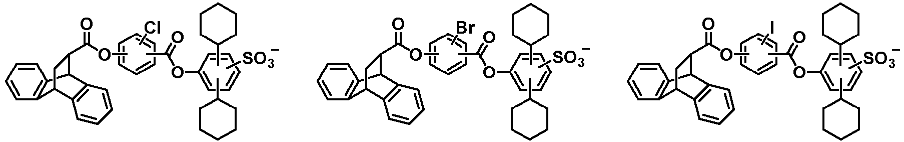

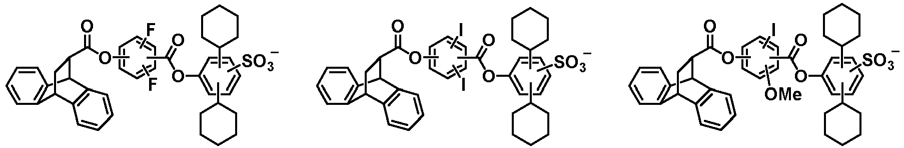

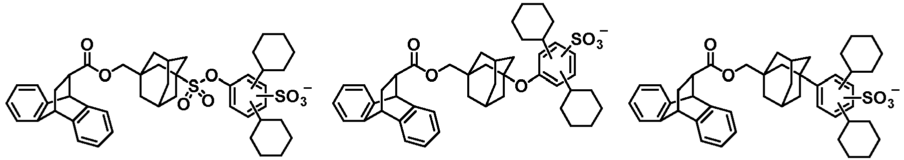

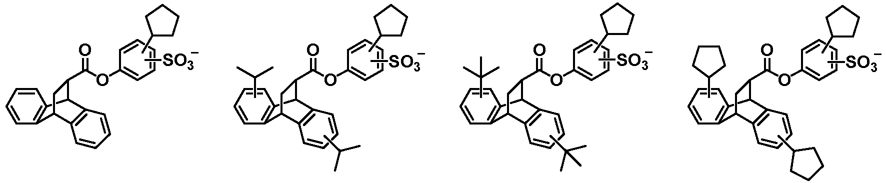

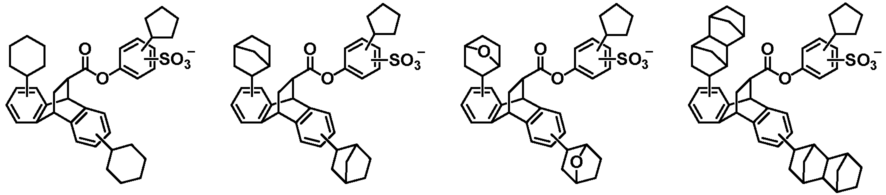

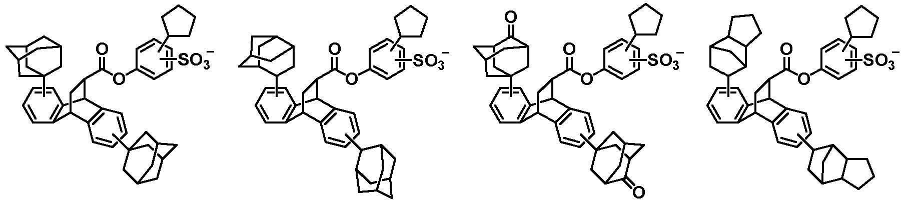

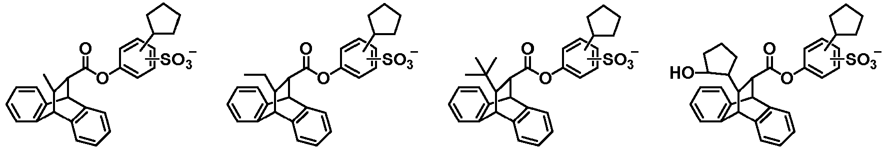

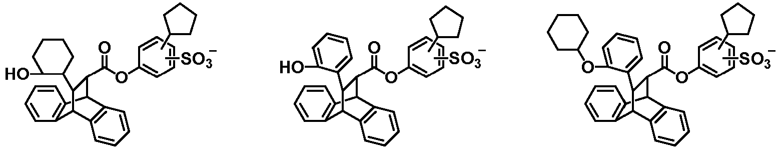

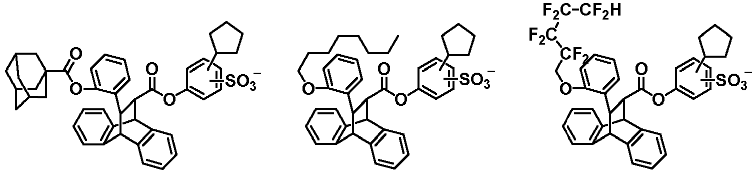

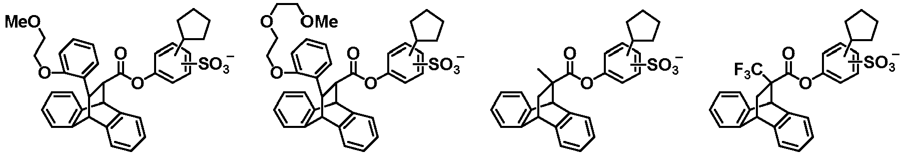

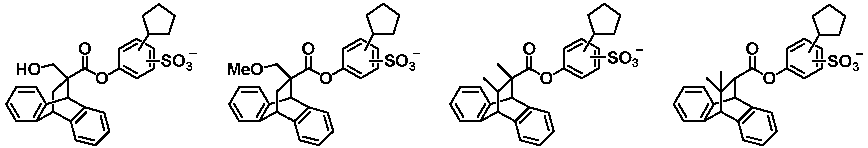

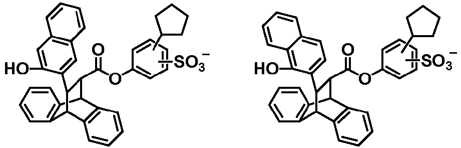

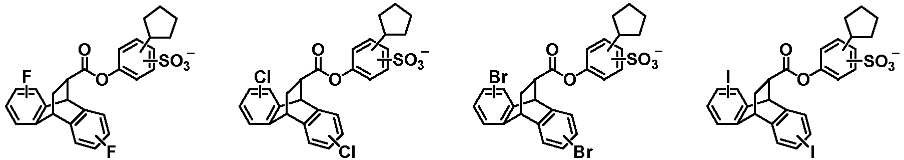

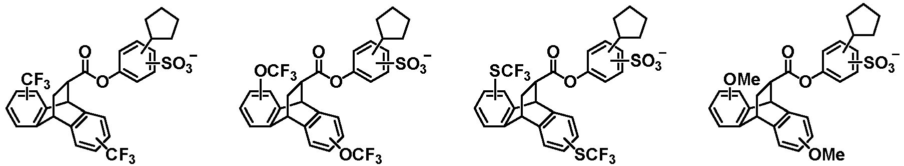

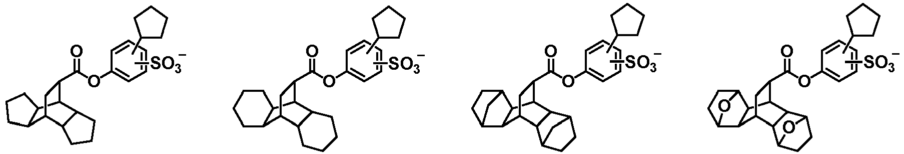

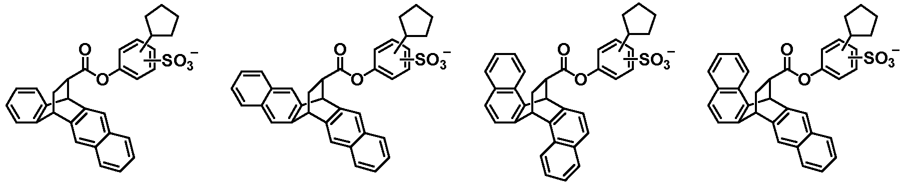

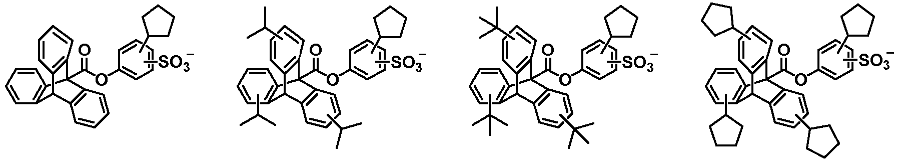

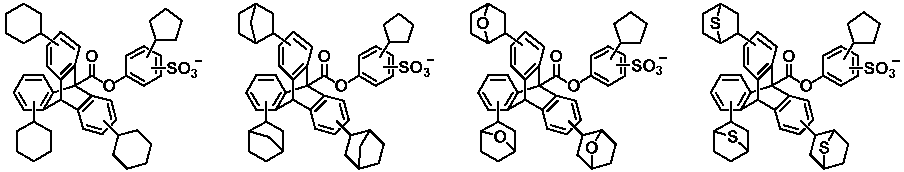

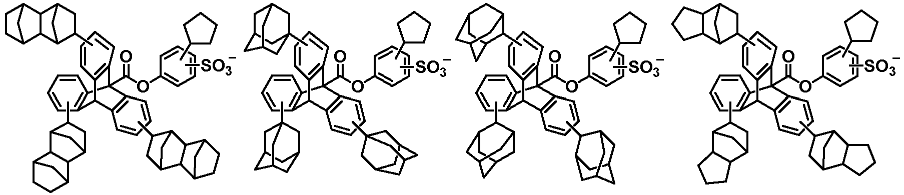

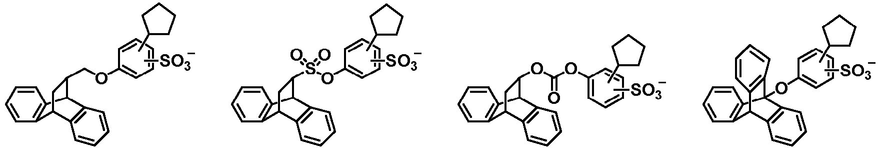

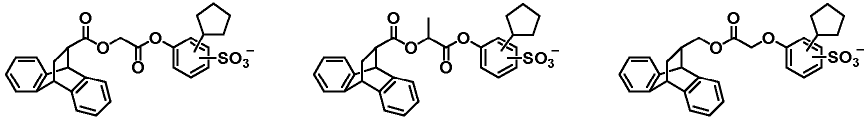

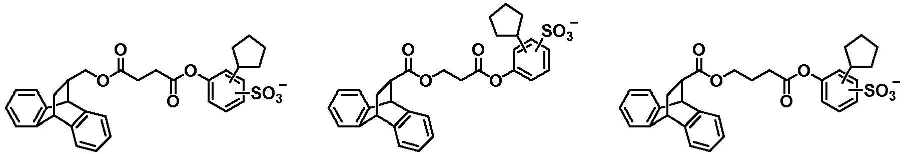

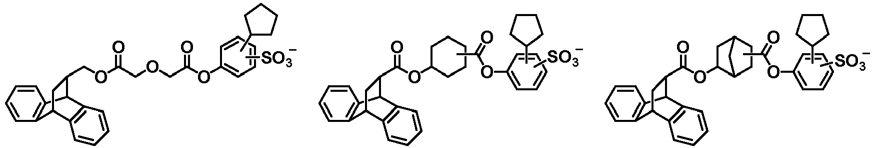

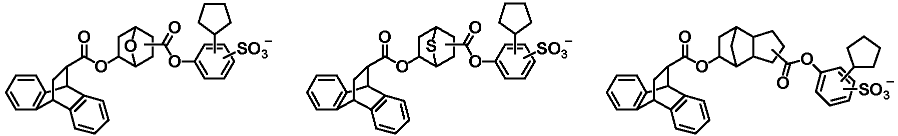

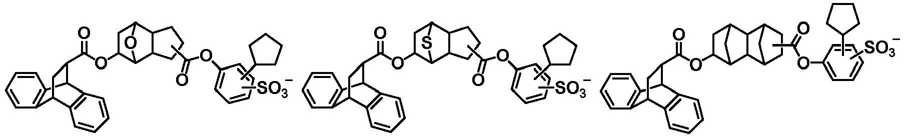

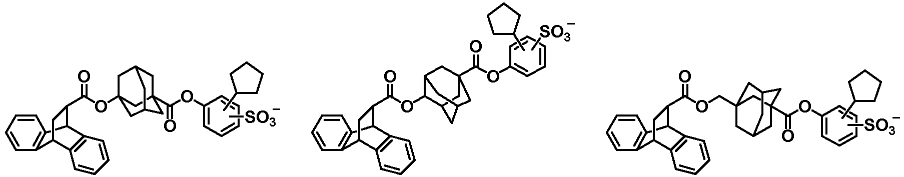

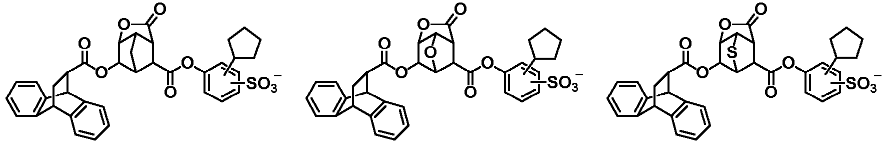

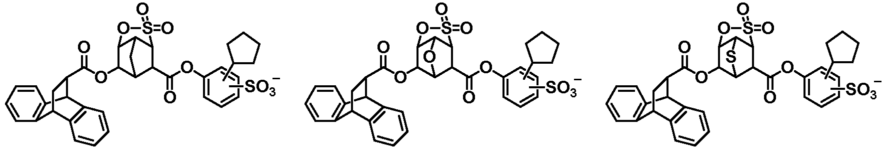

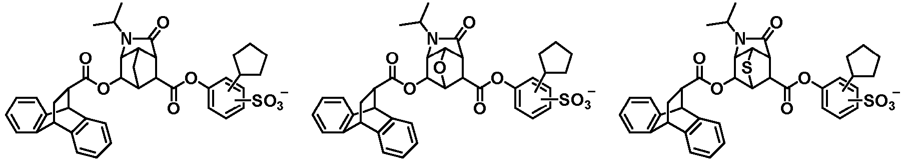

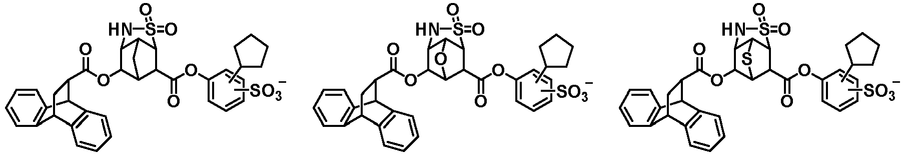

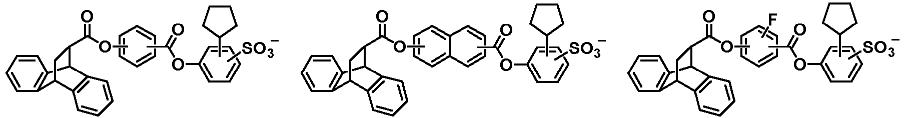

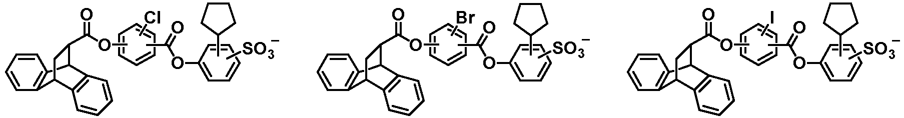

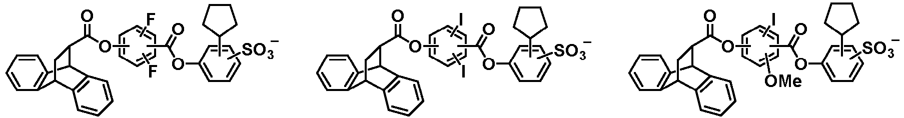

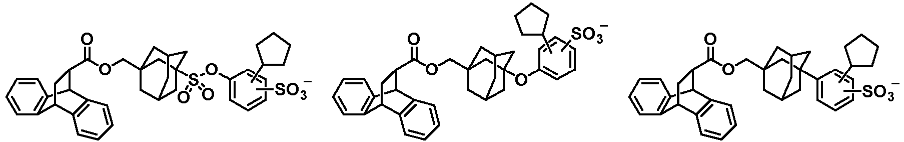

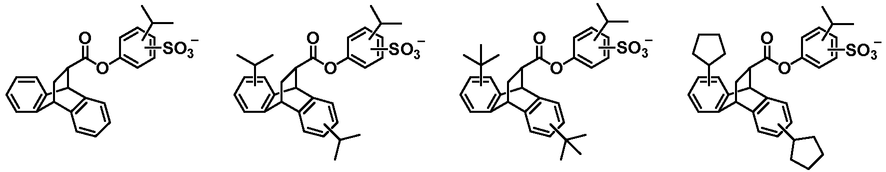

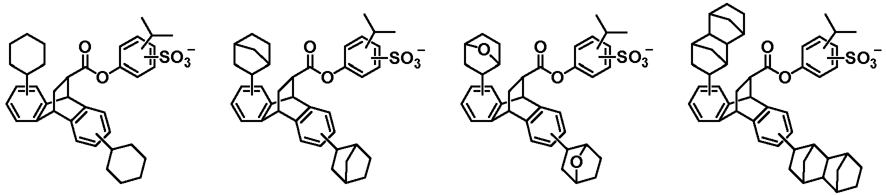

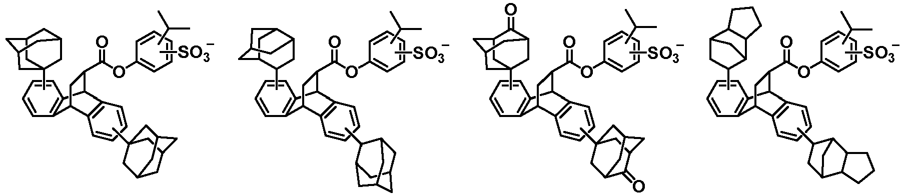

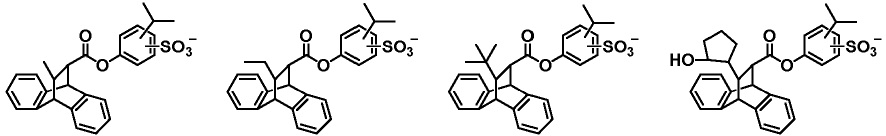

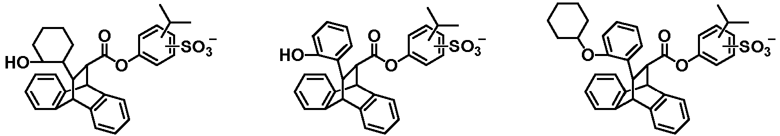

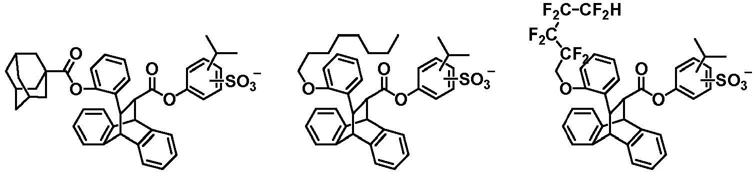

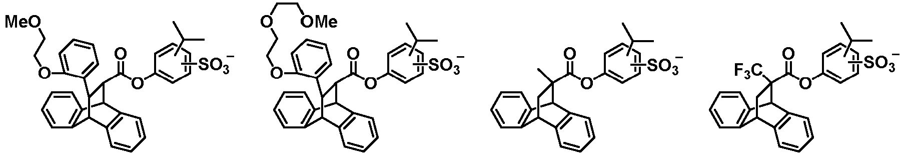

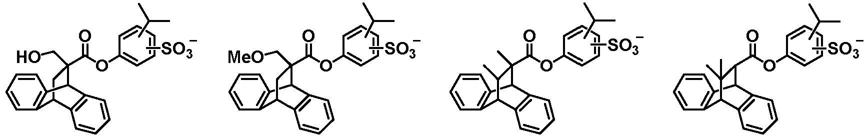

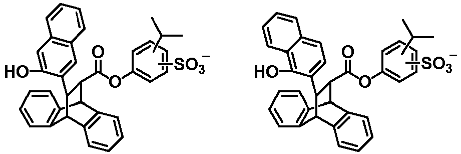

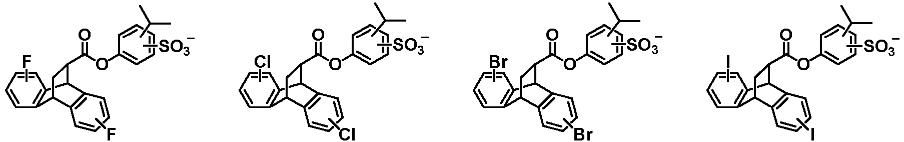

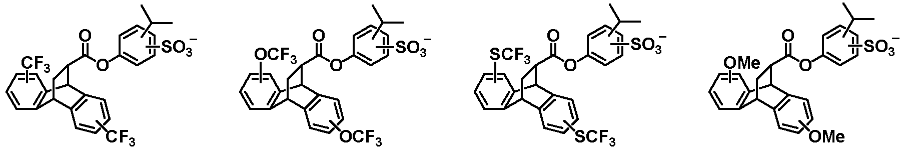

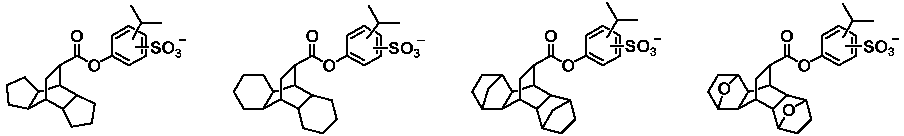

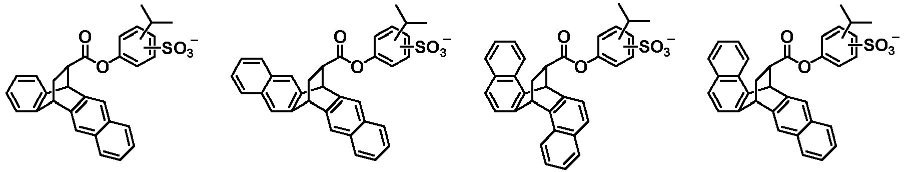

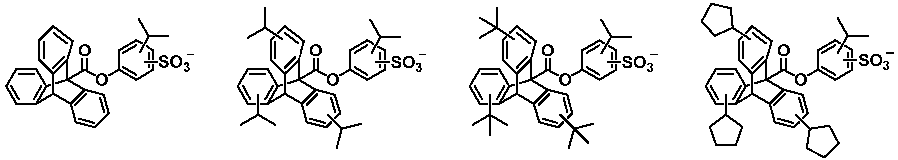

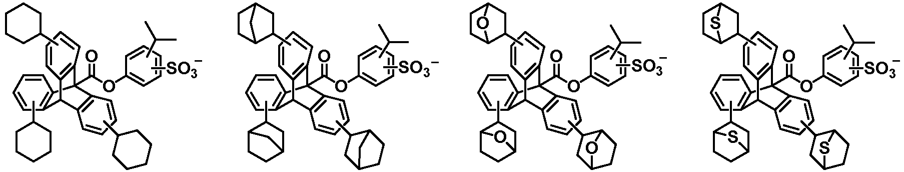

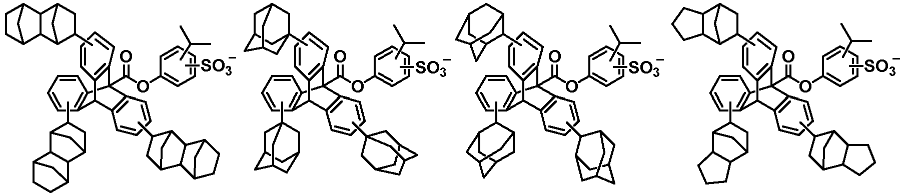

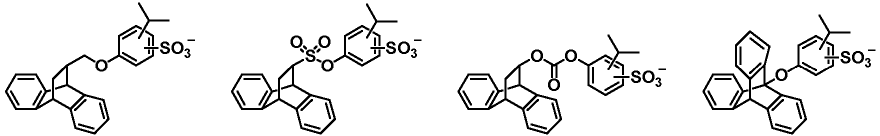

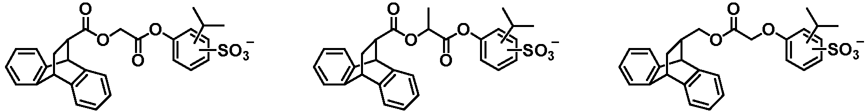

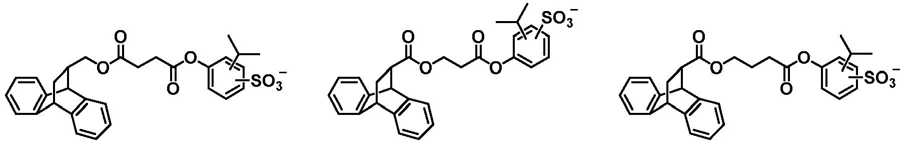

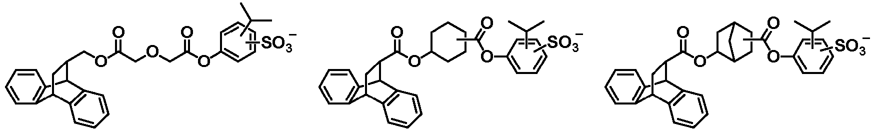

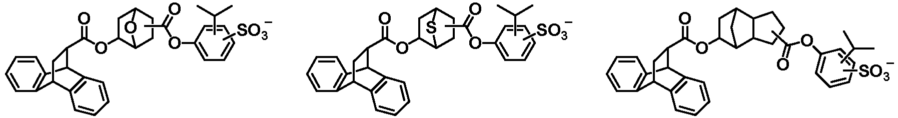

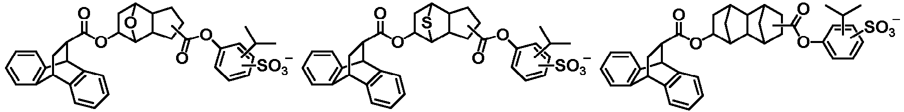

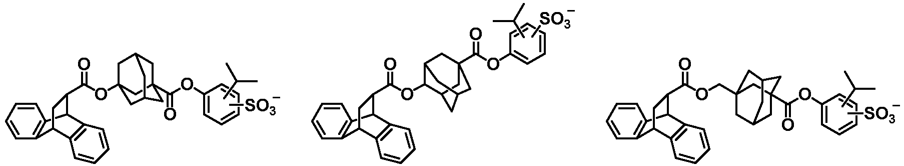

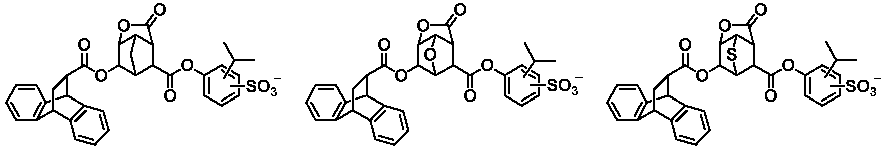

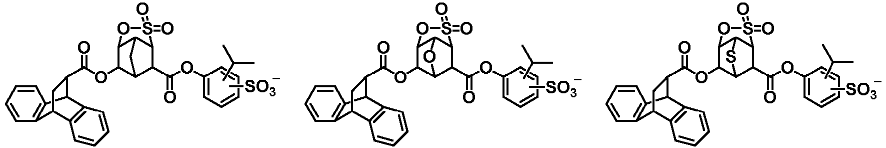

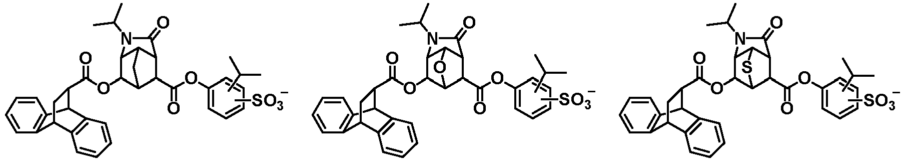

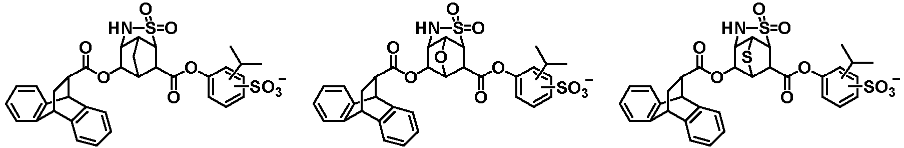

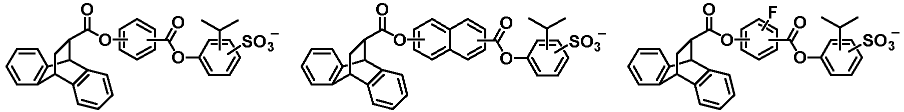

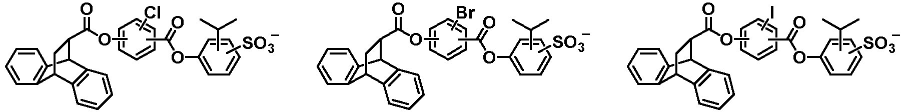

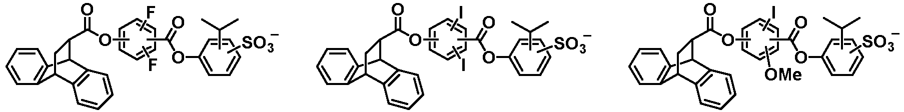

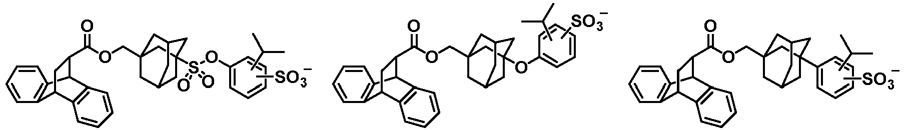

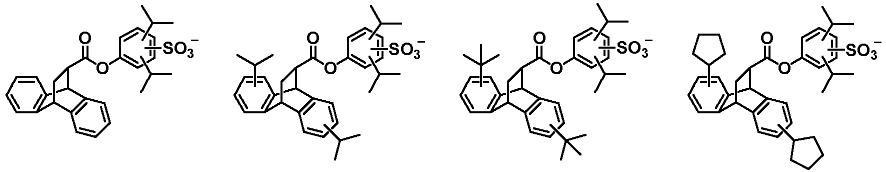

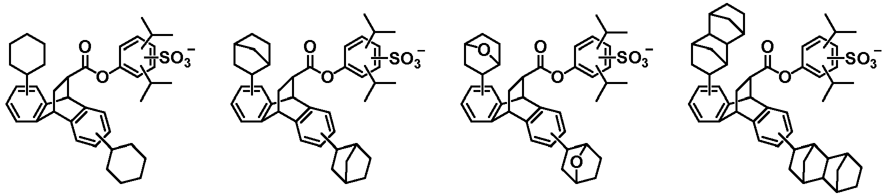

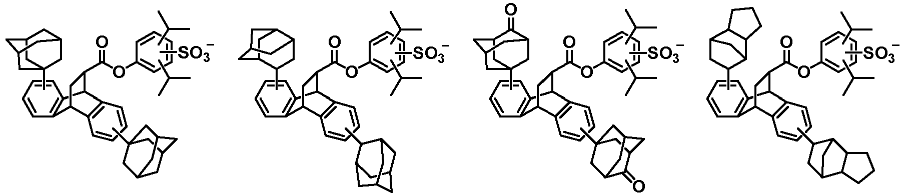

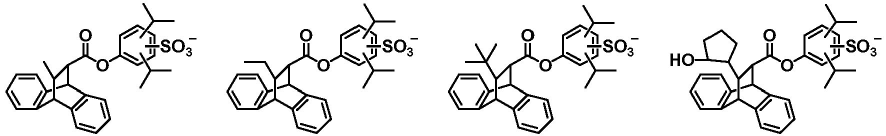

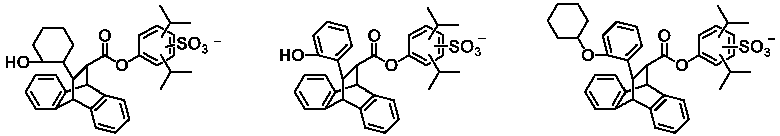

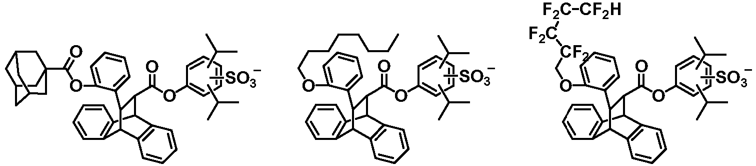

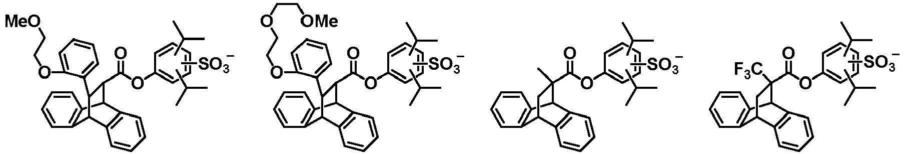

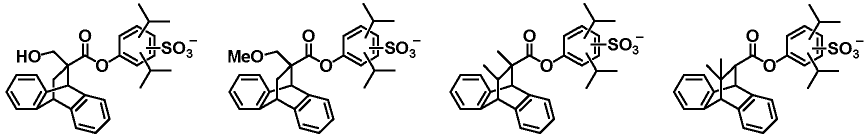

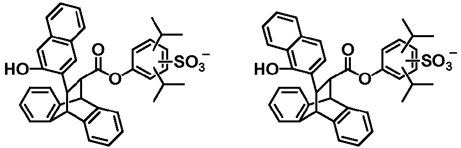

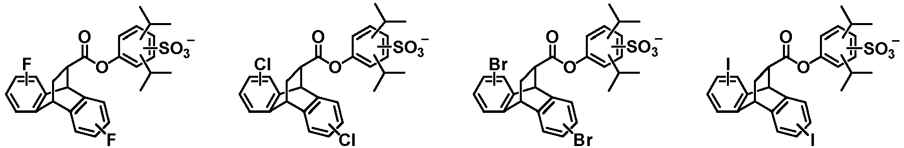

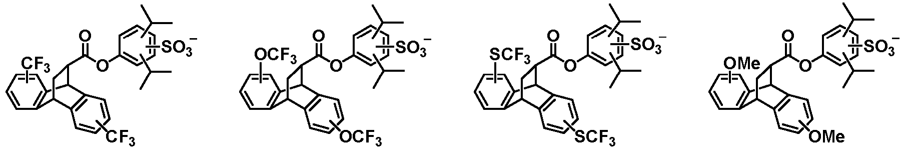

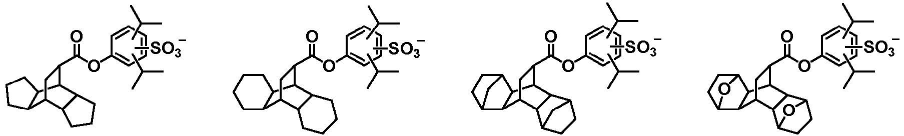

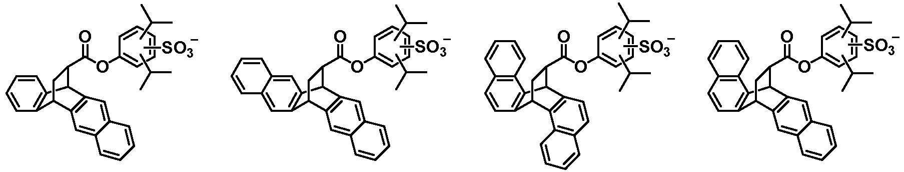

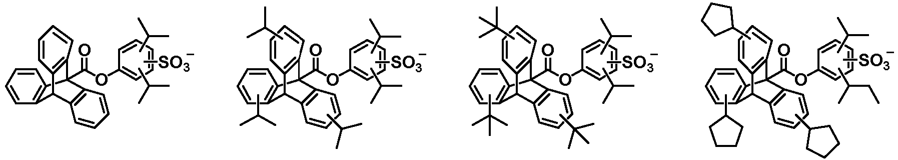

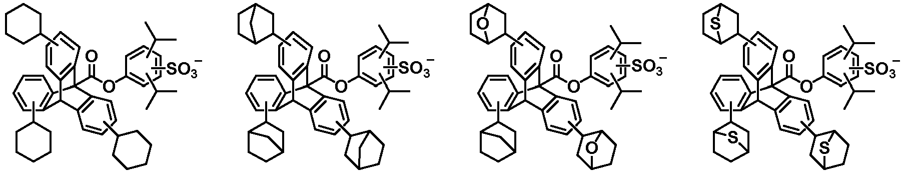

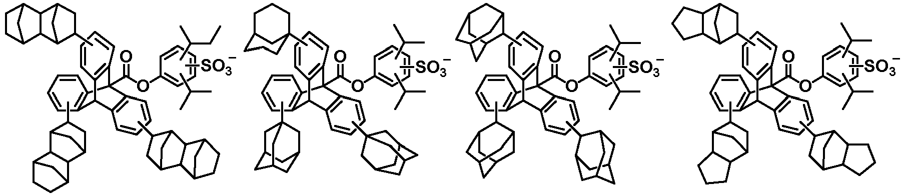

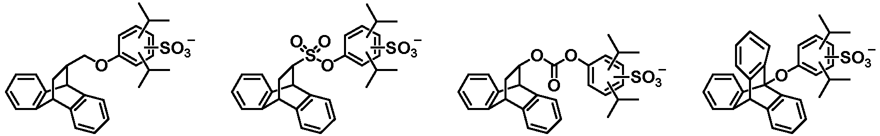

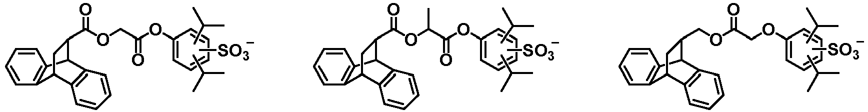

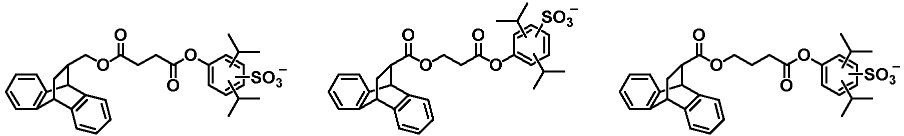

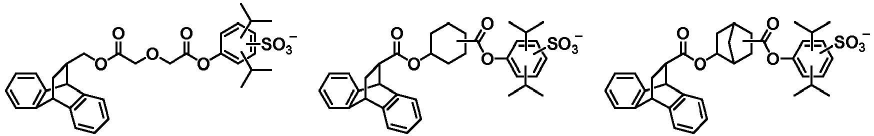

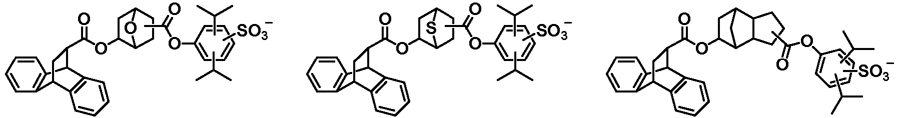

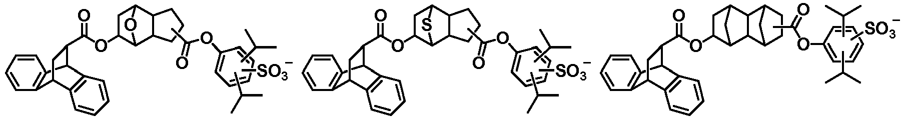

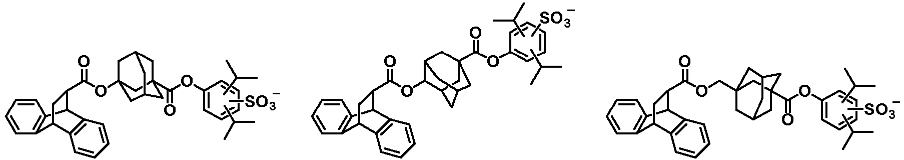

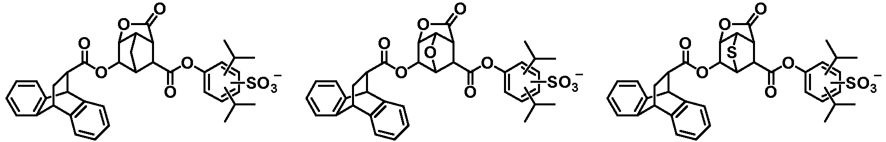

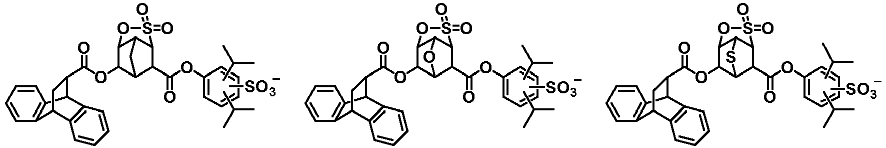

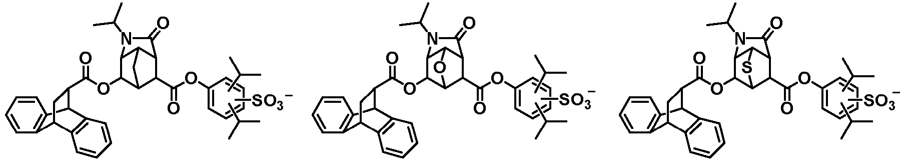

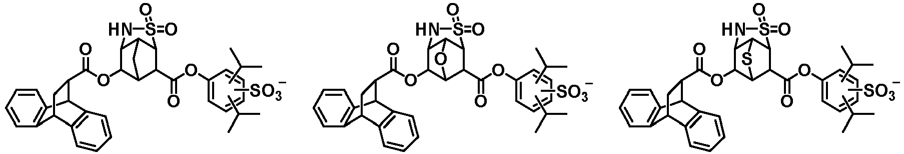

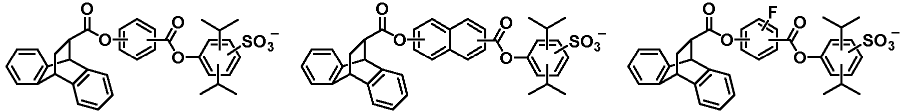

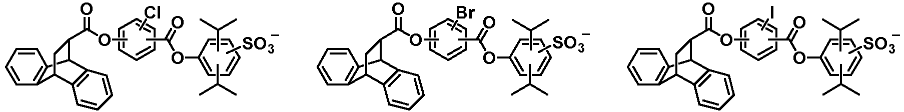

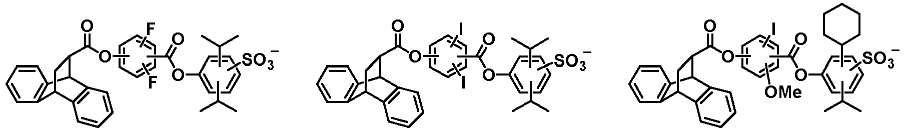

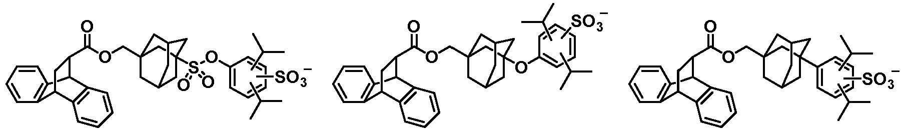

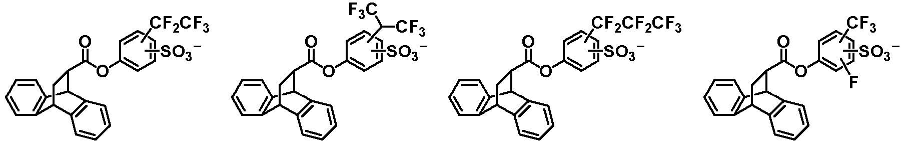

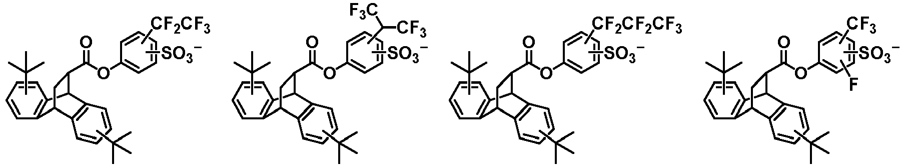

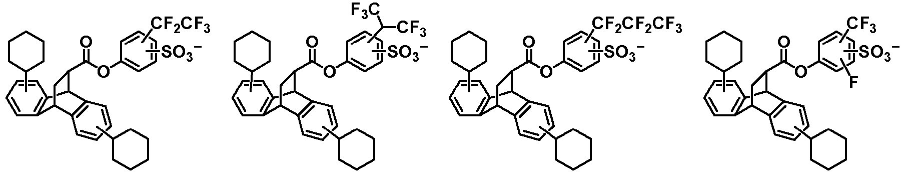

In addition, as the photoacid generator, a salt compound containing an anion having the formula (F1) is also preferred.

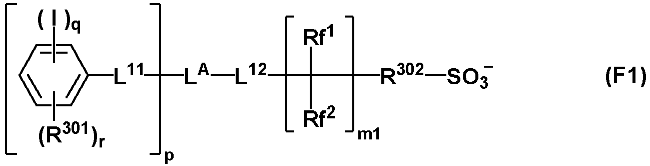

**In formula** (F1), m1 is 0 or 1, p is 1, 2, or 3, q is 1, 2, 3, 4, or 5, r is 0, 1, 2, or 3, and provided that 1 ≤ q+r ≤ 5.

In formula (F1), L¹¹ is a single bond, an ether bond, an ester bond, a sulfonate ester bond, a carbonate bond, or a carbamate bond.

In formula (F1), L¹² is an ether bond, an ester bond, a sulfonate ester bond, a carbonate bond, or a carbamate bond.

In formula (F1), L^{A} is a single bond or a C₁-C₂₀ hydrocarbylene group when p is 1, and is a C₁-C₂₀ (p+1)-valent hydrocarbon group when p is 2 or 3, and the hydrocarbylene group and the (p+1)-valent hydrocarbon group may contain at least one moiety selected from an ether bond, a carbonyl group, an ester bond, an amide bond, a sultone ring, a lactam ring, a carbonate bond, a halogen atom, a hydroxy group, and a carboxy group.

The C₁-C₂₀ hydrocarbylene group L^{A} may be saturated or unsaturated, and may be straight, branched, or cyclic. Specific examples thereof include C₁-C₂₀ alkanediyl groups such as a methanediyl group, an ethane-1,1-diyl group, an ethane-1,2-diyl group, a propane-1,3-diyl group, a butane-1,4-diyl group, a pentane-1,5-diyl group, a hexane-1,6-diyl group, a heptane-1,7-diyl group, an octane-1,8-diyl group, a nonane-1,9-diyl group, a decane-1,10-diyl group, an undecane-1,11-diyl group, and a dodecane-1,12-diyl group; C₃-C₂₀ cyclic saturated hydrocarbylene groups such as a cyclopentanediyl group, a cyclohexanediyl group, a norbornanediyl group, and an adamantanediyl group; C₂-C₂₀ unsaturated aliphatic hydrocarbylene groups such as a vinylene group and a propene-1,3-diyl group; C₆-C₂₀ arylene groups such as a phenylene group and a naphthylene group; and combinations thereof. The C₁-C₂₀ (p+1)-valent hydrocarbon group L^{A} may be saturated or unsaturated, and may be straight, branched, or cyclic. Specific examples thereof include groups obtained by further removing one or two hydrogen atoms from the specific examples of the C₁-C₂₀ hydrocarbylene group described above.

In formula (F1), Rf¹ and Rf² are each independently a hydrogen atom, a fluorine atom, or a trifluoromethyl group, and at least one of Rf¹ and Rf² is a fluorine atom or a trifluoromethyl group.

In formula (F1), R³⁰¹ is a hydroxy group, a carboxy group, a C₁-C₆ saturated hydrocarbyl group, a C₁-C₆ saturated hydrocarbyloxy group, a C₂-C₆ saturated hydrocarbylcarbonyloxy group, a fluorine atom, a chlorine atom, a bromine atom, -N(R^{301A})(R^{301B}), -N(R^{301C})-C(=O)-R^{301D}, or -N(R^{301C})-C(=O)-O-R^{301D}, R^{301A} and R^{301B} are each independently a hydrogen atom or a C₁-C₆ saturated hydrocarbyl group, R^{301C} is a hydrogen atom or a C₁-C₆ saturated hydrocarbyl group, and R^{301D} is a C₁-C₆ saturated hydrocarbyl group or a C₂-C₈ unsaturated aliphatic hydrocarbyl group.

The C₁-C₆ saturated hydrocarbyl groups R³⁰¹, R^{301A}, R^{301B}, and R^{301C} may be straight, branched, or cyclic, and specific examples thereof include C₁-C₆ alkyl groups such as a methyl group, an ethyl group, an n-propyl group, an isopropyl group, an n-butyl group, an isobutyl group, a sec-butyl group, a tert-butyl group, an n-pentyl group, and an n-hexyl group; and C₃-C₆ cyclic saturated hydrocarbyl groups such as a cyclopropyl group, a cyclobutyl group, a cyclopentyl group, and a cyclohexyl group. Examples of the saturated hydrocarbyl moiety of the C₁-C₆ saturated hydrocarbyloxy group R³⁰¹ are as exemplified above for the specific examples of the saturated hydrocarbyl group, and examples of the saturated hydrocarbyl moiety of the C₂-C₆ saturated hydrocarbylcarbonyloxy group R³⁰¹ include the C₁-C₃ saturated hydrocarbyl group among the specific examples of the C₁-C₆ saturated hydrocarbyl group described above.

The C₂-C₈ unsaturated aliphatic hydrocarbyl group R^{301D} may be straight, branched, or cyclic, and specific examples thereof include C₂-C₈ alkenyl groups such as a vinyl group, a propenyl group, a butenyl group, and a hexenyl group; C₂-C₈ alkynyl groups such as an ethynyl group, a propynyl group, and a butynyl group; and C₃-C₈ cyclic unsaturated aliphatic hydrocarbyl groups such as a cyclohexenyl group and a norbornenyl group.

In formula (F1), R³⁰² is a C₁-C₂₀ saturated hydrocarbylene group or a C₆-C₂₀ arylene group, some or all of hydrogen atoms of the saturated hydrocarbylene group may be substituted by a halogen atom other than a fluorine atom, and some or all of hydrogen atoms of the arylene group may be substituted by a substituent selected from a C₁-C₂₀ saturated hydrocarbyl group, a C₁-C₂₀ saturated hydrocarbyloxy group, a C₆-C₂₀ aryl group, a halogen atom, and a hydroxy group.

The C₁-C₂₀ hydrocarbylene group R³⁰² may be saturated or unsaturated, and may be straight, branched, or cyclic. Specific examples thereof are as exemplified above for the C₁-C₂₀ hydrocarbylene group L^{A}.

Specific examples of the C₆-C₂₀ arylene group R³⁰² include a phenylene group, a naphthylene group, a phenanthrenediyl group, and an anthracenediyl group. The hydrocarbyl moiety of the C₁-C₂₀ saturated hydrocarbyl group and the C₁-C₂₀ hydrocarbyloxy group which are the substituents of the arylene group may be straight, branched, or cyclic, and specific examples thereof include C₁-C₂₀ alkyl groups such as a methyl group, an ethyl group, an n-propyl group, an isopropyl group, an n-butyl group, an isobutyl group, a sec-butyl group, a tert-butyl group, an n-pentyl group, an n-hexyl group, an n-octyl group, an n-nonyl group, an n-decyl group, an undecyl group, a dodecyl group, a tridecyl group, a tetradecyl group, a pentadecyl group, a heptadecyl group, an octadecyl group, a nonadecyl group, and an icosyl group; and C₃-C₂₀ cyclic saturated hydrocarbyl groups such as a cyclopropyl group, a cyclopentyl group, a cyclohexyl group, a cyclopropylmethyl group, a 4-methylcyclohexyl group, a cyclohexylmethyl group, a norbornyl group, and an adamantyl group. Specific examples of the C₆-C₁₄ arylene group which is a substituent of the arylene group include a phenylene group, a naphthylene group, a phenanthrenediyl group, and an anthracenediyl group.

The anion having formula (F1) is preferably an anion having the formula (F2).

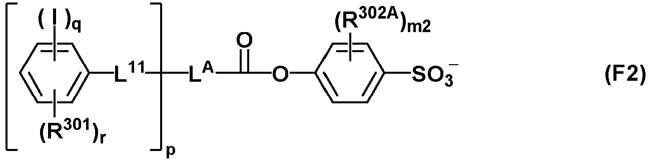

In formula (F2), p, q, r, L¹¹, L^{A}, and R³⁰¹ are as defined above, m2 is 1, 2, 3, or 4, and R^{302A} is a C₁-C₂₀ saturated hydrocarbyl group, a C₁-C₂₀ saturated hydrocarbyloxy group, a C₆-C₁₄ aryl group, a halogen atom, or a hydroxy group. When m2 is 2, 3, or 4, each R^{302A} may be identical or different.

Specific examples of the anion having formula (F1) are shown below, but are not limited thereto.

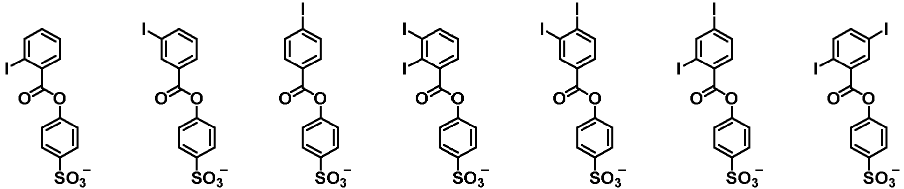

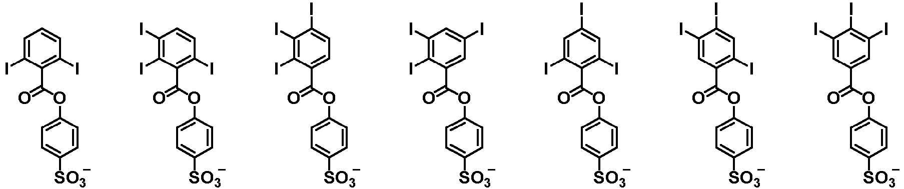

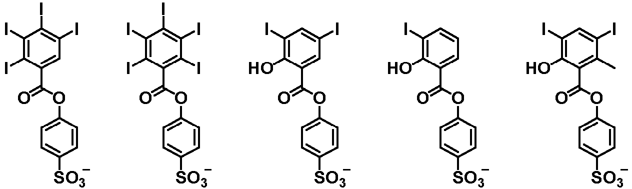

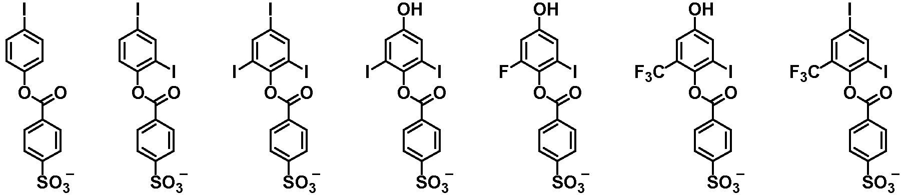

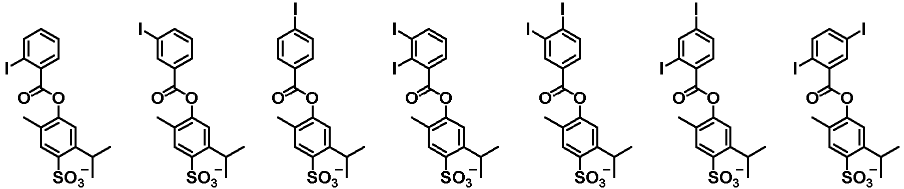

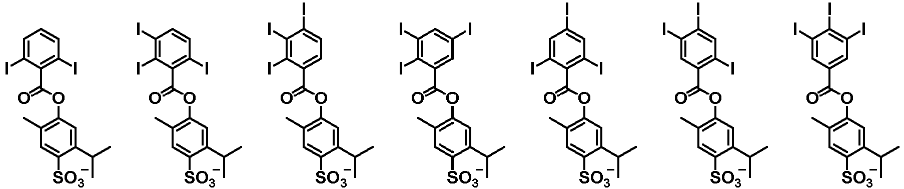

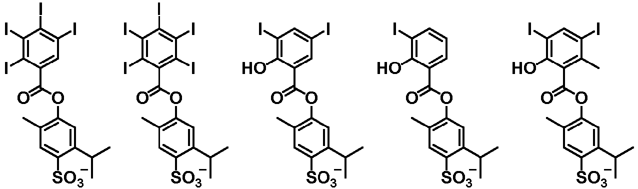

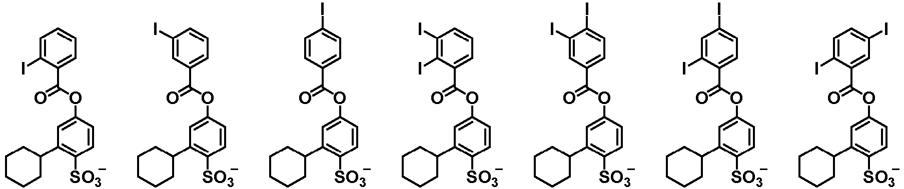

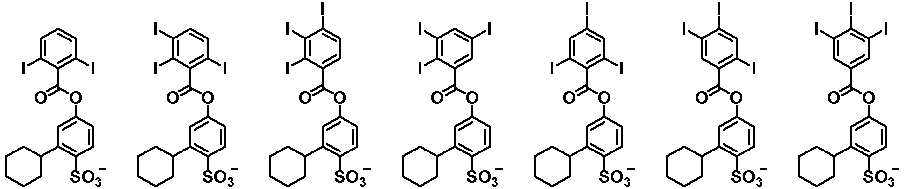

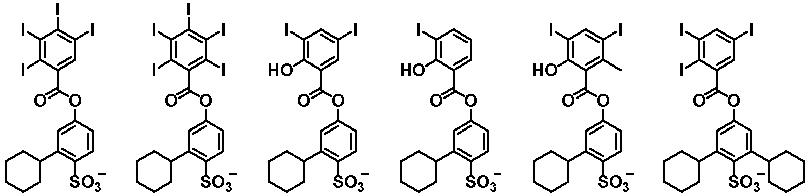

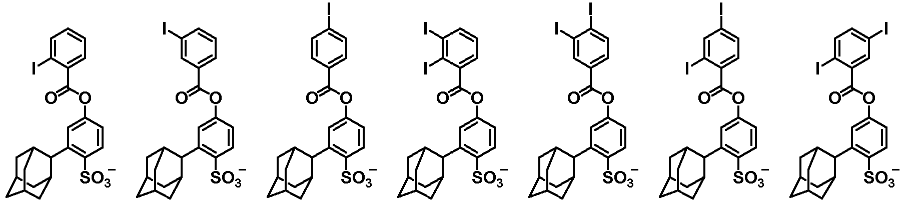

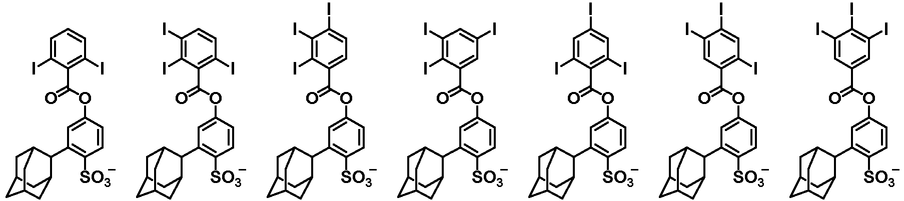

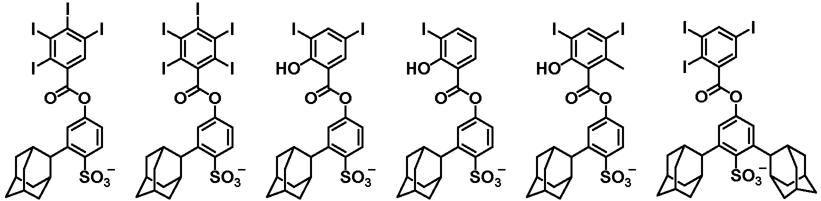

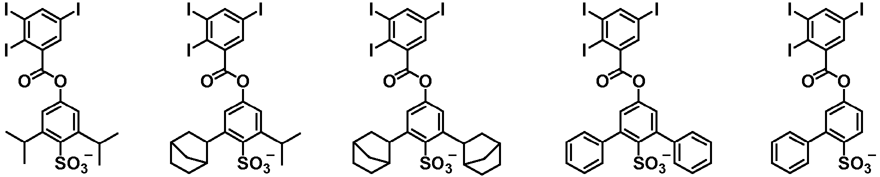

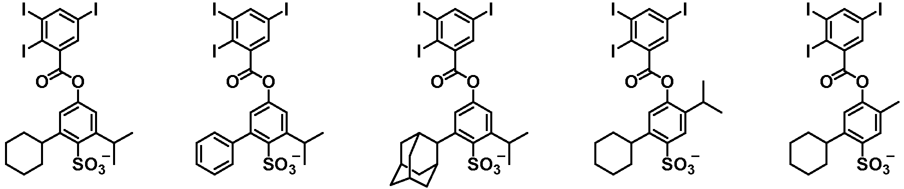

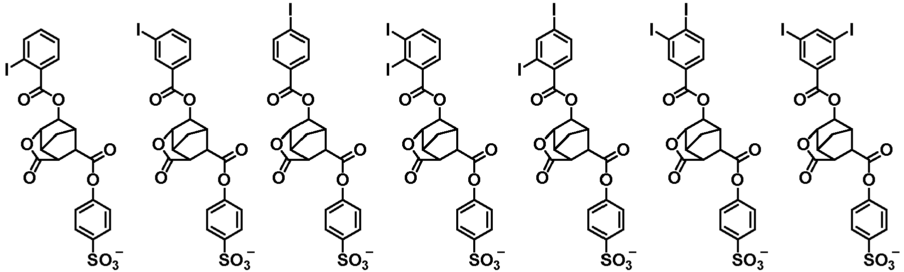

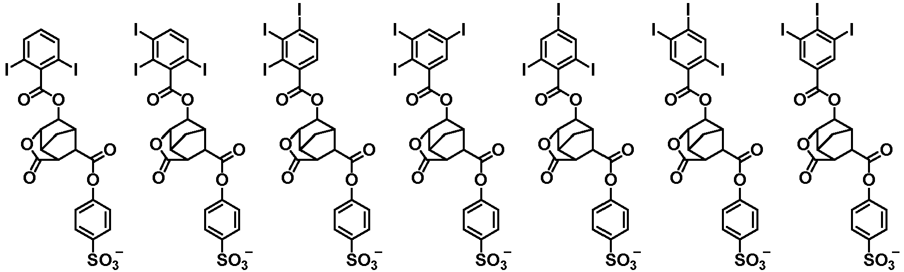

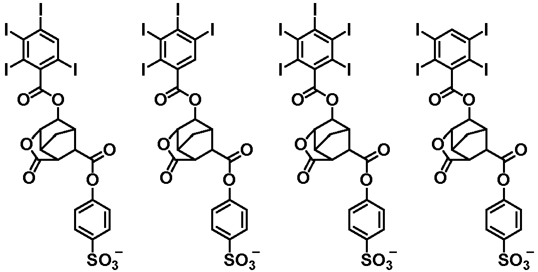

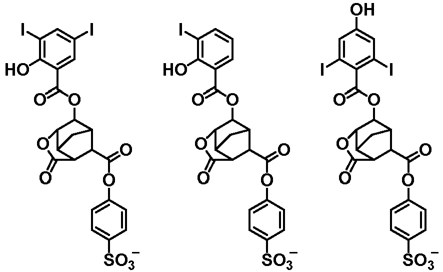

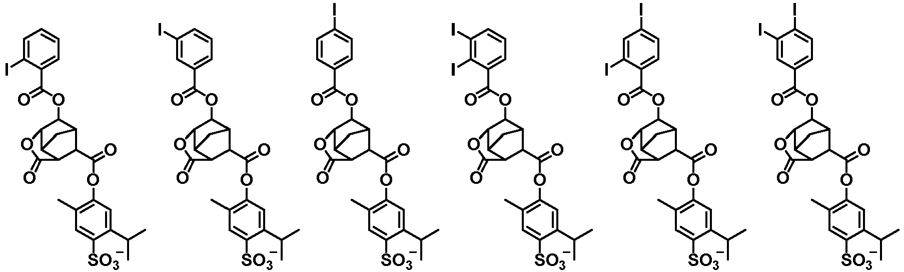

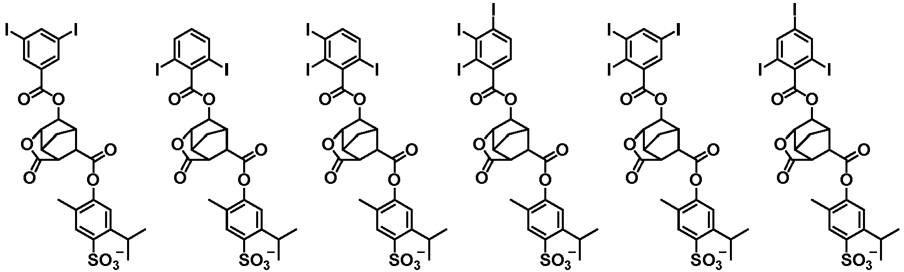

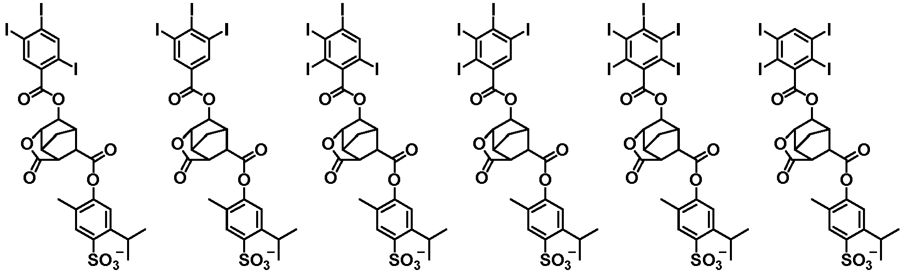

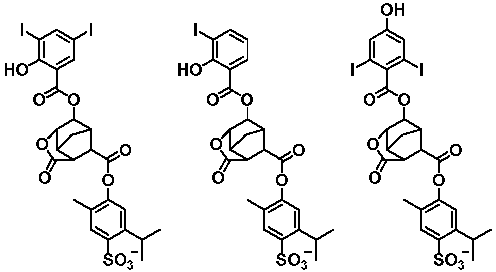

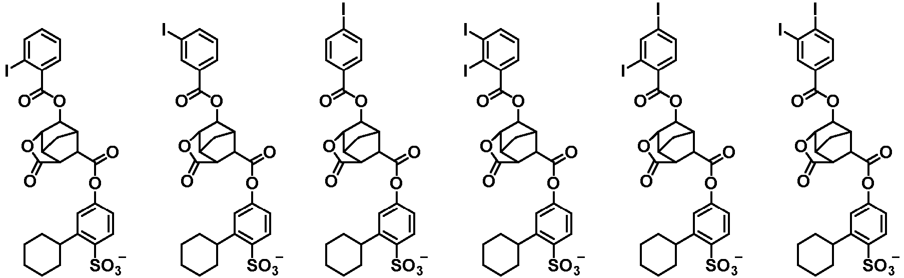

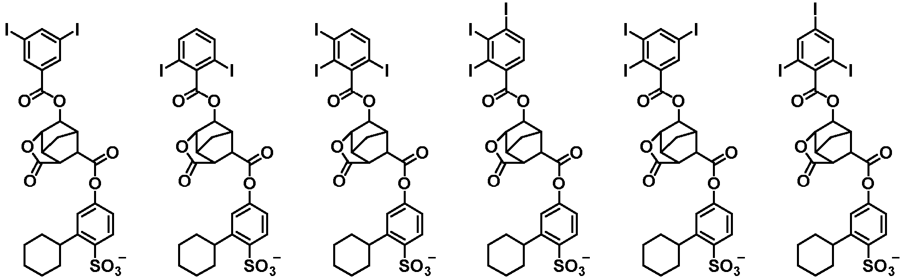

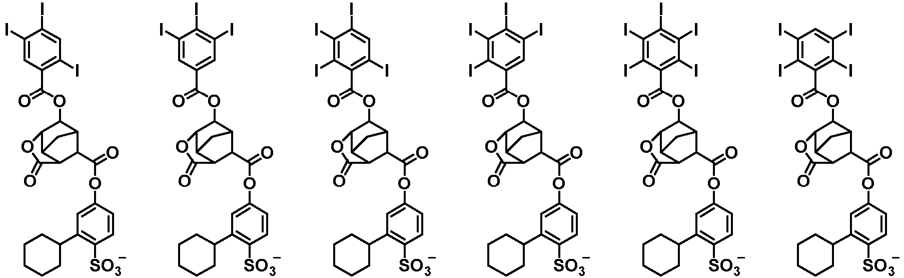

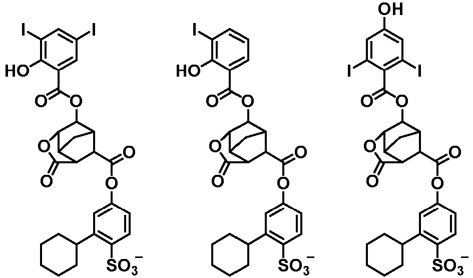

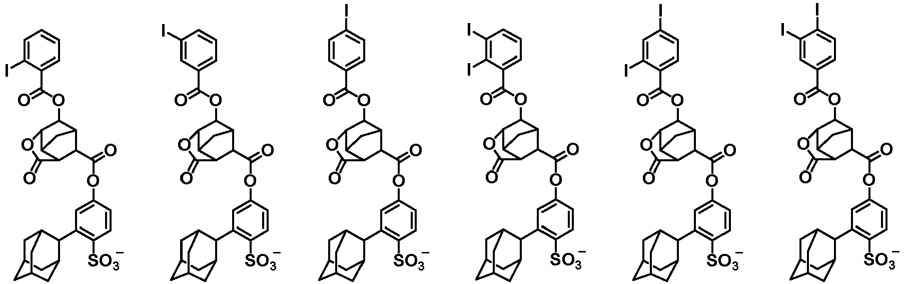

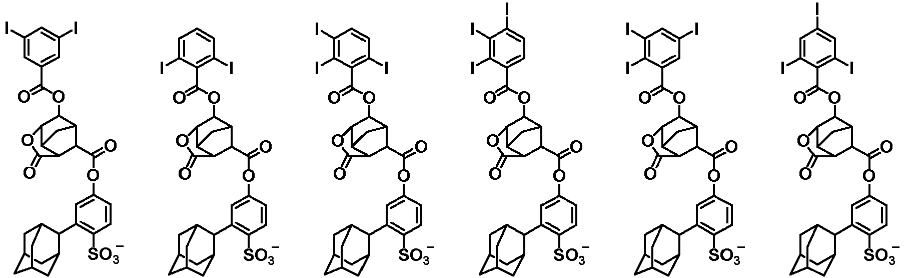

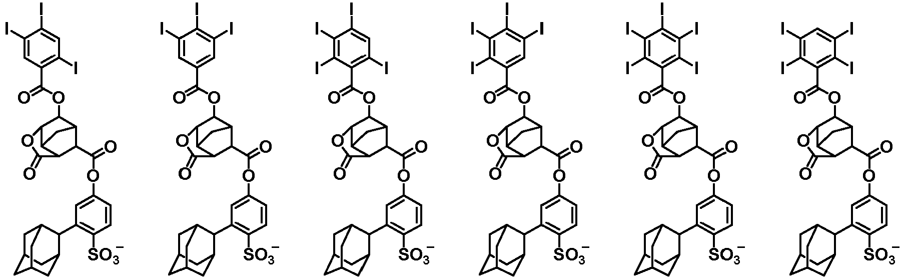

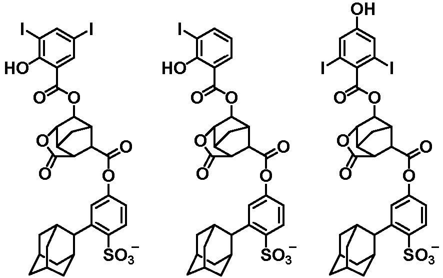

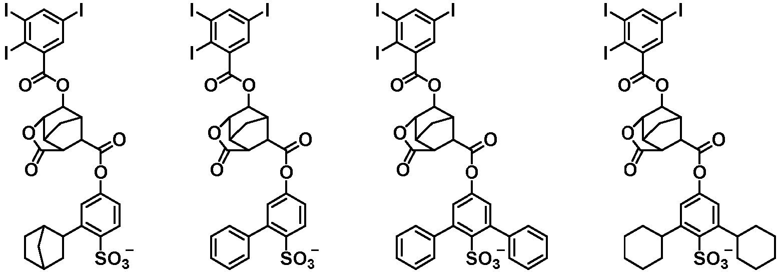

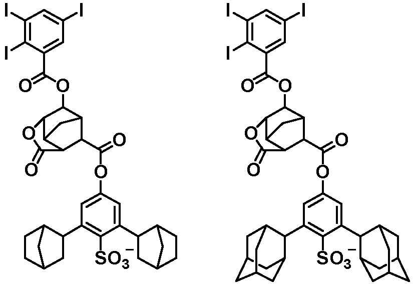

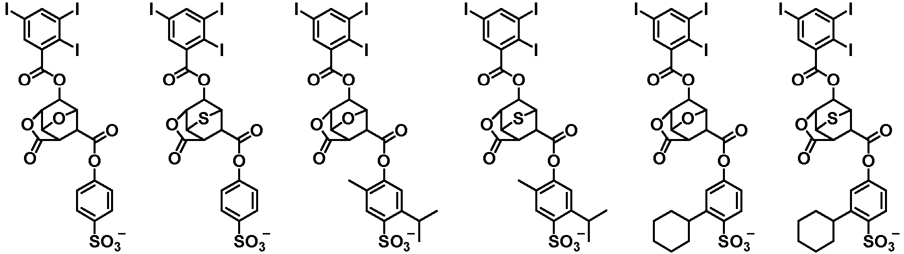

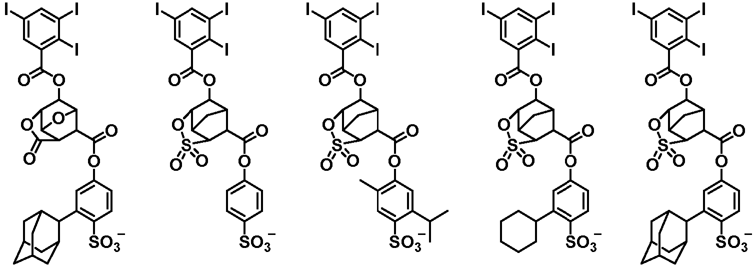

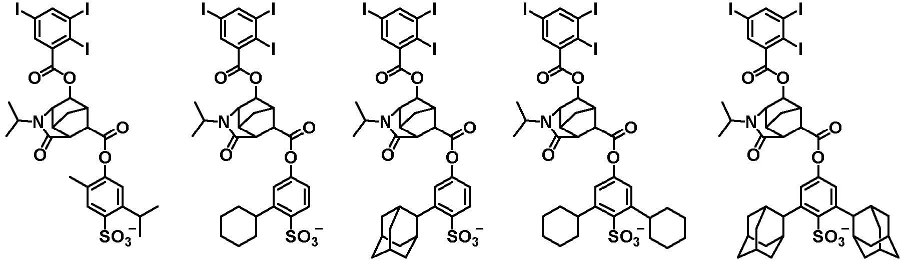

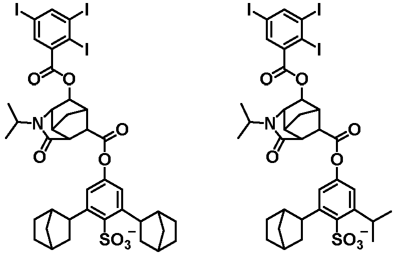

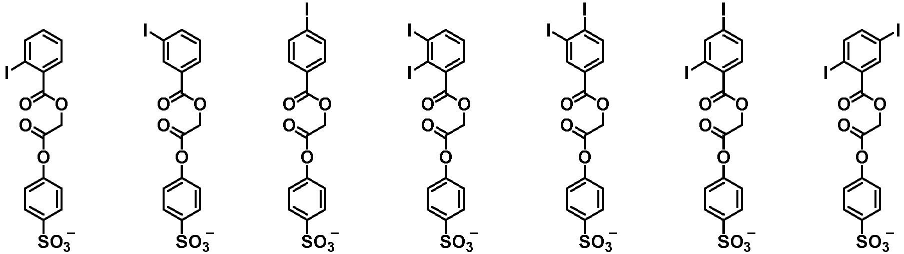

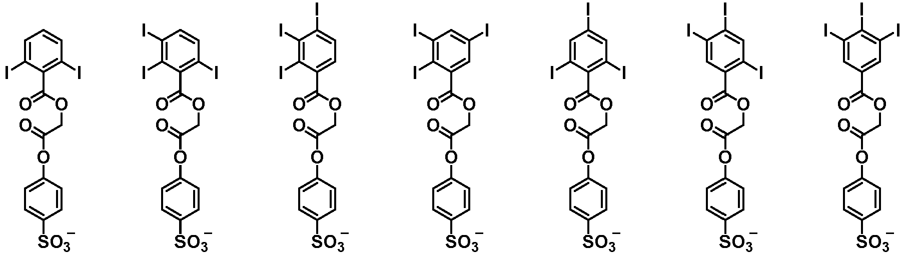

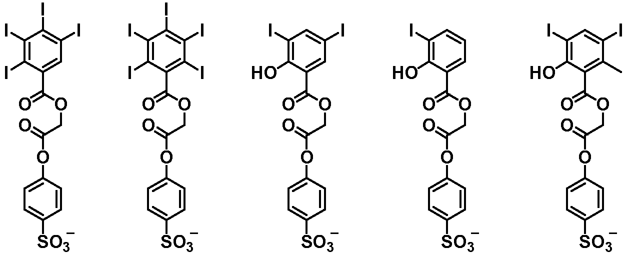

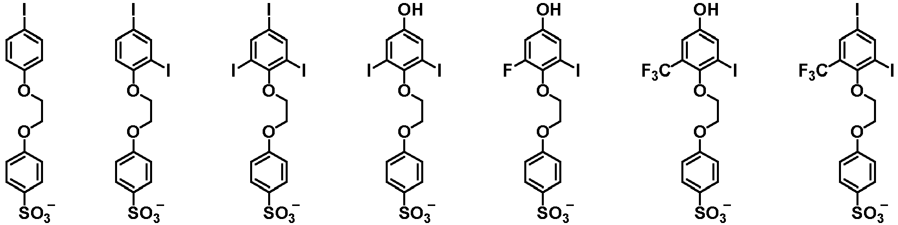

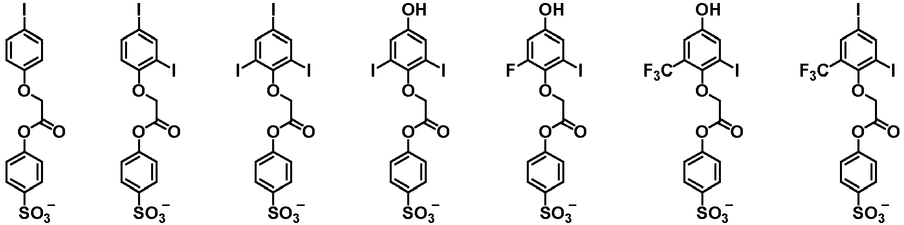

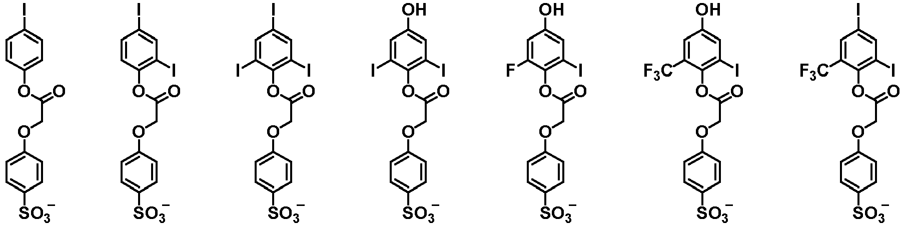

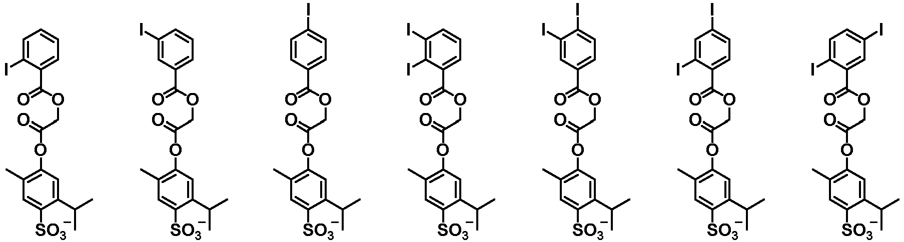

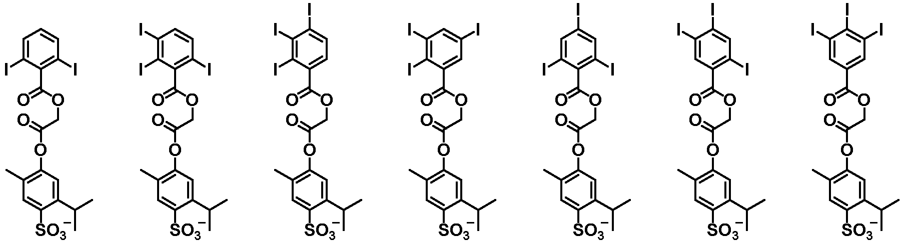

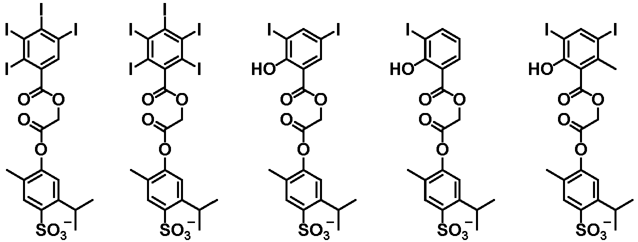

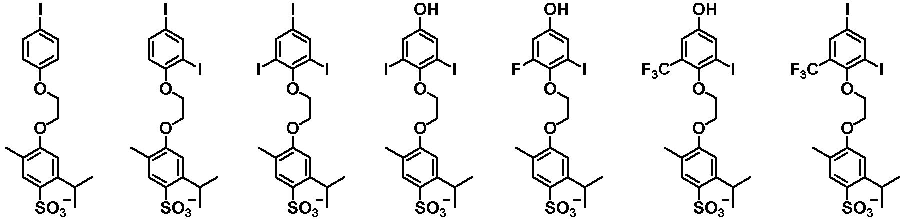

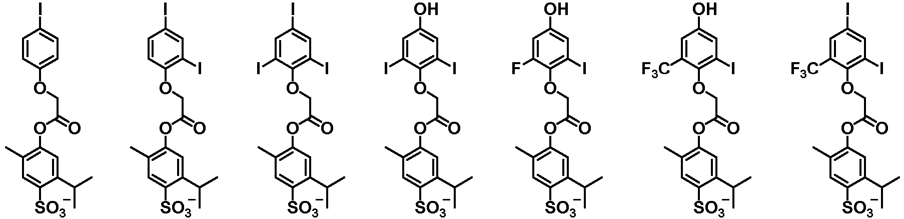

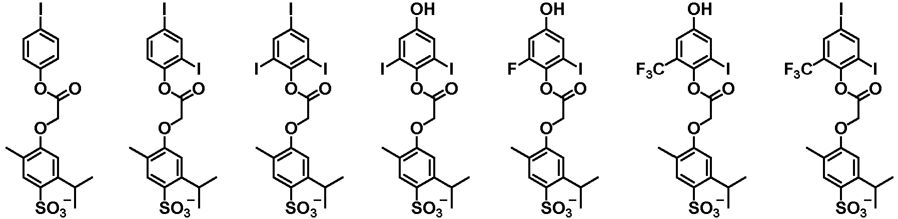

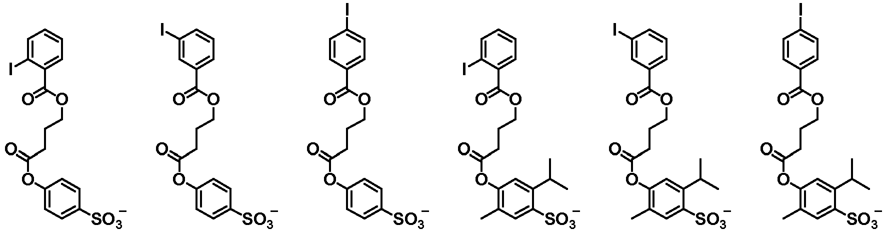

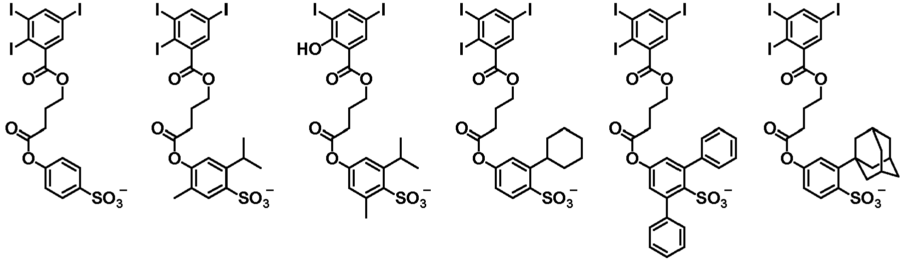

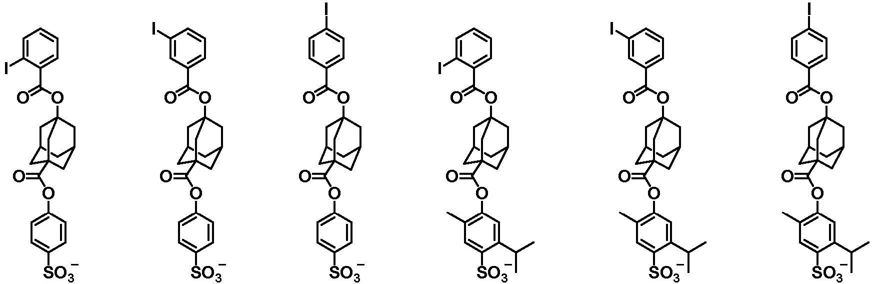

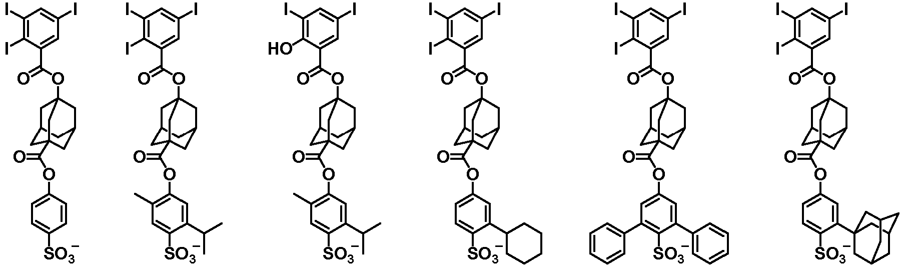

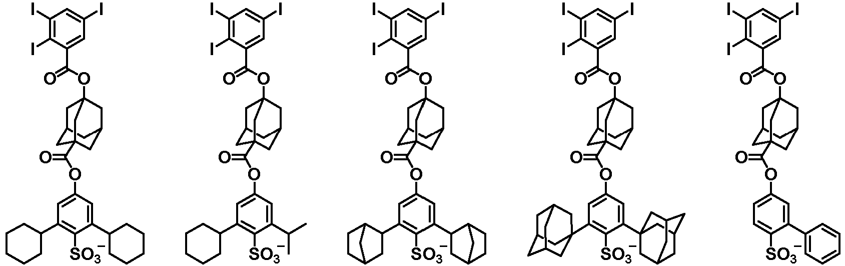

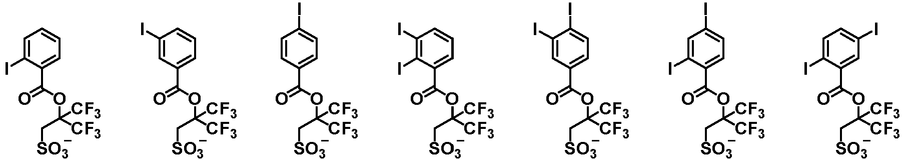

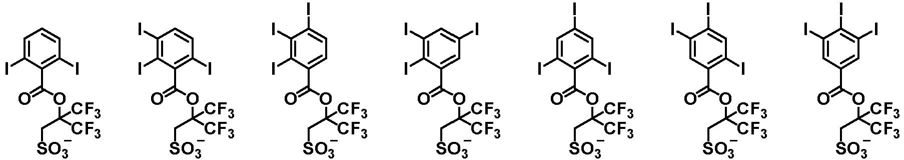

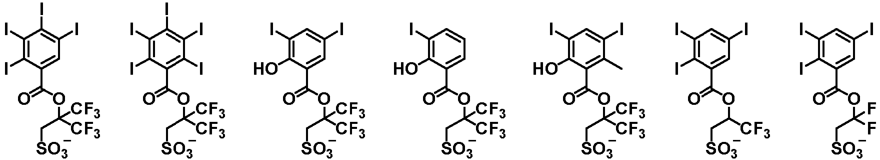

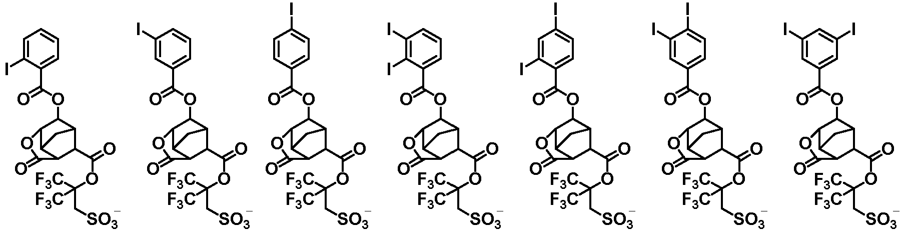

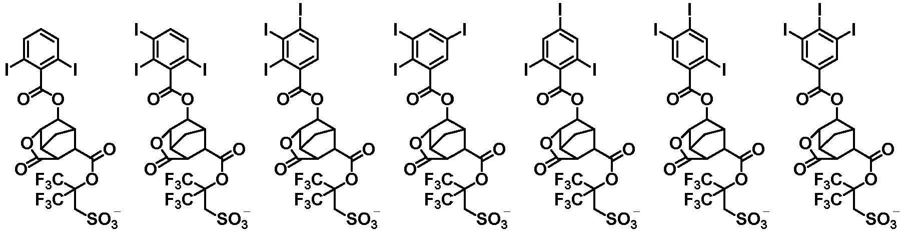

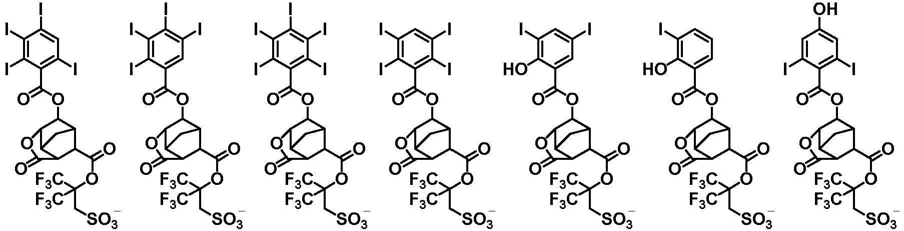

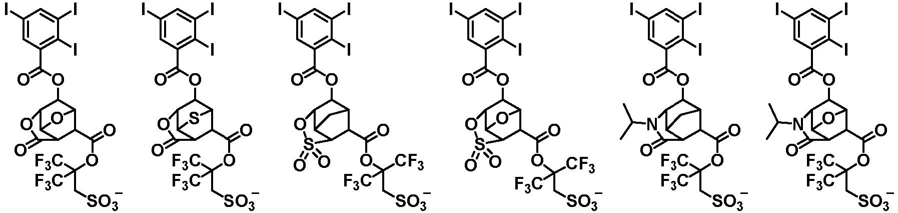

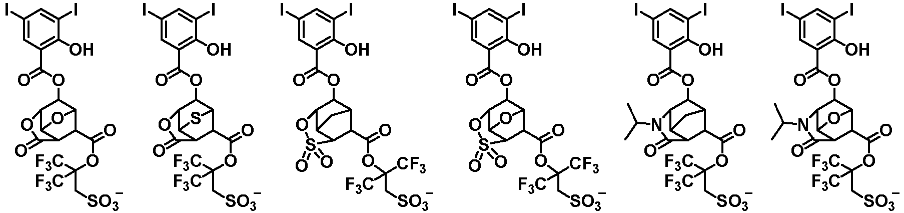

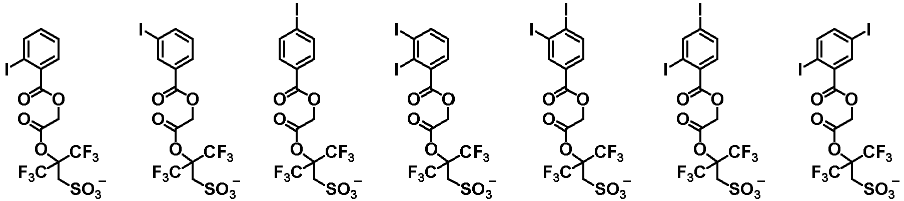

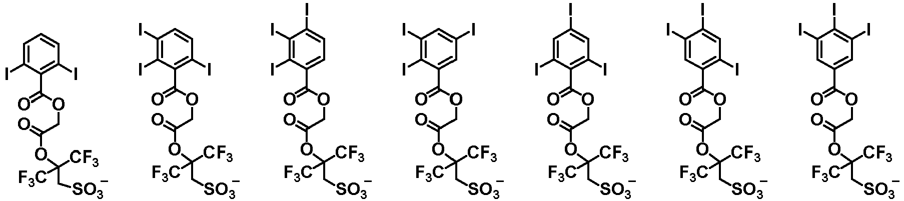

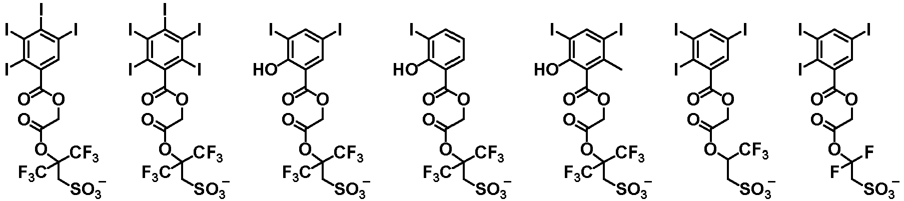

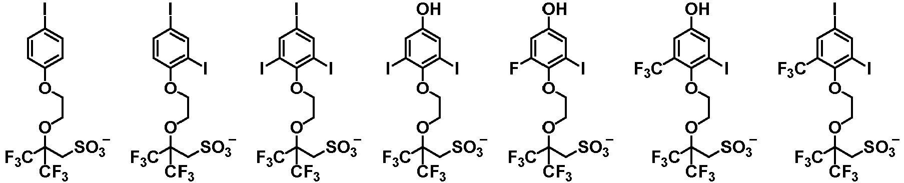

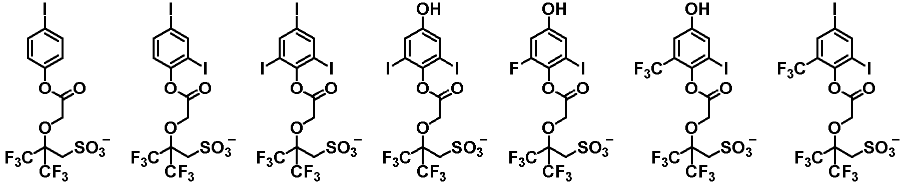

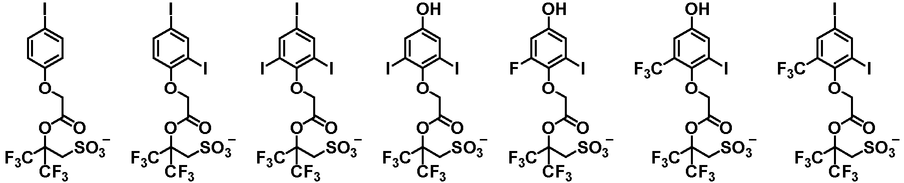

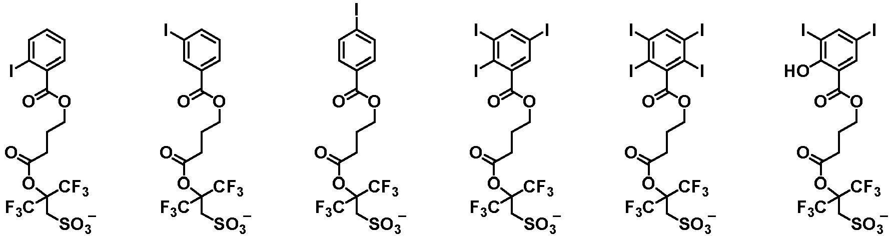

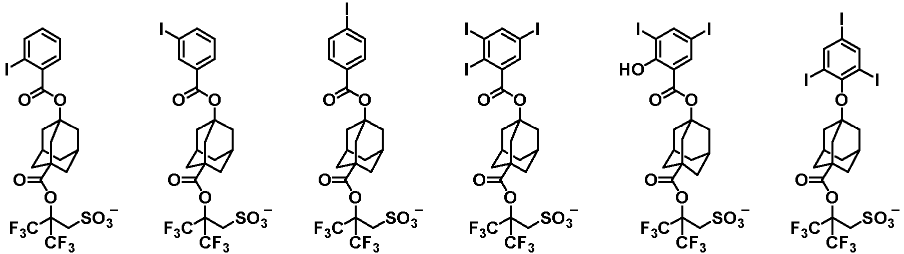

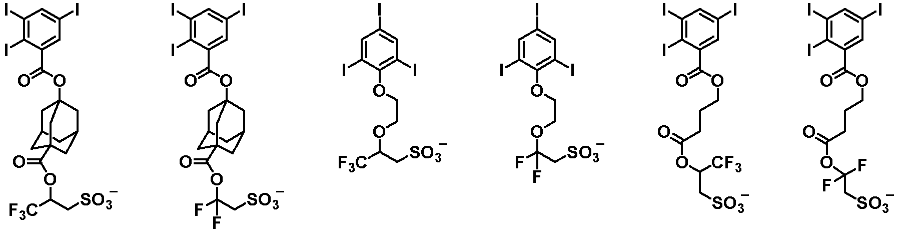

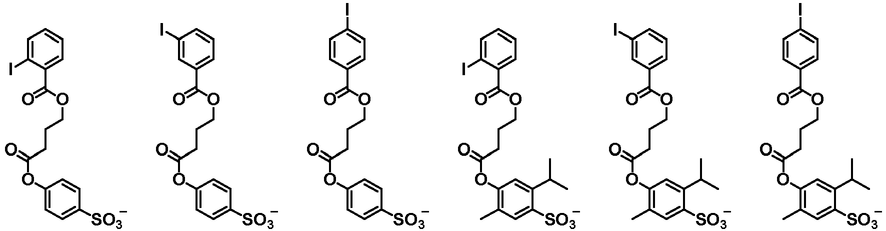

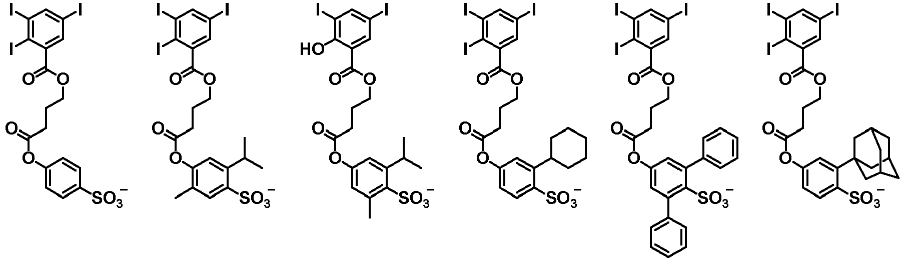

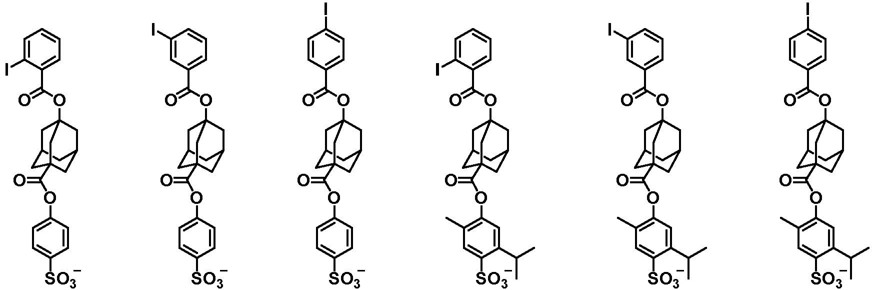

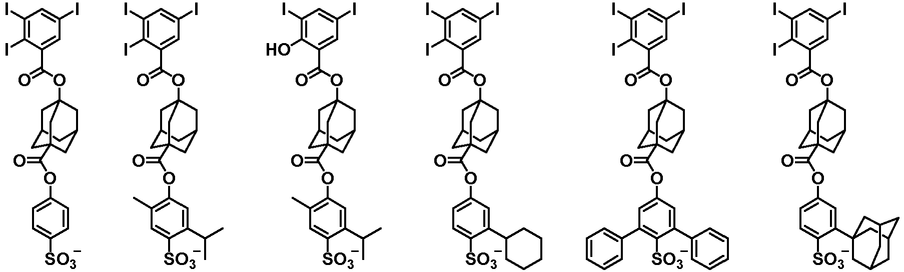

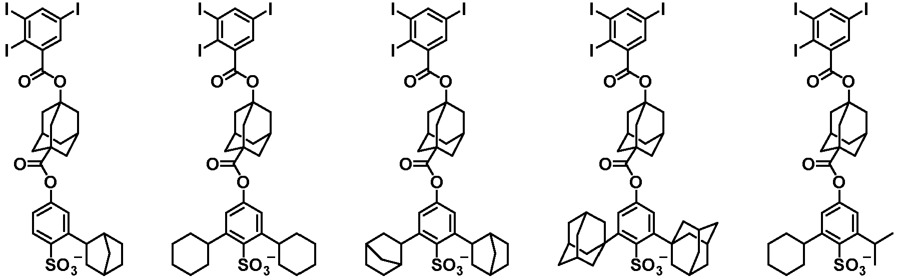

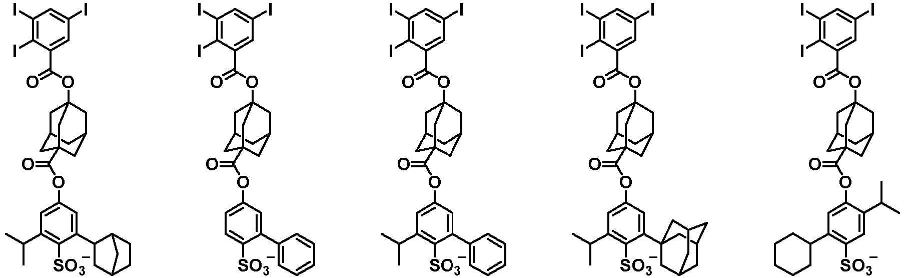

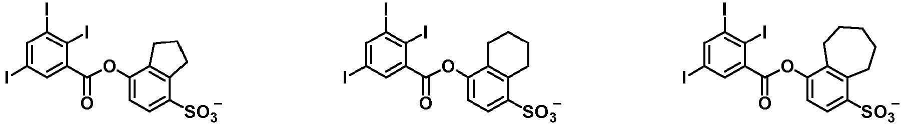

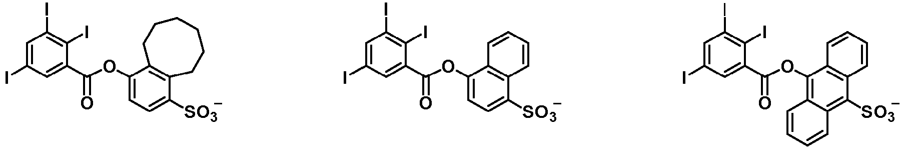

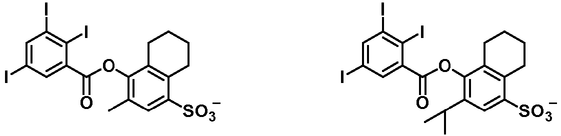

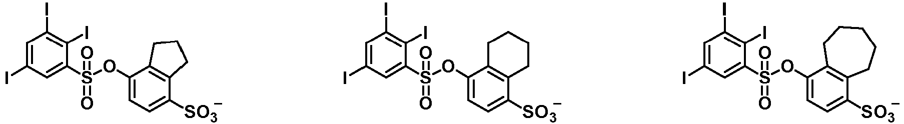

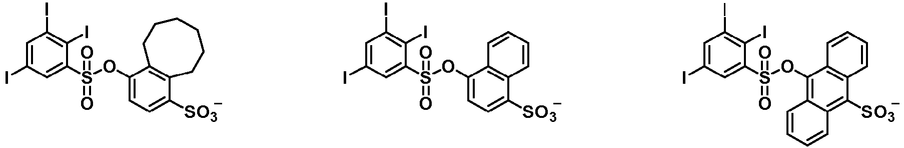

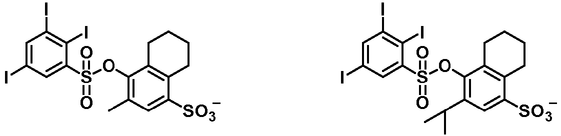

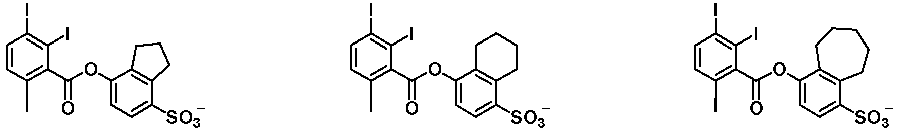

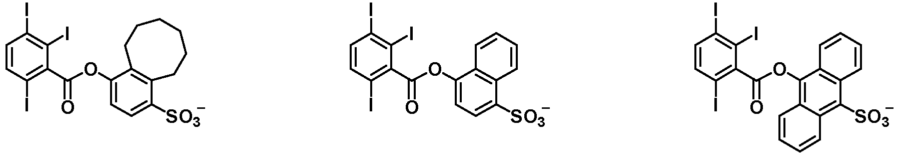

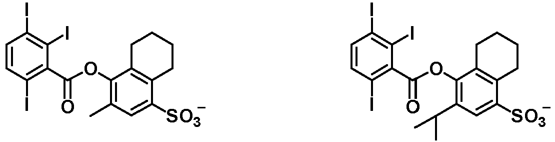

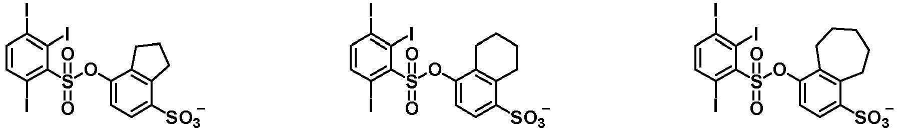

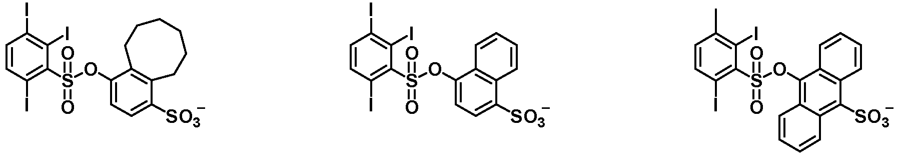

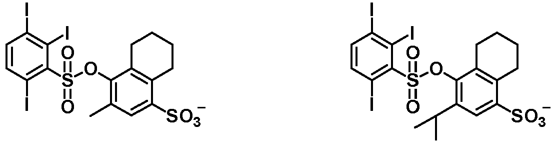

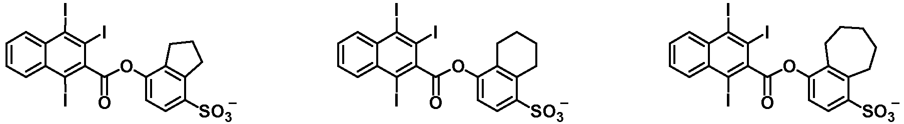

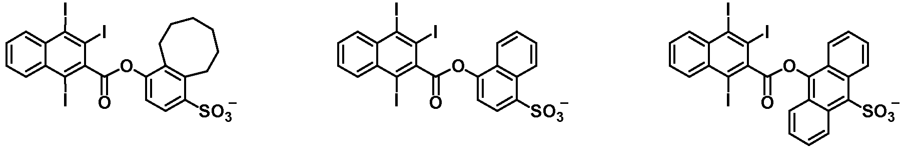

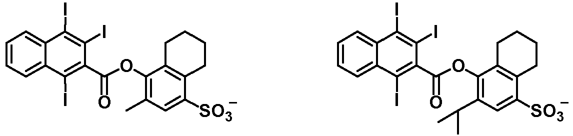

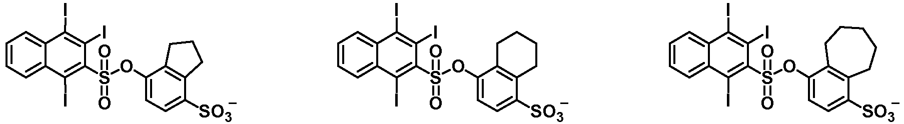

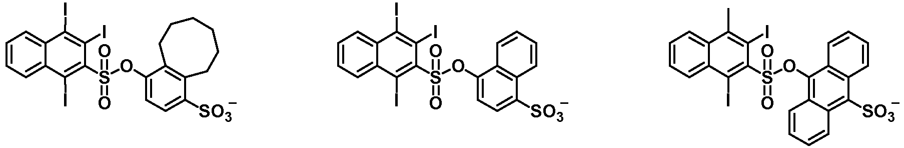

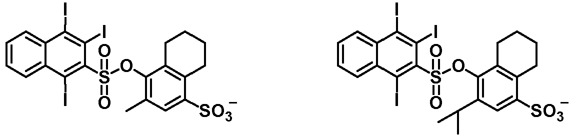

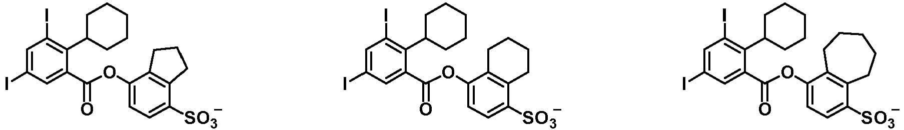

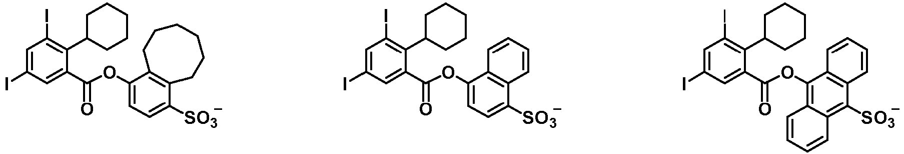

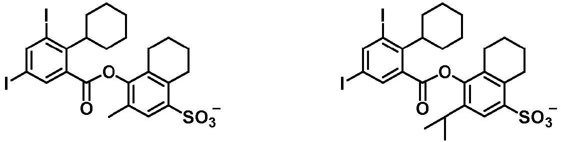

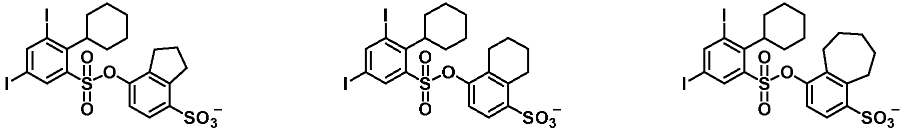

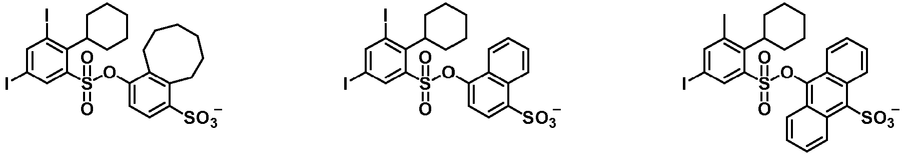

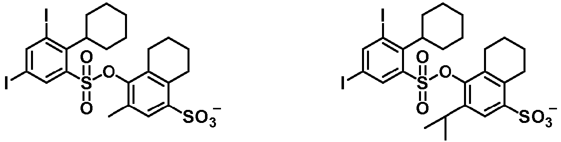

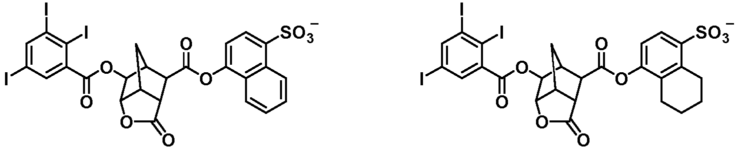

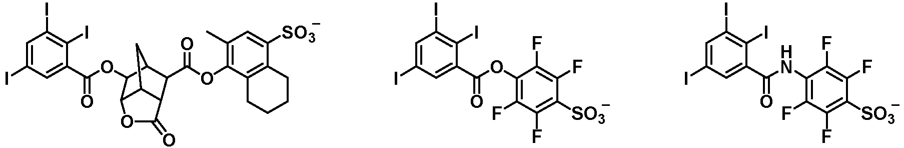

In the photoacid generator (F), a sulfonium cation or an iodonium cation is preferred as a cation that pairs with the anion described above. Specific examples of the sulfonium cation are as exemplified above for the sulfonium cation having formula (cation-1), but are not limited thereto. Specific examples of the iodonium cation are as exemplified above for the iodonium cation having formula (cation-2), but are not limited thereto.

The acid generated by the photoacid generator preferably has a pKa of -2.0 or more, more preferably -1.0 or more. The upper limit of pKa is preferably 2.0. The pKa value is calculated using pKa DB in software ACD/Chemsketch ver: 9.04 (Advanced Chemistry Development, Inc.).

In the chemically amplified negative resist composition of the present invention, the content of the photoacid generator (F) is preferably 1 to 10 parts by weight, more preferably 1 to 5 parts by weight per 80 parts by weight of the base polymer. By comprising the photoacid generator (F), it is possible to appropriately adjust the amount of acid generated in the exposed areas and the solubility inhibition property of the unexposed areas. The photoacid generator (F) may be of one type or a combination of plural types.

### (G) Surfactant

The chemically amplified negative resist composition of the present invention may comprise any conventional surfactants for facilitating to coat the composition to the substrate. Examples of the surfactants include PF-636 (Omnova Solutions Inc.) and FC-4430 (3M) as well as a number of known surfactants as described in JP-A 2004-115630. Any suitable one may be chosen therefrom. The content of the surfactant (G) in the chemically amplified negative resist composition of the present invention is preferably 0 to 5 parts by weight per 80 parts by weight of the base polymer (B). The surfactant (G) may be of one type or a combination of plural types.

### Resist pattern forming process

Another embodiment of the invention is a resist pattern forming process comprising the steps of: applying the chemically amplified negative resist composition onto a substrate to form a resist film thereon, exposing the resist film patternwise to high-energy radiation (that is, exposing the resist film using high-energy radiation), and developing the exposed resist film in an alkaline developer.

As the substrate, for example, a substrate for integrated circuit fabrication (Si, SiO, SiO₂, SiN, SiON, TiN, WSi, BPSG, SOG, organic antireflective coating, and the like) or a substrate for transmissive or reflective mask circuit fabrication (Cr, CrO, CrON, MoSi₂, Si, SiO, SiO₂, SiON, SiONC, CoTa, NiTa, TaBN, SnO₂, and the like) can be used. The chemically amplified negative resist composition is applied onto the substrate by a method such as spin coating so as to have a film thickness of 0.03 to 2 µm, and prebaked on a hotplate at preferably 60 to 150°C for 1 to 20 minutes, more preferably 80 to 140°C for 1 to 10 minutes to form a resist film.

Then, the resist film is exposed patternwise to high-energy radiation. Examples of the high-energy radiation include UV, deep-UV, excimer laser (KrF, ArF, and the like), EUV of wavelength 3 to 15 nm, X-ray, γ-ray, synchrotron radiation, and EB. In the present invention, it is preferable to perform exposure using EUV or EB.

On use of UV, deep-UV, excimer laser, EUV, X-ray, γ-ray, or synchrotron radiation as the high-energy radiation, the resist film is exposed through a mask having a desired pattern, preferably in a dose of 1 to 500 mJ/cm², more preferably 10 to 400 mJ/cm². On use of EB, a desired pattern may be written directly in a dose of preferably 1 to 500 µC/cm², more preferably 10 to 400 µC/cm².

The exposure may be performed by conventional lithography whereas the immersion lithography of holding a liquid, typically water between the mask and the resist film may be employed if desired. In the case of immersion lithography, a protective film which is insoluble in water may be used.

The resist film is then subjected to post-exposure baking (PEB) on a hotplate preferably at 60 to 150°C for 1 to 20 minutes, more preferably at 80 to 140°C for 1 to 10 minutes.

Thereafter, the resist film is developed with a developer in the form of an aqueous base solution, for example, 0.1 to 5 wt%, preferably 2 to 3 wt% aqueous solution of tetramethylammonium hydroxide (TMAH) preferably for 0.1 to 3 minutes, more preferably 0.5 to 2 minutes by conventional techniques such as dip, puddle, and spray techniques. In this way, a desired resist pattern is formed on the substrate.

From the chemically amplified negative resist composition of the present invention, a pattern with a high resolution and minimal LER can be formed. The chemically amplified negative resist composition of the present invention is particularly useful for pattern formation on a substrate having a material which is likely to cause pattern stripping or pattern collapse on the surface, because adhesion of a resist pattern is difficult to be obtained. Examples of such a substrate include a substrate in which a chromium compound containing one or more light elements selected from metal chromium, oxygen, nitrogen, and carbon is deposited on the outermost surface by sputtering, and a substrate containing SiO, SiOₓ, a tantalum compound, a molybdenum compound, a cobalt compound, a nickel compound, a tungsten compound, and a tin compound on the outermost surface. The chemically amplified negative resist composition of the present invention is particularly useful for pattern formation using a photomask blank as a substrate. At this time, the photomask blank may be a transmission type or a reflective type.

The mask blank of transmission type may be a photomask blank having a light shielding film of chromium-based material. It may be either a photomask blank for binary masks or a photomask blank for phase shift masks. In the case of the binary mask-forming photomask blank, the light-shielding film may include an antireflection layer of chromium-based material and a light-shielding layer. In one example, the antireflection layer on the surface layer side is entirely composed of a chromium-based material. In an alternative example, only a surface side portion of the antireflection layer on the surface layer side is composed of a chromium-based material and the remaining portion is composed of a silicon compound-based material which may contain a transition metal. In the case of the phase shift mask-forming photomask blank, it may include a phase shift film and a chromium-based light-shielding film thereon.

Photomask blanks having an outermost layer of chromium base material are well known as described in JP-A 2008-26500 and JP-A 2007-302873 and the references cited therein. Although the detail description is omitted herein, the following layer construction may be employed when a light-shielding film including an antireflective layer and a light shielding layer is composed of chromium base materials.

In the example where a light-shielding film including an antireflective layer and a light-shielding layer is composed of chromium base materials, layers may be stacked in the order of an antireflective layer and a light-shielding layer from the outer surface side, or layers may be stacked in the order of an antireflective layer, a light-shielding layer, and an antireflective layer from the outer surface side. Each of the antireflective layer and the light-shielding layer may be composed of multiple sub-layers. When the sub-layers have different compositions, the composition may be graded discontinuously or continuously from sub-layer to sub-layer. The chromium base material used herein may be metallic chromium or a material consisting of metallic chromium and a light element such as oxygen, nitrogen or carbon. Examples used herein include metallic chromium, chromium oxide, chromium nitride, chromium carbide, chromium oxynitride, chromium oxycarbide, chromium nitride carbide, and chromium oxide nitride carbide.

The mask blank of reflection type includes a substrate, a multilayer reflective film formed on one major surface (front surface) of the substrate, for example, a multilayer reflective film of reflecting exposure radiation such as EUV radiation, and an absorber film formed on the multilayer reflective film, for example, an absorber film of absorbing exposure radiation such as EUV radiation to reduce reflectivity. From the reflection type mask blank (reflection type mask blank for EUV lithography), a reflection type mask (reflection type mask for EUV lithography) having an absorber pattern (patterned absorber film) formed by patterning the absorber film is produced. The EUV radiation used in the EUV lithography has a wavelength of 13 to 14 nm, typically about 13.5 nm.

The multilayer reflective film is preferably formed contiguous to one major surface of a substrate. An underlay film may be disposed between the substrate and the multilayer reflective film as long as the benefits of the invention are not lost. The absorber film may be formed contiguous to the multilayer reflective film while a protective film (protective film for the multilayer reflective film) may be disposed between the multilayer reflective film and the absorber film, preferably contiguous to the multilayer reflective film, more preferably contiguous to the multilayer reflective film and the absorber film. The protective film is used for protecting the multilayer reflective film in a cleaning, tailoring or otherwise processing step. Also preferably, the protective film has an additional function of protecting the multilayer reflective film or preventing the multilayer reflective film from oxidation during the step of patterning the absorber film by etching. Besides, an electroconductive film, which is used for electrostatic chucking of the reflection type mask to an exposure tool, may be disposed below the other major surface (back side surface) which is opposed to the one major surface of the substrate, preferably contiguous to the other major surface. It is provided herein that a substrate has one major surface which is a front or upper side surface and another major surface which is a back or lower side surface. The terms "front and back" sides or "upper and lower" sides are used for the sake of convenience. One or another major surface may be either of the two major surfaces (film bearing surfaces) of a substrate, and in this sense, front and back or upper and lower are exchangeable. Specifically, the multilayer reflective film may be formed by any of the methods of JP-A 2021-139970 and the references cited therein.

The resist pattern forming process is successful in forming patterns having a very high resolution, reduced LER, rectangularity, and fidelity even on a substrate (typically mask blank of transmission or reflection type) whose outermost surface is made of a material tending to affect resist pattern profile such as a chromium, silicon, or tantalum containing material.

### EXAMPLES

Synthesis Examples, Examples, and Comparative Examples are given below for specifically illustrating the present invention although the present invention is not limited thereto. The device used is as follows.
MALDI TOF-MS: S3000 (JEOL, Ltd.)

### [1] Synthesis of onium salt monomer

### Example 1-1

### Synthesis of onium salt monomer PP-1

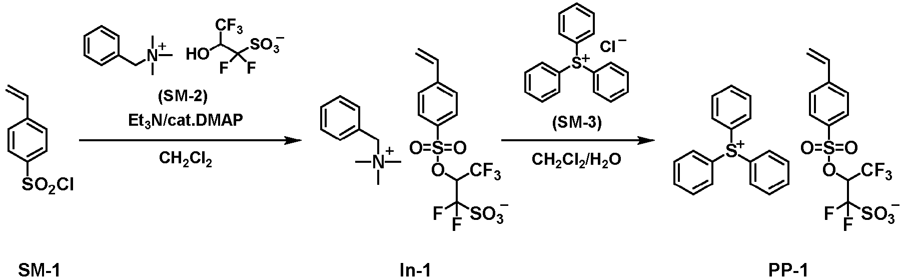

### (1) Synthesis of Intermediate In-1

In nitrogen atmosphere, a reactor was charged with 40.5 g of reactant SM-1, 83.5 g of reactant SM-2, 2.4 g of DMAP, and 250 g of methylene chloride and cooled in an ice bath. While the internal temperature of the reactor was kept at 20°C or lower, 26.3 g of triethylamine was added dropwise. After the dropwise addition, the temperature was raised to room temperature, and the reaction solution was aged for 12 hours. At the end of aging, water was added to stop the reaction, ordinary aqueous work-up was performed, the solvent was distilled off, and then, diisopropyl ether was added to wash the residue, thereby obtaining 82.9 g of Intermediate In-1 as an oily product (yield 76%).

### (2) Synthesis of onium salt monomer PP-1

In nitrogen atmosphere, a reactor was charged with 82.9 g of Intermediate In-2, 50.0 g of reactant SM-3, 300 g of methylene chloride, and 150 g of water, stirring was performed for 30 minutes, and then, the organic layer was taken out, washed with water, and then concentrated under reduced pressure. The concentrate was purified by silica gel chromatography, diisopropyl ether was added thereto, and recrystallization was performed to obtain 88.1 g of the target onium salt monomer PP-1 as white crystals (yield 88%).
MALDI TOF-MS:
POSITIVE M⁺263 (corresponding to C₁₈H₁₅S⁺)
NEGATIVE M⁻395 (corresponding to C₁₁H₈F₃O₆S₂⁻)

### Examples 1-2 to 1-7

### Synthesis of onium salt monomers PP-2 to PP-7

Onium salt monomers PP-2 to PP-7 represented by the formulae were synthesized using corresponding reactants and well-known organic synthesis reactions.

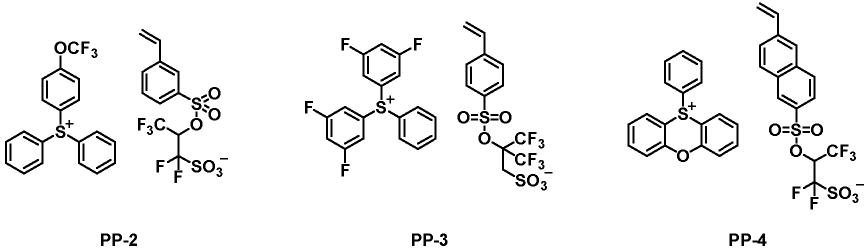

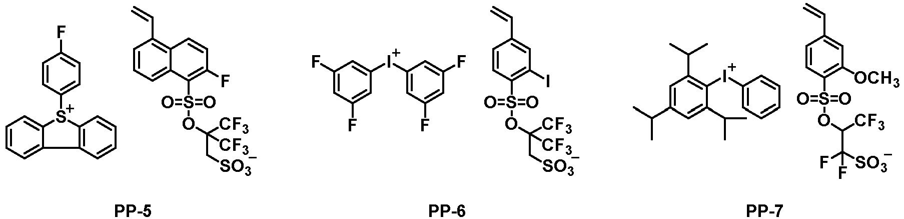

### Comparative Examples 1-1 to 1-4

### Synthesis of comparative onium salt monomers cPP-1 to cPP-4

Onium salt monomers cPP-1 to cPP-4 represented by the formulae were synthesized using corresponding reactants and well-known organic synthesis reactions.

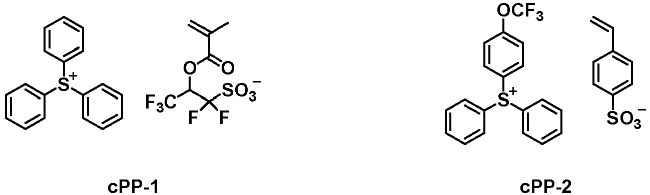

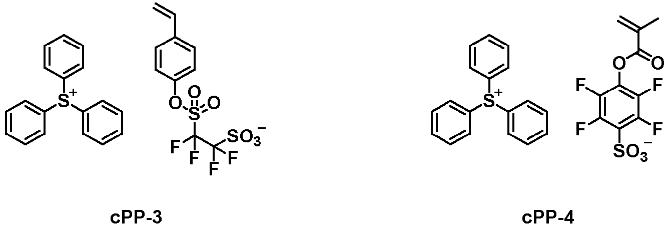

### [2] Synthesis of base polymer

### Example 2-1

### Synthesis of base polymer P-1

In nitrogen atmosphere, 71.1 g of monomer A-1, 10.6 g of monomer B-4, 18.3 g of monomer PP-1, 12.7 g of V-601 (Fujifilm Wako Pure Chemical Industries, Ltd.), and 120 g of PGME were placed in a flask to prepare a monomer-polymerization initiator solution. In nitrogen atmosphere, 60 g of PGME was placed in another flask, heating was performed to 80°C with stirring, and then, the monomer-polymerization initiator solution was added dropwise for 4 hours. After completion of the dropwise addition, stirring was continued for 18 hours while maintaining the temperature of the polymerization liquid at 80°C, and then, the polymerization liquid was cooled to room temperature. The resulting polymerization liquid was added dropwise to 5,000 g of a stirred mixed solvent of methanol and water (methanol:water = 1:9), and the precipitated polymer was separated by filtration. The resulting polymer was washed twice with 1,000 g of a mixed solvent of methanol and water (methanol:water = 1:9), and then vacuum-dried at 40°C for 20 hours to obtain 85.4 g of white powdery polymer P-1. The polymer P-1 was measured by ¹³C-NMR, ¹H-NMR, and GPC, and the following analysis results were obtained. Mw is measured by GPC versus polystyrene standards using DMF as a solvent.

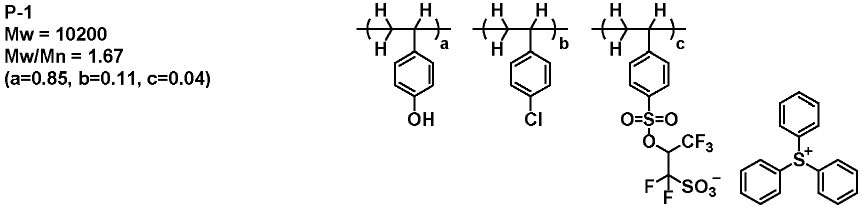

### Examples 2-2 to 2-36, Comparative Examples 2-1 to 2-30, and Synthesis Examples 1-1 to 1-7

### Synthesis of base polymers P-2 to P-36, comparative base polymers CP-1 to CP-30, and base polymers AP-1 to AP-7

Base polymers P-2 to P-36, comparative base polymers CP-1 to CP-30, and base polymers AP-1 to AP-7 shown in Tables 1 to 3 were synthesized in the same manner as in Example 2-1, except that the type and blending ratio of each monomer were changed. In Tables 1 to 3, the incorporation ratio indicates a molar ratio.

**Table 1**

| Polymer | Unit 1 | Incorporation ratio (mol%) | Unit 2 | Incorporation ratio (mol%) | Unit 3 | Incorporation ratio (mol%) | Unit 4 | Incorporation ratio (mol%) | Mw | Mw/Mn |
|---|---|---|---|---|---|---|---|---|---|---|
| P-1 | PP-1 | 4 | A-1 | 85 | B-4 | 11 | - | - | 10,000 | 1.82 |
| P-2 | PP-1 | 5 | A-1 | 75 | B-1 | 10 | B-4 | 10 | 15,100 | 1.81 |
| P-3 | PP-1 | 5 | A-1 | 75 | B-2 | 10 | B-4 | 10 | 14,500 | 1.84 |
| P-4 | PP-1 | 5 | A-1 | 75 | B-1 | 10 | B-5 | 10 | 14,300 | 1.81 |
| P-5 | PP-1 | 5 | A-1 | 75 | B-1 | 10 | B-6 | 10 | 14,200 | 1.83 |
| P-6 | PP-1 | 5 | A-2 | 75 | B-1 | 10 | B-4 | 10 | 14,300 | 1.80 |
| P-7 | PP-1 | 15 | A-3 | 60 | B-1 | 15 | B-4 | 10 | 14,700 | 1.81 |
| P-8 | PP-1 | 5 | A-1 | 70 | B-3 | 5 | C-1 | 20 | 15,200 | 1.82 |
| P-9 | PP-2 | 5 | A-1 | 70 | B-3 | 5 | C-1 | 20 | 15,300 | 1.81 |
| P-10 | PP-3 | 5 | A-1 | 70 | B-3 | 5 | C-1 | 20 | 15,200 | 1.83 |
| P-11 | PP-4 | 5 | A-1 | 70 | B-3 | 5 | C-1 | 20 | 15,800 | 1.79 |
| P-12 | PP-5 | 5 | A-1 | 70 | B-3 | 5 | C-1 | 20 | 16,000 | 1.78 |
| P-13 | PP-6 | 5 | A-1 | 70 | B-3 | 5 | C-1 | 20 | 15,700 | 1.81 |
| P-14 | PP-7 | 5 | A-1 | 70 | B-3 | 5 | C-1 | 20 | 15,400 | 1.83 |
| P-15 | PP-1 | 5 | A-2 | 70 | B-1 | 5 | C-1 | 20 | 16,400 | 1.81 |
| P-16 | PP-1 | 10 | A-2 | 65 | B-1 | 5 | C-1 | 20 | 16,700 | 1.84 |
| P-17 | PP-1 | 5 | A-1 | 70 | B-1 | 5 | C-2 | 20 | 16,900 | 1.86 |
| P-18 | PP-1 | 5 | A-1 | 75 | B-1 | 5 | C-3 | 15 | 16,800 | 1.82 |
| P-19 | PP-1 | 5 | A-1 | 50 | B-1 | 5 | C-4 | 40 | 16,600 | 1.84 |
| P-20 | PP-1 | 5 | A-1 | 70 | B-1 | 5 | C-5 | 20 | 16,500 | 1.85 |
| P-21 | PP-1 | 5 | A-1 | 45 | B-1 | 5 | C-6 | 45 | 16,600 | 1.88 |
| P-22 | PP-1 | 5 | A-1 | 70 | B-1 | 5 | C-7 | 20 | 16,400 | 1.81 |
| P-23 | PP-1 | 5 | A-1 | 65 | C-1 | 30 | - | - | 19,500 | 1.56 |
| P-24 | PP-1 | 1 | A-1 | 66 | B-1 | 8 | C-1 | 25 | 13,800 | 1.74 |
| P-25 | PP-1 | 2 | A-1 | 55 | B-1 | 8 | C-4 | 35 | 9,800 | 1.86 |
| P-26 | PP-1 | 1 | A-1 | 66 | B-1 | 8 | C-5 | 25 | 8,900 | 1.77 |
| P-27 | PP-1 | 3 | A-2 | 77 | C-1 | 20 | - | - | 13,000 | 1.81 |
| P-28 | PP-1 | 3 | A-2 | 67 | C-4 | 30 | - | - | 16,700 | 1.77 |
| P-29 | PP-1 | 3 | A-2 | 77 | C-5 | 20 | - | - | 15,700 | 1.76 |
| P-30 | PP-1 | 3 | A-2 | 62 | C-6 | 35 | - | - | 16,500 | 1.75 |
| P-31 | PP-1 | 3 | A-2 | 67 | C-7 | 30 | - | - | 14,800 | 1.79 |
| P-32 | PP-3 | 3 | A-1 | 77 | C-1 | 20 | - | - | 17,800 | 1.83 |
| P-33 | PP-3 | 3 | A-1 | 67 | C-4 | 30 | - | - | 16,600 | 1.82 |
| P-34 | PP-3 | 3 | A-1 | 77 | C-5 | 20 | - | - | 17,900 | 1.79 |
| P-35 | PP-3 | 3 | A-1 | 62 | C-6 | 35 | - | - | 16,500 | 1.84 |
| P-36 | PP-3 | 3 | A-1 | 67 | C-7 | 30 | - | - | 15,500 | 1.82 |

**Table 2**

| Polymer | Unit 1 | Incorporation ratio (mol%) | Unit 2 | Incorporation ratio (mol%) | Unit 3 | Incorporation ratio (mol%) | Unit 4 | Incorporation ratio (mol%) | Mw | Mw/Mn |
|---|---|---|---|---|---|---|---|---|---|---|
| CP-1 | cPP-1 | 4 | A-1 | 85 | B-4 | 11 | - | - | 10,500 | 1.83 |
| CP-2 | cPP-1 | 5 | A-1 | 75 | B-1 | 10 | B-4 | 10 | 15,300 | 1.82 |
| CP-3 | cPP-1 | 5 | A-1 | 75 | B-2 | 10 | B-4 | 10 | 14,900 | 1.83 |
| CP-4 | cPP-1 | 5 | A-1 | 75 | B-1 | 10 | B-5 | 10 | 14,200 | 1.84 |
| CP-5 | cPP-1 | 5 | A-1 | 75 | B-1 | 10 | B-6 | 10 | 14,700 | 1.84 |
| CP-6 | cPP-1 | 5 | A-2 | 75 | B-1 | 10 | B-4 | 10 | 14,800 | 1.83 |
| CP-7 | cPP-1 | 5 | A-1 | 70 | B-3 | 5 | C-1 | 20 | 15,100 | 1.81 |
| CP-8 | cPP-2 | 5 | A-1 | 70 | B-3 | 5 | C-1 | 20 | 15,600 | 1.83 |
| CP-9 | cPP-3 | 5 | A-1 | 70 | B-3 | 5 | C-1 | 20 | 15,600 | 1.84 |
| CP-10 | cPP-4 | 5 | A-1 | 70 | B-3 | 5 | C-1 | 20 | 15,400 | 1.77 |
| CP-11 | cPP-1 | 10 | A-2 | 65 | B-1 | 5 | C-1 | 20 | 16,800 | 1.82 |
| CP-12 | cPP-1 | 5 | A-1 | 70 | B-1 | 5 | C-2 | 20 | 16,800 | 1.84 |
| CP-13 | cPP-1 | 5 | A-1 | 75 | B-1 | 5 | C-3 | 15 | 16,400 | 1.85 |
| CP-14 | cPP-1 | 5 | A-1 | 50 | B-1 | 5 | C-4 | 40 | 16,500 | 1.82 |
| CP-15 | cPP-1 | 5 | A-1 | 70 | B-1 | 5 | C-5 | 20 | 16,200 | 1.84 |
| CP-16 | cPP-1 | 5 | A-1 | 45 | B-1 | 5 | C-6 | 45 | 16,900 | 1.87 |
| CP-17 | cPP-1 | 5 | A-1 | 70 | B-1 | 5 | C-7 | 20 | 16,100 | 1.79 |
| CP-18 | cPP-1 | 1 | A-1 | 66 | B-1 | 8 | C-1 | 25 | 13,500 | 1.75 |
| CP-19 | cPP-2 | 2 | A-1 | 55 | B-1 | 8 | C-4 | 35 | 9,600 | 1.84 |
| CP-20 | cPP-3 | 1 | A-1 | 66 | B-1 | 8 | C-5 | 25 | 8,400 | 1.79 |
| CP-21 | cPP-1 | 3 | A-2 | 77 | C-1 | 20 | - | - | 13,300 | 1.84 |
| CP-22 | cPP-1 | 3 | A-2 | 67 | C-4 | 30 | - | - | 16,400 | 1.75 |
| CP-23 | cPP-1 | 3 | A-2 | 77 | C-5 | 20 | - | - | 15,900 | 1.74 |
| CP-24 | cPP-1 | 3 | A-2 | 62 | C-6 | 35 | - | - | 16,300 | 1.79 |
| CP-25 | cPP-1 | 3 | A-2 | 67 | C-7 | 30 | - | - | 14,400 | 1.76 |
| CP-26 | cPP-2 | 3 | A-1 | 77 | C-1 | 20 | - | - | 17,400 | 1.81 |
| CP-27 | cPP-2 | 3 | A-1 | 67 | C-4 | 30 | - | - | 16,900 | 1.85 |
| CP-28 | cPP-2 | 3 | A-1 | 77 | C-5 | 20 | - | - | 17,500 | 1.81 |
| CP-29 | cPP-2 | 3 | A-1 | 62 | C-6 | 35 | - | - | 16,400 | 1.87 |
| CP-30 | cPP-2 | 3 | A-1 | 67 | C-7 | 30 | - | - | 15,100 | 1.78 |

**Table 3**

| Polymer | Unit 1 | Incorporation ratio (mol%) | Unit 2 | Incorporation ratio (mol%) | Unit 3 | Incorporation ratio (mol%) | Unit 4 | Incorporation ratio (mol%) | Mw | Mw/Mn |
|---|---|---|---|---|---|---|---|---|---|---|
| AP-1 | A-2 | 80 | B-2 | 10 | B-4 | 10 | - | - | 4,400 | 1.66 |
| AP-2 | A-1 | 80 | B-2 | 10 | B-4 | 10 | - | - | 4,300 | 1.65 |
| AP-3 | A-1 | 60 | B-1 | 10 | C-1 | 30 | - | - | 4,500 | 1.62 |
| AP-4 | A-1 | 45 | B-1 | 10 | C-4 | 45 | - | - | 4,400 | 1.61 |
| AP-5 | A-1 | 55 | B-1 | 10 | C-5 | 35 | - | - | 3,500 | 1.63 |
| AP-6 | A-1 | 50 | B-1 | 10 | C-6 | 40 | - | - | 4,500 | 1.64 |
| AP-7 | A-1 | 65 | B-1 | 10 | C-7 | 35 | - | - | 4,600 | 1.62 |

The structure of the repeat unit introduced into the polymer is shown below.

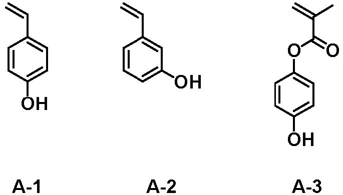

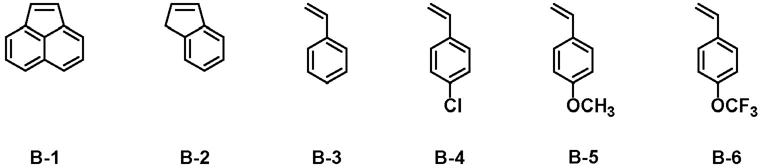

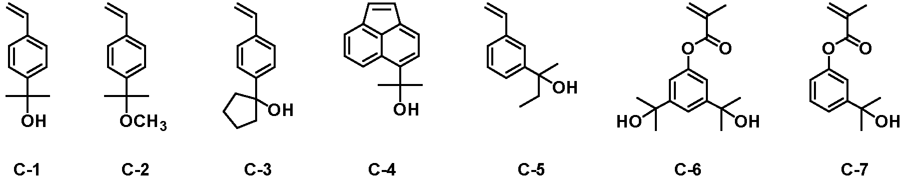

The dissolution rate of the polymer in an alkaline developer was calculated by spin-coating a polymer solution (polymer concentration: 12.0 wt%, solvent: PGME) on an 8-inch silicon wafer, baking the solution at 150°C for 90 seconds to form a film having a thickness of 2,000 nm, performing development with a 2.38 wt% TMAH aqueous solution at 23°C for 10 seconds, and measuring the film loss amount. As a result, the dissolution rates of the polymers P-1 to P-36 and AP-1 to AP-7 were 20 nm/sec or less. The dissolution rates of the comparative polymers CP-1 to CP-30 were 20 nm/sec or less.

### [3] Preparation of chemically amplified negative resist composition

### Examples 3-1 to 3-67 and Comparative Examples 3-1 to 3-61

Each component was dissolved in an organic solvent with the composition shown in Tables 4 to 9, and the resulting solution was filtered through a nylon filter having a size of 5 nm and a UPE filter having a size of sub-1nm to prepare a chemically amplified negative resist composition. The organic solvent is a mixed solvent of 920 parts by weight of PGMEA, 1,850 parts by weight of EL, and 1,850 parts by weight of PGME. In Tables 4 to 9, the crosslinker TMGU is tetramethoxymethyl glycoluril, and PF-636 is a surfactant PolyFox PF-636 (OMNOVA SOLUTIONS).

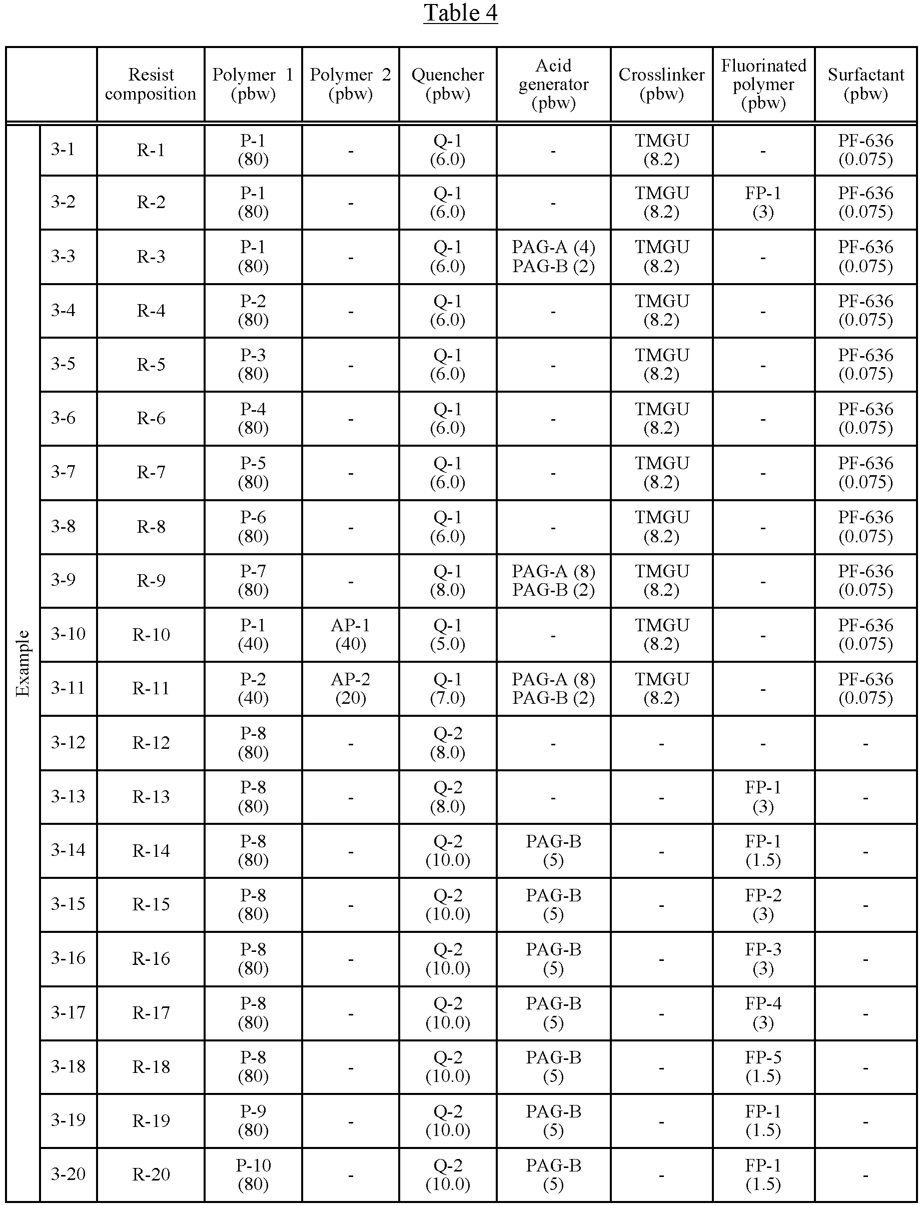

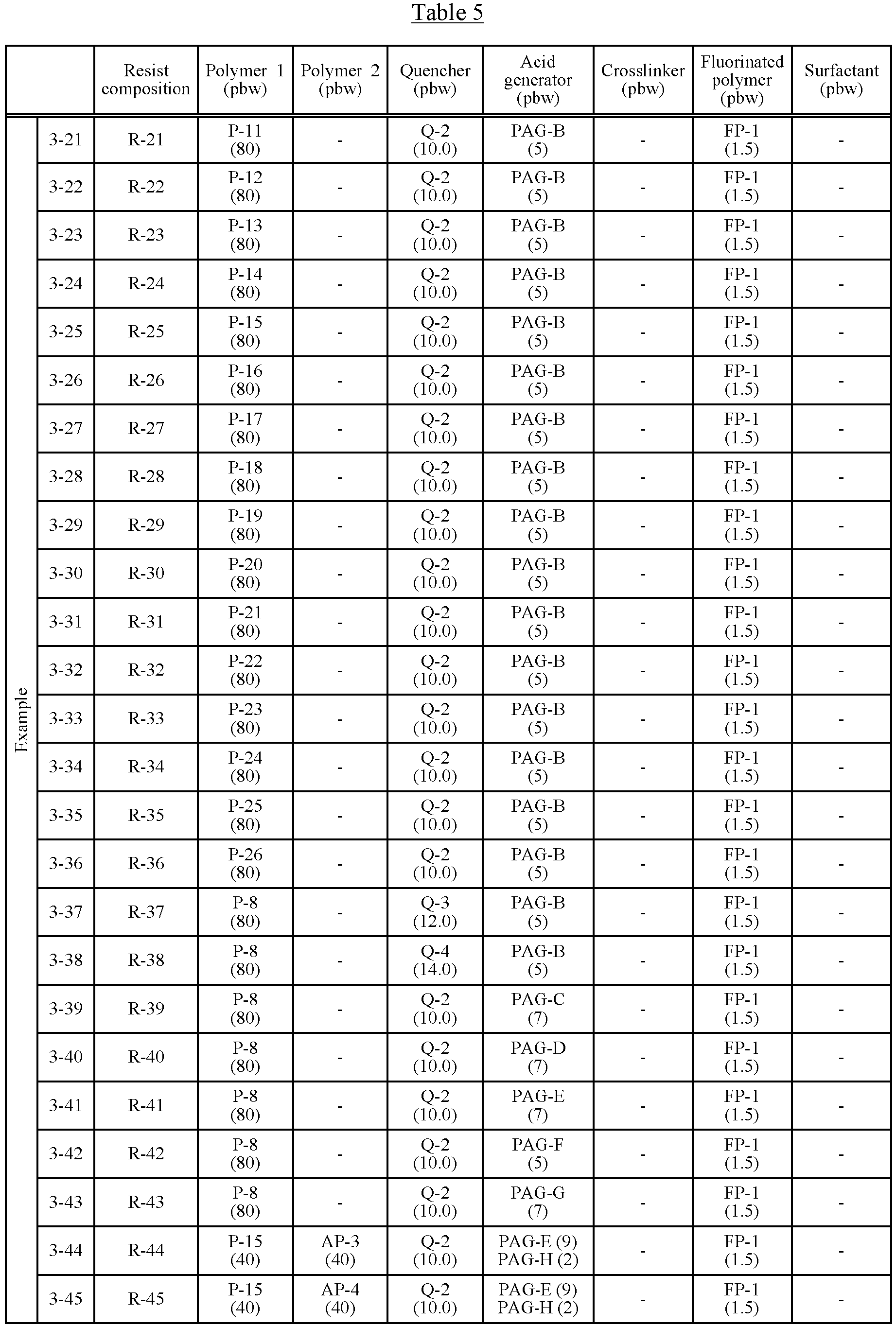

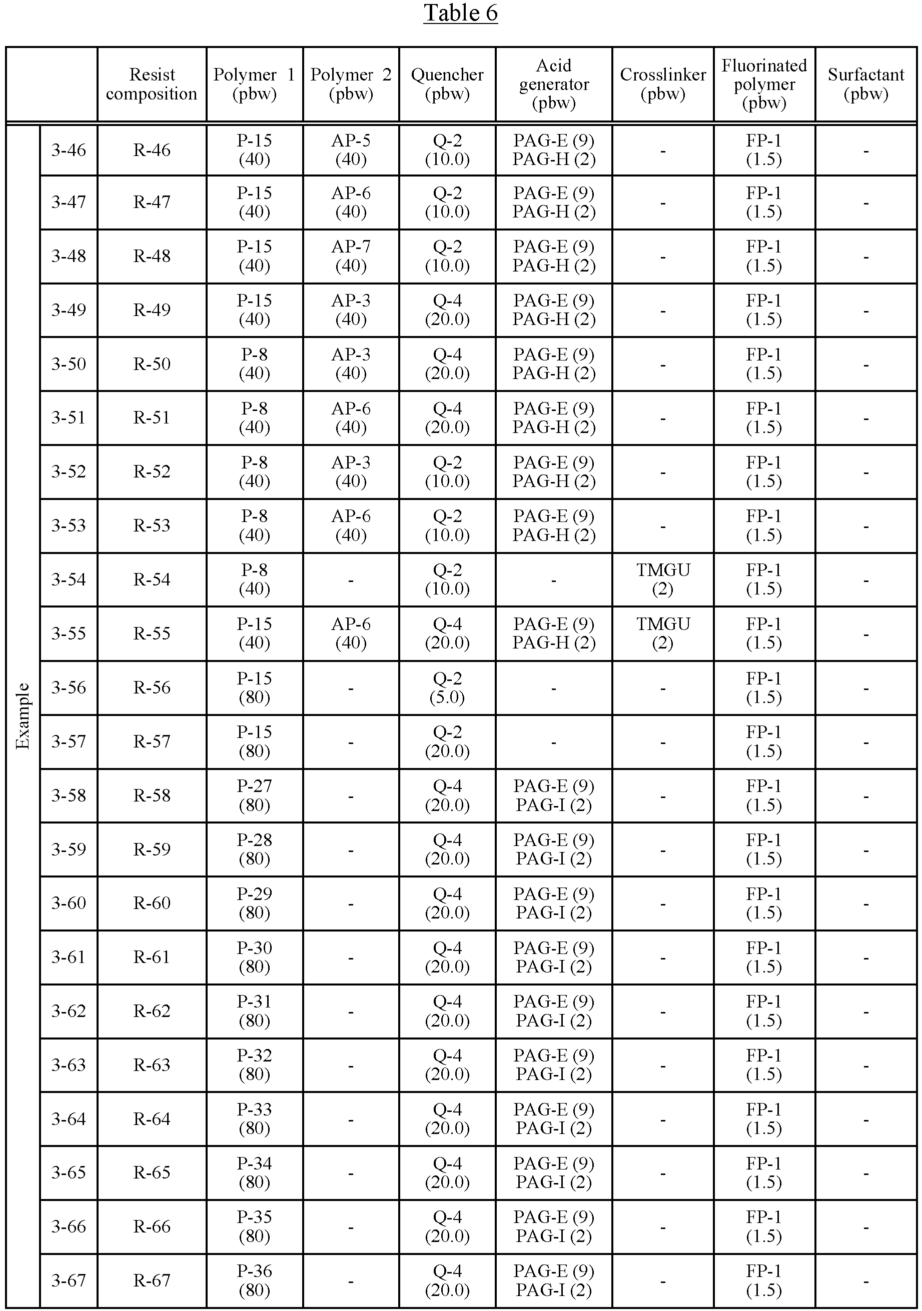

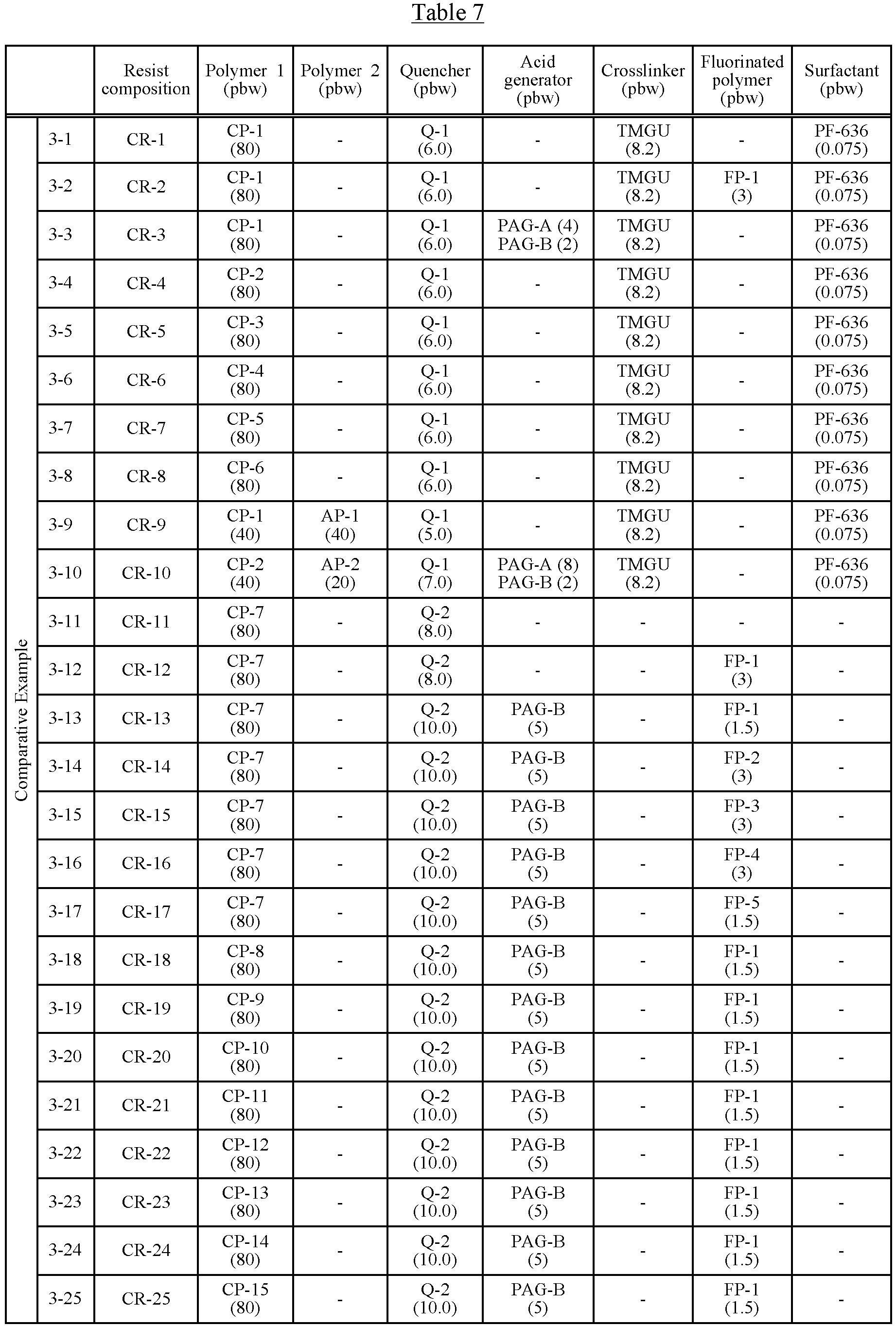

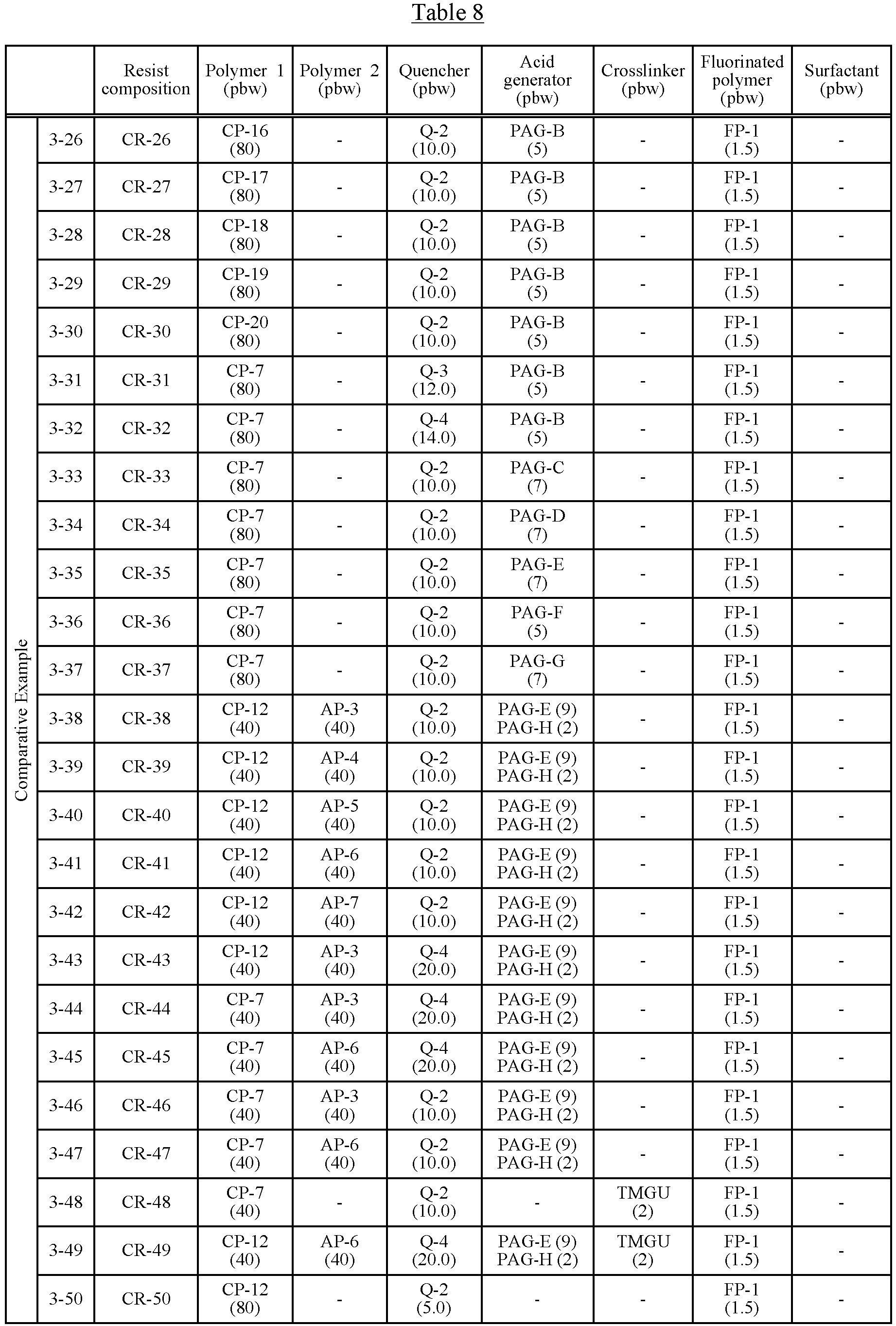

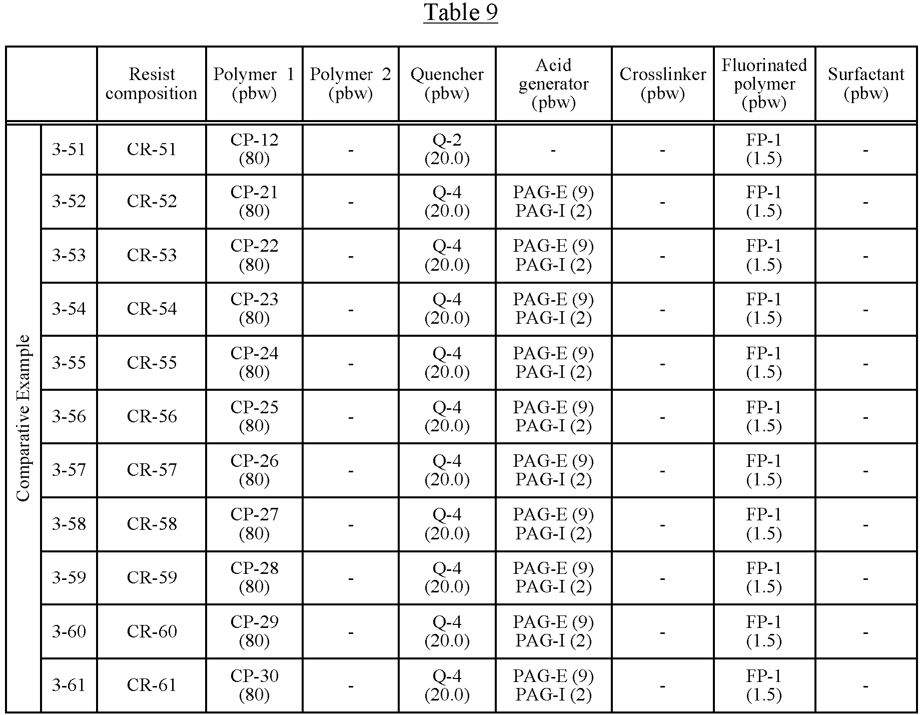

In Tables 4 to 9, the structures of quenchers Q-1 to Q-4, photoacid generators PAG-A to PAG-I, and polymers FP-1 to FP-5 are as follows.

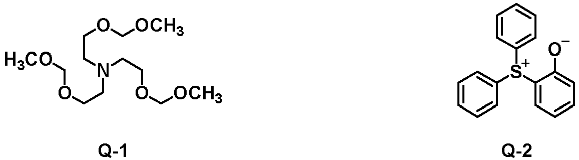

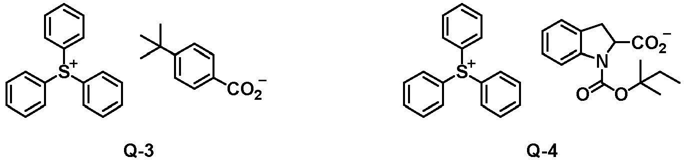

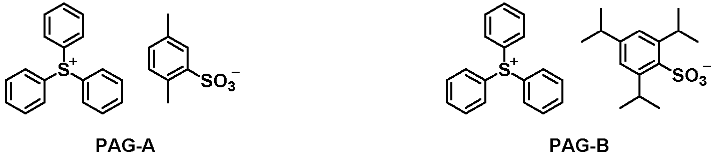

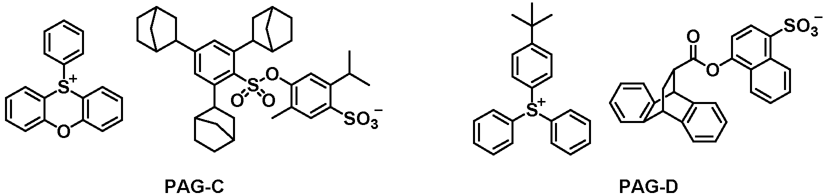

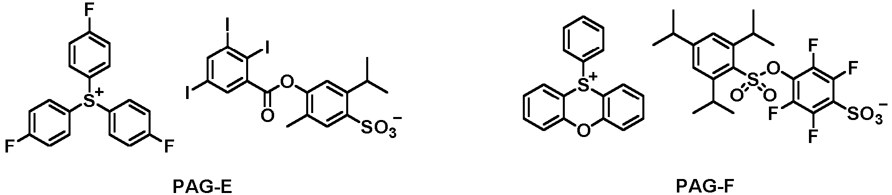

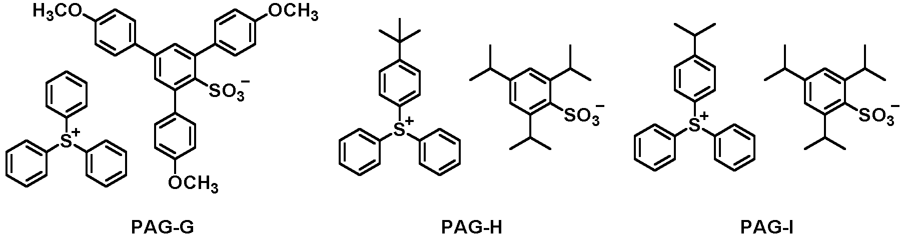

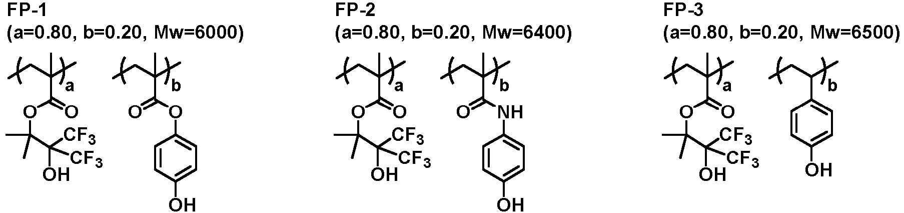

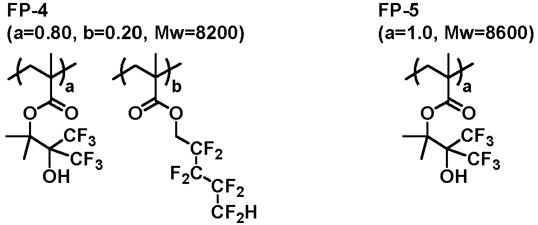

### [4] EB lithography test

### Examples 4-1 to 4-67 and Comparative Examples 4-1 to 4-61

Using ACT-M (Tokyo Electron Ltd.), each of the chemically amplified negative resist compositions (R-1 to R-67 and CR-1 to CR-61) was spin coated onto a mask blank for an EUV exposure mask of 152 mm squares having the outermost surface of chromium compound, and prebaked on a hotplate at 110°C for 600 seconds to form a resist film of 80 nm thick. The thickness of the resulting resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The resist film was further exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a negative pattern.

The resulting resist pattern was evaluated as follows. The resist pattern was evaluated as follows. The patterned mask blank was observed under a top-down scanning electron microscope (TDSEM). An LER of 200 nmLS was measured with an SEM while an exposure dose for resolving a 1:1 line-and-space (LS) of 200 nm at 1:1 was defined as an optimum dose (µC/cm²), a minimum dimension at an exposure dose for resolving LS of 200 nm at 1:1 was defined as LS resolution (maximum resolution), and a minimum dimension at an exposure dose for resolving a line width of a square of 200 nm square was defined as dot resolution (maximum resolution). Whether or not the pattern profile was rectangular was visually determined. The evaluation results of each resist composition are shown in Tables 10 to 13.

**Table 10**

| | | Resist composition | Eop (µC/cm²) | Maximum resolution (LS) (nm) | Maximum resolution (dot) (nm) | LER (nm) | Pattern profile |
|---|---|---|---|---|---|---|---|
| | 4-1 | R-1 | 150 | 40 | 80 | 4.0 | rectangular |
| | 4-2 | R-2 | 150 | 40 | 80 | 4.1 | rectangular |
| | 4-3 | R-3 | 150 | 40 | 80 | 4.2 | rectangular |
| | 4-4 | R-4 | 160 | 40 | 80 | 3.9 | rectangular |
| | 4-5 | R-5 | 150 | 40 | 80 | 3.9 | rectangular |
| | 4-6 | R-6 | 160 | 40 | 80 | 4.1 | rectangular |
| | 4-7 | R-7 | 150 | 40 | 80 | 4.2 | rectangular |
| | 4-8 | R-8 | 170 | 40 | 80 | 4.0 | rectangular |
| | 4-9 | R-9 | 180 | 40 | 80 | 4.1 | rectangular |
| | 4-10 | R-10 | 170 | 40 | 80 | 4.0 | rectangular |
| | 4-11 | R-11 | 170 | 40 | 80 | 3.9 | rectangular |
| | 4-12 | R-12 | 160 | 35 | 70 | 3.4 | rectangular |
| | 4-13 | R-13 | 170 | 35 | 70 | 3.4 | rectangular |
| | 4-14 | R-14 | 180 | 35 | 70 | 3.4 | rectangular |
| | 4-15 | R-15 | 160 | 35 | 70 | 3.5 | rectangular |
| | 4-16 | R-16 | 170 | 35 | 70 | 3.4 | rectangular |
| | 4-17 | R-17 | 160 | 35 | 70 | 3.4 | rectangular |
| Example | 4-18 | R-18 | 170 | 35 | 70 | 3.5 | rectangular |
| | 4-19 | R-19 | 180 | 35 | 70 | 3.4 | rectangular |
| | 4-20 | R-20 | 170 | 35 | 70 | 3.6 | rectangular |
| | 4-21 | R-21 | 180 | 35 | 70 | 3.4 | rectangular |
| | 4-22 | R-22 | 170 | 35 | 70 | 3.4 | rectangular |
| | 4-23 | R-23 | 170 | 35 | 70 | 3.5 | rectangular |
| | 4-24 | R-24 | 170 | 35 | 70 | 3.4 | rectangular |
| | 4-25 | R-25 | 170 | 35 | 70 | 3.6 | rectangular |
| | 4-26 | R-26 | 170 | 35 | 70 | 3.5 | rectangular |
| | 4-27 | R-27 | 170 | 35 | 70 | 3.5 | rectangular |
| | 4-28 | R-28 | 170 | 35 | 70 | 3.4 | rectangular |
| | 4-29 | R-29 | 170 | 35 | 70 | 3.5 | rectangular |
| | 4-30 | R-30 | 170 | 35 | 70 | 3.4 | rectangular |
| | 4-31 | R-31 | 170 | 35 | 70 | 3.4 | rectangular |
| | 4-32 | R-32 | 170 | 35 | 70 | 3.4 | rectangular |
| | 4-33 | R-33 | 170 | 35 | 70 | 3.6 | rectangular |
| | 4-34 | R-34 | 170 | 35 | 70 | 3.4 | rectangular |
| | 4-35 | R-35 | 170 | 35 | 70 | 3.4 | rectangular |

**Table 11**

| | | Resist composition | Eop (µC/cm²) | Maximum resolution (LS) (nm) | Maximum resolution (dot) (nm) | LER (nm) | Pattern profile |
|---|---|---|---|---|---|---|---|
| | 4-36 | R-36 | 170 | 35 | 70 | 3.4 | rectangular |
| | 4-37 | R-37 | 180 | 35 | 70 | 3.6 | rectangular |
| | 4-38 | R-38 | 180 | 35 | 70 | 3.4 | rectangular |
| | 4-39 | R-39 | 170 | 35 | 70 | 3.6 | rectangular |
| | 4-40 | R-40 | 170 | 35 | 70 | 3.4 | rectangular |
| | 4-41 | R-41 | 170 | 35 | 70 | 3.5 | rectangular |
| | 4-42 | R-42 | 170 | 35 | 70 | 3.4 | rectangular |
| | 4-43 | R-43 | 170 | 35 | 70 | 3.6 | rectangular |
| | 4-44 | R-44 | 180 | 35 | 70 | 3.4 | rectangular |
| | 4-45 | R-45 | 170 | 35 | 70 | 3.5 | rectangular |
| | 4-46 | R-46 | 170 | 35 | 70 | 3.4 | rectangular |
| | 4-47 | R-47 | 170 | 35 | 70 | 3.6 | rectangular |
| | 4-48 | R-48 | 170 | 35 | 70 | 3.4 | rectangular |
| | 4-49 | R-49 | 180 | 35 | 70 | 3.5 | rectangular |
| | 4-50 | R-50 | 170 | 35 | 70 | 3.4 | rectangular |
| Example | 4-51 | R-51 | 170 | 35 | 70 | 3.5 | rectangular |
| | 4-52 | R-52 | 170 | 35 | 70 | 3.6 | rectangular |
| | 4-53 | R-53 | 180 | 35 | 70 | 3.5 | rectangular |
| | 4-54 | R-54 | 180 | 35 | 70 | 3.4 | rectangular |
| | 4-55 | R-55 | 170 | 35 | 70 | 3.6 | rectangular |
| | 4-56 | R-56 | 180 | 35 | 70 | 3.5 | rectangular |
| | 4-57 | R-57 | 170 | 35 | 70 | 3.4 | rectangular |
| | 4-58 | R-58 | 170 | 35 | 70 | 3.6 | rectangular |
| | 4-59 | R-59 | 180 | 35 | 70 | 3.5 | rectangular |
| | 4-60 | R-60 | 170 | 35 | 70 | 3.5 | rectangular |
| | 4-61 | R-61 | 170 | 35 | 70 | 3.4 | rectangular |
| | 4-62 | R-62 | 170 | 35 | 70 | 3.6 | rectangular |
| | 4-63 | R-63 | 170 | 35 | 70 | 3.4 | rectangular |
| | 4-64 | R-64 | 180 | 35 | 70 | 3.4 | rectangular |
| | 4-65 | R-65 | 180 | 35 | 70 | 3.5 | rectangular |
| | 4-66 | R-66 | 170 | 35 | 70 | 3.6 | rectangular |
| | 4-67 | R-67 | 180 | 35 | 70 | 3.5 | rectangular |

**Table 12**

| | | Resist composition | Eop (µC/cm²) | Maximum resolution (LS) (nm) | Maximum resolution (dot) (nm) | LER (nm) | Pattern profile |
|---|---|---|---|---|---|---|---|
| | 4-1 | CR-1 | 150 | 65 | 100 | 5.8 | undercut |
| | 4-2 | CR-2 | 150 | 65 | 100 | 5.9 | undercut |
| | 4-3 | CR-3 | 150 | 65 | 100 | 5.8 | undercut |
| | 4-4 | CR-4 | 150 | 65 | 100 | 5.7 | undercut |
| | 4-5 | CR-5 | 150 | 60 | 100 | 5.6 | undercut |
| | 4-6 | CR-6 | 150 | 60 | 95 | 5.8 | undercut |
| | 4-7 | CR-7 | 150 | 65 | 100 | 5.5 | undercut |
| | 4-8 | CR-8 | 150 | 60 | 100 | 5.7 | undercut |
| | 4-9 | CR-9 | 150 | 60 | 100 | 5.6 | undercut |
| | 4-10 | CR-10 | 150 | 60 | 100 | 5.8 | undercut |
| | 4-11 | CR-11 | 170 | 50 | 80 | 5.0 | rectangular |
| | 4-12 | CR-12 | 170 | 50 | 80 | 4.8 | round head |
| | 4-13 | CR-13 | 170 | 55 | 80 | 4.8 | rectangular |
| | 4-14 | CR-14 | 180 | 50 | 80 | 4.4 | round head |
| | 4-15 | CR-15 | 170 | 50 | 80 | 4.8 | round head |
| | 4-16 | CR-16 | 170 | 50 | 80 | 4.5 | rectangular |
| | 4-17 | CR-17 | 170 | 50 | 80 | 4.7 | round head |
| Comparative Example | 4-18 | CR-18 | 170 | 50 | 80 | 4.8 | rectangular |
| | 4-19 | CR-19 | 170 | 50 | 85 | 4.4 | round head |
| | 4-20 | CR-20 | 170 | 50 | 80 | 4.5 | round head |
| | 4-21 | CR-21 | 180 | 50 | 80 | 4.8 | round head |
| | 4-22 | CR-22 | 170 | 50 | 80 | 4.7 | rectangular |
| | 4-23 | CR-23 | 180 | 50 | 80 | 4.8 | round head |
| | 4-24 | CR-24 | 170 | 55 | 80 | 4.7 | rectangular |
| | 4-25 | CR-25 | 170 | 50 | 80 | 4.4 | round head |
| | 4-26 | CR-26 | 170 | 50 | 80 | 4.5 | rectangular |
| | 4-27 | CR-27 | 170 | 50 | 85 | 4.4 | round head |
| | 4-28 | CR-28 | 170 | 50 | 80 | 4.8 | rectangular |
| | 4-29 | CR-29 | 170 | 50 | 80 | 4.7 | rectangular |
| | 4-30 | CR-30 | 180 | 50 | 80 | 4.8 | rectangular |
| | 4-31 | CR-31 | 170 | 50 | 85 | 4.4 | round head |
| | 4-32 | CR-32 | 170 | 50 | 80 | 4.4 | rectangular |
| | 4-33 | CR-33 | 170 | 50 | 80 | 4.8 | round head |
| | 4-34 | CR-34 | 170 | 55 | 80 | 4.8 | rectangular |
| | 4-35 | CR-35 | 170 | 50 | 80 | 4.8 | round head |

**Table 13**

| | | Resist composition | Eop (µC/cm²) | Maximum resolution (LS) (nm) | Maximum resolution (dot) (nm) | LER (nm) | Pattern profile |
|---|---|---|---|---|---|---|---|
| | 4-36 | CR-36 | 170 | 50 | 80 | 4.4 | rectangular |
| | 4-37 | CR-37 | 170 | 50 | 80 | 4.8 | round head |
| | 4-38 | CR-38 | 170 | 50 | 80 | 4.8 | rectangular |
| | 4-39 | CR-39 | 180 | 50 | 80 | 4.4 | rectangular |
| | 4-40 | CR-40 | 180 | 50 | 80 | 4.5 | round head |
| | 4-41 | CR-41 | 170 | 55 | 80 | 4.4 | round head |
| | 4-42 | CR-42 | 180 | 50 | 80 | 4.8 | rectangular |
| | 4-43 | CR-43 | 170 | 50 | 80 | 4.5 | rectangular |
| | 4-44 | CR-44 | 180 | 50 | 80 | 4.7 | round head |
| | 4-45 | CR-45 | 170 | 50 | 80 | 4.8 | round head |
| | 4-46 | CR-46 | 180 | 55 | 80 | 4.4 | rectangular |
| | 4-47 | CR-47 | 170 | 50 | 80 | 4.8 | rectangular |
| Comparative Example | 4-48 | CR-48 | 170 | 50 | 80 | 4.5 | round head |
| | 4-49 | CR-49 | 170 | 50 | 80 | 4.7 | rectangular |
| | 4-50 | CR-50 | 170 | 50 | 80 | 4.8 | rectangular |
| | 4-51 | CR-51 | 180 | 50 | 85 | 4.4 | rectangular |
| | 4-52 | CR-52 | 170 | 50 | 80 | 4.8 | round head |
| | 4-53 | CR-53 | 170 | 50 | 80 | 4.5 | round head |
| | 4-54 | CR-54 | 180 | 50 | 80 | 4.8 | rectangular |
| | 4-55 | CR-55 | 170 | 50 | 80 | 4.8 | round head |
| | 4-56 | CR-56 | 170 | 50 | 80 | 4.7 | rectangular |
| | 4-57 | CR-57 | 170 | 55 | 85 | 4.5 | round head |
| | 4-58 | CR-58 | 180 | 50 | 80 | 4.8 | round head |
| | 4-59 | CR-59 | 170 | 50 | 80 | 4.8 | rectangular |
| | 4-60 | CR-60 | 170 | 55 | 85 | 4.5 | rectangular |
| | 4-61 | CR-61 | 170 | 50 | 80 | 4.4 | round head |

### [5] Etching resistance test

### Examples 5-1 to 5-67 and Comparative Examples 5-1 to 5-61

Using ACT-M (Tokyo Electron Ltd.), each of the chemically amplified negative resist compositions (R-1 to R-67 and CR-1 to CR-61) was spin coated onto a photomask blank of 152 mm squares having the outermost surface of chromium, and prebaked on a hotplate at 110°C for 600 seconds to form a resist film of 120 nm thick. The thickness of the resulting resist film was measured by an optical film thickness measurement system Nanospec (Nanometrics Inc.). Measurement was made at 81 points in the plane of the blank substrate excluding a peripheral band extending 10 mm inward from the periphery, and an average film thickness and a film thickness range were computed therefrom.

The resulting coated substrate was dry-etched by a dry etching device (UNAXIS G4) under the following conditions, and the film loss rate (A/sec) derived from the residual film after etching was calculated. The results are shown in Tables 14 to 17.

| | |
|---|---|
| RF1 (RIE): | pulse 700 V |
| RF2 (ICP): | CW 400 W |
| Pressure: | 6 mTorr |
| Cl₂: | 185 sccm |
| O₂: | 55 sccm |
| He: | 9.25 sccm |
| Etching time: | 75 sec |

**Table 14**

| | | Resist composition | Rate (A/sec) |
|---|---|---|---|
| | 5-1 | R-1 | 5.3 |
| | 5-2 | R-2 | 5.2 |
| | 5-3 | R-3 | 5.5 |
| | 5-4 | R-4 | 5.3 |
| | 5-5 | R-5 | 5.5 |
| | 5-6 | R-6 | 5.3 |
| | 5-7 | R-7 | 5.2 |
| | 5-8 | R-8 | 5.3 |
| | 5-9 | R-9 | 5.2 |
| | 5-10 | R-10 | 5.4 |
| | 5-11 | R-11 | 5.2 |
| | 5-12 | R-12 | 5.5 |
| | 5-13 | R-13 | 5.5 |
| | 5-14 | R-14 | 5.5 |
| | 5-15 | R-15 | 5.2 |
| | 5-16 | R-16 | 5.5 |
| | 5-17 | R-17 | 5.5 |
| Example | 5-18 | R-18 | 5.2 |
| | 5-19 | R-19 | 5.5 |
| | 5-20 | R-20 | 5.3 |
| | 5-21 | R-21 | 5.4 |
| | 5-22 | R-22 | 5.5 |
| | 5-23 | R-23 | 5.2 |
| | 5-24 | R-24 | 5.3 |
| | 5-25 | R-25 | 5.4 |
| | 5-26 | R-26 | 5.3 |
| | 5-27 | R-27 | 5.2 |
| | 5-28 | R-28 | 5.5 |
| | 5-29 | R-29 | 5.2 |
| | 5-30 | R-30 | 5.5 |
| | 5-31 | R-31 | 5.5 |
| | 5-32 | R-32 | 5.2 |
| | 5-33 | R-33 | 5.5 |
| | 5-34 | R-34 | 5.2 |
| | 5-35 | R-35 | 5.2 |

**Table 15**

| | | Resist composition | Rate (A/sec) |
|---|---|---|---|
| | 5-36 | R-36 | 5.5 |
| | 5-37 | R-37 | 5.4 |
| | 5-38 | R-38 | 5.2 |
| | 5-39 | R-39 | 5.4 |
| | 5-40 | R-40 | 5.5 |
| | 5-41 | R-41 | 5.4 |
| | 5-42 | R-42 | 5.2 |
| | 5-43 | R-43 | 5.3 |
| | 5-44 | R-44 | 5.5 |
| | 5-45 | R-45 | 5.3 |
| | 5-46 | R-46 | 5.4 |
| | 5-47 | R-47 | 5.5 |
| | 5-48 | R-48 | 5.5 |
| | 5-49 | R-49 | 5.5 |
| | 5-50 | R-50 | 5.2 |
| | 5-51 | R-51 | 5.2 |
| Example | 5-52 | R-52 | 5.5 |
| | 5-53 | R-53 | 5.2 |
| | 5-54 | R-54 | 5.5 |
| | 5-55 | R-55 | 5.4 |
| | 5-56 | R-56 | 5.4 |
| | 5-57 | R-57 | 5.4 |
| | 5-58 | R-58 | 5.2 |
| | 5-59 | R-59 | 5.5 |
| | 5-60 | R-60 | 5.4 |
| | 5-61 | R-61 | 5.5 |
| | 5-62 | R-62 | 5.2 |
| | 5-63 | R-63 | 5.4 |
| | 5-64 | R-64 | 5.3 |
| | 5-65 | R-65 | 5.2 |
| | 5-66 | R-66 | 5.3 |
| | 5-67 | R-67 | 5.2 |

**Table 16**

| | | Resist composition | Rate (A/sec) |
|---|---|---|---|
| | 5-1 | CR-1 | 7.5 |
| | 5-2 | CR-2 | 7.3 |
| | 5-3 | CR-3 | 7.4 |
| | 5-4 | CR-4 | 7.5 |
| | 5-5 | CR-5 | 7.3 |
| | 5-6 | CR-6 | 7.3 |
| | 5-7 | CR-7 | 7.5 |
| | 5-8 | CR-8 | 7.4 |
| | 5-9 | CR-9 | 7.3 |
| | 5-10 | CR-10 | 7.3 |
| | 5-11 | CR-11 | 7.5 |
| | 5-12 | CR-12 | 7.5 |
| | 5-13 | CR-13 | 7.5 |
| | 5-14 | CR-14 | 7.4 |
| | 5-15 | CR-15 | 7.5 |
| | 5-16 | CR-16 | 7.3 |
| | 5-17 | CR-17 | 7.3 |
| Comparative Example | 5-18 | CR-18 | 5.6 |
| | 5-19 | CR-19 | 5.5 |
| | 5-20 | CR-20 | 7.2 |
| | 5-21 | CR-21 | 7.5 |
| | 5-22 | CR-22 | 7.5 |
| | 5-23 | CR-23 | 7.5 |
| | 5-24 | CR-24 | 7.5 |
| | 5-25 | CR-25 | 7.3 |
| | 5-26 | CR-26 | 7.5 |
| | 5-27 | CR-27 | 7.5 |
| | 5-28 | CR-28 | 7.4 |
| | 5-29 | CR-29 | 5.7 |
| | 5-30 | CR-30 | 5.6 |
| | 5-31 | CR-31 | 7.5 |
| | 5-32 | CR-32 | 7.3 |
| | 5-33 | CR-33 | 7.5 |
| | 5-34 | CR-34 | 7.4 |
| | 5-35 | CR-35 | 7.3 |

**Table 17**

| | | Resist composition | Rate (A/sec) |
|---|---|---|---|
| | 5-36 | CR-36 | 7.3 |
| | 5-37 | CR-37 | 7.5 |
| | 5-38 | CR-38 | 7.4 |
| | 5-39 | CR-39 | 7.5 |
| | 5-40 | CR-40 | 7.3 |
| | 5-41 | CR-41 | 7.3 |
| | 5-42 | CR-42 | 7.5 |
| | 5-43 | CR-43 | 7.5 |
| | 5-44 | CR-44 | 7.4 |
| | 5-45 | CR-45 | 7.5 |
| | 5-46 | CR-46 | 7.5 |
| | 5-47 | CR-47 | 7.5 |
| | 5-48 | CR-48 | 7.4 |
| Comparative Example | 5-49 | CR-49 | 7.5 |
| | 5-50 | CR-50 | 7.4 |
| | 5-51 | CR-51 | 7.3 |
| | 5-52 | CR-52 | 7.5 |
| | 5-53 | CR-53 | 7.5 |
| | 5-54 | CR-54 | 7.3 |
| | 5-55 | CR-55 | 7.5 |
| | 5-56 | CR-56 | 7.5 |
| | 5-57 | CR-57 | 5.8 |
| | 5-58 | CR-58 | 5.7 |
| | 5-59 | CR-59 | 5.7 |
| | 5-60 | CR-60 | 5.6 |
| | 5-61 | CR-61 | 5.8 |

All of the chemically amplified negative resist compositions (R-1 to R-67) of the present invention exhibited excellent resolution, LER, and pattern rectangularity. On the other hand, in the resist compositions (CR-1 to CR-61) of Comparative Examples, optimization of acid diffusion was insufficient, and deterioration was observed in resolution, LER, and pattern rectangularity. In addition, also in the dry etching evaluation using R-1 to R-67, the etching resistance was excellent, and it was suggested that the inclusion of the repeat units A1 in the polymer is effective in mask processing.

### [6] Development residue test

### Examples 6-1 to 6-67 and Comparative Examples 6-1 to 6-61

Using ACT-M (Tokyo Electron Ltd.), each of the chemically amplified negative resist compositions (R-1 to R-67 and CR-1 to CR-61) was spin coated onto a mask blank for an EUV exposure mask of 152 mm squares having the outermost surface of chromium compound, and prebaked on a hotplate at 110°C for 600 seconds to form a resist film of 80 nm thick. The thickness of the resulting resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

Each resist film was baked as it was at 120°C for 600 seconds without writing, and developed with a 2.38 wt% aqueous solution of tetramethylammonium hydroxide, and then, the development residue was evaluated with a mask defect inspection device M9650 (Lasertec Corporation). The total number of defects after development is shown in Tables 18 to 21.

**Table 18**

| | | Resist composition | Total number of defects after development |
|---|---|---|---|
| | 6-1 | R-1 | 320 |
| | 6-2 | R-2 | 340 |
| | 6-3 | R-3 | 300 |
| | 6-4 | R-4 | 340 |
| | 6-5 | R-5 | 350 |
| | 6-6 | R-6 | 330 |
| | 6-7 | R-7 | 290 |
| | 6-8 | R-8 | 380 |
| | 6-9 | R-9 | 400 |
| | 6-10 | R-10 | 380 |
| | 6-11 | R-11 | 390 |
| | 6-12 | R-12 | 320 |
| | 6-13 | R-13 | 280 |
| | 6-14 | R-14 | 310 |
| | 6-15 | R-15 | 280 |
| | 6-16 | R-16 | 270 |
| | 6-17 | R-17 | 300 |
| Example | 6-18 | R-18 | 350 |
| | 6-19 | R-19 | 370 |
| | 6-20 | R-20 | 400 |
| | 6-21 | R-21 | 320 |
| | 6-22 | R-22 | 310 |
| | 6-23 | R-23 | 270 |
| | 6-24 | R-24 | 280 |
| | 6-25 | R-25 | 260 |
| | 6-26 | R-26 | 310 |
| | 6-27 | R-27 | 350 |
| | 6-28 | R-28 | 320 |
| | 6-29 | R-29 | 330 |
| | 6-30 | R-30 | 340 |
| | 6-31 | R-31 | 310 |
| | 6-32 | R-32 | 300 |
| | 6-33 | R-33 | 270 |
| | 6-34 | R-34 | 340 |
| | 6-35 | R-35 | 330 |

**Table 19**

| | | Resist composition | Total number of defects after development |
|---|---|---|---|
| | 6-36 | R-36 | 320 |
| | 6-37 | R-37 | 270 |
| | 6-38 | R-38 | 280 |
| | 6-39 | R-39 | 290 |
| | 6-40 | R-40 | 260 |
| | 6-41 | R-41 | 310 |
| | 6-42 | R-42 | 340 |
| | 6-43 | R-43 | 360 |
| | 6-44 | R-44 | 410 |
| | 6-45 | R-45 | 320 |
| | 6-46 | R-46 | 380 |
| | 6-47 | R-47 | 300 |
| | 6-48 | R-48 | 280 |
| | 6-49 | R-49 | 340 |
| | 6-50 | R-50 | 270 |
| | 6-51 | R-51 | 310 |
| Example | 6-52 | R-52 | 250 |
| | 6-53 | R-53 | 310 |
| | 6-54 | R-54 | 340 |
| | 6-55 | R-55 | 370 |
| | 6-56 | R-56 | 310 |
| | 6-57 | R-57 | 300 |
| | 6-58 | R-58 | 280 |
| | 6-59 | R-59 | 310 |
| | 6-60 | R-60 | 370 |
| | 6-61 | R-61 | 320 |
| | 6-62 | R-62 | 310 |
| | 6-63 | R-63 | 270 |
| | 6-64 | R-64 | 290 |
| | 6-65 | R-65 | 260 |
| | 6-66 | R-66 | 310 |
| | 6-67 | R-67 | 330 |

**Table 20**

| | | Resist composition | Total number of defects after development |
|---|---|---|---|
| | 6-1 | CR-1 | 1,200 |
| | 6-2 | CR-2 | 1,180 |
| | 6-3 | CR-3 | 1,000 |
| | 6-4 | CR-4 | 1,290 |
| | 6-5 | CR-5 | 1,010 |
| | 6-6 | CR-6 | 1,070 |
| | 6-7 | CR-7 | 1,050 |
| | 6-8 | CR-8 | 1,010 |
| | 6-9 | CR-9 | 990 |
| | 6-10 | CR-10 | 1,020 |
| | 6-11 | CR-11 | 980 |
| | 6-12 | CR-12 | 1,040 |
| | 6-13 | CR-13 | 1,050 |
| | 6-14 | CR-14 | 1,100 |
| | 6-15 | CR-15 | 1,200 |
| | 6-16 | CR-16 | 1,160 |
| | 6-17 | CR-17 | 970 |
| Comparative Example | 6-18 | CR-18 | 1,200 |
| | 6-19 | CR-19 | 1,220 |
| | 6-20 | CR-20 | 1,150 |
| | 6-21 | CR-21 | 1,120 |
| | 6-22 | CR-22 | 1,030 |
| | 6-23 | CR-23 | 1,200 |
| | 6-24 | CR-24 | 950 |
| | 6-25 | CR-25 | 1,030 |
| | 6-26 | CR-26 | 1,060 |
| | 6-27 | CR-27 | 940 |
| | 6-28 | CR-28 | 900 |
| | 6-29 | CR-29 | 920 |
| | 6-30 | CR-30 | 1,090 |
| | 6-31 | CR-31 | 1,040 |
| | 6-32 | CR-32 | 930 |
| | 6-33 | CR-33 | 890 |
| | 6-34 | CR-34 | 950 |
| | 6-35 | CR-35 | 790 |

**Table 21**

| | | Resist composition | Total number of defects after development |
|---|---|---|---|
| | 6-36 | CR-36 | 920 |
| | 6-37 | CR-37 | 900 |
| | 6-38 | CR-38 | 1,050 |
| | 6-39 | CR-39 | 960 |
| | 6-40 | CR-40 | 860 |
| | 6-41 | CR-41 | 830 |
| | 6-42 | CR-42 | 950 |
| | 6-43 | CR-43 | 1,040 |
| | 6-44 | CR-44 | 1,020 |
| | 6-45 | CR-45 | 970 |
| | 6-46 | CR-46 | 920 |
| | 6-47 | CR-47 | 850 |
| | 6-48 | CR-48 | 820 |
| Comparative Example | 6-49 | CR-49 | 890 |
| | 6-50 | CR-50 | 920 |
| | 6-51 | CR-51 | 760 |
| | 6-52 | CR-52 | 980 |
| | 6-53 | CR-53 | 800 |
| | 6-54 | CR-54 | 820 |
| | 6-55 | CR-55 | 940 |
| | 6-56 | CR-56 | 920 |
| | 6-57 | CR-57 | 850 |
| | 6-58 | CR-58 | 980 |
| | 6-59 | CR-59 | 800 |
| | 6-60 | CR-60 | 850 |
| | 6-61 | CR-61 | 780 |

As is apparent from the results shown in Tables 18 to 21, the number of defects due to development residues can be greatly reduced by the polymer comprising the repeat units A1 as compared with the conventional negative resist composition.

The resist pattern forming process using the chemically amplified negative resist composition of the present invention is useful in photolithography for the fabrication of semiconductor devices and the processing of photomask blanks of transmission or reflection type.

## Claims

1. An onium salt monomer having the formula (A1): 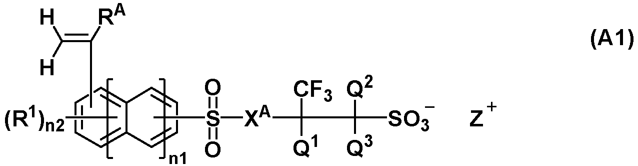 wherein n1 is 0 or 1, n2 is 0, 1, 2, 3, or 4,
R^{A} is a hydrogen atom, a fluorine atom, a methyl group, or a trifluoromethyl group,
R¹ is a C₁-C₂₀ hydrocarbyl group which may contain a halogen atom, a nitro group, a cyano group, a hydroxy group, or a heteroatom, when n2 is 2 or more, a plurality of R¹ may bond together to form a ring with the carbon atoms to which they are attached,
X^{A} is a single bond, -O-, or -N(H)-,
Q¹ to Q³ are each independently a hydrogen atom, a fluorine atom, or a trifluoromethyl group, and Z⁺ is an onium cation.

2. A polymer comprising repeat units derived from the onium salt monomer of claim 1.

3. The polymer of claim 2, further comprising repeat units having the formula (A2): 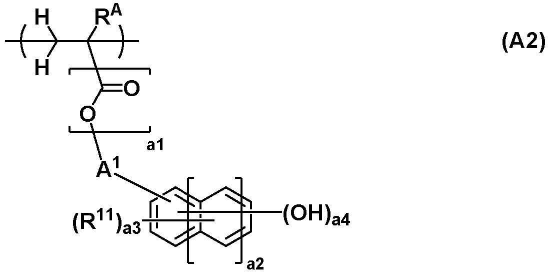 wherein a1 is 0 or 1, a2 is 0, 1, or 2, a3 is an integer satisfying 0 ≤ a3 ≤ 5+2(a2)-a4, a4 is 1, 2, or 3,
R^{A} is a hydrogen atom, a fluorine atom, a methyl group, or a trifluoromethyl group,
R¹¹ is a halogen atom, an optionally halogenated C₁-C₆ saturated hydrocarbyl group, an optionally halogenated C₁-C₆ saturated hydrocarbyloxy group, or an optionally halogenated C₂-C₈ saturated hydrocarbylcarbonyloxy group, and
A¹ is a single bond or a C₁-C₁₀ saturated hydrocarbylene group in which some -CH₂-may be replaced by -O-.

4. The polymer of claim 2 or 3, further comprising repeat units of at least one type selected from repeat units having the formula (A3) and repeat units having the formula (A4): 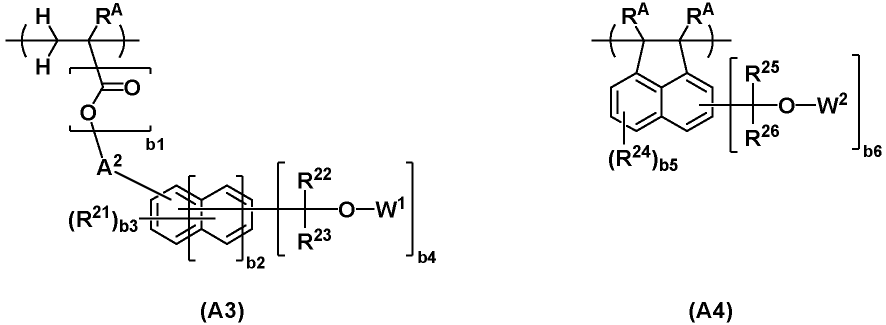 wherein b1 is 0 or 1, b2 is 0, 1, or 2, b3 is an integer satisfying 0 ≤ b3 ≤ 5+2(b2)-b4, b4 is 1, 2, or 3, b5 is 0, 1, or 2, b6 is 1 or 2,
R^{A} is each independently a hydrogen atom, a fluorine atom, a methyl group, or a trifluoromethyl group,
R²¹ is a C₁-C₂₀ hydrocarbyl group which may contain a halogen atom or a heteroatom,
R²² and R²³ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²² and R²³ are not hydrogen atoms at the same time, R²² and R²³ may bond together to form a ring with the carbon atoms to which they are attached, some -CH₂- in the ring may be replaced by -O- or -S-,
R²⁴ is a C₁-C₂₀ hydrocarbyl group which may contain a halogen atom or a heteroatom,
R²⁵ and R²⁶ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²⁵ and R²⁶ are not hydrogen atoms at the same time, R²⁵ and R²⁶ may bond together to form a ring with the carbon atoms to which they are attached, some -CH₂- in the ring may be replaced by -O- or -S-,
A² is a single bond or a C₁-C₁₀ saturated hydrocarbylene group in which some -CH₂-may be replaced by -O-, and
W¹ and W² are each independently a hydrogen atom, a C₁-C₁₀ aliphatic hydrocarbyl group, a C₂-C₁₀ aliphatic hydrocarbylcarbonyl group, or an optionally substituted C₆-C₁₃ aryl group.

5. The polymer of any one of claims 2 to 4, further comprising repeat units of at least one type selected from repeat units having the formula (A5), repeat units having the formula (A6), and repeat units having the formula (A7): 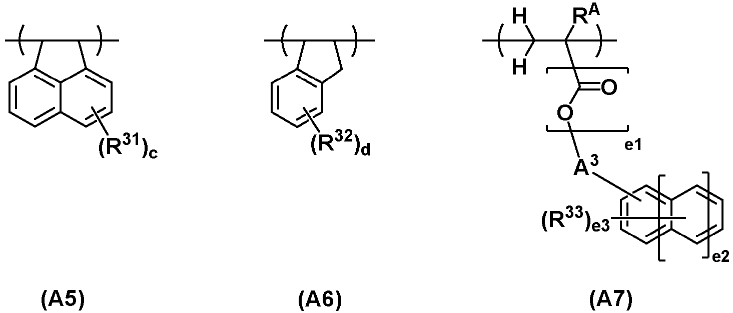 wherein c and d are each independently 0, 1, 2, 3, or 4, e1 is 0 or 1, e2 is 0, 1, or 2, e3 is 0, 1, 2, 3, 4, or 5,
R^{A} is a hydrogen atom, a fluorine atom, a methyl group, or a trifluoromethyl group,
R³¹ and R³² are each independently a hydroxy group, a halogen atom, an optionally halogenated C₁-C₈ saturated hydrocarbyl group, an optionally halogenated C₁-C₈ saturated hydrocarbyloxy group, or an optionally halogenated C₂-C₈ saturated hydrocarbylcarbonyloxy group,
R³³ is a C₁-C₂₀ saturated hydrocarbyl group, a C₁-C₂₀ saturated hydrocarbyloxy group, a C₂-C₂₀ saturated hydrocarbylcarbonyloxy group, a C₂-C₂₀ saturated hydrocarbyloxyhydrocarbyl group, a C₂-C₂₀ saturated hydrocarbylthiohydrocarbyl group, a halogen atom, a nitro group, a cyano group, a C₁-C₂₀ saturated hydrocarbylsulfinyl group, or a C₁-C₂₀ saturated hydrocarbylsulfonyl group, and
A³ is a single bond or a C₁-C₁₀ saturated hydrocarbylene group in which some -CH₂-may be replaced by -O-.

6. A chemically amplified negative resist composition comprising (A) a base polymer containing the polymer of any one of claims 2 to 5.

7. The chemically amplified negative resist composition of claim 6 wherein the base polymer further contains a polymer comprising repeat units having the formula (A2) and repeat units having the formula (A3) or (A4), but not repeat units having formula (A1): 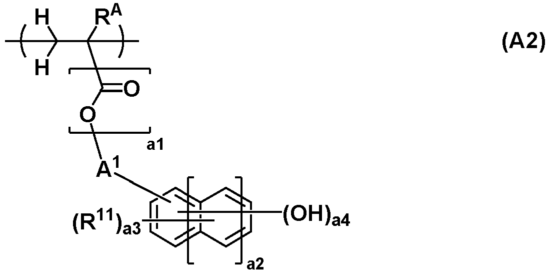
wherein a1 is 0 or 1, a2 is 0, 1, or 2, a3 is an integer satisfying 0 ≤ a3 ≤ 5+2(a2)-a4, a4 is 1, 2, or 3,
R^{A} is a hydrogen atom, a fluorine atom, a methyl group, or a trifluoromethyl group,
R¹¹ is a halogen atom, an optionally halogenated C₁-C₆ saturated hydrocarbyl group, an optionally halogenated C₁-C₆ saturated hydrocarbyloxy group, or an optionally halogenated C₂-C₈ saturated hydrocarbylcarbonyloxy group,
A¹ is a single bond or a C₁-C₁₀ saturated hydrocarbylene group in which some -CH₂-may be replaced by -O-, 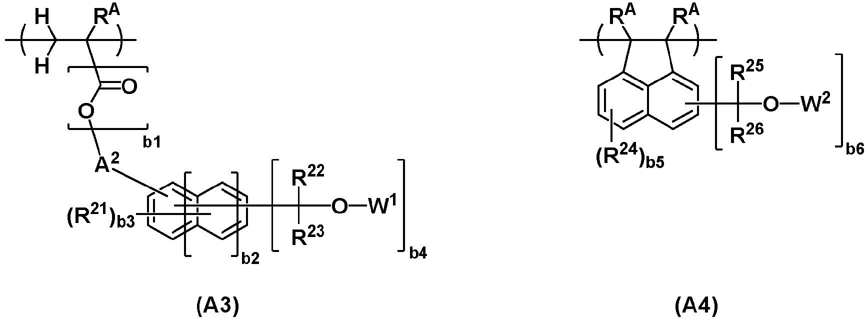
**wherein** b1 is 0 or 1, b2 is 0, 1, or 2, b3 is an integer satisfying 0 ≤ b3 ≤ 5+2(b2)-b4, b4 is 1, 2, or 3, b5 is 0, 1, or 2, b6 is 1 or 2,
R^{A} is each independently a hydrogen atom, a fluorine atom, a methyl group, or a trifluoromethyl group,
R²¹ is a C₁-C₂₀ hydrocarbyl group which may contain a halogen atom or a heteroatom,
R²² and R²³ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²² and R²³ are not hydrogen atoms at the same time, R²² and R²³ may bond together to form a ring with the carbon atoms to which they are attached, some -CH₂- in the ring may be replaced by -O- or -S-,
R²⁴ is a C₁-C₂₀ hydrocarbyl group which may contain a halogen atom or a heteroatom,
R²⁵ and R²⁶ are each independently a hydrogen atom, a C₁-C₁₅ saturated hydrocarbyl group which may be substituted with a hydroxy group or a C₁-C₆ saturated hydrocarbyloxy group, or an optionally substituted C₆-C₁₅ aryl group, provided that R²⁵ and R²⁶ are not hydrogen atoms at the same time, R²⁵ and R²⁶ may bond together to form a ring with the carbon atoms to which they are attached, some -CH₂- in the ring may be replaced by -O- or -S-,
A² is a single bond or a C₁-C₁₀ saturated hydrocarbylene group in which some -CH₂-may be replaced by -O-, and
W¹ and W² are each independently a hydrogen atom, a C₁-C₁₀ aliphatic hydrocarbyl group, a C₂-C₁₀ aliphatic hydrocarbylcarbonyl group, or an optionally substituted C₆-C₁₃ aryl group.

8. The chemically amplified negative resist composition of claim 6 or 7 further comprising (B) a crosslinker.

9. The chemically amplified negative resist composition of claim 6 or 7 which is free of a crosslinker.

10. The chemically amplified negative resist composition of any one of claims 6 to 9, further comprising (C) a fluorinated polymer comprising repeat units of at least one type selected from repeat units having the formula (C1), repeat units having the formula (C2), repeat units having the formula (C3), and repeat units having the formula (C4), and optionally repeat units of at least one type selected from repeat units having the formula (C5) and repeat units having the formula (C6): 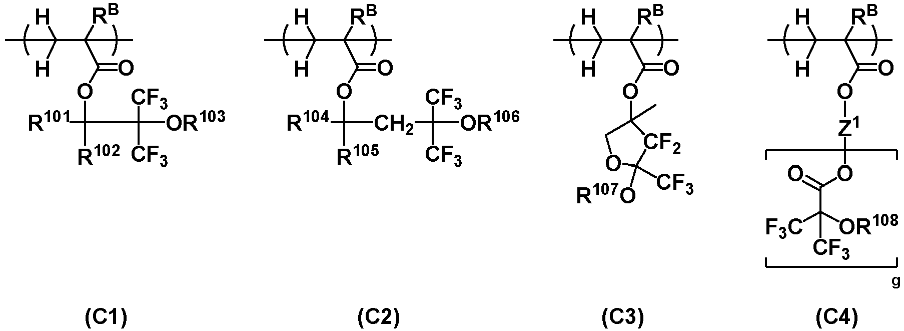 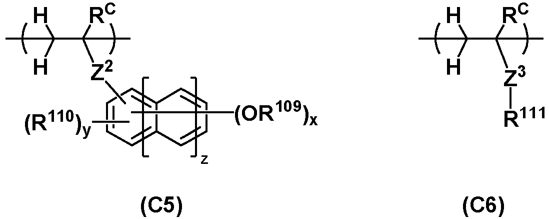 wherein x is 1, 2, or 3, y is an integer satisfying 0 ≤ y ≤ 5+2z-x, z is 0 or 1, g is 1, 2, or 3,
R^{B} is each independently a hydrogen atom, a fluorine atom, a methyl group, or a trifluoromethyl group,
R^{C} is each independently a hydrogen atom or a methyl group,
R¹⁰¹, R¹⁰², R¹⁰⁴, and R¹⁰⁵ are each independently a hydrogen atom or a C₁-C₁₀ saturated hydrocarbyl group,
R¹⁰³, R¹⁰⁶, R¹⁰⁷, and R¹⁰⁸ are each independently a hydrogen atom, a C₁-C₁₅ hydrocarbyl group, a C₁-C₁₅ fluorinated hydrocarbyl group, or an acid labile group, and when R¹⁰³, R¹⁰⁶, R¹⁰⁷, and R¹⁰⁸ each are a hydrocarbyl group or a fluorinated hydrocarbyl group, an ether bond or a carbonyl group may intervene in a carbon-carbon bond,
R¹⁰⁹ is a hydrogen atom or a C₁-C₅ straight or branched hydrocarbyl group in which a heteroatom-containing group may intervene in a carbon-carbon bond,
R¹¹⁰ is a C₁-C₅ straight or branched hydrocarbyl group in which a heteroatom containing group may intervene in a carbon-carbon bond,
R¹¹¹ is a C₁-C₂₀ saturated hydrocarbyl group in which at least one hydrogen atom is substituted by a fluorine atom, and in which some -CH₂- may be replaced by an ester bond or an ether bond,
Z¹ is a C₁-C₂₀ (g+1)-valent hydrocarbon group or C₁-C₂₀ (g+1)-valent fluorinated hydrocarbon group,
Z² is a single bond, *-C(=O)-O-, or *-C(=O)-NH-, * designates a point of attachment to the carbon atom in the backbone,
Z³ is a single bond, -O-, *-C(=O)-O-Z³¹-Z³²-, or *-C(=O)-NH-Z³¹-Z³²-, Z³¹ is a single bond or a C₁-C₁₀ saturated hydrocarbylene group,
Z³² is a single bond, an ester bond, an ether bond, or a sulfonamide bond, and
* designates a point of attachment to the carbon atom in the backbone.

11. The chemically amplified negative resist composition of any one of claims 6 to 10, further comprising (D) a quencher.

12. The chemically amplified negative resist composition of any one of claims 6 to 11, further comprising (E) an organic solvent.

13. The chemically amplified negative resist composition of any one of claims 6 to 12, further comprising (F) another photoacid generator.

14. A resist pattern forming process comprising the steps of: applying the chemically amplified negative resist composition of any one of claims 6 to 13 onto a substrate to form a resist film thereon, exposing the resist film patternwise to high-energy radiation, and developing the exposed resist film in an alkaline developer.

15. A mask blank of transmission or reflection type which is coated with the chemically amplified negative resist composition of any one of claims 6 to 13.
